# EUROPEAN PATENT SPECIFICATION

(11) **EP 3 057 950 B1**
(45) Date of publication and mention of the grant of the patent: **02.12.2020**
(21) Application number: 13786778.4
(22) Date of filing: 15.10.2013
(51) Int. Cl.: C07D 401/14, C07D 401/06, C07D 409/14, C07D 413/06, C07D 417/06, C07D 417/14, A61K 31/4709, A61P 29/00

(54) **PHENYL LINKED QUINOLINYL MODULATORS OF ROR-GAMMA-T**
PHENYLGEBUNDENE CHINOLINYLMODULATOREN VON ROR-GAMMA-T
MODULATEURS QUINOLINYLES À LIAISON PHÉNYLE DE ROR-GAMMA-T

(43) Date of publication of application: 24.08.2016
(73) Proprietor: Janssen Pharmaceutica NV, 2340 Beerse (BE)
(72) Inventor: BARBAY, Kent, Flourtown, Pennsylvania 19031 (US); EDWARDS, James P., San Diego, California 92129 (US); KREUTTER, Kevin D., Plainsboro, New Jersey 08536 (US); KUMMER, David A., San Diego, CA 92122 (US); MAHAROOF, Umar, North Wales, PA 19454 (US); NISHIMURA, Rachel, San Diego, California 92104 (US); URBANSKI, Maud, Flemington, New Jersey 08822 (US); VENKATESAN, Hariharan, San Diego, California 92128 (US); WANG, Aihua, Jamison, Pennsylvania 18929 (US); WOLIN, Ronald L., San Diego, California 92127 (US); WOODS, Craig R., San Diego, California 92122 (US); FOURIE, Anne, San Diego, California 92130 (US); XUE, Xiaohua, San Diego, California 92121 (US); CUMMINGS, Maxwell D., Ambler, Pennsylvania 19002 (US); LEONARD, Kristi A., Lansdale, Pennsylvania 19446 (US)
(74) Representative: Carpmaels & Ransford LLP
(86) International application number: PCT/US2013/065040
(87) International publication number: WO 2015/057203

(56) References cited:
- WO-A1-2014/062667
- WO-A2-2012/064744
- THEODORE M. KAMENECKA ET AL: "Synthetic modulators of the retinoic acid receptor-related orphan receptors", MEDCHEMCOMM, vol. 4, no. 5, 1 January 2013 (2013-01-01) , page 764, XP055069085, ISSN: 2040-2503, DOI: 10.1039/c3md00005b

## Description

### FIELD OF THE INVENTION

The invention is directed to substituted quinoline compounds, which are modulators of the nuclear receptor RORyt, pharmaceutical compositions, and methods for use thereof. More particularly, the RORyt modulators are useful for preventing, treating or ameliorating an RORyt mediated inflammatory syndrome, disorder or disease.

### BACKGROUND OF THE INVENTION

Retinoic acid-related nuclear receptor gamma t (RORyt) is a nuclear receptor, exclusively expressed in cells of the immune system, and a key transcription factor driving Th17 cell differentiation. Thl7 cells are a subset of CD4⁺ T cells, expressing CCR6 on their surface to mediate their migration to sites of inflammation, and dependent on IL-23 stimulation, through the IL-23 receptor, for their maintenance and expansion. Th17 cells produce several proinflammatory cytokines including IL-17A, IL-17F, IL-21, and IL-22 (Kom, T., E. Bettelli, et al. (2009). "IL-17 and Th17 Cells." Annu Rev Immunol 27: 485-517.), which stimulate tissue cells to produce a panel of inflammatory chemokines, cytokines and metalloproteases, and promote recruitment of granulocytes (Kolls, J. K. and A. Linden (2004). "Interleukin-17 family members and inflammation." Immunity 21(4): 467-76; Stamp, L. K., M. J. James, et al. (2004). "Interleukin-17: the missing link between T-cell accumulation and effector cell actions in rheumatoid arthritis" Immunol Cell Biol 82(1): 1-9). Th17 cells have been shown to be the major pathogenic population in several models of autoimmune inflammation, including collagen-induced arthritis (CIA) and experimental autoimmune encephalomyelitis (EAE) (Dong, C. (2006). "Diversification of T-helper-cell lineages: finding the family root of IL-17-producing cells." Nat Rev Immunol 6(4): 329-33; McKenzie, B. S., R. A. Kastelein, et al. (2006). "Understanding the IL-23-IL-17 immune pathway." Trends Immunol 27(1): 17-23.). RORyt-deficient mice are healthy and reproduce normally, but have shown impaired Thl7 cell differentiation *in vitro,* a significantly reduced Thl7 cell population *in vivo,* and decreased susceptibility to EAE (Ivanov, II, B. S. McKenzie, et al. (2006). "The orphan nuclear receptor RORgamma t directs the differentiation program of proinflammatory IL-17+ T helper cells." Cell 126(6): 1121-33.). Mice deficient for IL-23, a cytokine required for Thl7 cell survival, fail to produce Thl7 cells and are resistant to EAE, CIA, and inflammatory bowel disease (IBD) (Cua, D. J., J. Sherlock, et al. (2003). "Interleukin-23 rather than interleukin-12 is the critical cytokine for autoimmune inflammation of the brain." Nature 421(6924): 744-8.; Langrish, C. L., Y. Chen, et al. (2005). "IL-23 drives a pathogenic T cell population that induces autoimmune inflammation." J Exp Med 201(2): 233-40; Yen, D., J. Cheung, et al. (2006). "IL-23 is essential for T cell-mediated colitis and promotes inflammation via IL-17 and IL-6." J Clin Invest 116(5): 1310-6.). Consistent with these findings, an anti-IL23-specific monoclonal antibody blocks development of psoriasis-like inflammation in a murine disease model (Tonel, G., C. Conrad, et al. "Cutting edge: A critical functional role for IL-23 in psoriasis." J Immunol 185(10): 5688-91).

In humans, a number of observations support the role of the IL-23/Th17 pathway in the pathogenesis of inflammatory diseases. IL-17, the key cytokine produced by Th17 cells, is expressed at elevated levels in a variety of allergic and autoimmune diseases (Barczyk, A., W. Pierzchala, et al. (2003). "Interleukin-17 in sputum correlates with airway hyperresponsiveness to methacholine." Respir Med 97(6): 726-33.; Fujino, S., A. Andoh, et al. (2003). "Increased expression of interleukin 17 in inflammatory bowel disease." Gut 52(1): 65-70.; Lock, C., G. Hermans, et al. (2002). "Gene-microarray analysis of multiple sclerosis lesions yields new targets validated in autoimmune encephalomyelitis." Nat Med 8(5): 500-8.; Krueger, J. G., S. Fretzin, et al. "IL-17A is essential for cell activation and inflammatory gene circuits in subjects with psoriasis." J Allergy Clin Immunol 130(1): 145-154 e9.). Furthermore, human genetic studies have shown association of polymorphisms in the genes for Th17 cell-surface receptors, IL-23R and CCR6, with susceptibility to IBD, multiple sclerosis (MS), rheumatoid arthritis (RA) and psoriasis (Gazouli, M., I. Pachoula, et al. "NOD2/CARD15, ATG16L1 and IL23R gene polymorphisms and childhood-onset of Crohn's disease." World J Gastroenterol 16(14): 1753-8., Nunez, C., B. Dema, et al. (2008). "IL23R: a susceptibility locus for celiac disease and multiple sclerosis?" Genes Immun 9(4): 289-93.; Bowes, J. and A. Barton "The genetics of psoriatic arthritis: lessons from genome-wide association studies." Discov Med 10(52): 177-83; Kochi, Y., Y. Okada, et al. "A regulatory variant in CCR6 is associated with rheumatoid arthritis susceptibility." Nat Genet 42(6): 515-9.).

Ustekinumab (Stelara®), an anti-p40 monoclonal antibody blocking both IL-12 and IL-23, is approved for the treatment of adult patients (18 years or older), with moderate to severe plaque psoriasis, who are candidates for phototherapy or systemic therapy. Currently, monoclonal antibodies specifically targeting only IL-23, to more selectively inhibit the Thl7 subset, are also in clinical development for psoriasis (Garber K. (2011). "Psoriasis: from bed to bench and back" Nat Biotech 29, 563-566), further implicating the important role of the IL-23- and RORyt-driven Th17 pathway in this disease. Results from recent phase II clinical studies strongly support this hypothesis, as anti-IL-17 receptor and anti-IL-17 therapeutic antibodies both demonstrated high levels of efficacy in patients with chronic psoriasis (Papp, K. A., "Brodalumab, an anti-interleukin-17-receptor antibody for psoriasis." N Engl J Med 2012 366(13): 1181-9.; Leonardi, C., R. Matheson, et al. "Anti-interleukin-17 monoclonal antibody ixekizumab in chronic plaque psoriasis." N Engl J Med 366(13): 1190-9.). Anti-IL-17 antibodies have also demonstrated clinically relevant responses in early trials in RA and uveitis (Hueber, W., Patel, D.D., Dryja, T., Wright, A.M., Koroleva, I., Bruin, G., Antoni, C., Draelos, Z., Gold, M.H., Durez, P., Tak, P.P., Gomez-Reino, J.J., Foster, C.S., Kim, R.Y., Samson, C.M., Falk, N.S., Chu, D.S., Callanan, D., Nguyen, Q.D., Rose, K., Haider, A., Di Padova, F. (2010) Effects of AIN457, a fully human antibody to interleukin-17A, on psoriasis, rheumatoid arthritis, and uveitis. Sci Transl Med 2, 5272.).

All the above evidence supports inhibition of the Thl7 pathway by modulating RORyt activity as an effective strategy for the treatment of immune-mediated inflammatory diseases.

WO2012/064744 discloses tetrahydroquinoline and related compounds, pharmaceutical compositions, methods of inhibiting RORY activity, reducing the amount of IL-17 in a subject, and treating immune disorders and inflammatory disorders using such tetrahydroquinoline and related compounds.

WO2014/062667 discloses compounds that are phenyl linked quinolinyl modulators of RORyt.

MedChemCommun, 2013, 4, 764-776 provides a review of synthetic ROR ligand development.

### SUMMARY OF THE INVENTION

The present invention comprises compounds of Formula **I:** 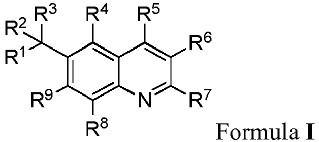 wherein:
R¹ is azetidinyl, pyrrolyl, pyrazolyl, imidazolyl, triazolyl, thiazolyl, pyridyl, pyridyl N-oxide, pyrazinyl, pyrimidinyl, pyridazyl, piperidinyl, tetrahydropyranyl, phenyl, oxazolyl, isoxazolyl, thiophenyl, benzoxazolyl, or quinolinyl; wherein said piperidinyl, pyridyl, pyridyl N-oxide, imidazolyl, phenyl, thiophenyl, benzoxazolyl, and pyrazolyl are optionally substituted with SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃, or OCH₂OCH₃; and optionally substituted with up to two additional substituents independently selected from the group consisting of Cl, OCH₃, and CH₃; and wherein said triazolyl, oxazolyl, isoxazolyl, and thiazolyl are optionally substituted with one or two CH₃ groups; and wherein said azetidinyl is optionally substituted with CO₂C(CH₃)₃, C(O)NH₂, CH₃, SO₂CH₃, or C(O)CH₃;
R² is 1-methyl-1,2,3-triazolyl, pyridyl, pyridyl-*N*-oxide, 1-methyl pyrazol-4-yl, pyrimidin-5-yl, pyridazyl, pyrazin-2-yl, oxazolyl, isoxazolyl, *N*-acetyl-azetidin-3-yl, *N-*methylsulfonyl-azetidin-3-yl, *N*-Boc-azetidin-3-yl, *N*-methyl-azetidin-3-yl, *N*-acetamidyl-azetidin-3-yl, *N*-acetyl piperidinyl, 1-*H*-piperidinyl, *N*-Boc-piperidinyl, *N*-C₍₁₋₂₎alkyl-piperidinyl, thiazol-5-yl, 1-methyl imidazol-2-yl, 1-(3-methoxypropyl)-imidazol-5-yl, or 1-C₍₁₋₂₎alkyl imidazol-5-yl; wherein said 1-C₍₁₋₂₎alkyl imidazol-5-yl is optionally substituted with up to two additional CH₃ groups, or one substituent selected from the group consisting of SCH₃, and Cl; and said pyridyl, and pyridyl-*N*-oxide are optionally substituted with up to two substituents independently selected from the group consisting of C(O)NH₂, -CN, OCH₃, CF₃, Cl, and CH₃; and said thiazol-5-yl, oxazolyl, and isoxazolyl are optionally substituted with up to two CH₃ groups; and said 1-methyl pyrazol-4-yl is optionally substituted with up to two additional CH₃ groups;
R³ is H, OH, OCH₃, NHCH₃, N(CH₃)₂, or NH₂;
R⁴ is H, or F;
R⁵ is H, Cl, -CN, CF₃, SCH₃, OC₍₁₋₃₎alkyl, OH, C₍₁₋₄₎alkyl, N(CH₃)OCH₃, NH(C₍₁₋₂₎alkyl), N(C₍₁₋₂₎alkyl)₂, NH-cyclopropyl, OCHF2, 4-hydroxy-piperidinyl, azetidin-1-yl, or fur-2-yl;
R⁶ is 2-chloro-thiophen-5-yl, 1-methyl-pyrazol-4-yl, phenyl, pyrimidinyl, or pyridyl, wherein said phenyl, pyrimidinyl, and pyridyl are optionally substituted with SO₂CH₃, NHSO₂CH₃, CF₃, F, Cl, -CN, OCH₃, or OCF₃;
R⁷ is H, Cl, -CN, C₍₁₋₄₎alkyl, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, OC₍₁₋₃₎alkyl, OCH₂-(1-methyl)-imidazol-2-yl, imidazol-2-yl, fur-2-yl, pyrazol-4-yl, pyrid-3-yl, or pyrimidin-5-yl; thiophen-3-yl, 1-methyl-indazol-5-yl, 1-methyl-indazol-6-yl, phenyl, or 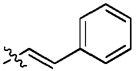 wherein said imidazolyl or pyrazolyl can be optionally substituted with a CH₃ group ;
A¹ is H or C₍₁₋₄₎alkyl;
A² is C₍₁₋₄₎alkyl, cyclopropyl, C₍₁₋₄₎alkylOC₍₁₋₄₎alkyl, C₍₁₋₄₎alkylOH, C(O)C₍₁₋₂₎alkyl, or OCH₃; or A¹ and A² may be taken together with their attached nitrogen to form a ring selected from the group consisting of: 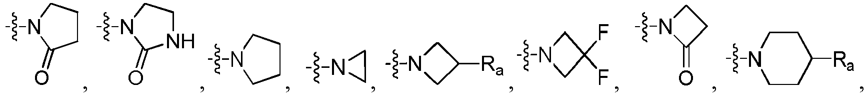 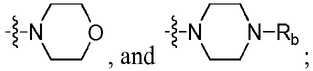
Rₐ is H, F, OCH₃, or OH;
R_{b} is CH₃, or phenyl;
R⁸ is H, CH₃, OCH₃, or F;
R⁹ is H, or F;
and wherein:
(i) R¹ is azetidinyl, wherein said azetidinyl is optionally substituted with CO₂C(CH₃)₃, C(O)NH₂, CH₃, SO₂CH₃, or C(O)CH₃; or
(ii) R² is *N*-acetyl-azetidin-3-yl, *N*-methylsulfonyl-azetidin-3-yl, *N*-Boc-azetidin-3-yl, *N*-methyl-azetidin-3-yl, or *N*-acetamidyl-azetidin-3-yl; or
(iii) R³ is NHCH₃, or N(CH₃)₂; or
(iv) R⁵ is NH-cyclopropyl, OCHF₂, azetidin-1-yl, or fur-2-yl; or
(v) R⁶ is phenyl substituted with NHSO₂CH₃, pyrimidinyl substituted with NHSO₂CH₃, or pyridyl substituted with NHSO₂CH₃; or
(vi) R⁷ is SO₂CH₃, 1-methyl-indazol-5-yl, 1-methyl-indazol-6-yl, or 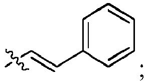 or
(vii) A² is cyclopropyl
and pharmaceutically acceptable salts thereof.

The present disclosure comprises compounds of Formula **I**. 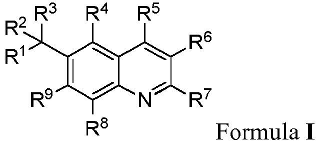 wherein:
R¹ is azetidinyl, pyrrolyl, pyrazolyl, imidazolyl, triazolyl, thiazolyl, pyridyl, pyridyl N-oxide, pyrazinyl, pyrimidinyl, pyridazyl, piperidinyl, tetrahydropyranyl, phenyl, oxazolyl, isoxazolyl, thiophenyl, benzoxazolyl, or quinolinyl; wherein said piperidinyl, pyridyl, pyridyl N-oxide, imidazolyl, phenyl, thiophenyl, benzoxazolyl, and pyrazolyl are optionally substituted with SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃, or OCH₂OCH₃; and optionally substituted with up to two additional substituents independently selected from the group consisting of Cl, OCH₃, and CH₃; and wherein said triazolyl, oxazolyl, isoxazolyl, and thiazolyl are optionally substituted with one or two CH₃ groups; and wherein said azetidinyl is optionally substituted with CO₂C(CH₃)₃, C(O)NH₂, CH₃, SO₂CH₃, or C(O)CH₃;
R² is 1-methyl-1,2,3-triazolyl, pyridyl, pyridyl-*N*-oxide, 1-methyl pyrazol-4-yl, pyrimidin-5-yl, pyridazyl, pyrazin-2-yl, oxazolyl, isoxazolyl, *N*-acetyl-azetidin-3-yl, *N-*methylsulfonyl-azetidin-3-yl, *N*-Boc-azetidin-3-yl, *N*-methyl-azetidin-3-yl, N-acetamidyl-azetidin-3-yl, *N*-acetyl piperidinyl, 1-*H*-piperidinyl, *N*-Boc-piperidinyl, *N*-C₍₁₋₂₎alkyl-piperidinyl (including *N*-methyl piperidin-4-yl), thiazol-5-yl, 1-methyl imidazol-2-yl, 1-(3-methoxypropyl)-imidazol-5-yl, or 1-C₍₁₋₂₎alkyl imidazol-5-yl; wherein said 1-C₍₁₋₂₎alkyl imidazol-5-yl is optionally substituted with up to two additional CH₃ groups, or one substituent selected from the group consisting of SCH₃, and Cl; and said pyridyl, and pyridyl-*N*-oxide are optionally substituted with up to two substituents independently selected from the group consisting of C(O)NH₂, -CN, OCH₃, CF₃, Cl, and CH₃; and said thiazol-5-yl, oxazolyl, and isoxazolyl are optionally substituted with up to two CH₃ groups; and said 1-methyl pyrazol-4-yl is optionally substituted with up to two additional CH₃ groups;
R³ is H, OH, OCH₃, NHCH₃, N(CH₃)₂, or NH₂;
R⁴ is H, or F;
R⁵ is H, Cl, -CN, CF₃, SCH₃, OC₍₁₋₃)alkyl, OH, C₍₁₋₄₎alkyl, N(CH₃)OCH₃, NH(C₍₁₋₂₎alkyl), N(C₍₁₋₂₎alkyl)₂, NH-cyclopropyl, OCHF₂, 4-hydroxy-piperidinyl, azetidin-1-yl, or fur-2-yl;
R⁶ is 2-chloro-thiophen-5-yl, 1-methyl-pyrazol-4-yl, phenyl, pyrimidinyl, or pyridyl, wherein said phenyl, pyrimidinyl, and pyridyl are optionally substituted with SO₂CH₃, NHSO₂CH₃, CF₃, F, Cl, -CN, OCH₃, or OCF₃;
R⁷ is H, Cl, -CN, C₍₁₋₄₎alkyl, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, OC₍₁₋₃₎alkyl, OCH₂-(1-methyl)-imidazol-2-yl, imidazol-2-yl, fur-2-yl, pyrazol-4-yl, pyrid-3-yl, or pyrimidin-5-yl; thiophen-3-yl, 1-methyl-indazol-5-yl, 1-methyl-indazol-6-yl, phenyl, or 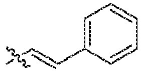 wherein said imidazolyl or pyrazolyl can be optionally substituted with a CH₃ group;
A¹ is H or C₍₁₋₄₎alkyl (including CH₃);
A² is C₍₁₋₄₎alkyl (including CH₃), cyclopropyl, C₍₁₋₄₎alkylOC₍₁₋₄₎alkyl, C₍₁₋₄)alkylOH, C(O)C₍₁₋₂)alkyl, or OCH₃; or A¹ and A² may be taken together with their attached nitrogen to form a ring selected from the group consisting of: 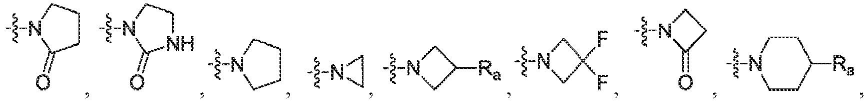 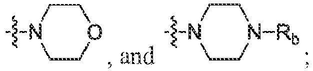
Rₐ is H, F, OCH₃, or OH;
R_{b} is CH₃, or phenyl;
R⁸ is H, CH₃, OCH₃, or F;
R⁹ is H, or F;
and pharmaceutically acceptable salts thereof;
provided that (4-chloro-3-phenylquinolin-6-yl)(1-methyl-1*H*-imidazol-5-yl)(pyridin-3-yl)methanamine, (4-chlorophenyl)(2,4-dichloro-3-(2-chlorophenyl)quinolin-6-yl)(1-methyl-1*H-*imidazol-2-yl)methanol, (4-chloro-3-phenylquinolin-6-yl)(1-methyl-1*H*-imidazol-5-yl)(pyridin-4-yl)methanamine, (4-chlorophenyl)(3-(2,6-dichlorophenyl)quinolin-6-yl)(1-methyl-1*H-*imidazol-5-yl)methanol, (4-chloro-3-phenylquinolin-6-yl)(2-(dimethylamino)pyridin-4-yl)(1-methyl- 1*H*-imidazol-2-yl)methanol, 4-(2-((4-chloro-6-((4-chlorophenyl)(hydroxy)(1-methyl-1*H-*imidazol-5-yl)methyl)-3-phenylquinolin-2-yl)oxy)ethyl)thiomorpholine 1,1-dioxide, 1-(2-((4-chloro-6-((4-chlorophenyl)(hydroxy)(1-methyl-1*H-*imidazol-5-yl)methyl)-3-phenylquinolin-2-yl)oxy)ethyl)pyrrolidin-2-one, (2-chloro-4-(dimethylamino)-3-phenylquinolin-6-yl)(pyridin-2-yl)(pyridin-4-yl)methanol, (4-chloro-3-phenylquinolin-6-yl)(2-fluoropyridin-4-yl)(1-methyl-1*H-*imidazol-2-yl)methanol, (4-chloro-2-(1-methyl-1*H*-pyrazol-4-yl)-3-phenylquinolin-6-yl)(4-chlorophenyl)(pyridin-3-yl)methanol, (2,4-dichloro-3-phenylquinolin-6-yl)di(pyridin-2-yl)methanol, 6-((3-chlorophenyl)(hydroxy)(2-(trifluoromethyl)pyridin-4-yl)methyl)-3-phenylquinoline-2-carbonitrile, (2,4-dichloro-8-methyl-3-phenylquinolin-6-yl)(1-methyl-1*H-*imidazol-4-yl)(6-methylpyridin-3-yl)methanol, (4-chlorophenyl)(2,4-dichloro-3-(2-chlorophenyl)quinolin-6-yl)(1-methyl-1*H*-imidazol-5-yl)methanol, (2,4-dichloro-3-phenylquinolin-6-yl)(phenyl)(pyridin-2-yl)methanol, (2,4-dichloro-3-phenylquinolin-6-yl)(oxazol-2-yl)(phenyl)methanol, the second eluting enantiomer of (4-methoxy-3-phenyl-2-(trifluoromethyl)quinolin-6-yl)(1-methyl-1*H*-imidazol-5-yl)(pyridin-2-yl)methanol, and the second eluting enantiomer of (4-chloro-2-methoxy-3-phenylquinolin-6-yl)(1-methyl-1*H-*imidazol-5-yl)pyrimidin-2-ylmethanol (when purified on a chiralcel OD column) are excluded.

### DETAILED DESCRIPTION OF THE INVENTION

The present invention comprises compounds of Formula I: 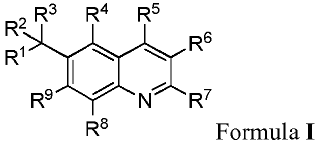 wherein:
R¹ is azetidinyl, pyrrolyl, pyrazolyl, imidazolyl, triazolyl, thiazolyl, pyridyl, pyridyl N-oxide, pyrazinyl, pyrimidinyl, pyridazyl, piperidinyl, tetrahydropyranyl, phenyl, oxazolyl, isoxazolyl, thiophenyl, benzoxazolyl, or quinolinyl; wherein said piperidinyl, pyridyl, pyridyl N-oxide, imidazolyl, phenyl, thiophenyl, benzoxazolyl, and pyrazolyl are optionally substituted with SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃, or OCH₂OCH₃; and optionally substituted with up to two additional substituents independently selected from the group consisting of Cl, OCH₃, and CH₃; and wherein said triazolyl, oxazolyl, isoxazolyl, and thiazolyl are optionally substituted with one or two CH₃ groups; and wherein said azetidinyl is optionally substituted with CO₂C(CH₃)₃, C(O)NH₂, CH₃, SO₂CH₃, or C(O)CH₃;
R² is 1-methyl-1,2,3-triazolyl, pyridyl, pyridyl-*N*-oxide, 1-methyl pyrazol-4-yl, pyrimidin-5-yl, pyridazyl, pyrazin-2-yl, oxazolyl, isoxazolyl, *N*-acetyl-azetidin-3-yl, *N-*methylsulfonyl-azetidin-3-yl, *N*-Boc-azetidin-3-yl, *N*-methyl-azetidin-3-yl, *N*-acetamidyl-azetidin-3-yl, *N*-acetyl piperidinyl, 1-*H*-piperidinyl, *N*-Boc-piperidinyl, *N*-C₍₁₋₂₎alkyl-piperidinyl, thiazol-5-yl, 1-methyl imidazol-2-yl, 1-(3-methoxypropyl)-imidazol-5-yl, or 1-C₍₁₋₂₎alkyl imidazol-5-yl; wherein said 1-C₍₁₋₂₎alkyl imidazol-5-yl is optionally substituted with up to two additional CH₃ groups, or one substituent selected from the group consisting of SCH₃, and Cl; and said pyridyl, and pyridyl-*N*-oxide are optionally substituted with up to two substituents independently selected from the group consisting of C(O)NH₂, -CN, OCH₃, CF₃, Cl, and CH₃; and said thiazol-5-yl, oxazolyl, and isoxazolyl are optionally substituted with up to two CH₃ groups; and said 1-methyl pyrazol-4-yl is optionally substituted with up to two additional CH₃ groups;
R³ is H, OH, OCH₃, NHCH₃, N(CH₃)₂, or NH₂;
R⁴ is H, or F;
R⁵ is H, Cl, -CN, CF₃, SCH₃, OC₍₁₋₃₎alkyl, OH, C₍₁₋₄₎alkyl, N(CH₃)OCH₃, NH(C₍₁₋₂₎alkyl), N(C₍₁₋₂₎alkyl)₂, NH-cyclopropyl, OCHF₂, 4-hydroxy-piperidinyl, azetidin-1-yl, or fur-2-yl;
R⁶ is 2-chloro-thiophen-5-yl, 1-methyl-pyrazol-4-yl, phenyl, pyrimidinyl, or pyridyl, wherein said phenyl, pyrimidinyl, and pyridyl are optionally substituted with SO₂CH₃, NHSO₂CH₃, CF₃, F, Cl, -CN, OCH₃, or OCF₃;
R⁷ is H, Cl, -CN, C₍₁₋₄₎alkyl, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, OC₍₁₋₃₎alkyl, OCH₂-(1-methyl)-imidazol-2-yl, imidazol-2-yl, fur-2-yl, pyrazol-4-yl, pyrid-3-yl, or pyrimidin-5-yl; thiophen-3-yl, 1-methyl-indazol-5-yl, 1-methyl-indazol-6-yl, phenyl, or 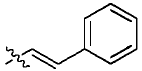 wherein said imidazolyl or pyrazolyl can be optionally substituted with a CH₃ group ;
A¹ is H or C₍₁₋₄)alkyl;
A² is C₍₁₋₄₎alkyl, cyclopropyl, C₍₁₋₄₎alkylOC₍₁₋₄₎alkyl, C₍₁₋₄₎alkylOH, C(O)C₍₁₋₂₎alkyl, or OCH₃; or A¹ and A² may be taken together with their attached nitrogen to form a ring selected from the group consisting of: 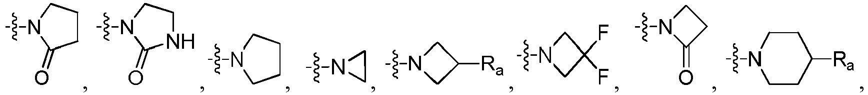 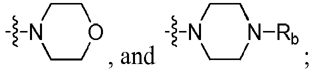
Rₐ is H, F, OCH₃, or OH;
R_{b} is CH₃, or phenyl;
R⁸ is H, CH₃, OCH₃, or F;
R⁹ is H, or F;
and wherein:
(i) R¹ is azetidinyl, wherein said azetidinyl is optionally substituted with CO₂C(CH₃)₃, C(O)NH₂, CH₃, SO₂CH₃, or C(O)CH₃; or
(ii) R² is *N*-acetyl-azetidin-3-yl, *N*-methylsulfonyl-azetidin-3-yl, *N*-Boc-azetidin-3-yl, *N*-methyl-azetidin-3-yl, or *N*-acetamidyl-azetidin-3-yl; or
(iii) R³ is NHCH₃, or N(CH₃)₂; or
(iv) R⁵ is NH-cyclopropyl, OCHF₂, azetidin-1-yl, or fur-2-yl; or
(v) R⁶ is phenyl substituted with NHSO₂CH₃, pyrimidinyl substituted with NHSO₂CH₃, or pyridyl substituted with NHSO₂CH₃; or
(vi) R⁷ is SO₂CH₃, 1-methyl-indazol-5-yl, 1-methyl-indazol-6-yl, or 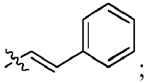 or
(vii) A² is cyclopropyl
and pharmaceutically acceptable salts thereof.

In another embodiment of the invention:
R¹ is imidazolyl, thiazolyl, pyridyl, pyrimidinyl, or phenyl; wherein said pyridyl, and said phenyl are optionally substituted with -CN, CF₃, F, or Cl; and wherein said imidazolyl, and thiazolyl are optionally substituted with one or two CH₃ groups;
R² is 1-methyl-1,2,3-triazol-5-yl, *N*-acetyl piperidin-4-yl, *N*-Boc-piperidin-4-yl, *N*-methyl piperidin-4-yl, 1-*H*-piperidin-4-yl, oxazol-2-yl, 2,4-dimethyl thiazol-5-yl, 1-methyl imidazol-2-yl, 1-methyl-imidazol-5-yl, or pyridyl; wherein said pyridyl is optionally substituted with CF₃;
R³ is H, OH;
R⁴ is H;
R⁵ is H, Cl, -CN, C₍₁₋₄₎alkyl, OC₍₁₋₂₎alkyl, SCH₃, N(CH₃)₂, or N(CH₃)OCH₃;
R⁶ is phenyl substituted with NHSO₂CH₃, pyrimidinyl substituted with NHSO₂CH₃, or pyridyl substituted with NHSO₂CH₃;
R⁷ is Cl, -CN, CF₃, SCH₃, OCH₂CF₃, NA¹A², N(CH₃)CH₂CH₂NA¹A², OC₍₁₋₃₎alkyl, OCH₂CH₂OCH₃, OCH₂CH₂NA¹A², or OCH₂CH₂NH₂;
A¹ is H, or CH₃;
A² is OCH₃, CH₃, CH₂CH₂OH, C(O)C₍₁₋₂₎alkyl, or CH₂CH₂OCH₃; or A¹ and A² may be taken together with their attached nitrogen to form a ring selected from the group consisting of: 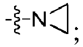
R⁸ is H, or CH₃;
R⁹ is H;
and pharmaceutically acceptable salts thereof.

The present disclosure comprises compounds of Formula I. 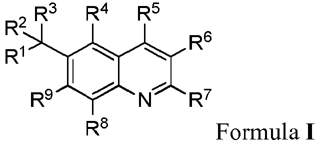 wherein:
R¹ is azetidinyl, pyrrolyl, pyrazolyl, imidazolyl, triazolyl, thiazolyl, pyridyl, pyridyl N-oxide, pyrazinyl, pyrimidinyl, pyridazyl, piperidinyl, tetrahydropyranyl, phenyl, oxazolyl, isoxazolyl, thiophenyl, benzoxazolyl, or quinolinyl; wherein said piperidinyl, pyridyl, pyridyl N-oxide, imidazolyl, phenyl, thiophenyl, benzoxazolyl, and pyrazolyl are optionally substituted with SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃, or OCH₂OCH₃; and optionally substituted with up to two additional substituents independently selected from the group consisting of Cl, OCH₃, and CH₃; and wherein said triazolyl, oxazolyl, isoxazolyl, and thiazolyl are optionally substituted with one or two CH₃ groups; and wherein said azetidinyl is optionally substituted with CO₂C(CH₃)₃, C(O)NH₂, CH₃, SO₂CH₃, or C(O)CH₃;
R² is 1-methyl-1,2,3-triazolyl, pyridyl, pyridyl-*N*-oxide, 1-methyl pyrazol-4-yl, pyrimidin-5-yl, pyridazyl, pyrazin-2-yl, oxazolyl, isoxazolyl, *N*-acetyl-azetidin-3-yl, *N-*methylsulfonyl-azetidin-3-yl, *N*-Boc-azetidin-3-yl, *N*-methyl-azetidin-3-yl, *N*-acetamidyl-azetidin-3-yl, *N*-acetyl piperidinyl, 1-*H*-piperidinyl, *N*-Boc-piperidinyl, *N*-C₍₁₋₂₎alkyl-piperidinyl (including *N*-methyl piperidin-4-yl), thiazol-5-yl, 1-methyl imidazol-2-yl, 1-(3-methoxypropyl)-imidazol-5-yl, or 1-C₍₁₋₂₎alkyl imidazol-5-yl; wherein said 1-C₍₁₋₂₎alkyl imidazol-5-yl is optionally substituted with up to two additional CH₃ groups, or one substituent selected from the group consisting of SCH₃, and Cl; and said pyridyl, and pyridyl-*N*-oxide are optionally substituted with up to two substituents independently selected from the group consisting of C(O)NH₂, -CN, OCH₃, CF₃, Cl, and CH₃; and said thiazol-5-yl, oxazolyl, and isoxazolyl are optionally substituted with up to two CH₃ groups; and said 1-methyl pyrazol-4-yl is optionally substituted with up to two additional CH₃ groups;
R³ is H, OH, OCH₃, NHCH₃, N(CH₃)₂, or NH₂;
R⁴ is H, or F;
R⁵ is H, Cl, -CN, CF₃, SCH₃, OC₍₁₋₃₎alkyl, OH, C₍₁₋₄₎alkyl, N(CH₃)OCH₃, NH(C₍₁₋₂₎alkyl), N(C₍₁₋₂₎alkyl)₂, NH-cyclopropyl, OCHF₂, 4-hydroxy-piperidinyl, azetidin-1-yl, or fur-2-yl;
R⁶ is 2-chloro-thiophen-5-yl, 1-methyl-pyrazol-4-yl, phenyl, pyrimidinyl, or pyridyl, wherein said phenyl, pyrimidinyl, and pyridyl are optionally substituted with SO₂CH₃, NHSO₂CH₃, CF₃, F, Cl, -CN, OCH₃, or OCF₃;
R⁷ is H, Cl, -CN, C₍₁₋₄₎alkyl, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, OC₍₁₋₃₎alkyl, OCH₂-(1-methyl)-imidazol-2-yl, imidazol-2-yl, fur-2-yl, pyrazol-4-yl, pyrid-3-yl, or pyrimidin-5-yl; thiophen-3-yl, 1-methyl-indazol-5-yl, 1-methyl-indazol-6-yl, phenyl, or 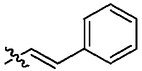 wherein said imidazolyl or pyrazolyl can be optionally substituted with a CH₃ group ;
A¹ is H or C₍₁₋₄₎alkyl (including CH₃);
A² is C₍₁₋₄₎alkyl (including CH₃), cyclopropyl, C₍₁₋₄₎alkylOC₍₁₋₄₎alkyl, C₍₁₋₄₎alkylOH, C(O)C₍₁₋₂₎alkyl, or OCH₃; or A¹ and A² may be taken together with their attached nitrogen to form a ring selected from the group consisting of: 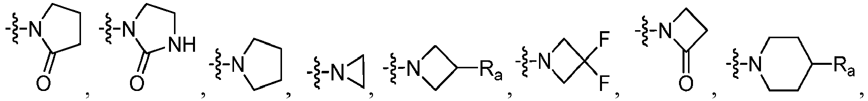 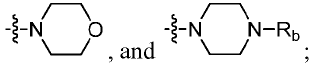
Rₐ is H, F, OCH₃, or OH;
R_{b} is CH₃, or phenyl;
R⁸ is H, CH₃, OCH₃, or F;
R⁹ is H, or F;
and pharmaceutically acceptable salts thereof;
provided that (4-chloro-3-phenylquinolin-6-yl)(1-methyl-1*H*-imidazol-5-yl)(pyridin-3-yl)methanamine, (4-chlorophenyl)(2,4-dichloro-3-(2-chlorophenyl)quinolin-6-yl)(1-methyl-1*H-*imidazol-2-yl)methanol, (4-chloro-3-phenylquinolin-6-yl)(1-methyl-1*H*-imidazol-5-yl)(pyridin-4-yl)methanamine, (4-chlorophenyl)(3-(2,6-dichlorophenyl)quinolin-6-yl)(1-methyl-1*H-*imidazol-5-yl)methanol, (4-chloro-3-phenylquinolin-6-yl)(2-(dimethylamino)pyridin-4-yl)(1-methyl-1*H*-imidazol-2-yl)methanol, 4-(2-((4-chloro-6-((4-chlorophenyl)(hydroxy)(1-methyl-1*H-*imidazol-5-yl)methyl)-3-phenylquinolin-2-yl)oxy)ethyl)thiomorpholine 1,1-dioxide, 1-(2-((4-chloro-6-((4-chlorophenyl)(hydroxy)(1-methyl-1*H*-imidazol-5-yl)methyl)-3-phenylquinolin-2-yl)oxy)ethyl)pyrrolidin-2-one, (2-chloro-4-(dimethylamino)-3-phenylquinolin-6-yl)(pyridin-2-yl)(pyridin-4-yl)methanol, (4-chloro-3-phenylquinolin-6-yl)(2-fluoropyridin-4-yl)(1-methyl-1*H-*imidazol-2-yl)methanol, (4-chloro-2-(1-methyl-1*H*-pyrazol-4-yl)-3-phenylquinolin-6-yl)(4-chlorophenyl)(pyridin-3-yl)methanol, (2,4-dichloro-3-phenylquinolin-6-yl)di(pyridin-2-yl)methanol, 6-((3-chlorophenyl)(hydroxy)(2-(trifluoromethyl)pyridin-4-yl)methyl)-3-phenylquinoline-2-carbonitrile, (2,4-dichloro-8-methyl-3-phenylquinolin-6-yl)(1-methyl-1H-imidazol-4-yl)(6-methylpyridin-3-yl)methanol, (4-chlorophenyl)(2,4-dichloro-3-(2-chlorophenyl)quinolin-6-yl)(1-methyl-1*H*-imidazol-5-yl)methanol, (2,4-dichloro-3-phenylquinolin-6-yl)(phenyl)(pyridin-2-yl)methanol, and (2,4-dichloro-3-phenylquinolin-6-yl)(oxazol-2-yl)(phenyl)methanol, the second eluting enantiomer of (4-methoxy-3-phenyl-2-(trifluoromethyl)quinolin-6-yl)(1-methyl-1*H*-imidazol-5-yl)(pyridin-2-yl)methanol, and the second eluting enantiomer of (4-chloro-2-methoxy-3-phenylquinolin-6-yl)(1-methyl-1*H-*imidazol-5-yl)pyrimidin-2-ylmethanol (when purified on a chiralcel OD column) are excluded.

In another aspect of the disclosure:
R¹ is imidazolyl, thiazolyl, pyridyl, pyrimidinyl, or phenyl; wherein said pyridyl, and said phenyl are optionally substituted with -CN, CF₃, F, or Cl; and wherein said imidazolyl, and thiazolyl are optionally substituted with one or two CH₃ groups;
R² is 1-methyl-1,2,3-triazol-5-yl, *N*-acetyl piperidin-4-yl, *N*-Boc-piperidin-4-yl, *N*-methyl piperidin-4-yl1-*H*-piperidin-4-yl, oxazol-2-yl, 2,4-dimethyl thiazol-5-yl, 1-methyl imidazol-2-yl, 1-methyl-imidazol-5-yl, or pyridyl; wherein said pyridyl is optionally substituted with CF₃;
R³ is H, OH;
R⁴ is H;
R⁵ is H, Cl, -CN, C₍₁₋₄₎alkyl, OC₍₁₋₂₎alkyl, SCH₃, N(CH₃)₂, or N(CH₃)OCH₃;
R⁶ is 2-chloro-thiophen-5-yl, 1-methyl-pyrazol-4-yl, phenyl, pyrimidinyl, or pyridyl, wherein said phenyl, pyrimidinyl, and pyridyl are optionally substituted with SO₂CH₃, NHSO₂CH₃, CF₃, Cl, -CN, OCF₃, or OCH₃;
R⁷ is Cl, -CN, CF₃, SCH₃, OCH₂CF₃, NA¹A², N(CH₃)CH₂CH₂NA¹A², OC₍₁₋₃₎alkyl, OCH₂CH₂OCH₃, OCH₂CH₂NA¹A², or OCH₂CH₂NH₂;
A¹ is H, or CH₃;
A² is OCH₃, CH₃, CH₂CH₂OH, C(O)C₍₁₋₂₎alkyl, or CH₂CH₂OCH₃; or A¹ and A² may be taken together with their attached nitrogen to form a ring selected from the group consisting of: 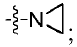
R⁸ is H, or CH₃;
R⁹ is H;
and pharmaceutically acceptable salts thereof;
provided that (4-chlorophenyl)(2,4-dichloro-3-(2-chlorophenyl)quinolin-6-yl)(1-methyl-1*H-*imidazol-5-yl)methanol, 6-((3-chlorophenyl)(hydroxy)(2-(trifluoromethyl)pyridin-4-yl)methyl)-3-phenylquinoline-2-carbonitrile, (2,4-dichloro-3-phenylquinolin-6-yl)di(pyridin-2-yl)methanol, (2,4-dichloro-3-phenylquinolin-6-yl)(phenyl)(pyridin-2-yl)methanol, (2-chloro-4-(dimethylamino)-3-phenylquinolin-6-yl)(pyridin-2-yl)(pyridin-4-yl)methanol, (4-chlorophenyl)(2,4-dichloro-3-(2-chlorophenyl)quinolin-6-yl)(1-methyl-1*H*-imidazol-2-yl)methanol, (2,4-dichloro-3-phenylquinolin-6-yl)(oxazol-2-yl)(phenyl)methanol, the second eluting enantiomer of (4-methoxy-3-phenyl-2-(trifluoromethyl)quinolin-6-yl)(1-methyl-1H-imidazol-5-yl)(pyridin-2-yl)methanol, and the second eluting enantiomer of (4-chloro-2-methoxy-3-phenylquinolin-6-yl)(1-methyl-1*H*-imidazol-5-yl)pyrimidin-2-ylmethanol (when purified on a chiralcel OD column) are excluded.

Another embodiment of the invention is a compound selected from the group consisting of: 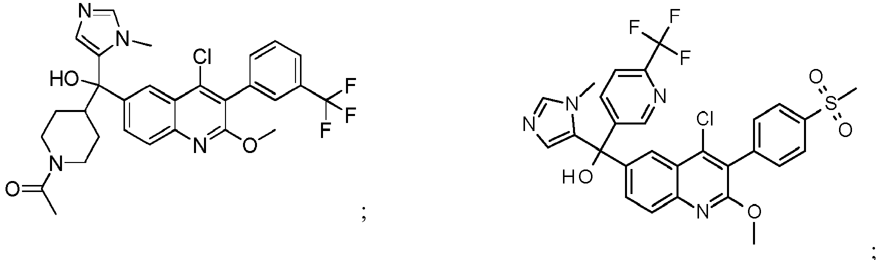 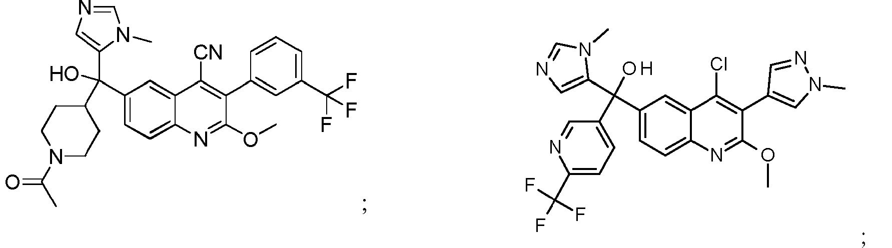 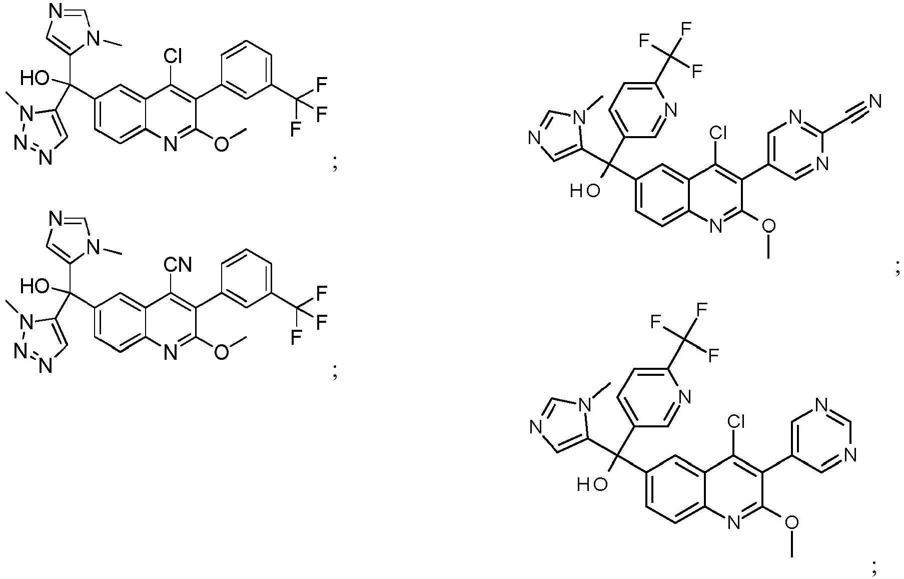 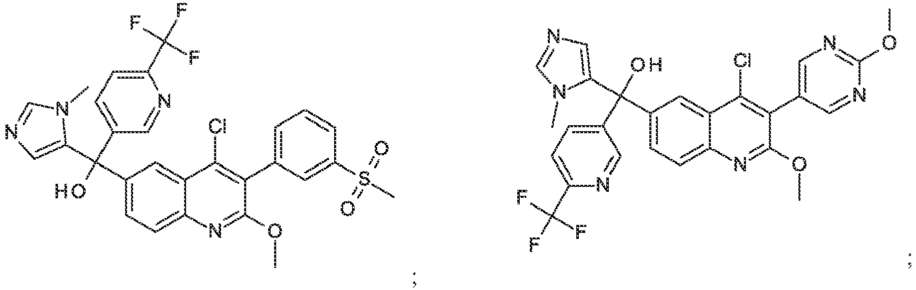 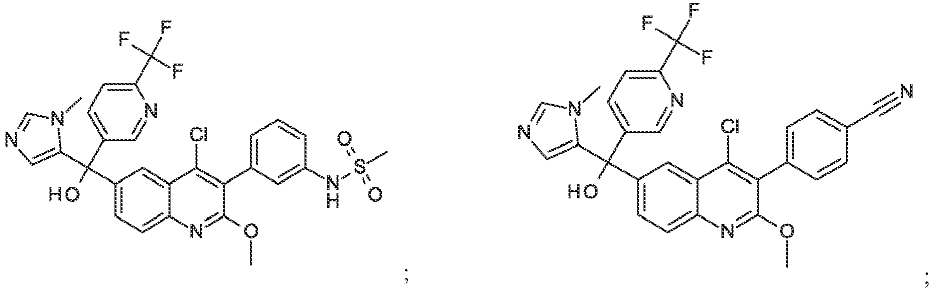 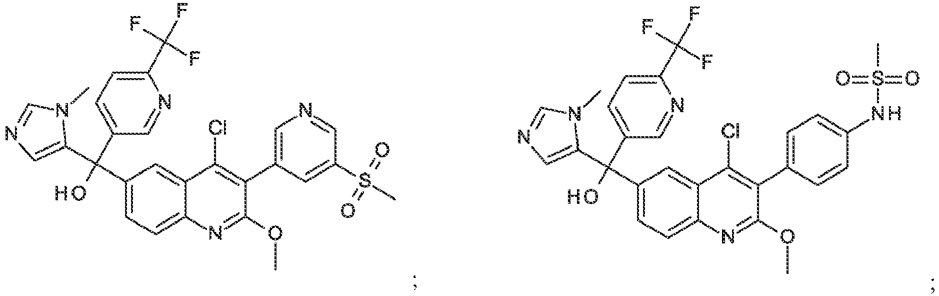 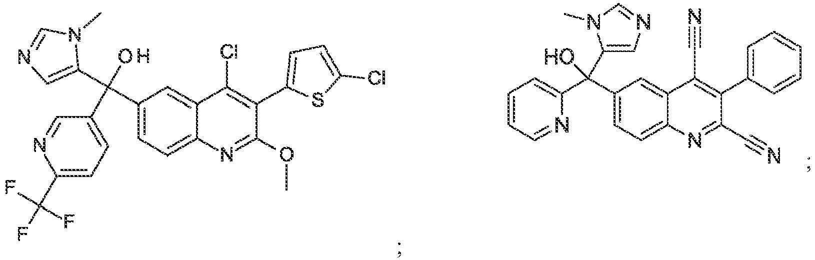 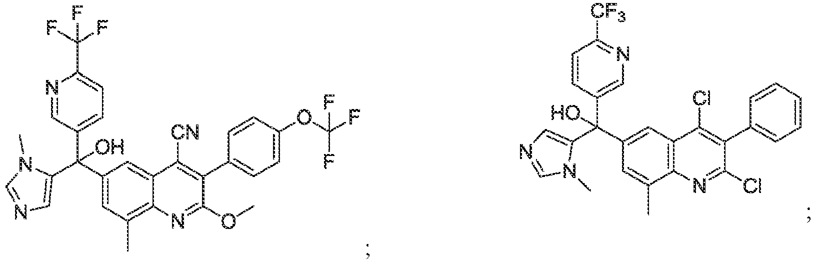 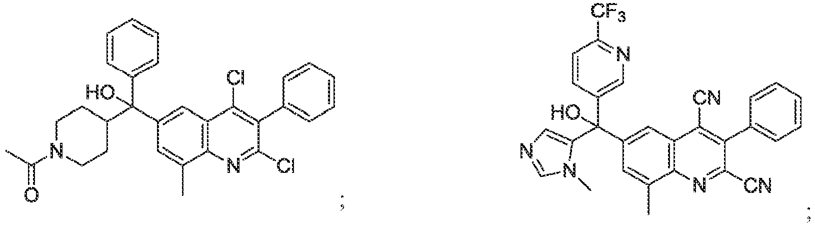 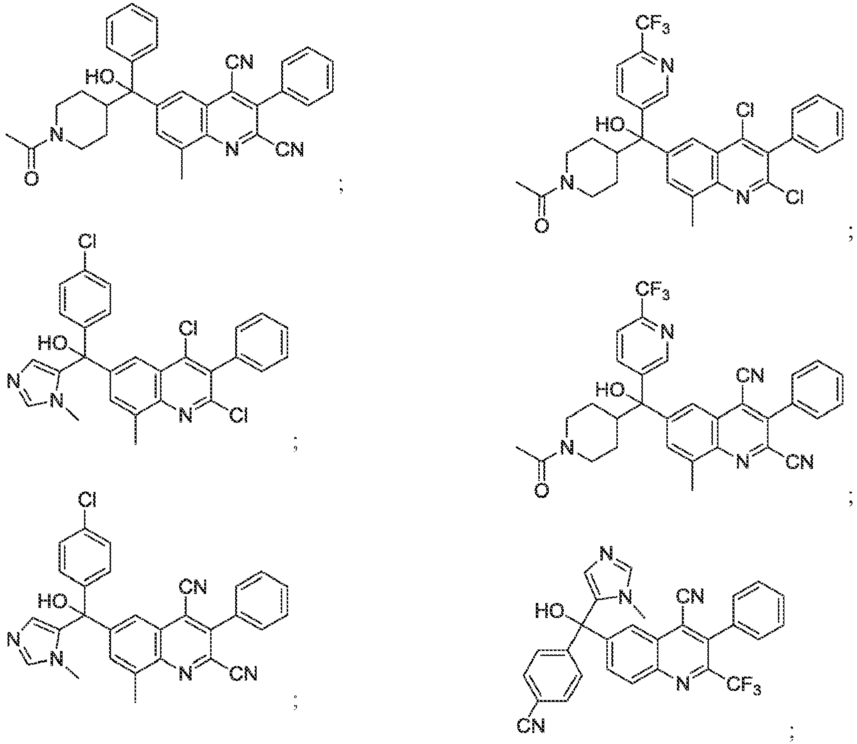 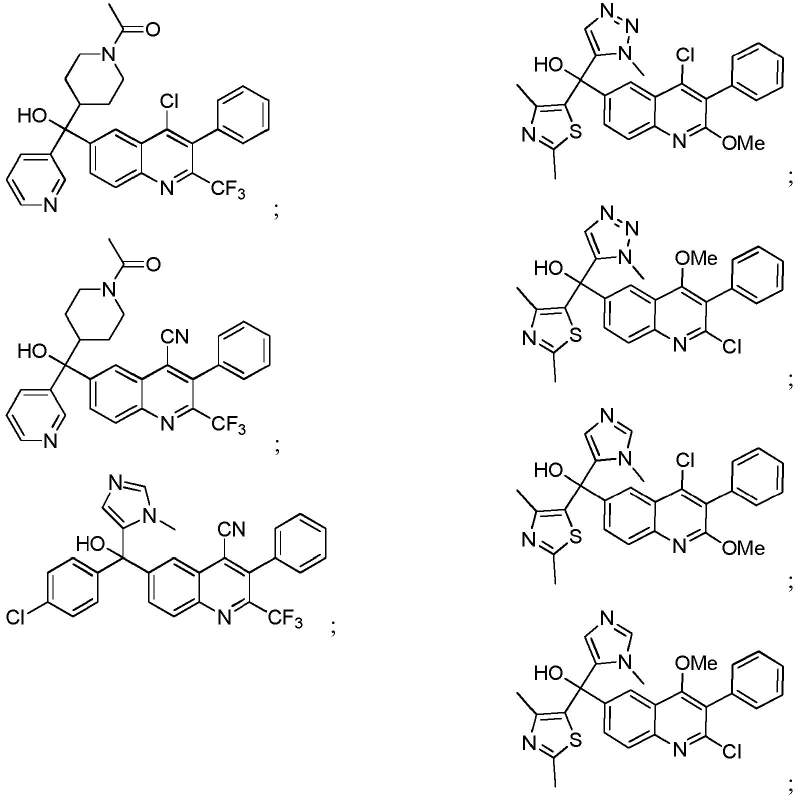 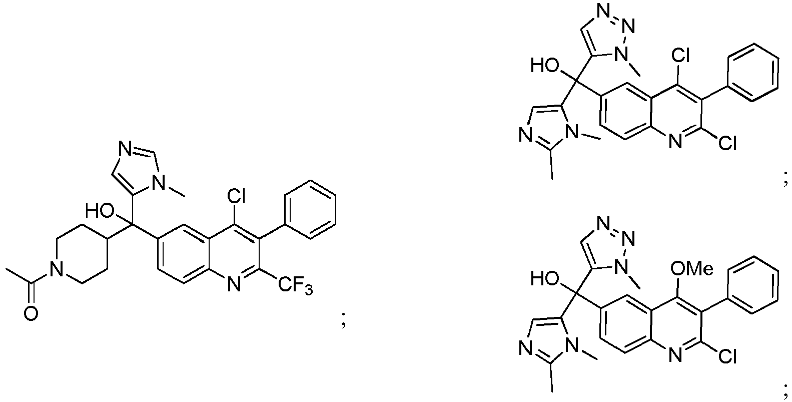 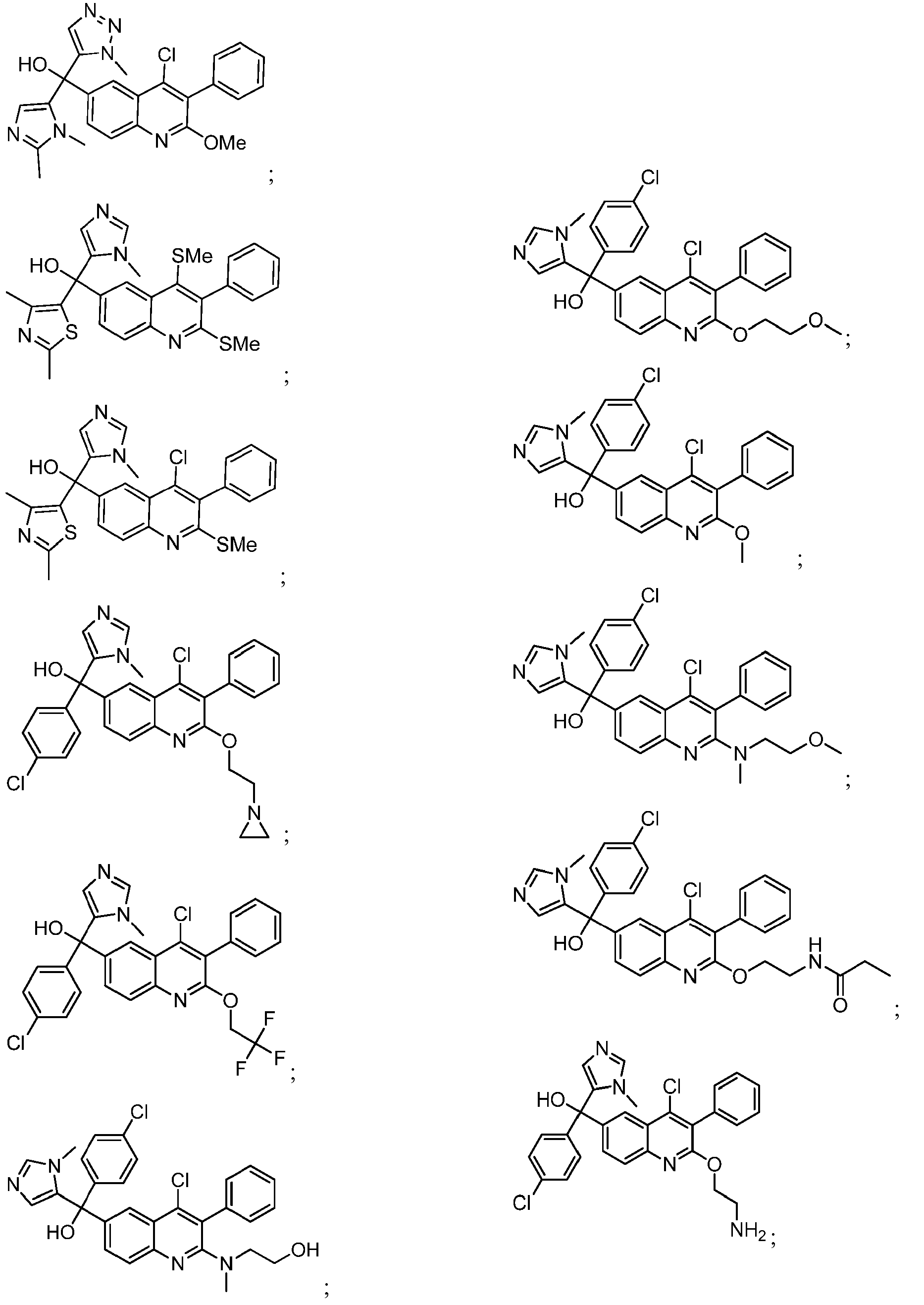 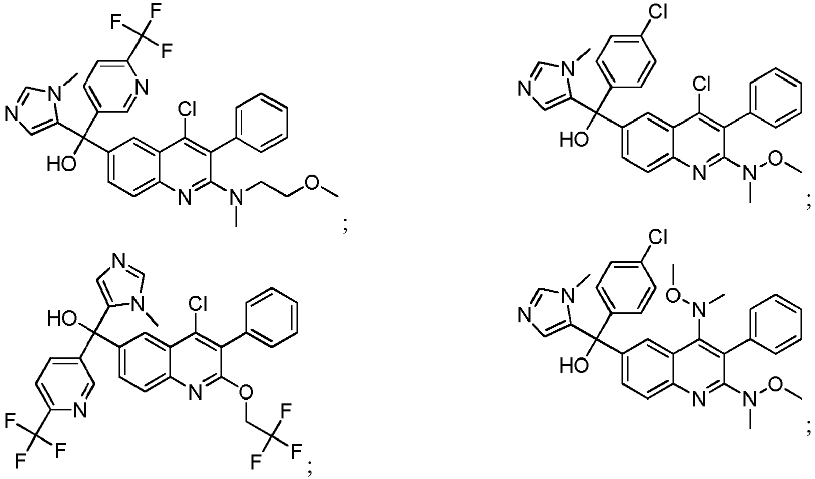 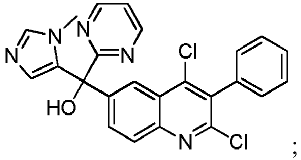 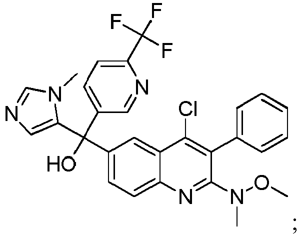 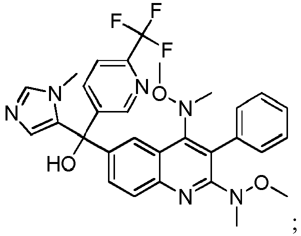 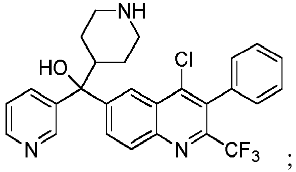 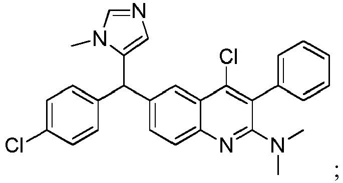 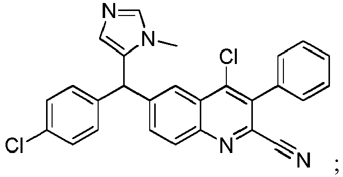 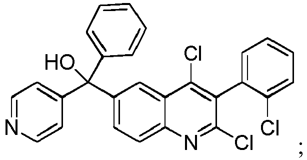 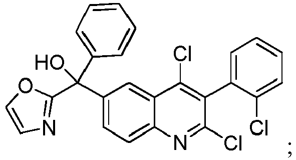 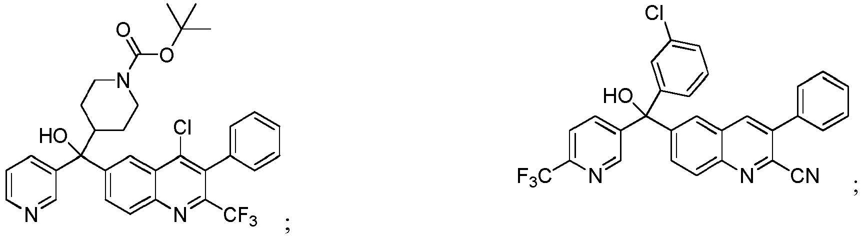 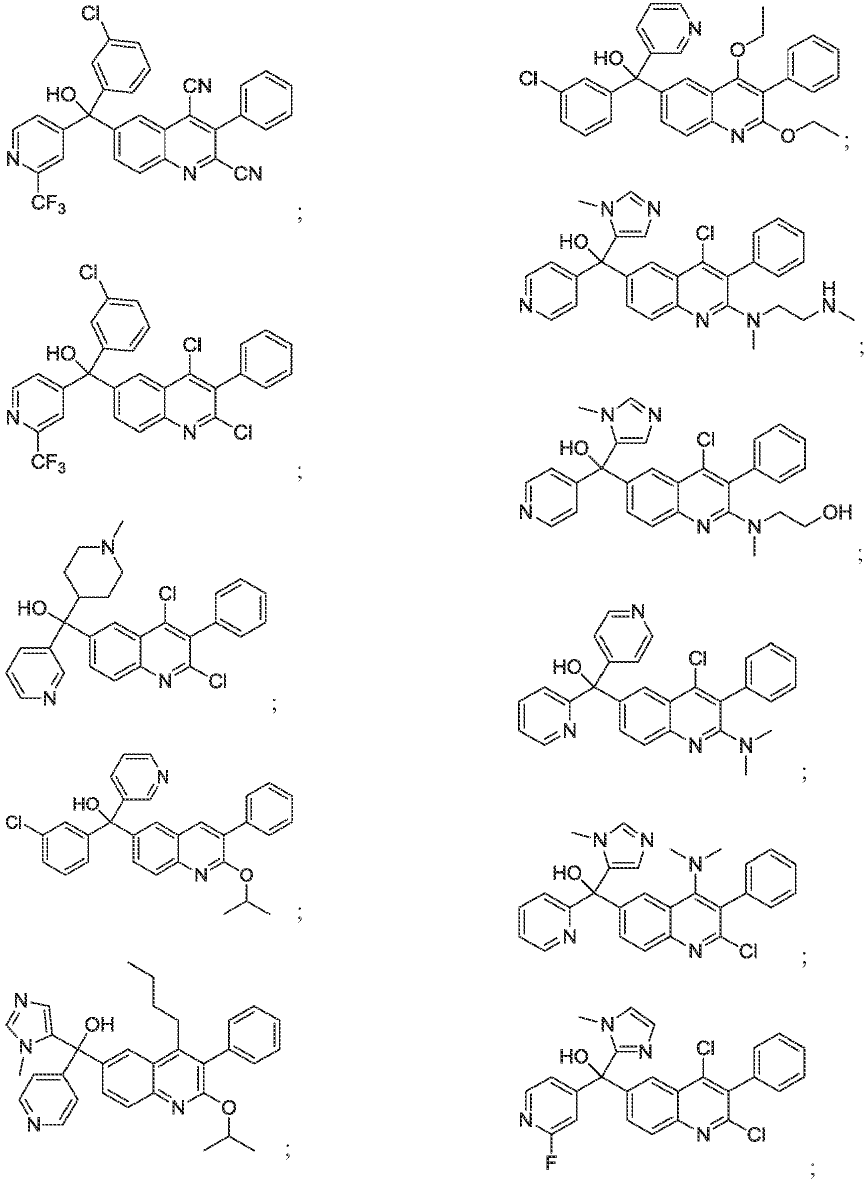 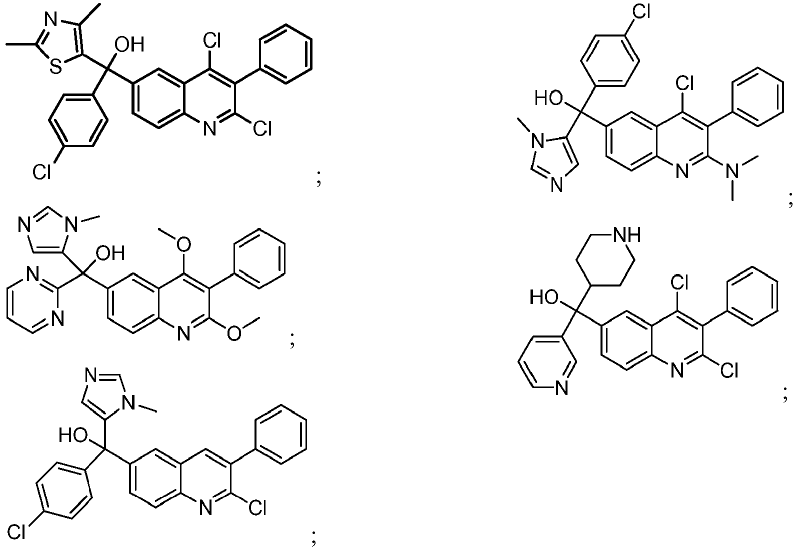 and pharmaceutically acceptable salts thereof.

The disclosure comprises a compound of Formula **I** and a pharmaceutically acceptable carrier.

The present disclosure also provides a method for treating or ameliorating an RORyt mediated inflammatory syndrome, disorder or disease comprising administering to a subject in need thereof an effective amount of a compound of Formula **I** or a form, composition or medicament thereof.

The present disclosure provides a method of preventing, treating or ameliorating a syndrome, disorder or disease, wherein said syndrome, disorder or disease is selected from the group consisting of: ophthalmic disorders, uveitis, atherosclerosis, rheumatoid arthritis, psoriasis, psoriatic arthritis, atopic dermatitis, multiple sclerosis, Crohn's Disease, ulcerative colitis, ankylosing spondylitis, nephritis, organ allograft rejection, fibroid lung, systic fibrosis, renal insufficiency, diabetes and diabetic complications, diabetic nephropathy, diabetic retinopathy, diabetic retinitis, diabetic microangiopathy, tuberculosis, chronic obstructive pulmonary disease, sarcoidosis, invasive *staphylococcia,* inflammation after cataract surgery, allergic rhinitis, allergic conjunctivitis, chronic urticaria, systemic lupus erythematosus, asthma, allergic asthma, steroid resistant asthma, neutrophilic asthma, periodontal diseases, periodonitis, gingivitis, gum disease, diastolic cardiomyopathies, cardiac infarction, myocarditis, chronic heart failure, angiostenosis, restenosis, reperfusion disorders, glomerulonephritis, solid tumors and cancers, chronic lymphocytic leukemia, chronic myelocytic leukemia, multiple myeloma, malignant myeloma, Hodgkin's disease, and carcinomas of the bladder, breast, cervix, colon, lung, prostate, or stomach comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is selected from the group consisting of: rheumatoid arthritis, psoriasis, chronic obstructive pulmonary disorder, psoriatic arthritis, ankylosing spondylitis, Crohn's disease, and ulcerative colitis.

The present disclosure provides a method of treating or ameliorating a syndrome, disorder or disease, wherein said syndrome, disorder or disease is selected from the group consisting of: rheumatoid arthritis, psoriasis, chronic obstructive pulmonary disorder, psoriatic arthritis, ankylosing spondylitis, Crohn's disease, and ulcerative colitis comprising administering to a subject in need thereof an effective amount of a compound of Formula I or a form, composition or medicament thereof.

The present disclosure provides a method of treating or ameliorating a syndrome, disorder or disease, wherein said syndrome, disorder or disease is selected from the group consisting of: rheumatoid arthritis, psoriasis, chronic obstructive pulmonary disorder, psoriatic arthritis, ankylosing spondylitis, Crohn's disease, neutrophilic asthma, steroid resistant asthma, multiple sclerosis, systemic lupus erythematosus, and ulcerative colitis comprising administering to a subject in need thereof an effective amount of a compound of Formula I or a form, composition or medicament thereof.

The present disclosure provides a method of treating or ameliorating a syndrome, disorder or disease, wherein said syndrome, disorder or disease is selected from the group consisting of: rheumatoid arthritis, and psoriasis comprising administering to a subject in need thereof an effective amount of a compound of Formula **I** or a form, composition or medicament thereof.

The present disclosure provides a method of treating or ameliorating a syndrome, disorder or disease, in a subject in need thereof comprising administering to the subject an effective amount of the compound of Formula **I** or composition or medicament thereof in a combination therapy with one or more anti-inflammatory agents, or immunosuppressive agents, wherein said syndrome, disorder or disease is selected from the group consisting of: rheumatoid arthritis, and psoriasis.

The present disclosure provides a method of treating or ameliorating a syndrome, disorder or disease, wherein said syndrome, disorder or disease is rheumatoid arthritis, comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is psoriasis comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is chronic obstructive pulmonary disorder comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is psoriatic arthritis comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is ankylosing spondylitis comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is Crohn's disease comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is ulcerative colitis comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is neutrophilic asthma comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is steroid resistant asthma comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is multiple sclerosis comprising administering to a subject in need thereof an effective amount of a compound of Formula **I** or a form, composition or medicament thereof.

The present disclosure provides a method of treating or ameliorating a syndrome, disorder or disease, wherein said syndrome, disorder or disease is systemic lupus erythematosus comprising administering to a subject in need thereof an effective amount of a compound of Formula **I** or a form, composition or medicament thereof.

The disclosure also relates to methods of modulating RORyt activity in a mammal by administration of an effective amount of at least one compound of Formula **I**.

The present disclosure provides a method of treating or ameliorating a syndrome, disorder or disease, wherein said syndrome, disorder or disease is selected from the group consisting of: inflammatory bowel diseases, rheumatoid arthritis, psoriasis, chronic obstructive pulmonary disorder, psoriatic arthritis, ankylosing spondylitis, neutrophilic asthma, steroid resistant asthma, multiple sclerosis, and systemic lupus erythematosus comprising administering to a subject in need thereof an effective amount of a compound of Formula **I** or a form, composition or medicament thereof.

The present disclosure provides a method of treating or ameliorating an inflammatory bowel disease, wherein said inflammatory bowel disease is Crohn's disease comprising administering to a subject in need thereof an effective amount of a compound of Formula **I** or a form, composition or medicament thereof.

The present disclosure provides a method of treating or ameliorating an inflammatory bowel diseases, wherein said inflammatory bowel disease is ulcerative colitis comprising administering to a subject in need thereof an effective amount of a compound of Formula **I** or a form, composition or medicament thereof.

### DEFINITIONS

The term "administering" with respect to the methods of the invention, means a method for therapeutically or prophylactically preventing, treating or ameliorating a syndrome, disorder or disease as described herein by using a compound of Formula **I** or a form, composition or medicament thereof. Such methods include administering an effective amount of said compound, compound form, composition or medicament at different times during the course of a therapy or concurrently in a combination form. The methods of the invention are to be understood as embracing all known therapeutic treatment regimens.

The term "subject" refers to a patient, which may be an animal, typically a mammal, typically a human, which has been the object of treatment, observation or experiment and is at risk of (or susceptible to) developing a syndrome, disorder or disease that is associated with abberant RORyt expression or RORyt overexpression, or a patient with an inflammatory condition that accompanies syndromes, disorders or diseases associated with abberant RORyt expression or RORyt overexpression.

The term "effective amount" means that amount of active compound or pharmaceutical agent that elicits the biological or medicinal response in a tissue system, animal or human, that is being sought by a researcher, veterinarian, medical doctor, or other clinician, which includes preventing, treating or ameliorating the symptoms of a syndrome, disorder or disease being treated.

As used herein, the term "composition" is intended to encompass a product comprising the specified ingredients in the specified amounts, as well as any product which results, directly or indirectly, from combinations of the specified ingredients in the specified amounts.

The term "alkyl" refers to both linear and branched chain radicals of up to 12 carbon atoms, preferably up to 6 carbon atoms, unless otherwise indicated, and includes, but is not limited to, methyl, ethyl, propyl, isopropyl, butyl, isobutyl, sec-butyl, tert-butyl, pentyl, isopentyl, hexyl, isohexyl, heptyl, octyl, 2,2,4-trimethylpentyl, nonyl, decyl, undecyl and dodecyl. Any alkyl group may be optionally substituted with one OCH₃, one OH, or up to two fluorine atoms.

The term "C_{(*α-b)*}" (where *α* and *b* are integers referring to a designated number of carbon atoms) refers to an alkyl, alkenyl, alkynyl, alkoxy or cycloalkyl radical or to the alkyl portion of a radical in which alkyl appears as the prefix root containing from *α* to *b* carbon atoms inclusive. For example, C₍₁₋₄₎ denotes a radical containing 1, 2, 3 or 4 carbon atoms.

The term "cycloalkyl" refers to a saturated or partially unsaturated monocyclic or bicyclic hydrocarbon ring radical derived by the removal of one hydrogen atom from a single ring carbon atom. Typical cycloalkyl radicals include cyclopropyl, cyclobutyl, cyclopentyl, cyclopentenyl, cyclohexyl, cyclohexenyl, cycloheptyl and cyclooctyl. Additional examples include C₍₃₋₆₎cycloalkyl, C₍₅₋₈₎cycloalkyl, decahydronaphthalenyl, and 2,3,4,5,6,7-hexahydro-lH-indenyl. Any cycloalkyl group may be optionally substituted with one OCH₃, one OH, or up to two fluorine atoms.

As used herein, the term "thiophenyl" is intended to describe the radical formed by removing a hydrogen atom from the molecule with the structure:

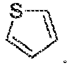

### PHARMACEUTICALLY ACCEPTABLE SALTS

Pharmaceutically acceptable acidic/anionic salts include, and are not limited to acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, glyceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate, pantothenate, phosphate/diphosphate, polygalacturonate, salicylate, stearate, subacetate, succinate, sulfate, tannate, tartrate, teoclate, tosylate and triethiodide. Organic or inorganic acids also include, and are not limited to, hydriodic, perchloric, sulfuric, phosphoric, propionic, glycolic, methanesulfonic, hydroxyethanesulfonic, oxalic, 2-naphthalenesulfonic, p-toluenesulfonic, cyclohexanesulfamic, saccharinic or trifluoroacetic acid.

Pharmaceutically acceptable basic/cationic salts include, and are not limited to aluminum, 2-amino-2-hydroxymethyl-propane-1,3-diol (also known as tris(hydroxymethyl)aminomethane, tromethane or "TRIS"), ammonia, benzathine, *t*-butylamine, calcium, calcium gluconate, calcium hydroxide, chloroprocaine, choline, choline bicarbonate, choline chloride, cyclohexylamine, diethanolamine, ethylenediamine, lithium, LiOMe, L-lysine, magnesium, meglumine, NH₃, NH₄OH, *N*-methyl-D-glucamine, piperidine, potassium, potassium-*t*-butoxide, potassium hydroxide (aqueous), procaine, quinine, sodium, sodium carbonate, sodium-2-ethylhexanoate, sodium hydroxide, triethanolamine or zinc.

### METHODS OF USE

The present disclosure is directed to a method for preventing, treating or ameliorating a RORyt mediated inflammatory syndrome, disorder or disease comprising administering to a subject in need thereof an effective amount of a compound of Formula I or a form, composition or medicament thereof.

Since RORyt is an N-terminal isoform of RORγ, it is recognized that compounds of the present invention which are modulators of RORyt are likely to be modulators of RORγ as well. Therefore the mechanistic description "RORyt modulators" is intended to encompass RORγ modulators as well.

When employed as RORyt modulators, the compounds of the invention may be administered in an effective amount within the dosage range of about 0.5 mg to about 10 g, preferably between about 0.5 mg to about 5 g, in single or divided daily doses. The dosage administered will be affected by factors such as the route of administration, the health, weight and age of the recipient, the frequency of the treatment and the presence of concurrent and unrelated treatments.

It is also apparent to one skilled in the art that the therapeutically effective dose for compounds of the present invention or a pharmaceutical composition thereof will vary according to the desired effect. Therefore, optimal dosages to be administered may be readily determined by one skilled in the art and will vary with the particular compound used, the mode of administration, the strength of the preparation, and the advancement of the disease condition. In addition, factors associated with the particular subject being treated, including subject age, weight, diet and time of administration, will result in the need to adjust the dose to an appropriate therapeutic level. The above dosages are thus exemplary of the average case. There can, of course, be individual instances where higher or lower dosage ranges are merited, and such are within the scope of this invention.

The compounds of Formula **I** may be formulated into pharmaceutical compositions comprising any known pharmaceutically acceptable carriers. Exemplary carriers include, but are not limited to, any suitable solvents, dispersion media, coatings, antibacterial and antifungal agents and isotonic agents. Exemplary excipients that may also be components of the formulation include fillers, binders, disintegrating agents and lubricants.

The pharmaceutically-acceptable salts of the compounds of Formula **I** include the conventional non-toxic salts or the quaternary ammonium salts which are formed from inorganic or organic acids or bases. Examples of such acid addition salts include acetate, adipate, benzoate, benzenesulfonate, citrate, camphorate, dodecylsulfate, hydrochloride, hydrobromide, lactate, maleate, methanesulfonate, nitrate, oxalate, pivalate, propionate, succinate, sulfate and tartrate. Base salts include ammonium salts, alkali metal salts such as sodium and potassium salts, alkaline earth metal salts such as calcium and magnesium salts, salts with organic bases such as dicyclohexylamino salts and salts with amino acids such as arginine. Also, the basic nitrogen-containing groups may be quaternized with, for example, alkyl halides.

The pharmaceutical compositions of the invention may be administered by any means that accomplish their intended purpose. Examples include administration by parenteral, subcutaneous, intravenous, intramuscular, intraperitoneal, transdermal, buccal or ocular routes. Alternatively or concurrently, administration may be by the oral route. Suitable formulations for parenteral administration include aqueous solutions of the active compounds in water-soluble form, for example, water-soluble salts, acidic solutions, alkaline solutions, dextrose-water solutions, isotonic carbohydrate solutions and cyclodextrin inclusion complexes.

The present invention also encompasses a method of making a pharmaceutical composition comprising mixing a pharmaceutically acceptable carrier with any of the compounds of the present invention. Additionally, the present invention includes pharmaceutical compositions made by mixing a pharmaceutically acceptable carrier with any of the compounds of the present invention.

### POLYMORPHS AND SOLVATES

Furthermore, the compounds of the present invention may have one or more polymorph or amorphous crystalline forms and as such are intended to be included in the scope of the invention. In addition, the compounds may form solvates, for example with water (i.e., hydrates) or common organic solvents. As used herein, the term "solvate" means a physical association of the compounds of the present invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. The term "solvate" is intended to encompass both solution-phase and isolatable solvates. Non-limiting examples of suitable solvates include ethanolates, methanolates, and the like.

It is intended that the present invention include within its scope polymorphs and solvates of the compounds of the present invention. Thus, the term "administering" shall encompass the means for treating, ameliorating or preventing a syndrome, disorder or disease described herein with the compounds of the present invention or a polymorph or solvate thereof, which would obviously be included within the scope of the invention albeit not specifically disclosed.

In another embodiment, the disclosure relates to a compound as described in Formula I for use as a medicament.

In another embodiment, the disclosure relates to the use of a compound as described in Formula I for the preparation of a medicament for the treatment of a disease associated with an elevated or aberrant RORyt activity.

The present disclosure includes within its scope prodrugs of the compounds of this invention. In general, such prodrugs will be functional derivatives of the compounds which are readily convertible *in vivo* into the required compound. Thus, the term "administering" shall encompass the treatment of the various disorders described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs", Ed. H. Bundgaard, Elsevier, 1985.

Furthermore, it is intended that within the scope of the present invention, any element, in particular when mentioned in relation to a compound of Formula **I**, shall comprise all isotopes and isotopic mixtures of said element, either naturally occurring or synthetically produced, either with natural abundance or in an isotopically enriched form. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O. The isotopes may be radioactive or nonradioactive. Radiolabelled compounds of Formula **I** may comprise a radioactive isotope selected from the group of ³H, ¹¹C, ¹⁸F, ¹²²I, ¹²³I, ¹²⁵I, ¹³¹I, ⁷⁵Br, ⁷⁶Br, ⁷⁷Br and ⁸²Br. Preferably, the radioactive isotope is selected from the group of ³H, ¹¹C and ¹⁸F.

Some compounds of the present invention may exist as atropisomers. Atropisomers are stereoisomers resulting from hindered rotation about single bonds where the steric strain barrier to rotation is high enough to allow for the isolation of the conformers. It is to be understood that all such conformers and mixtures thereof are encompassed within the scope of the present invention.

Where the compounds according to this invention have at least one stereocenter, they may accordingly exist as enantiomers or diastereomers. It is to be understood that all such isomers and mixtures thereof are encompassed within the scope of the present invention.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

### ABBREVIATIONS

Herein and throughout the application, the following abbreviations may be used.
- Å: angstrom
- Ac: acetyl
- Ac₂O: acetic anhydride
- Boc: tert-butyloxy carbonyl
- BHT: butylated hydroxytoluene
- br: broad
- Bu: butyl
- *n*-BuLi: *n*-butyl lithium
- *t*-BuOH: *tert*-butanol
- d: doublet
- dba: dibenzylideneacetone
- DCE: dichloroethane
- DCM: dichloromethane
- Dess-Martin periodinane: 1,1,1 -tris(acetyloxy)-1,1 -dihydro-1,2-benziodoxol-3-(1*H*)-one
- DIEA: *N*,*N*-diisopropylethylamine
- DMA: dimethylacetamide
- DME: dimethoxyethane
- DMF: *N,N*-dimethylformamide
- DMSO: dimethyl sulfoxide
- dppf: (diphenylphosphino)ferrocene
- Eaton's Reagent: 7.7 wt% phosphorus pentoxide solution in methanesulfonic acid
- EDCI: *N*-(3-dimethylaminopropyl)-*N'*-ethylcarbodiimide hydrochloride
- ESI: electrospray ionization
- Et: ethyl
- Et₂O: diethyl ether
- EtOAc: ethyl acetate
- EtOH: ethyl alcohol
- FCC: flash column chromatography
- HATU: *O*-(7-azabenzotriazol-1-yl)-*N,N,N,N'* -tetramethyluronium hexafluorophosphate
- HPLC: high pressure liquid chromatography
- Hunig's base: *N, N*-diisopropylethylamine
- Hz: hertz
- i-PrOH: isopropyl alcohol
- KHMDS: potassium bis(trimethylsilyl)amide
- LCMS: liquid chromatography-mass spectrometry
- LDA: lithium diisopropylamide
- m: multiplet
- M: molar (moles/liter)
- mCPBA: 3-chloroperbenzoic acid
- Me: methyl
- Meldrum's acid: 2,2-dimethyl-1,3-dioxane-4,6-dione
- MeOH: methanol
- MeONa: sodium methoxide
- MHz: megahertz
- min: minutes
- mL: milliliters
- MS: mass spectrometry
- MTBE: methyl tertiary butyl ether
- m/z: mass to charge ratio
- nm: nanometers
- NaO*i*Pr: sodium isopropoxide
- NBS: *N*-bromosuccinimide
- NMP: 1-methyl-2-pyrrolidinone
- NMR: nuclear magnetic resonance
- Ph: phenyl
- PPA: polyphosphoric acid
- ppm: parts per million
- Pr: propyl
- q: quartet
- RP-HPLC: reverse phase high pressure liquid chromatography
- s: singlet
- t: triplet
- TBAF: tetrabutylammonium fluoride
- TEA: triethylamine
- TEMPO: (2,2,6,6-tetramethylpiperidin-1-yl)oxidanyl
- TFA: trifluoroacetic acid
- THF: tetrahydrofuran
- TLC: thin layer chromatography
- UV: ultra-violet
- X-Phos: 2-dicyclohexylphosphino-2',4',6'-triisopropylbiphenyl

### GENERAL SCHEMES:

Compounds of Formula **I** in the present invention can be synthesized in accordance with the general synthetic methods known to those who are skilled in the art. The following reaction schemes are only meant to represent examples of the invention and are in no way meant to be a limit of the invention.

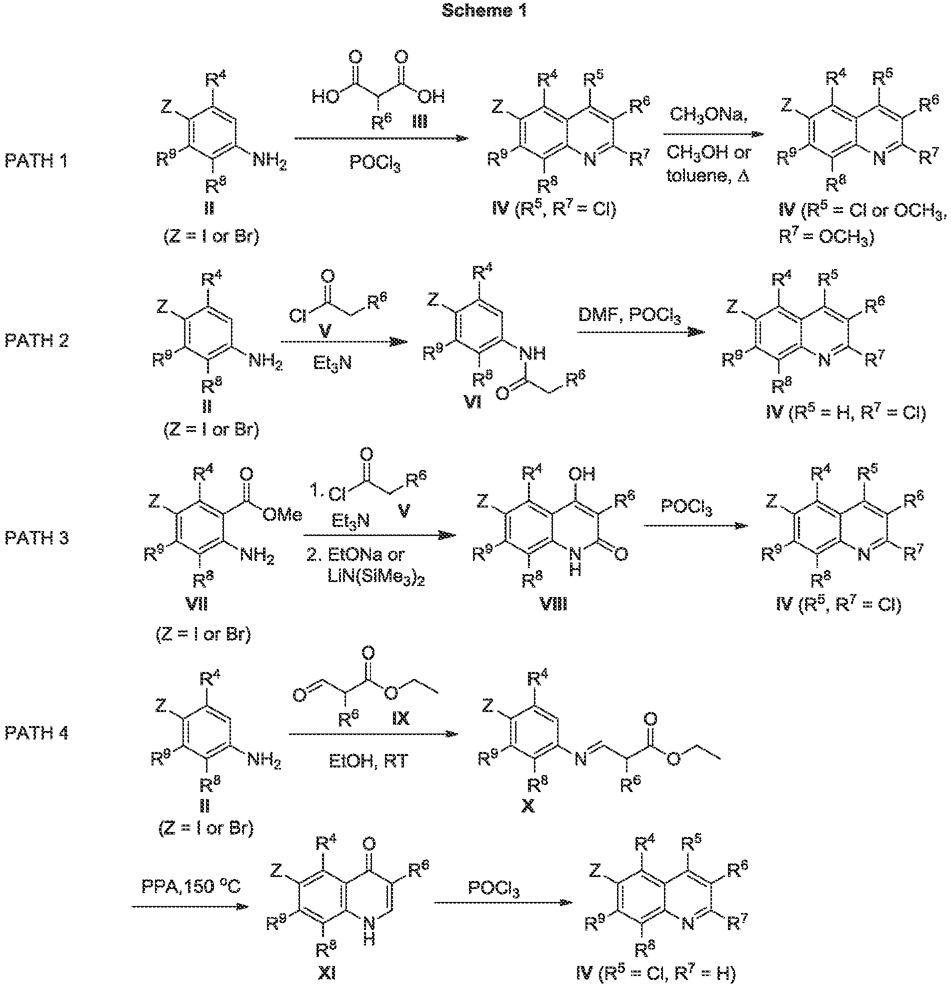

Scheme 1 describes the preparation of 6-bromo or 6-iodo-quinolines of Formula **IV** by various methods (path 1 to 4). In path 1, cyclization of 4-haloanilines **II** with commercially available 2-substituted malonic acids **III** can be done in refluxing phosphorus oxychloride to provide 6-haloquinolines **IV,** wherein R⁵ and R⁷ are Cl. Nucleophilic displacement of 2-chloro substitution with sodium methoxide in hot MeOH or toluene (Alan Osborne et.al. J. Chem. Soc. Perkin Trans. 1 (1993) 181 - 184 and J. Chem. Research (S), 2002, 4) gives 6-halo-2-methoxyquinolines **IV**. Path 2 illustrates the cyclization of amides **VI**, derived from acylation of 4-haloanilines **II** with substituted acid chlorides **V** (acid chlorides **V** are either commercially available or prepared from the corresponding carboxylic acid precursors by procedures known to those skilled in the art), in the presence of DMF in hot phosphorus oxychloride to generate 6-haloquinolines **IV,** wherein R⁵ is **H** and R⁷ is Cl. In path 3, methyl 2-aminobenzoates **VII** can undergo acylation with acid chlorides **V** to form an amide intermediate, which can be further treated with a base, such as sodium ethoxide or lithium bis(trimethylsilyl)amide, to afford 6-halo-4-hydroxyquinolin-2(1*H*)-ones **VIII.** Conversion of hydroxyquinolin-2(1*H*)-ones **VIII** to 2,4-dichloroquinolines **IV** can be carried out in refluxing phosphorus oxychloride. Path 4 describes the condensation of anilines **II** and aldehydes **IX** in ethanol to form compound **X** which can be further cyclized in polyphosphoric acid at high temperatures to give quinolinones **XI.** Conversion to the 4-chloroquinolines **IV** wherein R⁷ is H can be accomplished in phosphorus oxychloride as previously described.

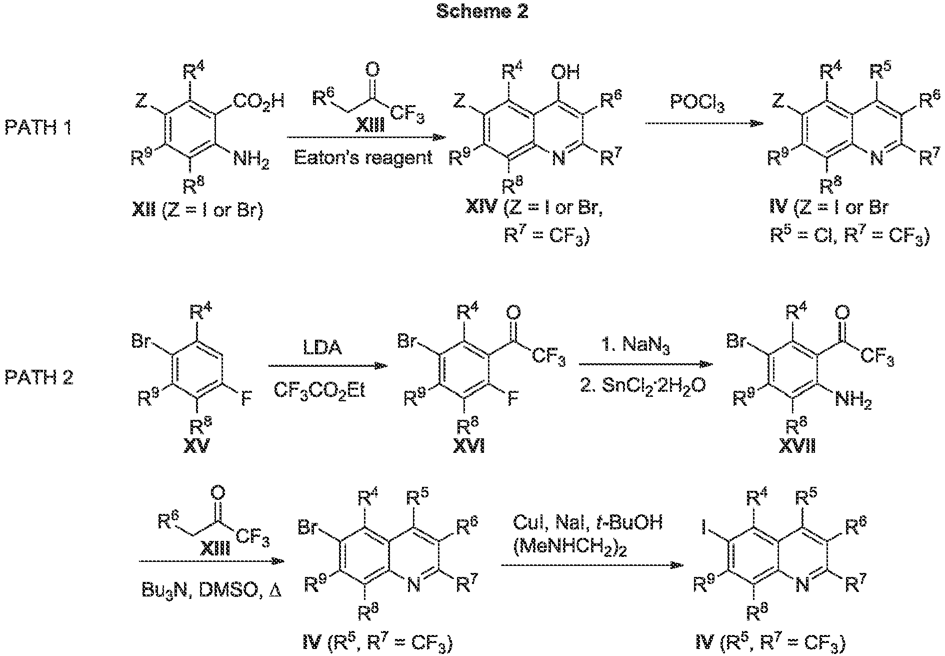

Scheme 2 illustrates the synthesis leading to 6-bromo or 6-iodoquinolines of Formula **IV** wherein R⁵ is Cl and R⁷ is CF₃ (path 1), and 6-iodoquinolines of Formula **IV** where R⁵ and R⁷ are CF₃ (path 2). In path 1, cyclization of 2-aminobenzoic acids **XII** with 1,1,1-trifluoropropan-2-ones **XIII** in Eaton's reagent at elevated temperatures yields 4-hydroxy-2-trifluoromethylquinolines **XIV,** which upon heating in phosphorus oxychloride at temperatures between 100 - 120 °C gives 6-bromo or 6-iodoquinolines **IV,** wherein R⁵ is Cl and R⁷ is CF₃. 6-Iodo-2,4-bis(trifluoromethyl)quinolines **IV** can be formed by the reaction sequence illustrated in path 2. Treatment of 1-bromo-4-fluorobenzenes **XV** with lithium diisopropylamide at - 78 °C followed by addition of ethyl trifluoroacetate provides 2-fluorophenyl-2,2,2-trifluoroethanones **XVI**. Anilines **XVII** can be prepared by displacing 2-fluoro in **XVI** with sodium azide followed by reduction with tin (II) chloride dihydrate. Cyclization of **XVII** with 1,1,1-trifluoropropan-2-ones **XIII** in the presence of tributylamine in a polar solvent, such as DMF or DMSO, at high temperatures can provide 6-bromo-2,4-bis(trifluoromethyl)quinolines **IV.** The 6-iodo-2,4-bis(trifluoromethyl)quinolines **IV** can then be subsequently obtained by conversion of 6-bromoquinoline **IV**, where R⁵ and R⁷ are CF₃, with NaI, CuI, and *N,N'*-dimethylethylenediamine in *t*-BuOH at high temperatures under microwave conditions.

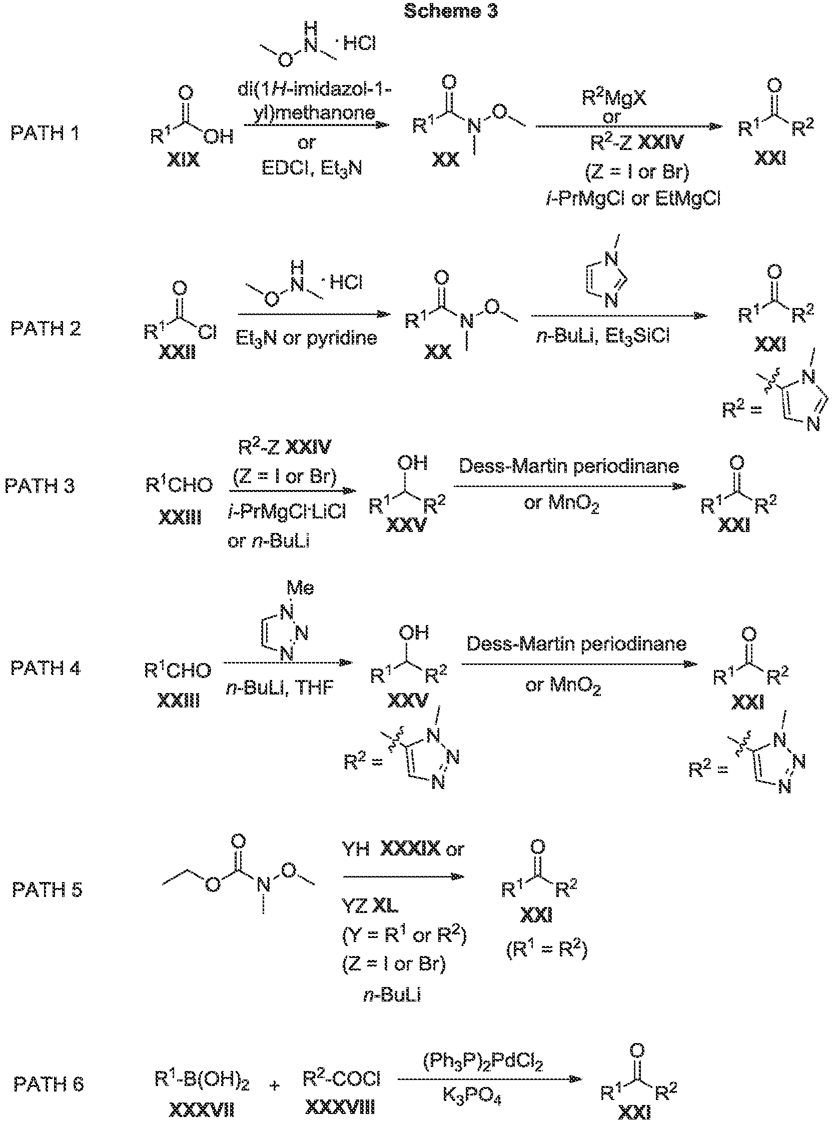

Scheme 3 illustrates synthetic routes (path 1 to 6) to ketones of Formula **XXI** wherein R¹ and R² are as described in the detailed description of the invention. In path 1, Weinreb amide **XX** can be prepared from acids **XIX** by reacting with *N,O*-dimethylhydroxylamine hydrochloride and 1,1-carbonyldiimidazole or with *N,O*-dimethylhydroxylamine hydrochloride in the presence of a base such as triethylamine or Hunig's base and a coupling reagent such as EDCI. The amides **XX** can be further treated with Grignard reagents such as R²MgX (X is Br or Cl) that can be obtained commercially or preformed by treatment of R²Z with organometallic reagents such as i-PrMgCl or EtMgCl in THF. Alternatively, Weinreb amides **XX** can be obtained from acyl chlorides **XXII** and N,O-dimethylhydroxylamine hydrochloride by using triethylamine or pyridine as a base. 1-Methyl-1*H*-imidazole can be treated with one equivalent of *n*-BuLi and one equivalent of chlorotriethylsilane at - 78 °C followed by an additional equivalent of *n*-BuLi, to which the Weinreb amides **XX** can be added to yield ketones **XXI** wherein R² is imidazolyl (path 2).

In path 3, halogen and metal exchange of bromides or iodides **XXIV** with *i*-PrMgCl.LiCl or n-BuLi, followed by addition of aldehydes **XXIII** affords alcohols **XXV**. Oxidation of **XXV** with Dess-Martin periodinane or MnO₂ can provide ketones **XXI**. In path 4, ketones **XXI,** where R² is triazolyl, can be prepared by treatment of 1-methyl-1*H*-1,2,3-triazole with *n*-BuLi followed by reaction with aldehydes **XXIII** to yield alcohols **XXV,** which could undergo oxidation with Dess-Martin periodinane or MnO₂. Path 5 exemplifies the preparation of symmetrical ketones **XXI,** wherein R¹ and R² are the same. As illustrated, an aryl or heteroaryl group containing an acidic proton **XXXIX** (Y = R¹ or R²) can be deprotonated in the presence of a strong base such as *n*-butyllithium once solubilized in a preferred solvent such as tetrahydrofuran at temperatures between 0 and -78 °C then added in excess to ethyl methoxy(methyl)carbamate to provide ketones **XXI** wherein R¹ and R² are the same. Aryl or heteroaryl bromide or iodide **XL** can also be lithiated through a lithium/halogen exchange with n-butyllithium before adding in excess to ethyl methoxy(methyl)carbamate as previously described to provide symmetrical ketones **XXI**. Path 6, which employs palladium catalyzed cross-coupling of arylboronic acids **XXXVII** with acid chlorides **XXXVIII** using K₃PO₄ as a base and (Ph₃P)₂PdCl₂ as a catalyst in a high boiling non-polar solvent such as toluene, can also be used to generate ketones **XXI.**

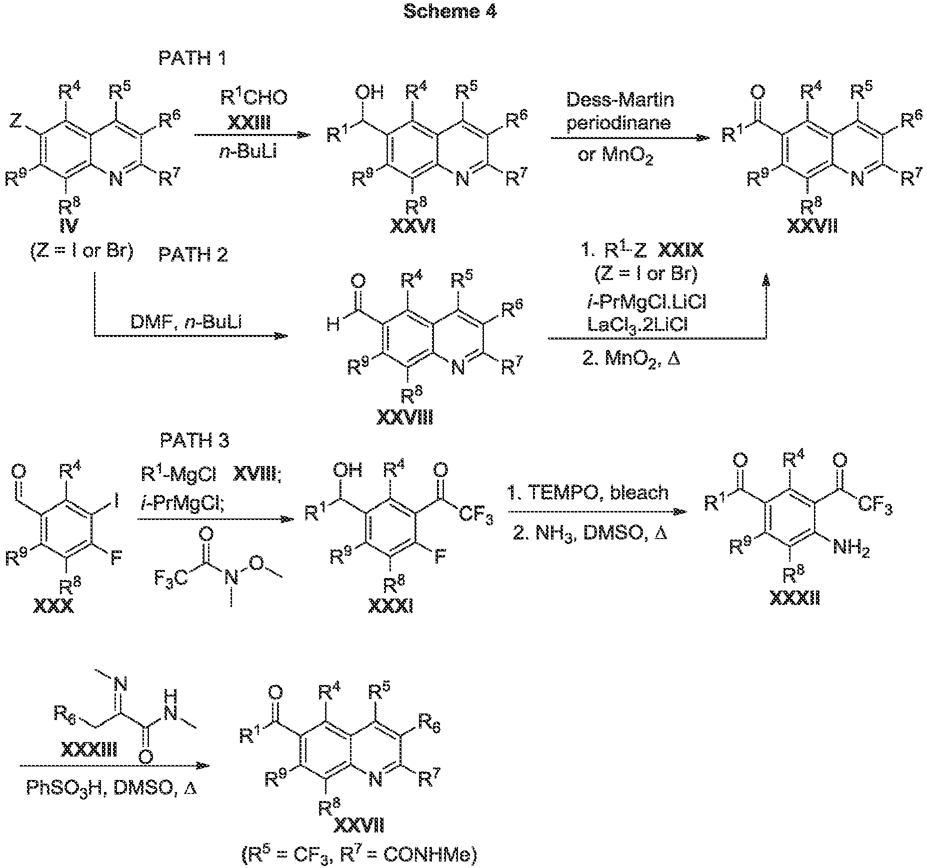

Synthesis leading to intermediate ketones **XXVII** may also be achieved via chemical routes shown in Scheme 4. In path 1, treatment of 6-bromo or 6-iodoquinolines **IV** with *n*-BuLi at - 78 °C followed by addition of aldehydes **XXIII** provides secondary alcohol quinolines **XXVI**, which can be oxidized to ketones **XXVII** with Dess-Martin periodinane or MnO₂. Alternatively, ketones **XXVII** may also be prepared by treatment of 6-haloquinolines **IV** with n-BuLi at -78 °C followed by quenching with DMF affording carboxaldehydes **XXVIII**. Ketones **XXVII** can be obtained in a two-step process by addition of aldehyde **XXVIII** to a reaction mixture of aryl iodides or bromides **XXIX** and *i*-PrMgCl·LiCl followed by oxidation with MnO₂ (path 2).

As illustrated in path 3, a one-pot reaction of aldehydes **XXX** and Grignard reagents such as R¹-MgCl **XVIII** followed by treatment with *i*-PrMgCl and addition of 2,2,2-trifluoro-*N*-methoxy-*N-*methylacetamide yields hydroxyl compounds **XXXI.** The hydroxyl group can be oxidized using bleach and TEMPO. Fluoro displacement can then be achieved with ammonia in hot DMSO to provide anilines **XXXII**. In the presence of benzenesulfonic acid, condensation of anilines **XXXII** and 2-(methylimino)butanamides **XXXIII** in hot DMSO furnishes ketoquinolines **XXVII** wherein R⁵ is CF₃ and R⁷ is CONHMe.

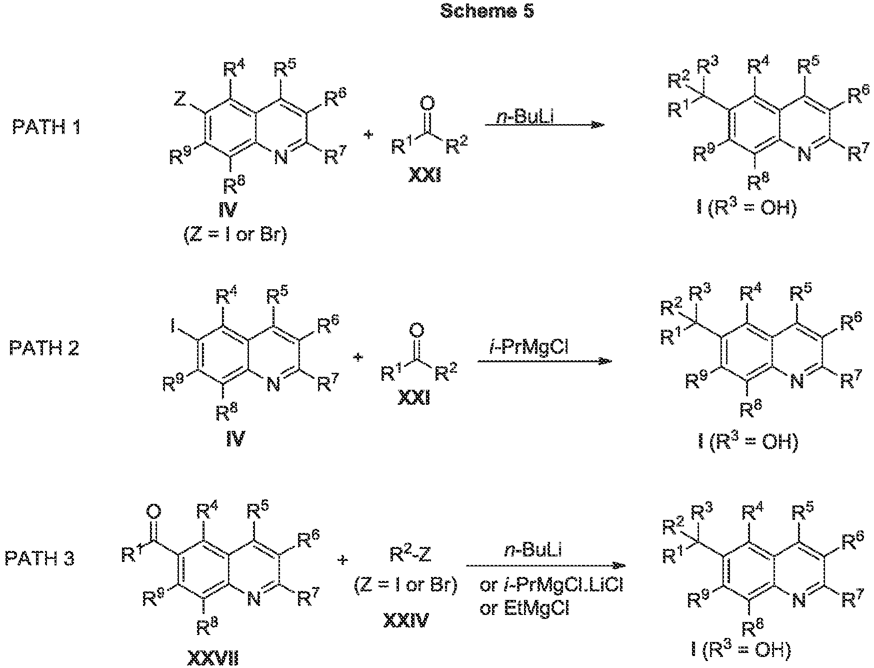

Scheme 5 illustrates synthetic routes leading to compounds of Formula **I** (path 1 to 3). As illustrated in path 1, a mixture of the 6-bromo or 6-iodoquinolines **IV** in an appropriate solvent such as THF can be either premixed with the ketones **XXI** at -78 °C followed by addition *of n-*BuLi or can be pretreated with *n*-BuLi at -78 °C prior to the addition of the ketones **XXI** to afford the tertiary alcohols of Formula **I,** wherein R³ is OH. In path 2, 6-iodoquinolines **IV** can be treated with *i*-PrMgCl followed by addition of ketone **XXI** to yield compounds of Formula **I** wherein R³ is OH. As shown in path 3, halogen-metal exchange of aryl halides (iodide or bromide) **XXIV** with an organometallic reagent, such as n-BuLi, *i*-PrMgCl.LiCl, or EtMgCl, at an appropriate temperature, such as -78 °C or 0 °C, followed by reaction with ketones **XXVII** may afford tertiary alcohol quinolines of Formula **I**. Compounds of Formula **I** wherein R² is *N-*Boc piperdinyl can be deprotected under acidic conditions using standard procedures known in the art then further functionalized on nitrogen by treatment with an anhydride or acylating agent such as acetylchloride to provide compounds of Formula **I** wherein R² is *N*-acetyl piperidin-4-yl.

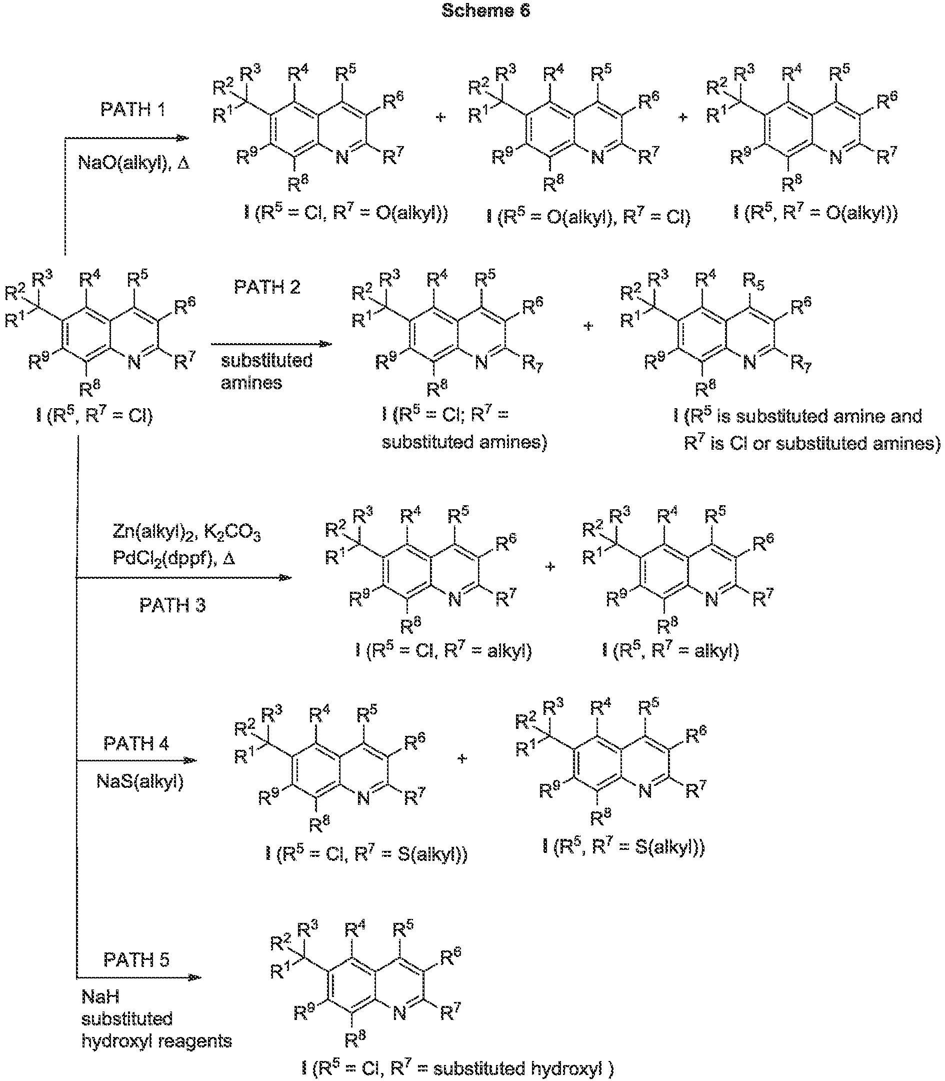

Scheme 6 illustrates methods used to synthesize compounds of Formula **I** wherein either the chlorine at R⁷ or at both R⁵ and R⁷ positions are replaced with nitrogen, oxygen, sulfur or alkyl groups. In path 1, 4 and 5, nucleophilic displacement of 2,4-dichloroquinolines **I** (R⁵ and R⁷ are Cl) with NaO(alkyl), NaS(alkyl), such as NaOMe, NaSMe, NaOEt, or NaO*ⁱ*Pr, in an appropriate solvent, such as MeOH, EtOH, *i*-PrOH or DMF at elevated temperatures or with substituted hydroxy reagents such as 2-methoxyethanol, 2,2,2-trifluoroethanol or amine substituted hydroxyl reagents in the presence of a base like sodium hydride in a non-polar solvent such as toluene provides compounds of Formula **I** wherein R⁵ is Cl and R⁷ is O(alkyl), S(alkyl), O(CH₂)₂OCH₃, OCH₂CF₃, or O(CH₂)₂NA¹A² wherein A¹ and A² are defined in the detailed description of the invention and compounds of Formula **I** wherein R⁵ and R⁷ are O(alkyl) or S(alkyl). Likewise, nucleophilic displacement of 2,4-dichloroquinolines **I** (R⁵ and R⁷ are Cl) with primary or secondary alkyl amines, heterocycle amines, or *N,O*-dimethylhydroxylamine in polar solvents such as MeOH, EtOH, or Et₂NCHO, or DMF provides quinolines of Formula **I** (path 2) wherein R⁵ is NH(alkyl), N(alkyl)₂, N(CH₃)OCH₃, or Cl, and R⁷ is NH(alkyl), N(alkyl)₂, N(CH₃)OCH₃, NA¹A², NHC₍₂₋₃₎alkylNA¹A² or N(CH₃)C₍₂₋₄₎alkylNA¹A², wherein A¹ and A² are as defined above. Replacement of chlorine at positions 2 and 4 of quinolines **I** (R⁵ and R⁷ are Cl) with alkyl groups could be carried out using Zn(alkyl)₂ in the presence of K₂CO₃ and a palladium catalyst, such as PdCl₂(dppf), to afford 2-alkyl and 2,4-dialkylquinolines **I** (path 3).

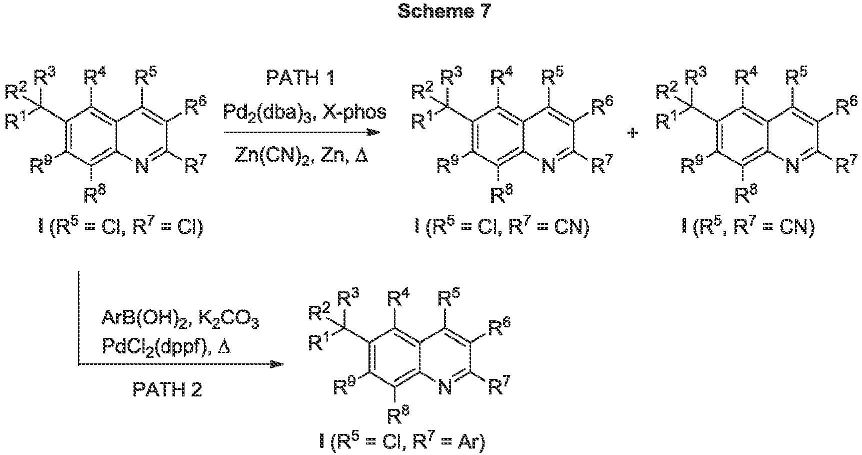

Synthetic routes to compounds of Formula **I,** wherein R⁵ is Cl or CN, and R⁷ is CN or aryl, are illustrated in Scheme 7. In path 1, cyanation of the 2,4-dichloroquinolines **I** with Zn(CN)₂ in the presence of Zn, a palladium catalyst, such as Pd₂(dba)₃, and a ligand, such as dppf or X-phos, at high temperatures can provide 2-CN and 2,4-diCN quinolines **I**. The 2,4-dichloroquinolines **I** can also undergo a Suzuki reactions with ArB(OH)₂ or ArB(OR)₂ and a palladium catalyst, such as PdCl₂(dppf), yielding compounds of Formula **I** wherein R⁷ is phenyl, substituted phenyl and five or six-membered heteroaryls such as furan, pyridine, pyridazine, pyrazine, pyrimidine, pyrrol, pyrazole or imidazole (path 2).

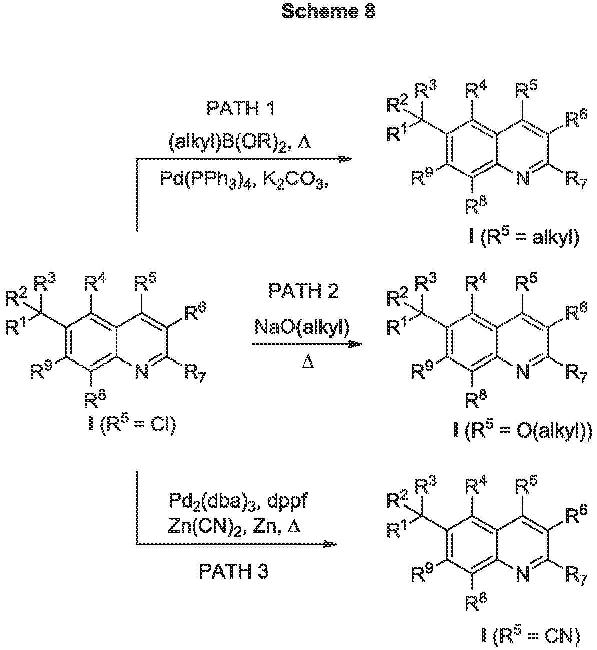

As illustrated in Scheme 8, compounds of Formula **I** wherein R⁵ is a chlorine can be further substituted by treatment with alkylboronic acids or esters under Suzuki reaction conditions (path 1), with sodium alkoxides (path 2), or with zinc cyanide (path 3) using conditions previously described to provide compounds of Formula I wherein R⁵ is alkyl, O(alkyl) or CN and R⁷ is as described above.

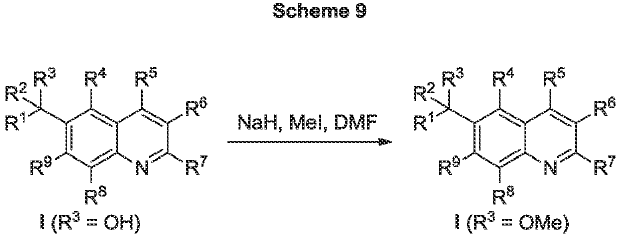

In Scheme 9, tertiary alcohols **I** can be treated with base, such as NaH, and alkylated with MeI in DMF to provide compounds of Formula **I** wherein R³ is OMe.

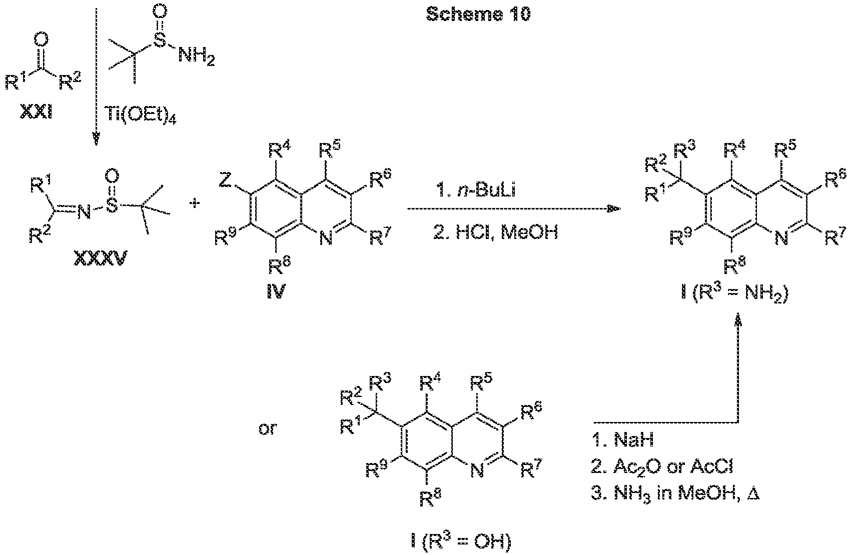

Synthetic routes to compounds of Formula **I,** wherein R³ is NH₂, are illustrated in Scheme 10. Ketimines **XXXV** may be prepared by Ti(OEt)₄ mediated condensation of ketones **XXI** with 2-methylpropane-2-sulfinamide in refluxing THF. Addition of *n*-BuLi to the reaction mixture of ketimines **XXXV** and 6-bromo or 6-iodoquinolines **IV** at - 78 °C followed by cleavage of *tert-*butanesulfinyl group with HCl in MeOH liberates amines **I.** Alternatively, compounds of Formula **I,** wherein R³ is OH, can be treated with sodium hydride followed by addition of acetic anhydride or acetyl chloride and stirred at room temperature over a 24 to 72 hour period to provide the intermediate acetate wherein R³ is OAc. The acetate can then be combined with a solution of ammonia in methanol and heated at temperatures between 60 and 85 °C to provide compounds of Formula **I**, wherein R³ is NH₂.

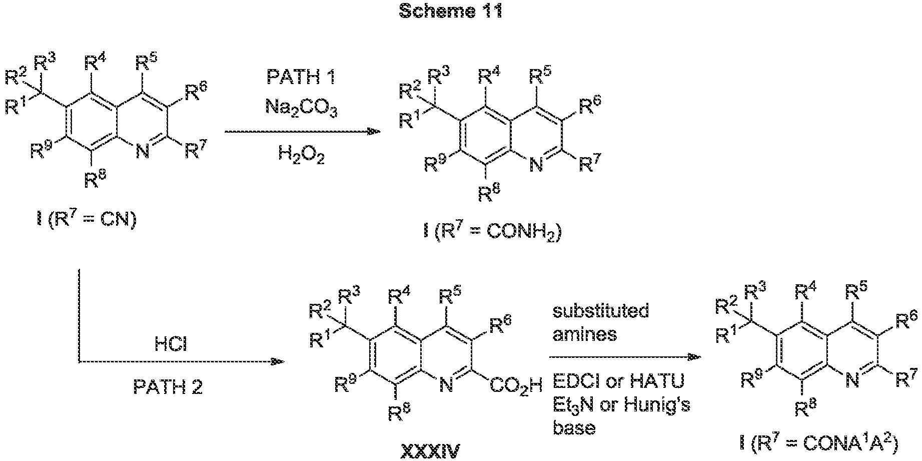

As shown in Scheme 11, the quinolines of Formula **I** wherein R⁷ is CN can be hydrolyzed as described in US20080188521 by treatment with sodium carbonate and hydrogen peroxide to provide compounds of Formula **I** wherein R⁷ is CONH₂ (path 1) or can be treated with a strong acid like HCl to convert CN to a carboxylic acid **XXXIV** (path 2). Once formed the acid can be further coupled to substituted amines using appropriated coupling reagents such as EDCI or HATU in the presence of a base such as triethylamine or Hunig's base to provide compounds of Formula **I** wherein R⁷ is CONA¹A².

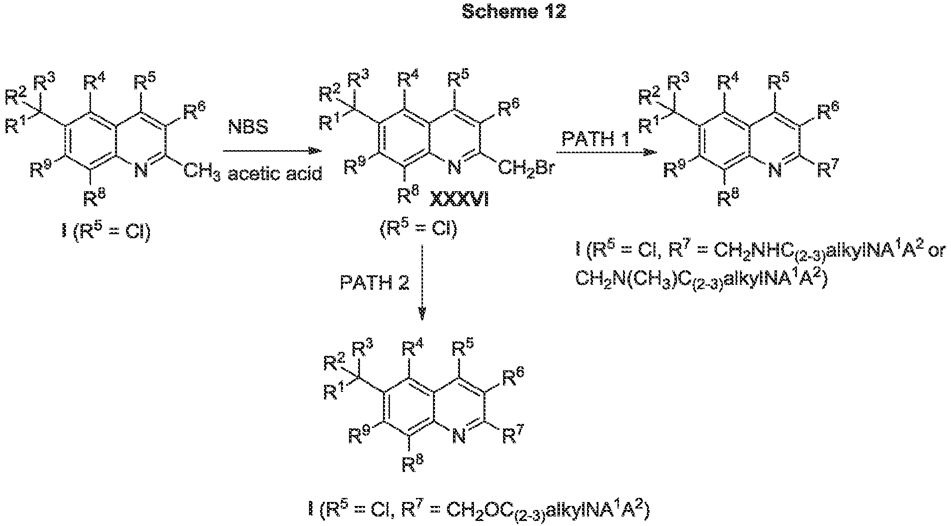

Synthesis of compounds of Formula **I,** wherein R⁷ is an aminoalkylaminomethylene or an aminoalkoxymethylene can be prepared from 2-methylquinolines as shown in Scheme 12. Bromination of 2-methylquinolines of Formula **I** can be accomplished with *N*-bromosuccinimide in acetic acid at elevated temperatures as described in WO2010151740, to provide the methylbromide intermediate **XXXVI.** Nucleophilic displacement of the bromide under basic conditions using procedures known in the art could afford compounds of Formula **I** wherein R⁷ is -CH₂NHC₍₂₋₃₎alkylNA¹A² or CH₂N(CH₃)C₍₂₋₃₎alkylNA¹A² (path 1) or CH₂OC₍₂₋₃₎alkylNA¹A² (path 2) and A¹ and A² are defined above.

Compounds of Formula **I** wherein R¹, R² or R⁶ are pyridyl can be treated with m-chloroperbenzoic acid in a chlorinated solvent at ambient to 40 °C to form the pyridyl-*N*-oxides of Formula **I.**

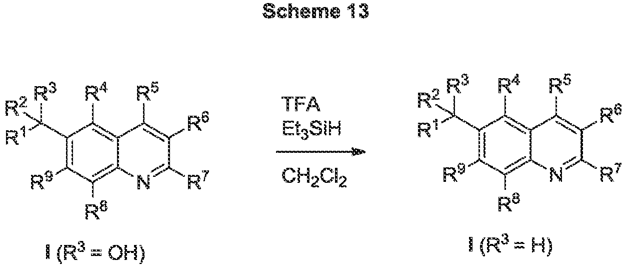

As shown in Scheme 13, compounds of the Formula **I** wherein R³ is H can be prepared by treating compounds of Formula **I** wherein R³ is OH with a hydride source such as triethylsilane and an acid such as trifluoroacetic acid in a solvent such as dichloromethane at room temperature or with heating (WO2009091735).

Scheme 14 outlines alternative synthetic methods to compounds of Formula **I.** Acylation of methyl 2-aminobenzoates **VII** (path 1) or 2-trifluoroketoanilines **XVII** (path 2) with benzyloxyacetyl chloride in the presence of a base such as triethylamine in a solvent such as dichloromethane can afford amides **XLI** and **XLV** respectively. Amides **XLI** and **XLV** can undergo an intramolecular cyclization reaction with a base such as potassium bis(trimethylsilyl)amide in a solvent such as tetrahydrofuran to provide the intermediate 6-haloquinolin-2(1*H*)-ones which can be converted with phosphorus oxychloride, as previously described, to compounds of Formula **IV** wherein R⁵ and R⁷ is Cl (resulting from path 1) and R⁵ is CF₃ and R⁷ is Cl (resulting from path 2). The coupling of 6-haloquinolines **IV** and ketones of Formula **XXI** to introduce R¹ and R² followed by displacement of the 2 or 4-chloro using procedures previously described provides quinolines of Formula **XLII** wherein R¹, R², R⁵ and R⁷ are defined in the detailed description of the invention. Palladium-catalyzed hydrogenation of compounds of Formula **XLII** that are substituted with a benzyloxy at C-3 can provide intermediate quinolin-3-ols **XLIII.** The quinolin-3-ols **XLIII** can be converted into the corresponding triflates **XLIV** with trifluoromethanesulfonic acid in the presence of a base, such as pyridine, in a solvent such as dichloromethane. The triflates **XLIV** can be converted into compounds of Formula **I,** wherein R⁶ is aryl or heteroaryl as defined above, by a palladium-catalyzed cross coupling with organoboron reagents of the formula R⁶B(OR)₂ in the presence of a base, such as potassium carbonate, in a solvent mixture such as 1,4-dioxane/water.

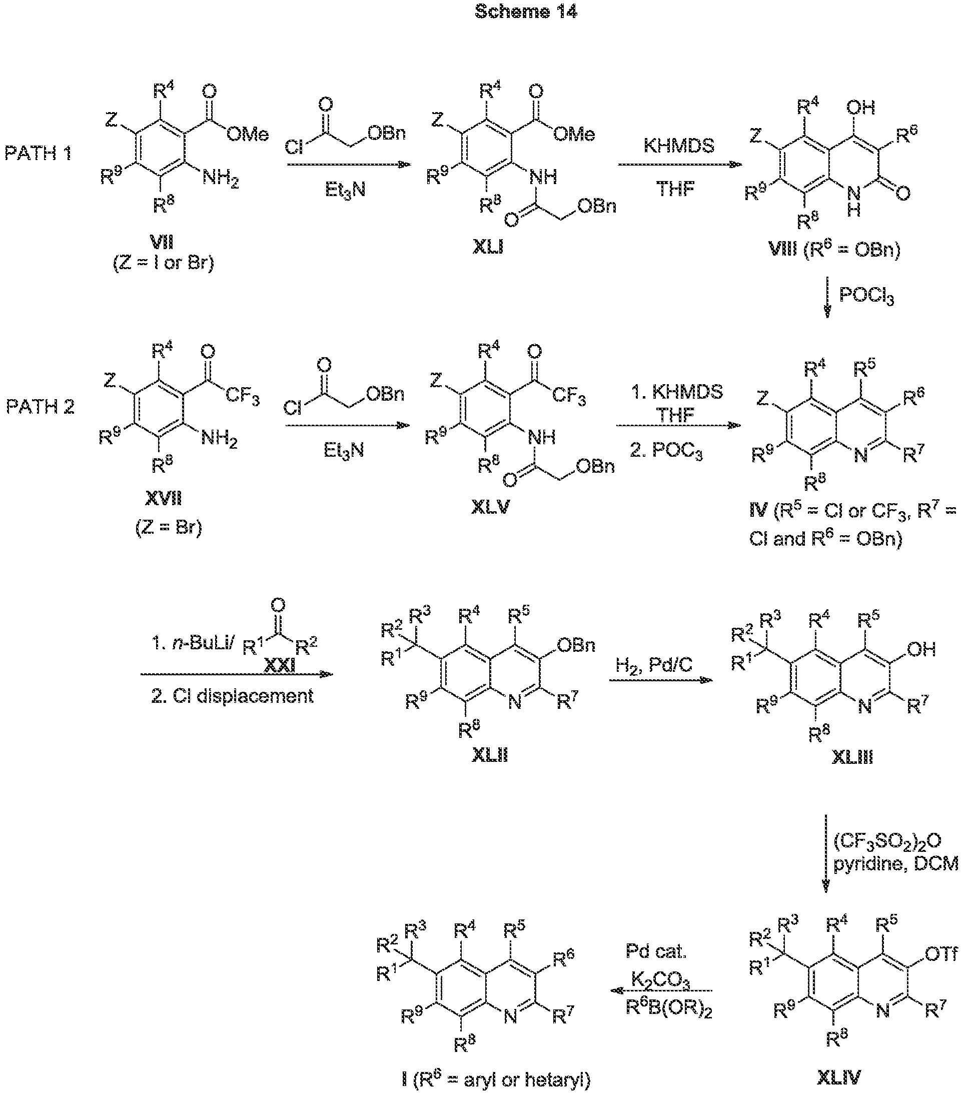

### EXAMPLES

Compounds of the present invention can be prepared by methods known to those who are skilled in the art. The following examples are only meant to represent examples of the invention and are in no way meant to be a limit of the invention.

### Intermediate 1: step a

### tert-Butyl 4-(hydroxy(1-methyl-1H-imidazol-5-yl)methyl)piperidine-1-carboxylate

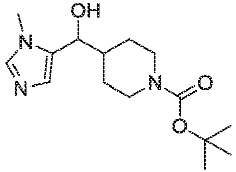

A solution of 5-bromo-1-methyl-1*H*-imidazole (25.0 g, 155 mmol; dried over 3Å molecular sieves, then filtered) in DCM (310 mL) was stirred in an ice bath while *i*PrMgCl (72 mL, 2.01 M solution in THF, 145 mmol) was added rapidly dropwise under argon via pressure-equalizing addition funnel. Residual *i*PrMgCl was rinsed down with 50 mL THF, and the ice bath was removed and the reaction stirred for 25 minutes. A solution of *tert*-butyl 4-formylpiperidine-1-carboxylate (27.6 g, 130 mmol) in THF (65 mL) was added dropwise over ∼5 minutes via pressure-equalizing addition funnel at room temperature. After stirring 1 hour at room temperature, the yellow mixture was quenched with 5 M aqueous NH₄Cl (250 mL) in one portion. The organic layer was dried (Na₂SO₄), filtered, and concentrated to provide the crude title compound as a clear light amber oil.

### Intermediate 1: step b

### tert-Butyl 4-(1-methyl-1H-imidazole-5-carbonyl)piperidine-1-carboxylate

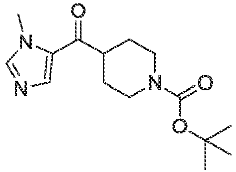

A homogeneous solution of *tert*-butyl 4-(hydroxy(1-methyl-1*H*-imidazol-5-yl)methyl)piperidine-1-carboxylate (32.2 g, 109 mmol, Intermediate 1: step a) in 1,4-dioxane (436 mL) was treated with MnO₂ (47.6 g, 547 mmol) and stirred at 100 °C open to the air overnight (17 hours). Since the reaction was only ∼50% complete by NMR, the reaction was cooled to room temperature and additional MnO₂ was added (48.0 g, 552 mmol) and the reaction stirred open to the air at 100 °C for 6.5 hours, then at room temperature for 18 days, then filtered through a pad of Celite® and the black filter cake washed with EtOAc. The crude filtrate was treated with a third portion of MnO₂ (28.5 g, 327 mmol) and stirred at room temperature overnight. The reaction was then filtered as above and concentrated to provide the crude title compound as a clear dark yellow oil. This oil was purified by FCC with an EtOAc to 50% acetone/EtOAc gradient to provide the title compound as a clear dark yellow oil.

### Intermediate 1: step c

### 1-(4-(1-Methyl-1H-imidazole-5-carbonyl)piperidin-1-yl)ethanone

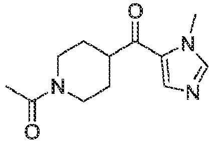

A homogeneous yellow solution of *tert*-butyl 4-(1-methyl-1*H*-imidazole-5-carbonyl)piperidine-1-carboxylate (10.1 g, 34.4 mmol, Intermediate 1: step b) in DCM (172 mL) was treated with TFA (26.4 mL, 344 mmol) and stirred at room temperature for 2.5 hours. The reaction was concentrated from toluene (2 × 100 mL), and the resulting clear light amber residue was taken up in DCM (344 mL) and TEA (23.9 mL, 172 mmol). Acetic anhydride (3.91 mL, 41.3 mmol) was added dropwise and the reaction stirred at room temperature for 1 hour. The reaction was concentrated under high vacuum and the residue was purified by FCC using 95:5 DCM/MeOH with 2% TEA as eluent. The combined fractions were concentrated, dissolved in DCM (200 mL), and washed with water (2 × 200 mL) to remove TEA. The organic layer was dried (Na₂SO₄), filtered, and concentrated. The residue was triturated with MTBE (75 mL) at reflux for 15 minutes and then allowed to cool to room temperature. The mixture was filtered and the off-white filter cake was washed with MTBE (2 × 3 mL) to provide, after air drying at 100 °C, the title compound as an off-white fine powder.

### Intermediate 2: step a

### 6-(Trifluoromethyl)nicotinoyl chloride

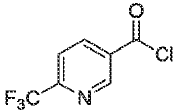

To a 1 L 3-neck flask equipped with an overhead stirrer, Claisen adaptor, nitrogen bubbler, 60 mL addition funnel, and thermocouple was added 6-(trifluoromethyl)nicotinic acid (45 g, 235.5 mmol), dichloromethane (540 mL) and DMF (0.910 mL, 11.77 mmol) via syringe. To this solution was added oxalyl chloride (24.51 mL, 282.56 mmol) and the reaction was allowed to stir at ambient temperature overnight. The reaction was then filtered and the clear filtrate was concentrated *in vacuo* to afford the title compound as a brownish semisolid.

### Intermediate 2: step b

### N-Methoxy-N-methyl-6-(trifluoromethyl)nicotinamide

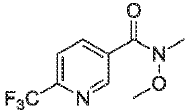

To a 1 L 3-neck flask equipped with an overhead stirrer, Claisen adaptor, nitrogen bubbler, 125 mL addition funnel, and thermocouple was added 6-(trifluoromethyl)nicotinoyl chloride (49.3 g, 235.2 mmol, Intermediate 2: step a), dichloromethane (493 mL), and N,O-dimethylhydroxylamine hydrochloride (25.63 g, 258.8 mmol). After the mixture was cooled to 7 °C, diisopropylethylamine (90.26 mL, 517.6 mmol) was added such that the addition temperature did not exceed 16 °C. After the addition, the reaction was allowed to warm to room temperature. The reaction was then transferred to a separatory funnel and the organic layer was washed with saturated aqueous NaHCO₃ (2 x 100 mL) followed by water (100 mL) and then dried over sodium sulfate, and filtered. The solvent was then removed *in vacuo* to afford the title compound as a brownish oil.

### Intermediate 2: step c

### (1-Methyl-1H-imidazol-5-yl)(6-(trifluoromethyl)pyridin-3-yl)methanone

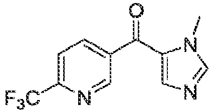

To a 3 L 4-neck flask equipped with an overhead stirrer, nitrogen bubbler, and thermocouple was added 5-bromo-1-methyl-1*H*-imidazole (47.96 g, 297.9 mmol), followed by THF (537 mL). To this room temperature solution was added isopropylmagnesium chloride/lithium chloride complex [1.3 M in THF] (246.8 mL, 320.8 mmol) (addition temperature maintained between 16.6 and 25 °C) to afford a milky suspension and the reaction was stirred for 60 minutes and then cooled to 5.3 °C in an ice bath. To this mixture was added a solution of *N*-methoxy-*N*-methyl-6-(trifluoromethyl)nicotinamide (53.66 g, 229.14 mmol, Intermediate 2: step b) in THF (268.3 mL) (addition temperature between 5.3 and 5.6 °C) to afford an orange mixture. After addition, the reaction was warmed to room temperature over 2 hours. After stirring at room temperature for 18 hours, THF (200 mL) was added and the reaction was stirred for 2 hours. The reaction was then cooled to 4 °C with an ice bath and carefully quenched with 2 N aqueous HCl to pH = 7, quenching temperature reached 12 °C. The mixture was diluted with ethyl acetate (500 mL), phases split and the organic layer was washed with brine (2 x 200 mL), dried over sodium sulfate, filtered, and the solvent was removed. Hot ether was added and suspension was filtered to provide the title compound as a solid.

### Intermediate 3

### 6-Bromo-2,4-dichloro-8-methyl-3-phenylquinoline

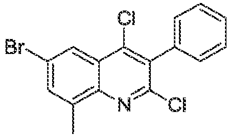

A mixture of 2-phenylmalonic acid (7.62 g, 42.3 mmol) and POCl₃ (32.8 mL, 352 mmol) was stirred at reflux (130 °C) for 10 minutes, and the resulting homogeneous yellow solution was cooled in an ice bath. 4-Bromo-2-methylaniline (6.56 g, 35.2 mmol) was added in one portion and the mixture was refluxed for 2 hours. The dark solution was allowed to cool to room temperature and was diluted with DCM (70 mL) and ice (100 mL), and stirred at room temperature for ∼5-10 minutes at which point exothermic POCl₃ hydrolysis commenced (ice bath cooling), and was then stirred at room temperature for another 30 minutes. The light yellow aqueous layer was extracted with DCM (1 × 30 mL), and the combined dark homogeneous organic layers were dried (Na₂SO₄), filtered, and concentrated with silica gel. The silica-adsorbed residue was dry load flash chromatographed with a 20% DCM/heptane to 100% DCM gradient to provide the title compound as an off-white solid.

### Intermediate 4

### 1-(4-Benzoylpiperidin-1-yl)ethanone

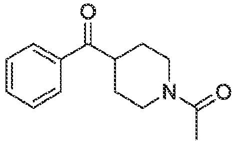

A mixture of phenyl(piperidin-4-yl)methanone hydrochloride (743 mg, 3.29 mmol) in dichloromethane (13.2 mL) and triethylamine (1.10 mL, 7.90 mmol) was treated with Ac₂O (0.373 mL, 3.95 mmol) dropwise over 1 minute in an ice bath under argon, and the resulting translucent mixture was immediately removed from the ice bath and stirred at room temperature overnight. The reaction was then extracted with 1 M aqueous HCl (1 × 8 mL) and 1 M aqueous NaOH (1 × 8 mL), and the organic layer was dried (Na₂SO₄), filtered, and concentrated to provide the title compound as a translucent beige oil that crystallized upon standing.

### Intermediate 5: step a

### 2-(3-(Trifluoromethyl)phenyl)acetyl chloride

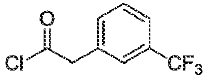

To a solution of 2-(3-(trifluoromethyl)phenyl)acetic acid (4.78 g, 23.4 mmol) in DCM (46 mL) was added oxalyl chloride (12.9 mL, 25.8 mmol). One drop of DMF was then added and the mixture was stirred for 1.5 hours at room temperature. The mixture was concentrated to afford the title compound.

### Intermediate 5: step b

### Methyl 5-bromo-2-(2-(3-(trifluoromethyl)phenyl)acetamido)benzoate

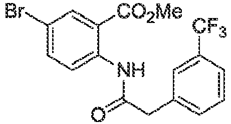

To a solution of 2-(3-(trifluoromethyl)phenyl)acetyl chloride (23.4 mmol assuming quantitative yield in previous step, Intermediate 5: step a) in DCM (50 mL) in an ice bath was added methyl 2-amino-5-bromobenzoate (4.90 g, 21.3 mmol) followed by triethylamine (6.51 mL, 46.8 mmol). The mixture was stirred at room temperature overnight. The reaction mixture was washed with saturated aqueous NH₄Cl then water. The organic phase was dried (Na₂SO₄), filtered, and concentrated and the residue was purified by flash column chromatography (50-70% DCM-heptanes). Some of the title compound was obtained in pure form from the column, along with mixed fractions containing the title compound and the starting aniline. The mixed fractions were left to stand in DCM:heptanes (1:1) overnight, forming crystalline needles of the title compound, which were collected by vacuum filtration and combined with clean fractions from the column.

### Intermediate 5: step c

### 6-Bromo-4-hydroxy-3-(3-(trifluoromethyl)phenyl)quinolin-2(1H)-one

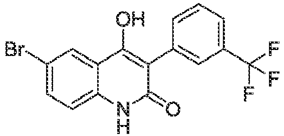

To a solution of methyl 5-bromo-2-(2-(3-(trifluoromethyl)phenyl)acetamido)benzoate (3.86 g, 9.28 mmol, Intermediate 5: step b) in THF (100 mL) at -78 °C was added KHMDS (0.5 M in toluene, 55.6 mL, 27.8 mmol) over 8 minutes. The resulting light yellow solution was stirred at - 78 °C for 5 minutes, then was transferred to an ice bath and stirred for 1 hour 15 minutes. The mixture was diluted with water and extracted once with EtOAc. The aqueous phase was acidified to pH 2 by addition of 1 N aqueous HCl solution. A white precipitate formed upon acidification and was collected by vacuum filtration and air dried to afford the title compound as a white powder.

### Intermediate 5: step d

### 6-Bromo-2,4-dichloro-3-(3-(trifluoromethyl)phenyl)quinoline

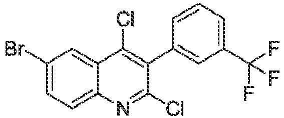

To a suspension of 6-bromo-4-hydroxy-3-(3-(trifluoromethyl)phenyl)quinolin-2(1*H*)-one (3.17 g, 8.25 mmol, Intermediate 5: step c) in acetonitrile (35 mL) was added POCl₃ (2.31 mL, 24.8 mmol). The resulting white suspension was heated at reflux under a drying tube for 2.5 hours. The mixture was allowed to cool to room temperature and stand for 2 hours. Precipitated solid was collected by vacuum filtration to afford the title compound as a white solid.

### Intermediate 5: step e

### 6-Bromo-4-chloro-2-methoxy-3-(3-(trifluoromethyl)phenyl)quinoline

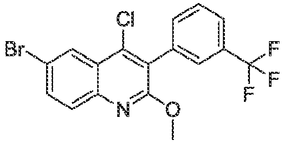

To a suspension of 6-bromo-2,4-dichloro-3-(3-(trifluoromethyl)phenyl)quinoline (1.48 g, 3.52 mmol, Intermediate 5: step d) in toluene (15 mL) in a sealed tube was added sodium methoxide (1.90 g, 35.2 mmol) in one portion. The resulting white suspension was heated in a 100 °C oil bath for 24 hours. An aqueous NaHCO₃ solution (10 wt. %, 27 mL) was added and the mixture was stirred for a few minutes, and the phases were separated. The aqueous phase was extracted twice with EtOAc. The organic extracts were washed with saturated aqueous NaCl. The organic phase was dried (Na₂SO₄), filtered, and concentrated. The crude product was purified by flash column chromatography (silica gel, 15-30% DCM-heptanes) to afford the title compound as a white powder.

### Intermediate 6: step a

### (1-Methyl-1H-1,2,3-triazol-5-yl)(1-methyl-1H-imidazol-5-yl)methanol

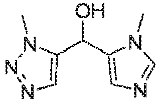

A solution of 1-methyl-1*H*-1,2,3-triazole (1.47 g, 17.7 mmol, PCT Int. Appl. 2008098104) in 20 mL THF was cooled to -40 °C in a dry-ice/acetonitrile bath. *n*-Butyllithium (1.6 M in hexane, 10.2 mL, 16.3 mmol) was added dropwise via syringe and the mixture was stirred at -40 °C for 30 minutes. A solution of 1-methyl-1*H*-imidazole-5-carbaldehyde (1.50 g, 13.6 mmol) in 10 mL THF was then added and the mixture was stirred for 5 minutes, then was transferred to an ice/water bath. After 1 hour, the mixture was quenched by addition of saturated aqueous NH₄Cl, diluted with water, and extracted twice with EtOAc. The aqueous phase, which contained the title compound, was then concentrated. The residue was purified by flash column chromatography (silica gel, gradient 3-10% MeOH-DCM) to afford the title compound as a light yellow foam.

### Intermediate 6: step b

### (1-Methyl-1H-1,2,3-triazol-5-yl)(1-methyl-1H-imidazol-5-yl)methanone

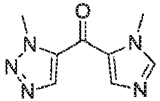

A mixture of (1-methyl-1*H*-1,2,3-triazol-5-yl)(1-methyl-1*H*-imidazol-5-yl)methanol (1.90 g, 9.83 mmol, Intermediate 6: step a) and manganese dioxide (5.04 g, 49.3 mmol) in 1,4-dioxane (100 mL) was stirred in a 100 °C oil bath under argon for 2 hours. The mixture was allowed to cool to room temperature, then was filtered through a pad of Celite®, rinsing with DCM. The filtrate was concentrated, yielding the title compound as a brown powder.

### Intermediate 7: step a

### Methyl 5-bromo-2-(2-phenylacetamido)benzoate

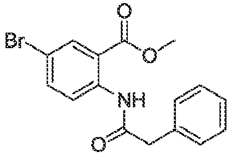

To a mixture of methyl 2-amino-5-bromobenzoate (9.00 g, 39.1 mmol) and Et₃N (7.6 mL, 54.8 mmol) in CH₂Cl₂ (90 mL) at 4 °C was added 2-phenylacetyl chloride (7.26 g, 46.9 mmol) dropwise. After completion of the addition, the cooling bath was removed and the mixture was stirred for 27 hours. TLC showed that some of the starting material methyl 2-amino-5-bromobenzoate still remained. More 2-phenylacetyl chloride (1.88 g, 12.2 mmol) and Et₃N (2.2 mL, 15.9 mmol) were added, and the mixture was stirred overnight. K₂CO₃ (aqueous) was added, the organic layer was separated, and the aqueous layer was extracted with CH₂Cl₂. The combined organic layers were washed with water, dried (Na₂SO₄), filtered, and concentrated *in vacuo.* CH₃CN (100 mL) was added, and the precipitated solid was filtered, washed with Et₂O, and dried to afford the title compound. The filtrate was concentrated *in vacuo,* and the solid was filtered, washed with Et₂O, and dried to provide additional title compound.

### Intermediate 7: step b

### 6-Bromo-4-hydroxy-3-phenylquinolin-2(1H)-one

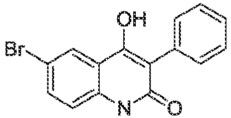

To a solution of methyl 5-bromo-2-(2-phenylacetamido)benzoate (7.71 g, 22.1 mmol, Intermediate 7: step a) in THF (50 mL) at -78 °C was added 1.0 M lithium bis(trimethylsilyl)amide in hexane (48.7 mL, 48.7 mmol) slowly, and the color changed from colorless to clear red. The mixture, at -78 °C, was warmed to room temperature over 4 hours, during which time the color changed to cloudy yellow. The reaction was quenched with water, and acidified with 37% HCl until pH ∼ 5. The precipitated solid was filtered, washed with water and Et₂O, and air dried to provide the title compound. Another crop precipitated from the filtrate after standing overnight. The solid was collected by filtration, washing with water and Et₂O, and air drying to afford additional title compound.

### Intermediate 7: step c

### 6-Bromo-2,4-dichloro-3-phenylquinoline

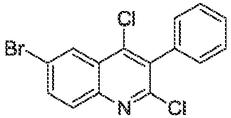

A solution of 6-bromo-4-hydroxy-3-phenylquinolin-2(1*H*)-one (8.50 g, 26.9 mmol, Intermediate 7: step b) in phosphoryl trichloride (51 mL, 547 mmol) was heated at 107 °C for 3.5 hours, and then cooled to room temperature. After evaporation of POCl₃ *in vacuo,* concentrated NH₄OH (aqueous) was added dropwise at 4 °C until pH 9. The precipitated solid was filtered, washed with water, and dried at 50 °C under vacuum overnight to provide the title compound.

### Intermediate 8: step a

### 4-Chloro-N-methoxy-N-methylbenzamide

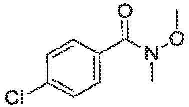

Pyridine (27.6 mL, 343 mmol) was added to *N,O*-dimethylhydroxylamine hydrochloride (16.7 g, 172 mmol) in DCM (400 mL). 4-Chlorobenzoyl chloride (20 mL, 156 mmol) was then added and the mixture was stirred at room temperature for 3 days. Solids were removed by vacuum filtration, washing with DCM. The filtrate was washed with 1 N aqueous HCl followed by water. The organic phase was dried (Na₂SO₄), filtered, and concentrated, affording the crude title compound as a colorless liquid which was used without purification.

### Intermediate 8: step b

### (4-Chlorophenyl)(1-methyl-1H-imidazol-5-yl)methanone

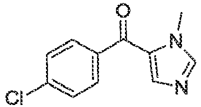

Ethyl magnesium bromide (3.0 M in diethyl ether, 21.5 mL, 64.4 mmol) was added via syringe over a few minutes to a clear colorless solution of 5-bromo-1-methyl-1*H-*imidazole (10.4 g, 64.4 mmol) in THF (100 mL) under a nitrogen atmosphere in an ice bath. A white precipitate formed during the addition. The mixture was removed from the ice bath and was stirred for 20 minutes, then was again cooled in an ice bath before addition of 4-chloro-*N*-methoxy-*N*-methylbenzamide (10.7 g, 53.6 mmol, Intermediate 8: step a). The resulting white suspension was stirred overnight at room temperature. The reaction was quenched by addition of saturated aqueous NH₄Cl and diluted with water. The mixture was partially concentrated to remove THF and was diluted with DCM. The mixture was acidified to pH 1 with 1 N aqueous HCl, then neutralized with saturated aqueous NaHCO₃. The phases were separated and the aqueous phase was further extracted with DCM. The organic extracts were washed with water, then were dried (Na₂SO₄), filtered, and concentrated, affording a white solid. The crude product was triturated with a mixture of EtOAc:heptanes (1:1, 150 mL). The precipitated solid was collected by vacuum filtration, washing with heptanes, to afford the title compound.

### Intermediate 9: step a

### Methyl 5-bromo-2-(2-(pyridin-2-yl)acetamido)benzoate

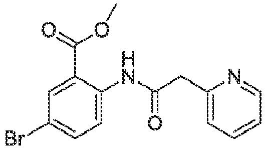

Into a 250-mL round-bottom flask was placed a solution of methyl 2-amino-5-bromobenzoate (5 g, 21.73 mmol), 2-(pyridin-2-yl)acetic acid hydrochloride (4.5 g, 25.92 mmol), HATU (10 g, 26.30 mmol) and DIEA (8.5 g, 65.77 mmol) in *N,N-*dimethylformamide (100 mL). The resulting solution was stirred overnight at 20 °C. The reaction was then quenched by the addition of 100 mL of water. The resulting solution was extracted with 3x100 mL of dichloromethane and the organic layers combined and dried over anhydrous sodium sulfate, filtered and concentrated under vacuum. The residue was applied onto a silica gel column with dichloromethane/methanol (1:50) to afford the title compound as a red solid.

### Intermediate 9: step b

### 6-Bromo-3-(pyridin-2-yl)quinoline-2,4(1H, 3H)-dione

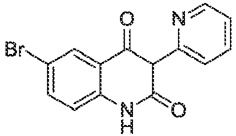

Into a 250-mL round-bottom flask was placed a solution of methyl 5-bromo-2-[2-(pyridin-2-yl)acetamido]benzoate (3 g, 7.73 mmol, Intermediate 9: step a) and MeONa (11.7 mL, 31 mmol, 2.64 M in MeOH) in tetrahydrofuran (30 mL). The resulting solution was stirred overnight at 20 °C. Then, the solvents were removed by a rotary evaporator and 50 mL water was added to the residue. The pH of the resulting mixture was adjusted to 7 with 1 M aqueous HC1. The solid was collected by filtration and dried under vacuum to afford the title compound as a white solid which was used in the next step without further purification.

### Intermediate 9: step c

### 6-Bromo-2,4-dichloro-3-(pyridin-2-yl)quinoline

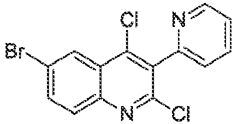

Into a 100-mL round-bottom flask was placed a solution of 6-bromo-3-(pyridin-2-yl)quinoline-2,4(1*H*, 3*H*)-dione (2.54 g, 7.21 mmol, Intermediate 9: step b) in POCl₃ (50 mL). The resulting solution was stirred for 3 hours at 120 °C. The reaction was then quenched by the addition of 50 mL of water. The resulting solution was extracted with 3x50 mL of ethyl acetate and the organic layers combined and dried over anhydrous sodium sulfate, filtered and concentrated to afford the title compound as a white solid.

### Intermediate 10: step a

### Methyl 5-bromo-2-(2-(pyridin-3-yl)acetamido)benzoate

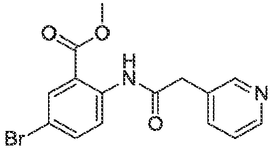

Into a 250-mL round-bottom flask was placed a solution of methyl 2-amino-5-bromobenzoate (5 g, 21.73 mmol, 100%), 2-(pyridin-3-yl)acetic acid hydrochloride (4.5 g, 25.92 mmol), HATU (10 g, 26.30 mmol 100%) and DIEA (8.5 g, 65.77 mmol, 100%) in *N,N*-dimethylformamide (100 mL). The resulting solution was stirred overnight at 20 °C. The reaction was then quenched by the addition of 100 mL of water. The resulting solution was extracted with 3x100 mL of dichloromethane and the organic layers combined and dried over anhydrous sodium sulfate and concentrated under vacuum. The residue was applied onto a silica gel column with ethyl acetate/petroleum ether (1:2-1:1) to provide the title compound as a yellow solid.

### Intermediate 10: step b

### 6-Bromo-3-(pyridin-3-yl)quinoline-2,4(1H, 3H)-dione

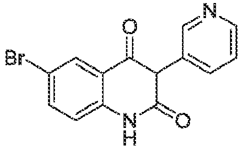

Into a 100-mL round-bottom flask, was placed a solution of methyl 5-bromo-2-[2-(pyridin-3-yl)acetamido]benzoate (3 g, 7.73 mmol, Intermediate 10: step a) and MeONa (11.7 mL, 31 mmol, 2.64 M in MeOH) in tetrahydrofuran (30 mL). The resulting solution was stirred overnight at 20 °C. The solids were collected by filtration and washed with water (3 x 5 mL). The solids were dried under vacuum to provide the title compound as a white solid.

### Intermediate 10: step c

### 6-Bromo-2,4-dichloro-3-(pyridin-3-yl)quinoline

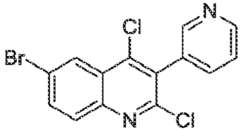

Into a 100-mL round-bottom flask, was placed a solution of 6-bromo-3-(pyridin-3-yl)quinoline-2,4(1*H*, 3*H*)-dione (700 mg, 1.99 mmol, Intermediate 10: step b) in phosphorus trichloride (20 mL). The resulting solution was stirred for 3 hours at 120 °C. The resulting mixture was concentrated under vacuum. The reaction was then quenched by the addition of 20 mL of water. The resulting solution was extracted with 3x20 mL of ethyl acetate and the organic layers combined and dried over anhydrous sodium sulfate, filtered and concentrated to afford the crude title compound as a white solid.

### Intermediate 11: step a

### Methyl 2-(2-(benzyloxy)acetamido)-5-bromobenzoate

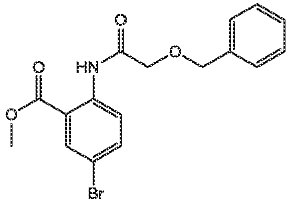

To a solution of methyl-2-amino-5-bromobenzoate (15 g, 62.6 mmol) in DCM (241 mL) at 0 °C was added benzyloxyacetyl chloride (12.5 mL, 75.1 mmol) followed by Et₃N (20 mL, 144 mmol) dropwise. The resulting white suspension was stirred at room temperature for 3 hours. The mixture was then washed with saturated aqueous NH₄Cl (200 mL) followed by water (200 mL). The organics were dried (Na₂SO₄), filtered and concentrated to dryness. The crude solid was triturated with MeOH (90 mL) and dried under vacuum to afford the title compound as a white solid.

### Intermediate 11: step b

### 3-(Benzyloxy)-6-bromo-4-hydroxyquinolin-2(1H)-one

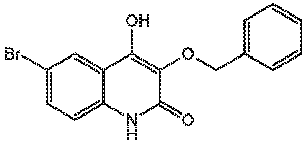

To a solution of methyl 2-(2-(benzyloxy)acetamido)-5-bromobenzoate (15 g, 39.7 mmol, Intermediate 11: step a) in THF (198 mL) was added KHMDS (1 M in THF, 119 mL, 119 mmol). The resulting solution was stirred at room temperature for 40 minutes and then additional KHMDS (19.8 mL, 19.8 mmol) was added and stirring continued at room temperature for 2 hours. The mixture was quenched with water (225 mL) and the layers were separated. The aqueous layer was acidified with 1 N aqueous HCl to pH 2-3. Some of the title compound precipitated out of solution and was collected by filtration. The aqueous was then extracted with EtOAc (3 x 200 mL). The organics were combined with the solid collected previously and sonicated. The solution was dried (Na₂SO₄), filtered and concentrated to dryness to provide the title compound as a yellow solid.

### Intermediate 11: step c

### 3-(Benzyloxy)-6-bromo-2,4-dichloroquinoline

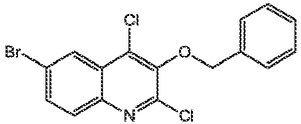

To a suspension of 3-(benzyloxy)-6-bromo-4-hydroxyquinolin-2(1*H*)-one (12.4 g, 35.8 mmol, Intermediate 11: step b) in acetonitrile (119 mL) was added POCl₃ (10 mL, 107.5 mmol) followed by 2,6-lutidine (6.26 mL, 53.7 mmol) dropwise. The suspension was heated to 100 °C for 4 hours, then the reaction was allowed to cool to room temperature. The solids were filtered, rinsed with MeOH and dried under vacuum to afford the title compound as a tan solid.

### Intermediate 11: step d

### (3-(Benzyloxy)-2,4-dichloroquinolin-6-yl)(1-methyl-1H-imidazol-5-yl)(6-(trifluoromethyl)pyridin-3-yl)methanol

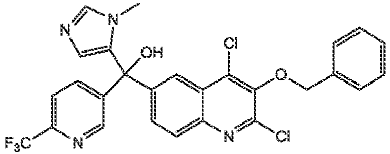

A solution of 3-(benzyloxy)-6-bromo-2,4-dichloroquinoline (2.57 g, 6.71 mmol, Intermediate 11: step c) in THF (100 mL) was cooled to -78 °C, during which it became a white suspension. Then, *n*BuLi (1.6 M in hexanes, 5.87 mL, 9.39 mmol) was added dropwise and the resulting dark red solution was stirred for 10 minutes at -78 °C. To this mixture was added a solution of (1-methyl-1*H*-imidazol-5-yl)(6-(trifluoromethyl)pyridin-3-yl)methanone (2.23 g, 8.72 mmol, Intermediate 2: step c) in THF (30 mL) over 4 minutes and the resulting mixture stirred at -78 °C for 2 minutes. The dry-ice/acetone bath was then replaced with an ice bath and the mixture was stirred for an additional 45 minutes. The reaction was then quenched with water and extracted with EtOAc. The organics were combined, dried (MgSO₄), filtered and concentrated to dryness to afford the crude product which was purified by FCC (4% MeOH/DCM) to provide the title compound.

### Intermediate 11: step e

### (3-(Benzyloxy)-4-chloro-2-methoxyquinolin-6-yl)(1-methyl-1H-imidazol-5-yl)(6-(trifluoromethyl)pyridin-3-yl)methanol

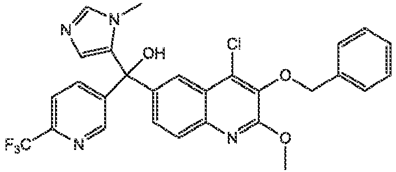

To a mixture of (3-(benzyloxy)-2,4-dichloroquinolin-6-yl)(1-methyl-1*H*-imidazol-5-yl)(6-(trifluoromethyl)pyridin-3-yl)methanol (2.46 g, 4.4 mmol, Intermediate 11: step d) in methanol (24.6 mL) was added NaOMe (0.5 M in MeOH, 8.8 mL, 4.4 mmol) and the resulting suspension heated to 65 °C for 8 hours. The mixture was then cooled to room temperature and concentrated to dryness. Water was added and the mixture acidified with 2 N aqueous HCl to ∼pH 2. The aqueous was then extracted with EtOAc. The organics were combined and washed with water, saturated aqueous NaHCO₃ and brine. The organics were then dried (MgSO₄), filtered and concentrated to dryness to afford the title compound which was used without further purification.

### Intermediate 11: step f

### 4-Chloro-6-(hydroxy(1-methyl-1H-imidazol-5-yl)(6-(trifluoromethyl)pyridin-3-yl)methyl)-2-methoxyquinolin-3-ol

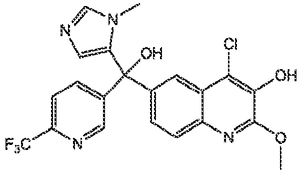

To a solution of (3-(benzyloxy)-4-chloro-2-methoxyquinolin-6-yl)(1-methyl-1*H*-imidazol-5-yl)(6-(trifluoromethyl)pyridin-3-yl)methanol (2.56 g, 4.61 mmol, Intermediate 11: step e) in MeOH (97 mL) was added 10% Pd/C (246 mg, 0.23 mmol). The reaction vessel was evacuated and then placed under an atmosphere of hydrogen for 1.5 hours. The mixture was then flushed with N₂ and filtered through a pad of Celite®). The filtrate was concentrated to dryness, then DCM was added and the solution concentrated to dryness. The resulting solid was dried in the oven. The solid was then purified by FCC (15% MeOH/DCM) to provide the title compound.

### Intermediate 11: step g

### 4-Chloro-6-(hydroxy(1-methyl-1H-imidazol-5-yl)(6-(trifluoromethyl)pyridin-3-yl)methyl)-2-methoxyquinolin-3-yl trifluoromethanesulfonate

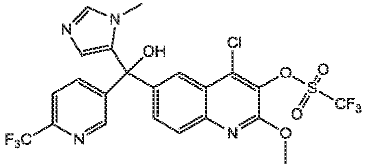

To a suspension of 4-chloro-6-(hydroxy(1-methyl-1*H*-imidazol-5-yl)(6-(trifluoromethyl)pyridin-3-yl)methyl)-2-methoxyquinolin-3-ol (750 mg, 1.61 mmol, Intermediate 11: step f) in CH₂Cl₂ (15 mL) was added pyridine (390 µL, 4.84 mmol) and the reaction became a solution. The solution was cooled to 0 °C and trifluoromethanesulfonic anhydride (683 mg, 2.42 mmol) was added dropwise. The reaction was stirred at 0 °C for 1 hour, then the ice bath was removed and stirring continued for an additional hour. Trifluoromethanesulfonic anhydride (683 mg, 2.42 mmol) was then added and the mixture stirred at room temperature for 1 hour. The solution was poured into a mixture of 1 N aqueous HCl (20 mL) and ice and then the aqueous was extracted with CH₂Cl₂. The organics were washed with water followed by saturated aqueous NaHCO₃ and brine. The aqueous layers were combined and back-extracted with EtOAc. The EtOAc layers were combined and washed with water followed by saturated aqueous NaHCO₃ and brine. The CH₂Cl₂ and EtOAc layers were combined, dried (MgSO₄), filtered and concentrated to dryness. The crude material was purified by FCC (0-5% MeOH/CH₂Cl₂) to provide the title compound.

### Intermediate 12: step a

### (1-Methyl-1H-imidazol-5-yl)(pyridin-2-yl)methanol

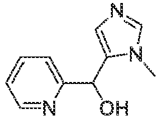

A solution of isopropylmagnesium chloride/lithium chloride complex (1.3 M in THF, 19.5 mL, 25.35 mmol) was added dropwise by syringe to a solution of 5-bromo-1-methyl-1*H*-imidazole (4.12 g, 25.58 mmol) in dry THF (130 mL) at 0 °C. After 15 minutes, the Grignard solution was added via cannulation to a solution of picolinaldehyde (2.0 mL, 20.93 mmol) in dry THF (55 mL) at 0 °C. The reaction mixture was stirred for 5 minutes at 0 °C, then warmed to room temperature for 1 hour. The reaction mixture was then cooled in an ice bath and quenched with saturated aqueous ammonium chloride. The mixture was partitioned between brine and ethyl acetate. The separated aqueous phase was further extracted with ethyl acetate. The organic phase was dried (Na₂SO₄), filtered, and concentrated. The crude product was purified by flash column chromatography (silica gel, 0-5% MeOH-DCM) to provide the title compound as a white solid.

### Intermediate 12: step b

### (1-Methyl-1H-imidazol-5-yl)(pyridin-2-yl)methanone

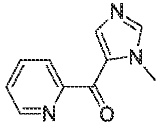

A heterogenous mixture of (1-methyl-1*H*-imidazol-5-yl)(pyridin-2-yl)methanol (1.41 g, 7.45 mmol, Intermediate 12: step a) and manganese dioxide (3.24 g, 37.27 mmol) in 1,4-dioxane (52 mL) was stirred at 100 °C for 2 hours. The reaction mixture was then cooled to room temperature, filtered through Celite®, washed with DCM, and concentrated to provide the title compound as an off-white solid.

### Intermediate 13: step a

### Diethyl 2-(4-(trifluoromethoxy)phenyl)malonate

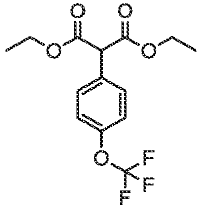

CuI (0.26 g, 1.378 mmol), 2-picolinic acid (0.24 g, 1.969 mmol) and cesium carbonate (19.24 g, 59.061 mmol) were combined in a reaction vessel and the flask was evacuated and re-filled with argon (3 times). 1,4-Dioxane was then added followed by diethylmalonate (6 mL, 39.374 mmol) and 1-iodo-4-(trifluoromethoxy)benzene (3 mL, 19.687 mmol). The resulting yellow suspension was stirred at room temperature for 48 hours and quenched with saturated NH₄Cl. The mixture was extracted with EtOAc (2x). The combined organic extracts were dried over Na₂SO₄, filtered, and concentrated to provide the title compound.

### Intermediate 13: step b

### 2-(4-(Trifluoromethoxy)phenyl)malonic acid

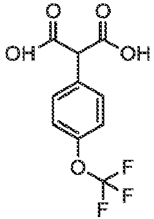

A mixture of diethyl 2-(4-(trifluoromethoxy)phenyl)malonate (5.6 g, 17.486 mmol, Intermediate 13: step a) and an aqueous 3 M NaOH solution was stirred in a 100 °C oil bath for 1 hour, cooled to room temperature, poured into ice water and acidified with 6 N aqueous HCl. The aqueous mixture was extracted with EtOAc. The EtOAc extract was dried over Na₂SO₄, filtered, and evaporated *in vacuo* to provide the title compound.

### Intermediate 13: step c

### 6-Bromo-2,4-dichloro-8-methyl-3-(4-(trifluoromethoxy)phenyl)quinoline

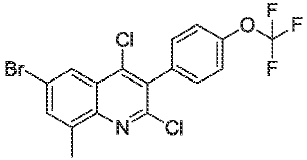

A mixture of 2-(4-(trifluoromethoxy)phenyl)malonic acid (3.1 g, 11.74 mmol, Intermediate 13: step b), 4-bromo-2-methylaniline (2.18 g, 11.74 mmol) and POCl₃ (10 mL) was heated at 105 °C for 3 hours, cooled to room temperature, concentrated under reduced pressure then slowly poured into ice water. A NH₄OH solution was added to a basic pH (pH 8 - 9). The precipitates were collected by filtration, rinsed with H₂O and dried under high vacuum. The resulting tan solids were dissolved in DCM and chromatographed (heptane/DCM) to provide the title compound.

### Intermediate 13: step d

### 6-Bromo-4-chloro-2-methoxy-8-methyl-3-(4-(trifluoromethoxy)phenyl)quinoline

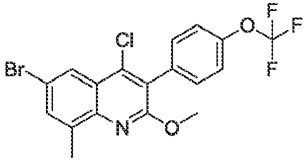

A mixture of 6-bromo-2,4-dichloro-8-methyl-3-(4-(trifluoromethoxy)phenyl)quinoline (1.97 g, 4.37 mmol, Intermediate 13: step c) and sodium methoxide (1.18 g, 21.84 mmol) in toluene (20 mL) was heated in a sealed tube at 110 °C for 24 hours, cooled to room temperature, diluted with DCM, stirred at room temperature for 30 minutes and filtered through Celite® rinsing several times with DCM. The solvents were removed under reduced pressure and the off-white solid product precipitated from MeOH, filtered and dried to provide the title compound.

### Intermediate 14: step a

### tert-Botyl 4-(hydroxy(6-(trifluoromethyl)pyridin-3-yl)methyl)piperidine-1-carboxylate

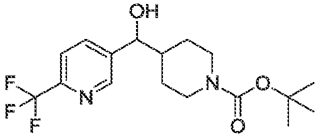

A solution of isopropylmagnesium chloride (2.0 M in THF, 40.3 mL, 80.6 mmol) was added dropwise by syringe to a solution of 5-bromo-2-(trifluoromethyl)pyridine (19.5 g, 86.3 mmol) in dry THF (12 mL) at 2 °C. After 30 minutes, *tert*-butyl 4-formylpiperidine-1-carboxylate (12.3 g, 57.3 mmol) was added to the Grignard solution at 2 °C as a solid. The reaction mixture was warmed to 10 °C over 1.5 hours after which it was quenched with saturated aqueous ammonium chloride solution. The mixture was partitioned between water and ethyl acetate. The separated aqueous phase was further extracted with ethyl acetate and washed with saturated aqueous NaCl solution. The organic phase was dried (MgSO₄), filtered, and concentrated and used crude in the next step.

### Intermediate 14: step b

### tert-Botyl 4-(6-(trifluoromethyl)nicotinoyl)piperidine-1-carboxylate

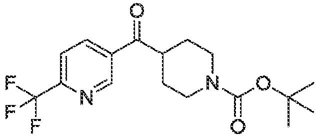

Dess-Martin periodinane reagent (30.0 g, 70.8 mmol) was added to a solution of *tert*-butyl 4-(hydroxy(6-(trifluoromethyl)pyridin-3-yl)methyl)piperidine-1-carboxylate (Intermediate 14: step a, 17.8 g, 49.5 mmol) in DCM at room temperature and the mixture was stirred for 2 hours. The reaction mixture was diluted with DCM and washed with saturated aqueous solution of NaHCO₃. The organic phase was dried (MgSO₄), filtered, and concentrated. The crude product was purified by flash column chromatography (silica gel, 0-60% EtOAc-hexanes) to provide the title compound that was 90% pure by NMR and was carried on to the next step.

### Intermediate 14: step c

### Piperidin-4-yl(6-(trifluoromethyl)pyridin-3-yl)methanone

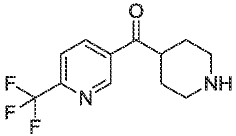

TFA (34.4 mL, 449.3 mmol) was added to a solution of *tert*-butyl 4-(6-(trifluoromethyl)nicotinoyl)piperidino-1-carboxylate (Intermediate 14: step b, 16.1 g, 44.9 mmol) in DCM (450 mL) and the resulting solution was stirred at room temperature for 3 hours. The mixture was concentrated to remove most of the TFA on the rotary evaporator and a mixture of EtOAc/hexanes was added. The white solid that precipitated was filtered and dried and used in the next step.

### Intermediate 14: step d

### 1-(4-(6-(Trifluoromethyl)nicotinoyl)piperidin-1-yl)ethanone

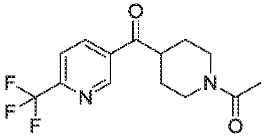

TEA (32.1 mL, 230.9 mmol) was added to a solution of piperidin-4-yl(6-(trifluoromethyl)pyridin-3-yl)methanone (Intermediate 14: step c, 14.3 g, 38.5 mmol) in DCM (427 mL) followed by acetic anhydride (5.28 mL, 55.8 mmol). The mixture was stirred for 2 hours and transferred to a separatory funnel and washed with 100 mL of aqueous 2 M NaH₂PO₄ solution. The organic layer was dried (MgSO₄), filtered and concentrated. The crude product was purified by flash column chromatography (silica gel, 0-3% MeOH-DCM) to provide the title compound.

### Intermediate 15: step a

### N-Methoxy-N-methylpyrimidine-2-carboxamide

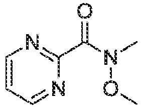

Sodium pyrimidine-2-carboxylate (4.00 g, 27.4 mmol), imidazole hydrochloride (3.15 g, 30.1 mmol), and 1-carbonyldiimidazole (5.26 g, 31.5 mmol) were slurried in acetonitrile (30 mL) at room temperature under an N₂ atmosphere. The mixture was then warmed to 52 °C over 30 minutes. Evolution of carbon dioxide was seen when reaction mixture reached approximately 50 °C. The mixture was then stirred at 52 °C for approximately 2 hours. The reaction was then cooled to room temperature, then *N,O*-dimethylhydroxylamine hydrochloride (3.54 g, 35.6 mmol) was then added slowly, portion wise over approximately 15 minutes and a mild exotherm was seen after each addition. The contents were stirred at room temperature overnight. To the reaction mixture was then added deionized water (25 mL) and dichloromethane (25 mL). 6 M Aqueous hydrochloric acid was added dropwise to acidify the aqueous layer to approximately pH 1. The organic phase was then separated and the aqueous phase was extracted twice with dichloromethane. The combined organics were washed with 2 M aqueous hydrochloric acid, separated, then the acidic layer was extracted twice with dichloromethane. The combined organic phases were washed with a saturated, aqueous NaHCO₃ solution, then dried over MgSO₄, filtered and the solvent was removed by distillation under reduced pressure to provide the title compound.

### Intermediate 15: step b

### (1-Methyl-1H-imidazol-5-yl)(pyrimidin-2-yl)methanone

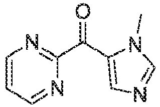

5-Bromo-1-methyl-1*H-*imidazole (6.66 g, 41.4 mmol) was added to a round bottom flask followed by tetrahydrofuran (150 mL) under an N₂ atmosphere. The contents were cooled to 0 °C in an ice water bath. EtMgBr (3.0 M solution in THF, 13.3 mL, 39.8 mmol) was added slowly via syringe over approximately 5 minutes, then the ice bath was removed and contents allowed to warm and stirred at room temperature for approximately 30 minutes. The vessel was then re-cooled to 0 °C and a solution of *N*-methoxy-*N*-methylpyrimidine-2-carboxamide (3.09 g, 15.9 mmol, Intermediate 15: step a) in THF (20 mL) was cannulated into the reaction vessel. The contents were allowed to stir at 0 °C, then slowly warmed to room temperature, then heated to 40 °C in an oil bath and heated with stirring at that temperature for approximately 36 hours. The contents were then cooled to 0 °C, quenched with a saturated aqueous NH₄Cl solution, diluted with ethyl acetate and transferred to a separatory funnel. The aqueous layer was separated, extracted twice with EtOAc, then the combined organic phases were dried over MgSO₄, filtered, then distilled under reduced pressure to afford an amber oil. The crude product was purified by flash column chromatography (silica gel, 0-10% DCM / (10% of a 2 M NH₃ MeOH in DCM)) to provide the title compound.

### Intermediate 16: step a

### (2,4-Dimethylthiazol-5-yl)(1-methyl-1H-1,2,3-triazol-5-yl)methanol

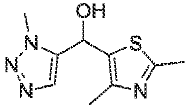

1-Methyl-1*H*-1,2,3-triazole was prepared according to the literature reference WO2008/98104. To a 2 L flask containing 1-methyl-1*H-*1,2,3-triazole (9 g, 108.3 mmol) was added THF (1500 mL) and the solution was cooled to -40 °C. To this colorless homogeneous solution was added n-butyllithium (2.5 M in hexanes, 45 mL, 112.5 mmol) dropwise which immediately afforded a dark brown viscous mixture. The mixture was kept between -10 to -20 °C for 60 minutes, then a THF solution of 2,4-dimethylthiazole-5-carbaldehyde (17.2 g, 121.8 mmol in 200 mL THF) was introduced via cannula. Once the aldehyde was added the reaction was allowed to warm to room temperature. After 3 hours, the reaction was quenched by pouring it into a saturated solution of aqueous NH₄Cl. The aqueous portion was extracted with EtOAc in portions, 7 x 400 mL. The combined organics were washed with brine, dried over MgSO₄, filtered and concentrated to afford a brown oil. Chromatography on silica gel (10% acetone-DCM increasing to 50% acetone and increasing to 10% MeOH-DCM) provided the title compound as an amber solid.

### Intermediate 16: step b

### (2,4-Dimethylthiazol-5-yl)(1-methyl-1H-1,2,3-triazol-5-yl)methanone

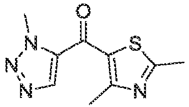

To a 500 mL flask containing (2,4-dimethylthiazol-5-yl)(1-methyl-1*H*-1,2,3-triazol-5-yl)methanol (10.5 g, 46.8 mmol, Intermediate 16: step a) was added 1,4-dioxane (400 mL) and the contents were warmed to form a homogeneous solution. Activated MnO₂ (18 g, 207 mmol) was added and the dark brownish mixture was heated to reflux in an aluminum heating mantle under an atmosphere of N₂. After 1.5 hours, the contents were filtered while still hot through Celite® and rinsed with warm THF. The resulting light orange solution was concentrated and passed through a silica gel column (25% acetone-DCM) to provide the title compound as a light orange solid.

### Intermediate 17: step a

### N-Methoxy-N,2,4-trimethylthiazole-5-carboxamide

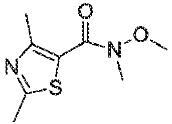

To a flask containing 2,4-dimethylthiazole-5-carboxylic acid (2.5 g, 15.9 mmol) was added DCM (75 mL) to give a suspension. DMF (3 mL) was added which resulted in a homogeneous solution. Then, carbonyldiimidazole (2.84 g, 17.5 mmol) was introduced and the mixture was stirred at room temperature for 2 hours. *N,O*-dimethylhydroxylamine HCl (1.9 g, 19.9 mmol) was then added and the mixture was stirred at room temperature for 18 hours, at which time the reaction mixture was diluted with water and 1 N aqueous NaOH and the aqueous portion was extracted with DCM (4 x 50 mL). The combined organics were washed with brine, dried over Na₂SO₄, filtered and concentrated. Chromatography on silica gel (10% EtOAc-DCM increasing to 40% EtOAc) provided the title compound as a colorless oil.

### Intermediate 17: step b

### (2,4-Dimethylthiazol-5-yl)(1-methyl-1H-imidazol-5-yl)methanone

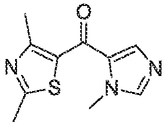

To a flask containing 5-bromo-1-methyl-1*H*-imidazole (390 mg, 2.42 mmol) was added THF (8 mL) and the solution was cooled to 0 °C. To this solution was added isopropylmagnesium chloride-LiCl complex (1.3 M in THF, 2.5 mL, 3.25 mmol) which resulted in a white suspension. The reaction mixture was stirred in a 0 °C bath for 30 minutes, then a THF (2 mL) solution of *N*-methoxy-*N*,2,4-trimethylthiazole-5-carboxamide (550 mg, 2.75 mmol, Intermediate 17: step a) was introduced. The reaction mixture was stirred at 50 °C for 18 hours, cooled to room temperature and quenched with aqueous NH₄Cl solution. The aqueous portion was extracted with DCM (4 x 50 mL) and the combined organics were washed with brine, dried over Na₂SO₄, filtered and concentrated. Chromatography on silica gel (25% EtOAc-DCM increasing to 5% MeOH-DCM) afforded the title compound as an amber solid.

### Intermediate 18: step a

### (2,4-Dichloro-3-phenylquinofin-6-yl)(1,2-dimethyl-1H-imidazol-5-yl)methanol

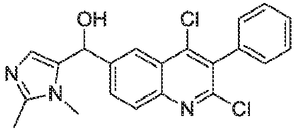

To a flask containing 6-bromo-2,4-dichloro-3-phenylquinoline (4 g, 11.33 mmol, Intermediate 7: step c) was added THF (200 mL) to give a homogeneous clear solution. The solution was cooled in a dry-ice bath and *n*-BuLi (2.5 M in hexanes, 4.25 mL, 10.63 mmol) was added which resulted in an immediate reddish-brownish mixture. After 2 minutes, a THF solution of 1,2-dimethyl-1*H-*imidazole-5-carbaldehyde (1.75 g, 14.1 mmol in 10 mL THF) was added and the reaction mixture became a light yellow color. The -78 °C bath was replaced with a 0 °C ice-bath and after 40 minutes, the reaction mixture was quenched with aqueous NH₄Cl solution and the aqueous portion was extracted with EtOAc (4 x 100 mL). The combined organics were washed with brine, dried over MgSO₄, filtered and concentrated. The resulting solid was triturated with Et₂O to provide the title compound as an off white powder.

### Intermediate 18: step b

### (2,4-Dichloro-3-phenylquinolin-6-yl)(1,2-dimethyl-1H-imidazol-5-yl)methanone

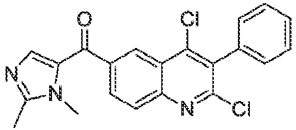

To a flask containing (2,4-dichloro-3-phenylquinolin-6-yl)(1,2-dimethyl-1*H*-imidazol-5-yl)methanol (520 mg, 1.31 mmol, Intermediate 18: step a) was added 1,4-dioxane (12 mL) followed by manganese dioxide (475 mg, 5.46 mmol). The mixture was heated to reflux for 3.25 hours and then the contents were filtered through a Celite® pad, while still warm, and the Celite® pad was rinsed with warm THF. The effluent was concentrated under reduced pressure to provide the title compound as an off white foam.

### Intermediate 19: step a

### 6-Bromo-3-phenyl-2-(trifluoromethyl)quinolin-4-ol

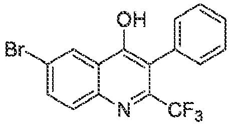

A mixture of 2-amino-5-bromobenzoic acid (3.01 g, 13.9 mmol), 1,1,1-trifluoro-3-phenylpropan-2-one (3.11 g, 16.5 mmol), and Eaton's reagent (9.3 mL) in a sealed tube was heated at 100 °C for 4 hours. The reaction mixture was then allowed to cool to room temperature, water was added slowly, and the mixture was stirred vigorously for about 15 minutes. The precipitated solid was collected by filtration, washed with water, and dried to provide the title compound.

### Intermediate 19: step b

### 6-Bromo-4-chloro-3-phenyl-2-(trifluoromethyl)quinoline

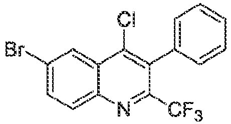

A solution of 6-bromo-3-phenyl-2-(trifluoromethyl)quinolin-4-ol (8.29 g, 22.5 mmol, Intermediate 19: step a) in phosphoryl trichloride (25 mL, 269 mmol) was heated at 110 °C for 2 hours, and concentrated in vacuo. Dichloromethane and ice-water were added, and the mixture was basified at 4 °C with concentrated NH₄OH until pH ∼ 10. The organic layer was separated and the aqueous layer was extracted with CH₂Cl₂. The combined organic phases were dried (Na₂SO₄), concentrated, and purified by flash column chromatography (120 g silica gel column, 2 - 9% EtOAc in heptanes) to afford the title compound as a light yellow solid.

### Intermediate 20: step a

### 6-Iodo-3-phenyl-2-(trifluoromethyl)quinolin-4-ol

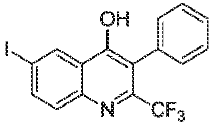

A mixture of 2-amino-5-iodobenzoic acid (5.20 g, 19.8 mmol), 1,1,1-trifluoro-3-phenylpropan-2-one (3.95 g, 21.0 mmol), and Eaton's reagent (12 mL) in a sealed tube was heated at 100 °C for 2 hours. More 1,1,1-trifluoro-3-phenylpropan-2-one (1.60 g, 8.50 mmol) was added and the mixture was heated for another 2 hours. The reaction was then cooled to room temperature, ice water was added, and the mixture was stirred vigorously for about 20 minutes. 50% Aqueous NaOH and concentrated NH₄OH solutions were added until pH 9. The resulting dark brown gummy material that formed was diluted with DCM which resulted in a solid precipitate that was collected by filtration. The solids were washed with water and Et₂O and air dried to provide the title compound.

### Intermediate 20: step b

### 4-Chloro-6-iodo-3-phenyl-2-(trifluoromethyl)quinoline

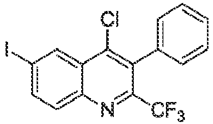

A solution of 6-iodo-3-phenyl-2-(trifluoromethyl)quinolin-4-ol (1.54 g, 3.71 mmol, Intermediate 20: step a) in phosphoryl trichloride (5 mL, 53.8 mmol) was heated at 110 °C for 1 hour 45 minutes, and then cooled to room temperature. Ice-water was added, and the mixture was basified at 4 °C with 50% aqueous NaOH and concentrated NH₄OH until pH 9. The precipitated solid was filtered, washed with water and Et₂O, and dried to provide the title compound. The filtrate was separated and the aqueous layer was extracted with CH₂Cl₂. The combined organic phases were dried (Na₂SO₄), concentrated, and purified by flash column chromatography (80 g silica gel column, 0 - 5% EtOAc in heptanes), affording a mixture of the title compound and desiodo by-product, 4-chloro-3-phenyl-2-(trifluoromethyl)quinoline, in about 8:1 ratio as a thick oil, which solidified over night.

### Intermediate 21

### tert-Butyl 4-nicotinoylpiperidine-1-carboxylate

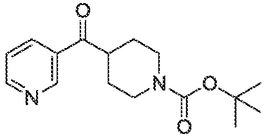

A mixture of piperidin-4-yl(pyridin-3-yl)methanone hydrochloride (397 mg, 1.75 mmol), di*-tert-*butyl dicarbonate (710 mg, 3.25 mmol), *N,N-*dimethylpyridin-4-amine (28 mg, 0.23 mmol) and Et₃N (1.2 mL, 8.6 mmol) in THF (15 mL) and CH₂Cl₂ (5 mL) was stirred for 3 days and then concentrated. The residue was purified by flash column chromatography (40 g silica gel column, 50-70% EtOAc in heptane) to provide the title compound as a clear oil.

### Intermediate 22: step a

### (3-Chlorophenyl)(6-(trifluoromethyl)pyridin-3-yl)methanone

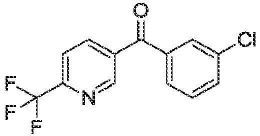

To a solution of *N*-methoxy-*N*-methyl-6-(trifluoromethyl)nicotinamide (1.23 g, 5.25 mmol, Intermediate 2: step b) in THF (12 mL) at 4 °C was added 0.5 M (3-chlorophenyl)magnesium bromide in THF (12.7 mL, 6.35 mmol). The mixture was stirred at 4 °C to room temperature overnight, and quenched with NH₄Cl (aqueous). The organic layer was separated, and the aqueous layer was extracted with CH₂Cl₂. The combined organic phases were dried (Na₂SO₄), filtered, concentrated, and purified by flash column chromatography (40 g silica gel column, 0-70% EtOAc in heptanes) to provide the title compound as an oil, which solidified upon standing.

### Intermediate 22: step b

### (3-Chlorophenyl)(2,4-dichloro-3-phenylquinolin-6-yl)(6-(trifluoromethyl)pyridin-3-yl)methanol

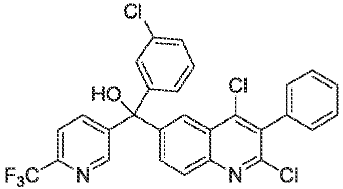

To a solution of 6-bromo-2,4-dichloro-3-phenylquinoline (422 mg, 1.20 mmol, Intermediate 7: step c) and (3-chlorophenyl)(6-(trifluoromethyl)pyridin-3-yl)methanone (340 mg, 1.19 mmol, Intermediate 22: step a) in THF (8 mL) at -78 °C was added 1.6 M n-BuLi in hexane (1.14 mL, 1.82 mmol). The mixture was stirred at -78 °C to 10 °C for 2 hours and then quenched with NH₄Cl (aqueous). The organic layer was separated, and the aqueous layer was extracted with CH₂Cl₂. The combined organic phases were dried (Na₂SO₄), filtered, concentrated, and purified by flash column chromatography (24 g silica gel column, 30 - 40% EtOAc in heptane) to provide the title compound as a white solid.

### Intermediate 23: step a

### N-Methoxy-N-methyl-2-(trifluoromethyl)isonicotinamide

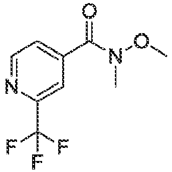

To a suspension of 2-(trifluoromethyl)isonicotinic acid (1.03 g, 5.39 mmol), *N,O-*dimethylhydroxylamine hydrochloride (0.800 g, 8.20 mmol) and *N*'(ethylcarbonimidoyl)-*N,N-*dimethyl-1,3-propanediamine hydrochloride (EDCI, 1.35 g, 7.04 mmol) in CH₂Cl₂ (30 mL) was added Et₃N (1.90 mL, 13.7 mmol), and the mixture immediately turned clear. After stirring at room temperature overnight, NH₄Cl (aqueous) was added. The mixture was stirred vigorously for a while, and white solid was then filtered off. The organic layer was separated, and the aqueous layer was extracted with CH₂Cl₂. The combined organic phases were washed with brine, and the aqueous layer was back extracted with CH₂Cl₂. The organic phases were combined, dried (Na₂SO₄), filtered, concentrated, and purified by flash column chromatography (40 g silica gel column, 40-70% EtOAc in heptanes) to afford the title compound as a clear oil.

### Intermediate 23: step b

### (3-Chlorophenyl)(2-(trifluoromethyl)pyridin-4-yl)methanone

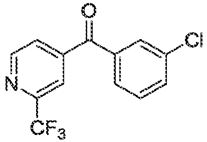

The title compound was prepared using *N*-methoxy-*N*-methyl-2-(trifluoromethyl)isonicotinamide (Intermediate 23: step a) in place of *N*-methoxy-*N*-methyl-6-(trifluoromethyl)nicotinamide (Intermediate 2: step b) according to the procedure described in Intermediate 22: step a.

### Intermediate 24

### (3-Chlorophenyl)(2,4-dichloro-3-phenylquinolin-6-yl)(pyridin-3-yl)methanol

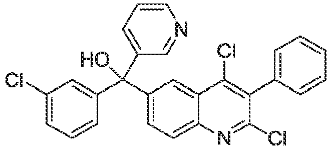

The title compound was prepared using (3-chlorophenyl)(pyridin-3-yl)methanone in place of (3-chlorophenyl)(6-(trifluoromethyl)pyridin-3-yl)methanone (Intermediate 22: step a) according to the procedure described in Intermediate 22: step b.

### Intermediate 25: step a

### N-Methoxy-N-methylisonicotinamide

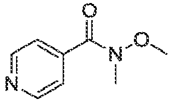

A suspension of 4-picolinic acid (3.00 g, 24.4 mmol) and 1,1-carbonyldiimidazole (4.74 g, 29.2 mmol) in CH₂Cl₂ (35 mL) was stirred for ∼ 40 minutes, at which time it became a clear solution. After the addition of *N,O*-dimethylhydroxylamine hydrochloride (2.85 g, 29.2 mmol), the mixture was stirred at room temperature for 22 hours. Water was added, the organic layer was separated, and the aqueous layer was extracted with CH₂Cl₂. The combined organic layers were washed with water once, and the aqueous layer was back extracted with CH₂Cl₂. The organic phases were combined, dried (Na₂SO₄), filtered, concentrated, and purified by flash column chromatography (80 g silica gel column, 100% EtOAc) to provide the title compound as a clear oil.

### Intermediate 25: step b

### (1-Methyl-1H-imidazol-5-yl)(pyridin-4-yl)methanone

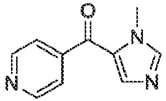

To a heat-gun dried flask containing 1-methyl-1*H*-imidazole (2.2 mL, 27.7 mmol) and THF (13 mL) at -78 °C was added 1.6 M *n*-BuLi in hexane (18.5 mL, 29.6 mmol). After stirring at -78 °C for 40 minutes, neat chlorotriethylsilane (4.9 mL, 29.2 mmol) was introduced slowly. The mixture was stirred at -78 °C for 1 hour. 1.6 M n-BuLi in hexane (18 mL, 28.8 mmol) was added, and stirring became very difficult. The cooling bath was removed, and stirring was continued for a while before the temperature reached around 10 °C. The mixture was re-cooled to -78 °C, a solution of *N*-methoxy-*N*-methylisonicotinamide (3.82 g, 23.0 mmol, Intermediate 25: step a) in THF (28 mL) was added via cannula, and stirring stopped. The cooling bath was removed, and the stirring was continued for 40 minutes before room temperature was reached. The reaction was quenched with a few drops of MeOH. Brine was added, the organic layer was separated, and the aqueous layer was extracted with CH₂Cl₂. The combined organic phases were dried (Na₂SO₄), filtered, concentrated, and purified by flash column chromatography (silica gel, 50-100% EtOAc in heptane, then 5-10% MeOH in CH₂Cl₂) to afford the title compound as an off-white solid.

### Intermediate 25: step c

### (2,4-Dichloro-3-phenylquinolin-6-yl)(1-methyl-1H-imidazol-5-yl)(pyridin-4-yl)methanol•TFA

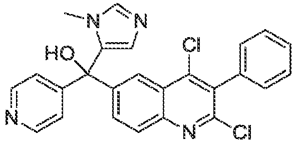

To a solution of 6-bromo-2,4-dichloro-3-phenylquinoline (1.46 g, 4.14 mmol, Intermediate 7: step c) and (1-methyl-1*H*-imidazol-5-yl)(pyridin-4-yl)methanone (778 mg, 4.16 mmol, Intermediate 25: step b) in THF (65 mL) at -78 °C was added 1.6 M *n*-BuLi in hexane (3.90 mL, 6.24 mmol). After stirring at -78 °C to room temperature overnight, the mixture was quenched with NH₄Cl (aqueous). The organic layer was separated, and the aqueous layer was extracted with CH₂Cl₂. The combined organic phases were dried (Na₂SO₄), filtered, concentrated, and purified by flash column chromatography (80 g silica gel column, 100% EtOAc, then 5-10% MeOH in CH₂Cl₂) and then reverse phase HPLC (water/acetonitrile/0.1% TFA) to afford the title compound.

### Intermediate 26

### (4-Butyl-2-chloro-3-phenylquinolin-6-yl)(1-methyl-1H-imidazol-5-yl)(pyridin-4-yl)methanol•TFA

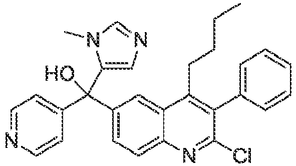

The title compound was isolated as a by-product from the reaction that formed (2,4-dichloro-3-phenylquinolin-6-yl)(1-methyl-1*H*-imidazol-5-yl)(pyridin-4-yl)methanol (Intermediate 25: step c).

### Intermediate 27

### (2,4-Dichloro-3-phenylquinolin-6-yl)(pyridin-2-yl)(pyridin4-yl)methanol•TFA

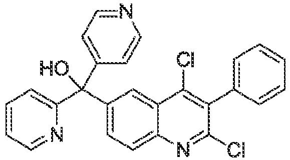

The title compound was prepared using pyridin-2-yl(pyridin-4-yl)methanone in place of (1-methyl-1*H*-imidazol-5-yl)(pyridin-4-yl)methanone (Intermediate 25: step b) according to the procedure described in Intermediate 25: step c.

### Intermediate 28: step a

### 2-Fluoro-N-methoxy-N-methylisonicotinamide

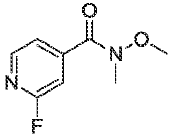

The title compound was prepared using 2-fluoroisonicotinic acid in place of 4-picolinic acid according to the procedure described in Intermediate 25: step a.

### Intermediate 28: step b

### (2-Fluoropyridin-4-yl)(1-methyl-1H-imidazol-2-yl)methanone

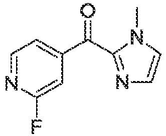

The title compound was prepared using 2-fluoro-*N*-methoxy-*N*-methylisonicotinamide (Intermediate 28: step a) in place of *N*-methoxy-*N*-methylisonicotinamide (Intermediate 25: step a) according to the procedure described in Intermediate 25: step b.

### Intermediate 29

### tert-Butyl 4-((2,4-dichloro-3-phenylquinolin-6-yl)(hydroxy)(pyridin-3-yl)methyl)piperidine-1-carboxylate

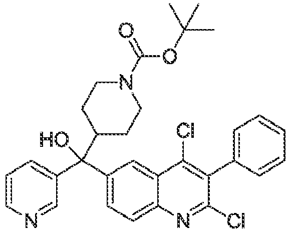

To a solution of 6-bromo-2,4-dichloro-3-phenylquinoline (174 mg, 0.490 mmol, Intermediate 7: step c) and *tert*-butyl 4-nicotinoylpiperidine-1-carboxylate (143 mg, 0.490 mmol, Intermediate 21) in THF (5 mL) at -78 °C was added 1.6 M *n*-BuLi in hexane (0.47 mL, 0.75 mmol). The mixture was stirred at -78 °C to 0 °C for 3 hours and quenched with NH₄Cl (aqueous). The organic layer was separated, and the aqueous layer was extracted with CH₂Cl₂. The combined organic phases were dried (Na₂SO₄), filtered, concentrated, and purified by flash column chromatography (40 g silica gel column, 50 - 70% EtOAc in heptane) to provide the title compound as a solid.

### Intermediate 30

### (4-Chlorophenyl)(2,4-dimethylthiazol-5-yl)methanone

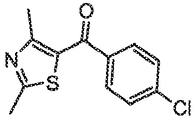

To a flask containing N-methoxy-N,2,4-trimethylthiazole-5-carboxamide (510 mg, 2.55 mmol, Intermediate 17: step a) was added THF (18 mL) and the solution was cooled to 0 °C. To this solution was added (4-chlorophenyl)magnesium chloride (1 M in diethylether, 3.3 mL, 3.3 mmol). A homogeneous yellow mixture resulted and was allowed to warm to room temperature over 15 minutes. After 2 hours, the reaction mixture was quenched with aqueous NH₄Cl solution and the aqueous portion was extracted with EtOAc (4 x 50 mL). The combined organics were washed with brine, dried over MgSO₄, filtered and concentrated to dryness. Chromatography on silica gel (100% DCM increasing to 10% EtOAc-DCM) provided the title compound as a white solid.

### Intermediate 31: step a

### N-(4-Bromophenyl)-2-phenylacetamide

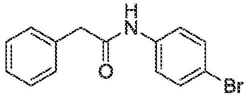

Into a 250-mL round-bottom flask were placed a solution of 4-bromoaniline (17.1 g, 99.42 mmol) and triethylamine (30 g, 297 mmol) in dichloromethane (100 mL). Then, 2-phenylacetyl chloride (18.6 g, 120 mmol) was added dropwise with stirring, and the resulting solution was stirred at room temperature for 6 hours. The solids were filtered out, and the filtrate was concentrated under vacuum. The crude product was purified by re-crystallization from ethyl acetate to provide the title compound as a white solid.

### Intermediate 31: step b

### 6-Bromo-2-chloro-3-phenylquinoline

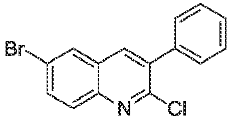

Into a 50-mL round-bottom flask were placed *N,N*-dimethylformamide (2.15 g, 29.45 mmol) and phosphoryl trichloride (20.3 g, 132.7 mmol) and the mixture was cooled to 0°C. Then, *N*-(4-bromophenyl)-2-phenylacetamide (5.7 g, 17.87 mmol, Intermediate 23: step a) was added in portions. The resulting solution was heated at 80 °C for 5 hours, then concentrated under vacuum, followed by dilution with 50 mL of H₂O, and then extraction with 3 x 50 mL of ethyl acetate. The combined organic layers were dried over anhydrous sodium sulfate, filtered and concentrated *in vacuo.* The residue was purified by FCC (1:10 EtOAc / petroleum ether) to afford the title compound as a yellow solid.

### Intermediate 32: step a

### Methyl 5-bromo-2-(2-(2-chlorophenyl)acetamido) benzoate

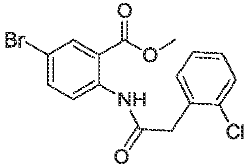

The title compound was prepared using 2-chlorophenylacetyl chloride in place of 2-phenylacetyl chloride using the procedure described for Intermediate 7: step a.

### Intermediate 32: step b

### 6-Bromo-3-(2-chlorophenyl)-4-hydroxyquinolin-2(1H)-one

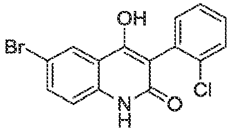

The title compound was prepared using methyl 5-bromo-2-(2-(2-chlorophenyl)acetamido) benzoate (Intermediate 32: step a) in place of 5-bromo-2-(2-phenylacetamido) benzoate using the procedure described for Intermediate 7: step b.

### Intermediate 32: step c

### 6-Bromo-2,4-dichloro-3-(2-chlorophenyl)quinoline

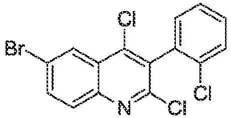

The title compound was prepared using 6-bromo-3-(2-chlorophenyl)-4-hydroxyquinolin-2(1*H*)-one (Intermediate 32: step b) in place of 6-bromo-4-hydroxy-3-phenylquinolin-2(1*H*)-one using the procedure described for Intermediate 7: step c.

### Example 1a: 1-(4-((4-Chloro-2-methoxy-3-(3-(trifluoromethyl)phenyl)quinolin-6-yl)(hydroxy)(1-methyl-1H-imidazol-5-yl)methyl)piperidin-1-yl)ethanone

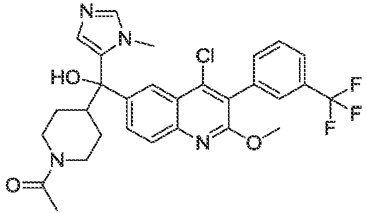

n-Butyllithium (1.6 M in hexane, 0.585 mL, 0.937 mmol) was added over 1 minute to a solution of 6-bromo-4-chloro-2-methoxy-3-(3-(trifluoromethyl)phenyl)quinoline (409.8 mg, 0.984 mmol, Intermediate 5: step e) in THF (3 mL) at -78 °C. After 1 minute, a solution of 1-(4-(1-methyl-1*H*-imidazole-5-carbonyl)piperidin-1-yl)ethanone (231.4 mg, 0.984 mmol, Intermediate 1: step c) in THF (5 mL) was added via cannula. The mixture was stirred at -78 °C for 10 minutes, then was transferred to an ice bath and stirred for 45 minutes. The reaction mixture was quenched by addition of saturated aqueous NH₄Cl, diluted with water, and extracted with EtOAc (3x). The organic phase was dried (Na₂SO₄), filtered, and concentrated to dryness. The residue was purified by flash column chromatography (silica gel, 0-8% MeOH-DCM) to afford the target compound as a white solid. MS m/e 573.3 [M+H]⁺.

1-(4-((4-Chloro-2-methoxy-3-(3-(trifluoromethyl)phenyl)quinolin-6-yl)(hydroxy)(1-methyl-1*H-*imidazol-5-yl)methyl)piperidin-1-yl)ethanone was purified by chiral HPLC (Chiralcel OD-H, EtOH) to give 2 enantiomers. **Example 1b:** (first enantiomer to elute off chiral column) ¹H NMR (400 MHz, CDCl₃, 1.2:1 mixture of rotamers, minor rotamer peaks marked *) δ 8.21 (s, 1H), 8.16* (br. s., 1H), 7.85* (d, *J* = 2.53 Hz, 1H), 7.83 (d, *J* = 2.53 Hz, 1H), 7.72* (s, 1H), 7.70 (s, 1H), 7.59 - 7.66 (m, 4H), 7.54 - 7.58 (m, 2H), 7.41 - 7.51 (m, 2H), 7.34 (s, 2H), 7.23 (s, 2H), 4.78 (d, *J* = 12.63 Hz, 1H), 4.61* (d, *J* = 12.63 Hz, 1H), 4.01 (s, 6H), 3.95 (d, *J* = 13.14 Hz, 1H), 3.76* (d, *J* = 11.12 Hz, 1H), 3.28 (s, 3H), 3.27* (s, 3H), 3.11 - 3.24* (m, 1H), 2.94 - 3.04 (m, 1H), 2.56 - 2.70 (m, 3H), 2.40 - 2.56 (m, 3H), 2.25 - 2.38 (m, 2H), 2.05 (s, 3H)*, 2.03 (s, 3H), 1.13 - 1.44 (m, 6H); MS m/e 573.2 [M+H]⁺ and **Example 1c:** (second enantiomer to elute off chiral column) ¹H NMR (400 MHz, CDCl₃, 1.2:1 mixture of rotamers, minor rotamer peaks marked *) δ 8.21 (s, 1H), 8.16* (br. s., 1H), 7.84* (d, *J* = 3.03 Hz, 1H), 7.82 (d, *J* = 2.53 Hz, 1H), 7.71* (s, 1H), 7.69 (s, 1H), 7.58 - 7.65 (m, 4H), 7.52 - 7.58 (m, 2H), 7.40 - 7.51 (m, 2H), 7.29 - 7.35 (m, 2H), 7.22 (s, 2H), 4.77 (d, *J* = 13.1 Hz, 1H), 4.60 (d, *J* = 13.6 Hz, 1H), 4.00 (s, 6H), 3.93 (d, *J* = 12.63 Hz, 1H), 3.74* (d, *J* = 13.14 Hz, 1H), 3.27 (s, 3H), 3.26* (s, 3H), 3.12 - 3.22 (m, 1H), 2.92 - 3.05 (m, 1H), 2.80 (s, 1H), 2.57 - 2.74 (m, 2H), 2.39 - 2.55 (m, 3H), 2.22 - 2.39 (m, 2H), 2.04* (s, 3H), 2.02 (s, 3H), 1.12 - 1.46 (m, 6H); MS m/e 573.2 [M+H]⁺.

### Example 2a: 6-((1-Acetylpiperidin-4-yl)(hydroxy)(1-methyl-1H-imidazol-5-yl)methyl)-2-methoxy-3-(3-(trifluoromethyl)phenyl)quinoline-4-carbonitrile

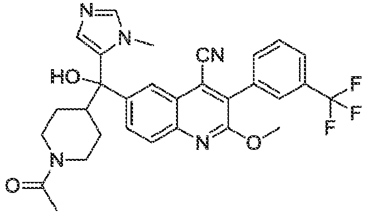

A round bottom flask was charged with 1-(4-((4-chloro-2-methoxy-3-(3-(trifluoromethyl)phenyl)quinolin-6-yl)(hydroxy)(1-methyl-1*H*-imidazol-5-yl)methyl)piperidin-1-yl)ethanone (158 mg, 0.276 mmol, Example 1a), Zn(CN)₂ (58.3 mg, 0.496 mmol), Pd₂(dba)₃ (37.9 mg, 0.041 mmol), zinc nanopowder (5.4 mg, 0.083 mmol), and dicyclohexyl(2',4',6'-triisopropyl-[1,1'-biphenyl]-2-yl)phosphine (X-Phos, 27.1 mg, 0.055 mmol). The flask was evacuated and re-filled with argon (three cycles). Dimethylacetamide (1.4 mL, sparged with argon for 30 minutes) was then added and the mixture was heated at 120 °C for 4 hours. The mixture was cooled to room temperature and was filtered through Celite®, washing with EtOAc. The filtrate was washed sequentially with 2 M aqueous NH₄OH, water, and saturated aqueous NaCl. The organic phase was dried (Na₂SO₄), filtered, and concentrated to dryness. The residue was purified by flash column chromatography (silica gel, 1-8% MeOH-DCM) to afford the title compound. MS m/e 564.3 [M+H]⁺.

6-((1-Acetylpiperidin-4-yl)(hydroxy)(1-methyl-1*H*-imidazol-5-yl)methyl)-2-methoxy-3-(3-(trifluoromethyl)phenyl)quinoline-4-carbonitrile was purified by chiral HPLC (Chiralcel OD-H, EtOH) to give 2 enantiomers. **Example 2b:** (first enantiomer to elute off chiral column) ¹H NMR (400 MHz, CDCl₃, 1.1:1 mixture of rotamers, minor rotamer peaks marked *) δ 8.28 (s, 1H), 8.24* (s, 1H), 7.84 - 7.91 (m, 2H), 7.71 - 7.84 (m, 6H), 7.63 - 7.71 (m, 2H), 7.43 - 7.50 (m, 2H), 7.29 - 7.33 (m, 2H), 7.21 (s, 2H), 4.76 (d, *J* = 14.65 Hz, 1H), 4.59* (d, *J* = 11.12 Hz, 1H), 4.06* (s, 6H), 3.94 (d, *J* = 12.63 Hz, 1H), 3.68 - 3.80 (m, 1H), 3.29 (s, 3H), 3.27* (s, 3H), 3.22 (s, 1H), 3.13 - 3.22* (m, 1H), 3.10* (s, 1H), 2.93 - 3.04 (m, 1H), 2.58 - 2.71 (m, 1H), 2.23 - 2.57 (m, 5H), 2.04 (s, 3H), 2.03 (s, 3H), 1.12 - 1.47 (m, 6H); MS m/e 564.2 [M+H]⁺ and **Example 2c:** (second enantiomer to elute off chiral column) ¹H NMR (400 MHz, CDCl₃, 1.1:1 mixture of rotamers, minor rotamer peaks marked *) δ 8.28 (s, 1H), 8.24* (s, 1H), 7.88-7.90* (m, 1H), 7.85-7.87 (m, 1H), 7.71 - 7.83 (m, 6H), 7.64 - 7.71 (m, 2H), 7.42 - 7.50 (m, 2H), 7.31-7.34 (m, 2H), 7.22 (s, 2H), 4.77 (d, *J* = 13.14 Hz, 1H), 4.60* (d, *J* = 14.15 Hz, 1H), 4.07 (s, 6H), 3.94* (d, *J* = 14.15 Hz, 1H), 3.68 - 3.80 (m, 1H), 3.28 (s, 3H), 3.27* (s, 3H), 3.13 - 3.23 (m, 1H), 2.96 - 3.05* (m, 1H), 2.95 (s, 1H), 2.84* (s, 1H), 2.58 - 2.70 (m, 1H), 2.22 - 2.57 (m, 5H), 2.05* (s, 3H), 2.03 (s, 3H), 1.17 - 1.45 (m, 6H); MS m/e 564.2 [M+H]⁺.

### Example 3a: (4-Chloro-2-methoxy-3-(3-(trifluoromethyl)phenyl)quinolin-6-yl)(1-methyl-1H-1,2,3-triazol-5-yl)(1-methyl-1H-imidazol-5-yl)methanol

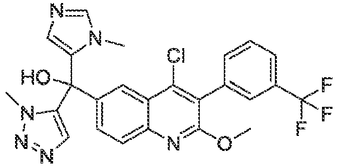

*n*-Butyllithium (1.6 M in hexane, 0.547 mL, 0.876 mmol) was added over 1 minute to a solution of 6-bromo-4-chloro-2-methoxy-3-(3-(trifluoromethyl)phenyl)quinoline (383 mg, 0.919 mmol, Intermediate 5: step e) in THF (3 mL) at -78 °C. After 5 minutes, a solution of (1-methyl-1*H-*1,2,3-triazol-5-yl)(1-methyl-1*H*-imidazol-5-yl)methanone (176 mg, 0.919 mmol, Intermediate 6, step b) in THF (15 mL) was added via cannula. The mixture was stirred at -78 °C for 5 minutes, then was transferred to an ice bath and stirred for 30 minutes. The reaction mixture was quenched by addition of saturated aqueous NH₄Cl, diluted with water, and extracted with EtOAc (3x). The organic phase was washed twice with water. The organic phase was dried (Na₂SO₄), filtered, and concentrated. The residue was purified by flash column chromatography (silica gel, 1-7% MeOH-DCM) to afford the target compound as a white solid. MS m/e 529.2 [M+H]⁺.

(4-Chloro-2-methoxy-3-(3-(trifluoromethyl)phenyl)quinolin-6-yl)(1-methyl-1*H*-1,2,3-triazol-5-yl)(1-methyl-1*H*-imidazol-5-yl)methanol was purified by chiral SFC (Chiralpack IC, 75% CO₂, 25% (0.2% isopropylamine-2-propanol)) to give 2 enantiomers. The enantiomers were then further purified individually on plug silica gel columns (0-5% MeOH-DCM). **Example 3b:** (first enantiomer to elute off chiral column) ¹H NMR (400 MHz, CDCl₃) δ 8.30 (br. s., 1H), 7.82 - 8.09 (br. s., 1H), 7.78 (d, *J* = 9.1 Hz, 1 H), 7.70 - 7.75 (m, 1H), 7.61 - 7.68 (m, 2H), 7.36 (br. s., 1H), 7.26 - 7.32 (m, 1H), 7.13 (br. s., 1H), 6.19 (br. s., 1H), 3.98 (s, 3H), 3.91 (s, 3H), 3.52 (s, 3H); MS m/e 529.2 [M+H]⁺ and **Example 3c:** (second enantiomer to elute off chiral column) ¹H NMR (400 MHz, CDCl₃) δ 8.31 (br. s., 1H), 7.79 - 8.05 (br. s., 1H), 7.78 (d, *J* = 8.6 Hz, 1 H), 7.71 - 7.75 (m, 1H), 7.61 - 7.69 (m, 2H), 7.36 (br. s., 1H), 7.26 - 7.32 (m, 1H), 7.12 (br. s., 1H), 6.19 (br. s., 1H), 3.98 (s, 3H), 3.91 (s, 3H), 3.52 (s, 3H); MS m/e 529.2 [M+H]⁺.

### Example 4a: 6-(Hydroxy(1-methyl-1H-1,2,3-triazol-5-yl)(1-methyl-1H-imidazol-5-yl)methyl)-2-methoxy-3-(3-(trifluoromethyl)phenyl)quinoline-4-carbonitrile

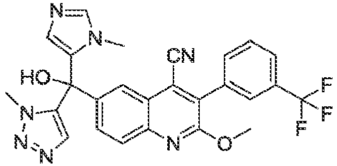

A round bottom flask was charged with (4-chloro-2-methoxy-3-(3-(trifluoromethyl)phenyl)quinolin-6-yl)(1-methyl-1*H*-1,2,3-triazol-5-yl)(1-methyl-1*H*-imidazol-5-yl)methanol (96.6 mg, 0.183 mmol, Example 3a), Zn(CN)₂ (38.6 mg, 0.329 mmol), Pd₂(dba)₃ (25.1 mg, 0.027 mmol), zinc nanopowder (3.6 mg, 0.055 mmol), and dicyclohexyl(2',4',6'-triisopropyl-[1,1'-biphenyl]-2-yl)phosphine (X-Phos, 18.0 mg, 0.037 mmol). The flask was evacuated and re-filled with argon (three cycles). Dimethylacetamide (1.8 mL, sparged with argon for 30 minutes) was then added and the mixture was heated at 120 °C for 9 hours. The mixture was cooled to room temperature and was filtered through Celite®, washing with EtOAc. The filtrate was washed sequentially with 2 M aqueous NH₄OH, water, and saturated aqueous NaCl. The organic phase was dried (Na₂SO₄), filtered, and concentrated. To improve conversion, the crude product was resubjected to the reaction conditions as described above for an additional 4.5 hours, then was worked up as described above. The residue was partially purified by flash column chromatography (silica gel, 2-8% MeOH-DCM) then was further purified by RP-HPLC (10-90% CH₃CN-H₂O, 0.1% TFA). HPLC fractions were basified (saturated aqueous NaHCO₃), partially concentrated, and extracted with DCM (3x). The organic extract was dried over Na₂SO₄, filtered, and concentrated to afford the title compound. MS m/e 519.9 [M+H]⁺. 6-(Hydroxy(1-methyl-1*H*-1,2,3-triazol-5-yl)(1-methyl-1*H*-imidazol-5-yl)methyl)-2-methoxy-3-(3-(trifluoromethyl)phenyl)quinoline-4-carbonitrile was purified by chiral SFC (Chiralpack IC, 90% CO₂, 10% (0.2% isopropylamine-EtOH)) to give 2 enantiomers. The enantiomers were then further purified on plug silica gel columns (0-5% MeOH-DCM). **Example 4b:** (first enantiomer to elute off chiral column) ¹H NMR (400 MHz, CDCl₃) δ 8.27 - 8.31 (m, 1H), 7.88 (d, *J* = 8.6 Hz, 1H), 7.84 (s, 1H), 7.77 - 7.82 (m, 1H), 7.73 - 7.77 (m, 1H), 7.66 - 7.73 (m, 1H), 7.35 (dd, *J* = 8.6, 1.5 Hz, 1H), 7.16 (s, 1H), 6.33 (br. s., 1H), 6.25 (br. s., 1H), 4.07 (s, 3H), 3.93 (s, 3H), 3.53 (s, 3H); MS m/e 5202 [M+H]⁺ and **Example 4c:** (second enantiomer to elute off chiral column) ¹H NMR (400 MHz, CDCl₃) δ 8.29 (d, J= 2.0 Hz, 1H), 7.90 (d, *J* = 8.6 Hz, 1H), 7.84 (s, 1H), 7.77 - 7.81 (m, 1H), 7.73 - 7.77 (m, 1H), 7.66 - 7.73 (m, 1H), 7.37 (dd, *J* = 9.1, 2.0 Hz, 1H), 7.33 (s, 1H), 7.19 (s, 1H), 6.30 (br. s., 1H), 5.54 (br. s., 1H), 4.08 (s, 3H), 3.95 (s, 3H), 3.55 (s, 3H); MS m/e 520.0 [M+H]⁺.

### Example 5: (4-Chlorophenyl)(2,4-dichloro-3-(pyridin-2-yl)quinolin-6-yl)(1-methyl-1H-imidazol-5-yl)methanol•TFA (Reference)

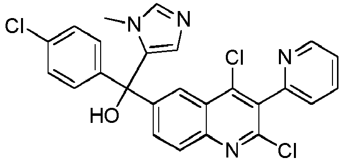

Into a 100-mL round-bottom flask was placed a solution of 6-bromo-2,4-dichloro-3-(pyridin-2-yl)quinoline (380 mg, 1.07 mmol, Intermediate 9: step c) in tetrahydrofuran (10 mL). This was followed by the addition of *n*-BuLi (0.43 mL, 1.28 mmol, 3.0 M in hexanes) dropwise with stirring at -78 °C. The resulting solution was stirred for 30 minutes at -78 °C. To this was added a solution of 5-[(4-chlorophenyl)carbonyl]-1-methyl-1*H*-imidazole (199 mg, 0.90 mmol, Intermediate 8: step b) in tetrahydrofuran (10 mL) dropwise with stirring at -78 °C. The resulting solution was allowed to react, with stirring, for an additional 2 hours at room temperature. The reaction was then quenched by the addition of 10 mL of saturated aqueous NH₄Cl. The resulting mixture was concentrated under vacuum. The crude product was purified by Prep-HPLC [(1#waters-2767-1): Column, Sunfire prep C18, 5 µm, 19 x 100 mm; mobile phase, 0.05% TFA in water and MeOH (45% MeOH up to 65% in 10 minutes); Detector, UV 254 nm] to afford the title compound trifluoroacetic acid salt as a white solid. MS (ES, *m*/*z*) 495 [M+H]⁺; ¹H NMR (300 MHz, CDCl₃ + D₂O) δ 8.86 - 8.69 (m, 1H), 8.50 (br. s., 1H), 8.40 (br. s., 0.5H), 8.25 (br. s., 0.5H), 8.07 (d, *J* = 8.6 Hz, 1H), 7.97 (t, *J* = 7.5 Hz, 1H), 7.85 - 7.69 (m, 1H), 7.60 - 7.43 (m, 2H), 7.43 - 7.30 (m, 4H), 6.70 (br. s., 0.5H), 6.65 (br. s., 0.5H), 3.59 (br. s., 3H).

### Example 6: (4-Chlorophenyl)(2,4-dichloro-3-(pyridin-3-yl)quinolin-6-yl)(0-methyl-1H-imidazol-5-yl)methanol•TFA (Reference)

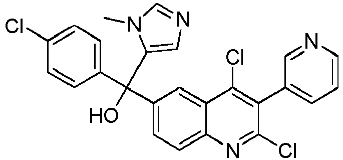

Into a 100-mL round-bottom flask purged and maintained with an inert atmosphere of nitrogen, was placed a solution of 6-bromo-2,4-dichloro-3-(pyridin-3-yl)quinoline (160 mg, 0.45 mmol, Intermediate 10: step c) in tetrahydrofuran (10 mL). This was followed by the addition of *t*-BuLi (0.85 mL, 1.35 mmol, 1.6 M in pentane) dropwise with stirring at -78 °C. The resulting solution was stirred for 30 minutes at -78 °C. To this was added a solution of 5-[(4-chlorophenyl)carbonyl]-1-methyl-1*H*-imidazole (121 mg, 0.55 mmol, Intermediate 8, step b) in tetrahydrofuran (10 mL) dropwise with stirring at -78 °C. The resulting solution was stirred for 30 minutes at -78 °C. The resulting solution was allowed to react, with stirring, for an additional 30 minutes at -40 °C. The resulting solution was allowed to react, with stirring overnight at room temperature. The reaction was then quenched by the addition of 10 mL of saturated aqueous NH₄Cl. The resulting solution was extracted with 3x10 mL of ethyl acetate and the organic layers combined and dried over anhydrous sodium sulfate, filtered and concentrated under vacuum. The crude product was purified by Flash-Prep-HPLC [(IntelFlash-1): Column, silica gel; mobile phase, 0.05% TFA in water and CH₃CN (22% CH₃CN up to 44% within 10 minutes; Detector, UV 254 nm] to afford the title compound trifluoroacetic acid salt as an off-white solid. MS (ES, *m*/*z*) 495 [M+H]⁺ and 517 [M+Na]⁺; ¹H NMR (400 MHz, CD₃OD) δ 9.03 (s, 1H), 8.78-8.62 (m, 2H), 8.39-8.36 (m, 1H), 8.15-8.07 (m, 2H), 7.97-7.94 (m, 1H), 7.80-7.76 (m, 1H), 7.54-7.45 (m, 4H), 7.00 (br. s., 1H), 3.74 (d, *J* = 11.2 Hz, 3H).

### Example 7a: {4-Chloro-2-methoxy-3-[4-(methylsulfonyl)phenyl]quinolin-6-yl}(1-methyl-1H-imidazol-5-yl)[6-(trifluoromethyl)pyridin-3-yl]methanol

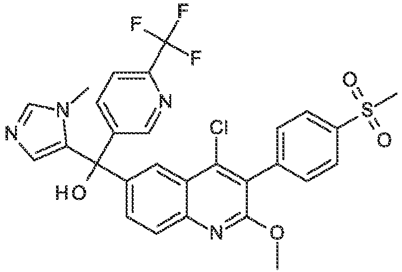

A mixture of 4-chloro-6-(hydroxy(1-methyl-1*H*-imidazol-5-yl)(6-(trifluoromethyl)pyridin-3-yl)methyl)-2-methoxyquinolin-3-yl trifluoromethanesulfonate (70 mg, 0.12 mmol, Intermediate 11: step g), (4-(methylsulfonyl)phenyl)boronic acid (35 mg, 0.18 mmol), PdCl₂(dppf) (9 mg, 0.012 mmol) and K₂CO₃ (16 mg, 0.12 mmol) was sparged with nitrogen three times. To this mixture was added 1,4-dioxane (2 mL) and water (0.3 mL) and the suspension purged with nitrogen. The resulting solution was heated to 65 °C for 15 hours. The reaction was allowed to cool to room temperature and concentrated to dryness. The residue was dissolved in DMSO, filtered and purified by reverse-phase HPLC (acetonitrile / water + TFA). The acidic fractions were neutralized by diluting with EtOAc and washing with saturated aqueous NaHCO₃ followed by water. The organics were dried (MgSO₄), filtered and concentrated to dryness to provide the title compound. ¹H NMR (400 MHz, CD₃OD) δ 8.79 - 8.76 (m, 1H), 8.24 - 8.20 (m, 1H), 8.08 - 8.05 (m, 2H), 8.05 - 8.01 (m, 1H), 7.96 - 7.93 (m, 1H), 7.84 (d, *J* = 8.3 Hz, 1H), 7.76 - 7.72 (m, 2H), 7.65 - 7.62 (m, 2H), 6.35 (s, 1H), 4.00 (s, 3H), 3.49 (s, 3H), 3.20 (s, 3H); MS (ESI): mass calcd. for C₂₈H₂₂ClF₃N₄O₄S, 602.1; m/z found, 603.2 [M+H]⁺.

{4-Chloro-2-methoxy-3-[4-(methylsulfonyl)phenyl]quinolin-6-yl}(1-methyl-1*H*-imidazol-5-yl)[6-(trifluoromethyl)pyridin-3-yl]methanol was purified by HPLC (Kromasil C18 100 Å, 5 µM column, Mobile phase: 35-100% acetonitrile/water + 0.25% ammonium bicarbonate) followed by FCC (0-5% MeOH/DCM) to give 2 enantiomers. The first eluting enantiomer was **Example 7b:** ¹H NMR (400 MHz, CD₃OD) δ 8.81 - 8.77 (m, 1H), 8.23 (d, *J* = 2.0 Hz, 1H), 8.09 - 8.06 (m, 2H), 8.06 - 8.02 (m, 1H), 7.97 - 7.93 (m, 1H), 7.85 (d, *J* = 8.2 Hz, 1H), 7.78 - 7.72 (m, 2H), 7.66 - 7.62 (m, 2H), 6.37 (s, 1H), 4.01 (s, 3H), 3.50 (s, 3H), 3.21 (s, 3H); MS (ESI): mass calcd. for C₂₈H₂₂ClF₃N₄O₄S, 602.1; m/z found, 603.2 [M+H]⁺ and the second eluting enantiomer was **Example 7c:** ¹H NMR (400 MHz, CD₃OD) δ 8.80 - 8.77 (m, 1H), 8.22 (d, *J* = 2.1 Hz, 1H), 8.09 - 8.05 (m, 2H), 8.05 - 8.01 (m, 1H), 7.96 - 7.92 (m, 1H), 7.85 (d, *J* = 8.2 Hz, 1H), 7.77 - 7.71 (m, 2H), 7.66 - 7.61 (m, 2H), 6.36 (s, 1H), 4.00 (s, 3H), 3.49 (s, 3H), 3.21 (s, 3H); MS (ESI): mass calcd. for C₂₈H₂₂ClF₃N₄O₄S, 602.1; m/z found, 603.2 [M+H]⁺.

### Example 8: [4-Chloro-2-methoxy-3-(1-methyl-1H-pyrazol-4-yl)quinolin-6-yl](1-methyl-1H-imidazol-5-yl)[6-(trifluoromethyl)pyridin-3-yl]methanol•TFA

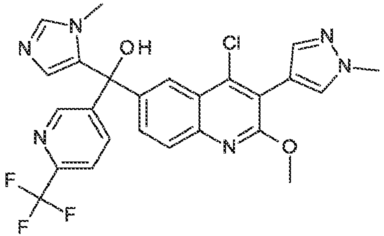

The title compound was prepared using 1-methyl-4-(4,4,5,5-tetramethyl-1,3,2-dioxaborolan-2-yl)-1*H*-pyrazole in place of (4-(methylsulfonyl)phenyl)boronic acid using the procedure described for Example 7a. ¹H NMR (400 MHz, CD₃OD) δ 9.03 (s, 1H), 8.82 (d, *J* = 2.3 Hz, 1H), 8.25 (d, *J* = 2.1 Hz, 1H), 8.10 - 8.06 (m, 1H), 8.03 (s, 1H), 7.93 (d, *J* = 8.7 Hz, 1H), 7.90 (d, *J* = 8.3 Hz, 1H), 7.85 (s, 1H), 7.66 (dd, *J* = 8.7,2.1 Hz, 1H), 7.08 - 7.06 (m, 1H), 4.09 (s, 3H), 3.98 (s, 3H), 3.71 (s, 3H); MS (ESI): mass calcd. for C₂₅H₂₀ClF₃N₆O₂, 528.1; m/z found, 529.2 [M+H]⁺.

### Example 9a: 5-(4-Chloro-6-{hydroxy(1-methyl-1H-imidazol-5-yl)[6-(trifluoromethyl)pyridin-3-yl]methyl}-2-methoxyquinolin-3-yl)pyrimidine-2-carbonitrile•TFA

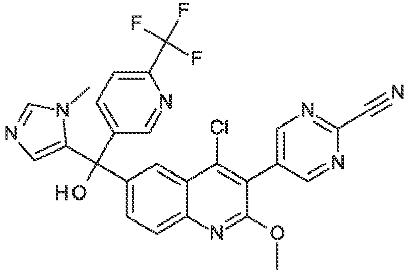

The title compound was prepared using 5-(4,4,5,5-tetramethyl-1,3,2-dioxaborolan-2-yl)pyrimidine-2-carbonitrile in place of (4-(methylsulfonyl)phenyl)boronic acid using the procedure described for Example 7a. ¹H NMR (400 MHz, CD₃OD) δ 9.06 - 9.02 (m, 3H), 8.84 - 8.79 (m, 1H), 8.33 - 8.29 (m, 1H), 8.12 - 8.08 (m, 1H), 8.02 (d, *J* = 8.9 Hz, 1H), 7.90 (d, *J* = 8.2 Hz, 1H), 7.82 - 7.77 (m, 1H), 7.10 (s, 1H), 4.06 (s, 3H), 3.71 (s, 3H); MS (ESI): mass calcd. for C₂₆H₁₇ClF₃N₇O₂, 551.1; m/z found, 552.2 [M+H]⁺.
5-(4-Chloro-6-{hydroxy(1-methyl-1*H*-imidazol-5-yl)[6-(trifluoromethyl)pyridin-3-yl]methyl)-2-methoxyquinolin-3-yl)pyrimidine-2-carbonitrile was purified by HPLC (CHIRACEL OD 20 µM Daicel column, Mobile phase: 100% EtOH) followed by FCC (0-10% MeOH/DCM) to give 2 enantiomers. The first eluting enantiomer was **Example 9b:** ¹H NMR (400 MHz, CD₃OD) δ 9.05 (s, 2H), 8.77 (m, 1H), 8.25 (d, *J* = 2.1 Hz, 1H), 8.04 (dd, *J* = 8.2, 2.2 Hz, 1H), 7.98 - 7.94 (m, 1H), 7.85 (d, *J* = 8.2 Hz, 1H), 7.79 (dd, *J* = 8.9, 2.1 Hz, 1H), 7.76 - 7.72 (m, 1H), 6.36 (s, 1H), 4.05 (s, 3H), 3.48 (s, 3H); MS (ESI): mass calcd. for C₂₆H₁₇ClF₃N₇O₂, 551.1; m/z found, 552.2 [M+H]⁺ and the second eluting enantiomer was **Example 9c:** ¹H NMR (400 MHz, CD₃OD) δ 9.05 (s, 2H), 8.80 - 8.76 (m, 1H), 8.26 (d, *J* = 2.1 Hz, 1H), 8.06 - 8.02 (m, 1H), 7.97 (d, *J* = 8.8 Hz, 1H), 7.85 (d, *J* = 8.3 Hz, 1H), 7.79 (dd, *J* = 8.9, 2.1 Hz, 1H), 7.75 (s, 1H), 6.36 (s, 1H), 4.06 (s, 3H), 3.49 (s, 3H); MS (ESI): mass calcd. for C₂₆H₁₇ClF₃N₇O₂, 551.1; m/z found, 552.2 [M+H]⁺.

### Example 10: (4-Chloro-2-methoxy-3-pyrimidin-5-ylquinolin-6-yl)(1-methyl-1H-imidazol-5-yl)[6-(trifluoromethyl)pyridin-3-yl]methanol•TFA

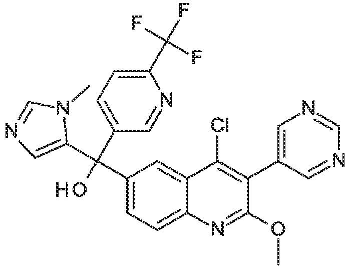

The title compound was prepared using pyrimidin-5-ylboronic acid in place of (4-(methylsulfonyl)phenyl)boronic acid using the procedure described for Example 7a. ¹H NMR (400 MHz, CD₃OD) δ 9.23 (s, 1H), 9.04 (s, 1H), 8.88 (s, 2H), 8.84 - 8.81 (m, 1H), 8.32 - 8.28 (m, 1H), 8.12 - 8.08 (m, 1H), 8.03 - 8.00 (m, 1H), 7.92 - 7.88 (m, 1H), 7.80 - 7.76 (m, 1H), 7.11 - 7.09 (m, 1H), 4.05 (s, 3H), 3.72 (s, 3H); MS (ESI): mass calcd. for C₂₅H₁₈ClF₃N₆O₂, 526.1; m/z found, 527.1 [M+H]⁺.

### Example 11: {4-Chloro-2-methoxy-3-[3-(methylsuffonyl)phenyl]quinolin-6-yl)(1-methyl-1H-imidazol-5-yl)[6-(trifluoromethyl)pyridin-3-yl]methanol•TFA

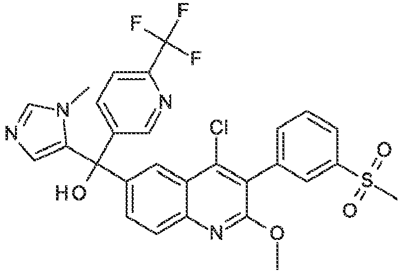

The title compound was prepared using (3-(methylsulfonyl)phenyl)boronic acid in place of (4-(methylsulfonyl)phenyl)boronic acid using the procedure described for Example 7a. ¹H NMR (400 MHz, CD₃OD) δ 9.02 (s, 1H), 8.84 - 8.81 (m, 1H), 8.30 - 8.27 (m, 1H), 8.12 - 8.07 (m, 1H), 8.06 - 8.03 (m, 1H), 8.00 (d, *J* = 8.8 Hz, 1H), 7.97 - 7.95 (m, 1H), 7.90 (d, *J* = 8.2 Hz, 1H), 7.79 - 7.71 (m, 3H), 7.10 - 7.07 (m, 1H), 4.02 (s, 3H), 3.72 (s, 3H), 3.18 (s, 3H); MS (ESI): mass calcd. for C₂₈H₂₂ClF₃N₄O₄S, 602.1; m/z found, 603.2 [M+H]⁺.

### Example 12: N-[3-(4-Chloro-6-fhydroxy(l-methyl-1H-imidazol-5-yl)[6-(trifluoromethyl)pyridin-3-yl]methyl}-2-methoxyquinolin-3-yl)phenyl]methanesulfonamide•TFA

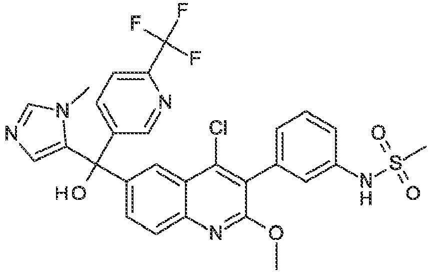

The title compound was prepared using (3-(methylsulfonamido)phenyl)boronic acid in place of (4-(methylsulfonyl)phenyl)boronic acid using the procedure described for Example 7a. ¹H NMR (400 MHz, CD₃OD) δ 9.03 (s, 1H), 8.85 - 8.81 (m, 1H), 8.27 - 8.24 (m, 1H), 8.11 - 8.06 (m, 1H), 7.99 (d, *J* = 8.7 Hz, 1H), 7.90 (d, *J* = 8.3 Hz, 1H), 7.75 - 7.70 (m, 1H), 7.48 - 7.42 (m, 1H), 7.31 - 7.27 (m, 1H), 7.26 - 7.23 (m, 1H), 7.14 - 7.10 (m, 1H), 7.09 - 7.07 (m, 1H), 4.01 (s, 3H), 3.72 (s, 3H), 2.99 (s, 3H); MS (ESI): mass calcd. for C₂₈H₂₃ClF₃N₅O₄S, 617.1; m/z found, 618.0 [M+H]⁺.

### Example 13: {4-Chloro-2-methoxy-3-[5-(methylsuflonyl)pyridin-3-yl]quinolin-6-yl)(1-methyl-1H-imidazol-5-yl)[6-(trifluoromethyl)pyridin-3-yl]methanol•TFA

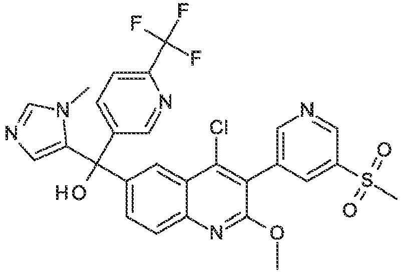

The title compound was prepared using (5-(methylsulfonyl)pyridin-3-yl)boronic acid in place of (4-(methylsulfonyl)phenyl)boronic acid using the procedure described for Example 7a. ¹H NMR (400 MHz, CD₃OD) δ 9.19 - 9.15 (m, 1H), 9.04 (s, 1H), 8.91 - 8.87 (m, 1H), 8.82 (d, *J* = 2.2 Hz, 1H), 8.46 - 8.43 (m, 1H), 8.31 (d, *J* = 2.1 Hz, 1H), 8.13 - 8.08 (m, 1H), 8.02 (d, *J* = 8.8 Hz, 1H), 7.90 (d, *J* = 8.3 Hz, 1H), 7.80 - 7.76 (m, 1H), 7.12 - 7.09 (m, 1H), 4.05 (s, 3H), 3.72 (s, 3H), 3.28 (s, 3H); MS (ESI): mass calcd. for C₂₇H₂₁ClF₃N₅O₄S, 603.1; m/z found, 604.0 [M+H]⁺.

### Example 14a: [4-Chloro-3-(5-chlorothiophen-2-yl)-2-methoxyquinofin-6-yl](1-methyl-1H-imidazol-5-yl)[6-(trifluoromethyl)pyridin-3-yl]methanol•TFA

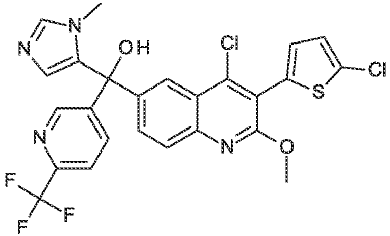

A mixture of 4-chloro-6-(hydroxy(1-methyl-1*H*-imidazol-5-yl)(6-(trifluoromethyl)pyridin-3-yl)methyl)-2-methoxyquinolin-3-yl trifluoromethanesulfonate (40 mg, 0.067 mmol, Intermediate 11: step g), (5-chlorothiophen-2-yl)boronic acid (16 mg, 0.1 mmol), PdCl₂(dppf) (5 mg, 0.007 mmol) and K₂CO₃ (9 mg, 0.067 mmol) was sparged with nitrogen three times. To this mixture was added 1,4-dioxane (1.2 mL) and water (0.17 mL) and the suspension purged with nitrogen. The resulting solution was heated to 65 °C for 15 hours. The mixture was then heated to 75 °C for 6 hours after which it was allowed to cool to room temperature. The solution was diluted with EtOAc, washed with water, dried (MgSO₄), filtered and concentrated to dryness. The crude material was purified by reverse-phase HPLC (acetonitrile / water + TFA) to provide the title compound. ¹H NMR (400 MHz, CD₃OD) δ 9.03 (s, 1H), 8.84 - 8.81 (m, 1H), 8.26 (d, *J* = 2.2 Hz, 1H), 8.11 - 8.06 (m, 1H), 7.96 (d, *J* = 8.7 Hz, 1H), 7.90 (d, *J* = 8.2 Hz, 1H), 7.74 - 7.71 (m, 1H), 7.09 - 7.04 (m, 3H), 4.06 (s, 3H), 3.71 (s, 3H); MS (ESI): mass calcd. for C₂₅H₁₇Cl₂F₃N₄O₂S, 564.0; m/z found, 565.0 [M+H]⁺.
[4-Chloro-3-(5-chlorothiophen-2-yl)-2-methoxyquinolin-6-yl](1-methyl-1*H*-imidazol-5-yl)[6-(trifluoromethyl)pyridin-3-yl]methanol was purified by HPLC (Kromasil C18 100 Å, 5 µM column, Mobile phase: 35-100% acetonitrile/water + 0.25% ammonium bicarbonate) to give 2 enantiomers. The first eluting enantiomer was **Example 14b:** ¹H NMR (400 MHz, CD₃OD) δ 8.79 - 8.77 (m, 1H), 8.21 (d, *J=* 2.0 Hz, 1H), 8.04 - 8.00 (m, 1H), 7.90 (d, *J* = 8.8 Hz, 1H), 7.84 (d, *J* = 8.2 Hz, 1H), 7.74 (s, 1H), 7.72 (dd, *J=* 8.8, 2.1 Hz, 1H), 7.08 - 7.06 (m, 1H), 7.05 - 7.02 (m, 1H), 6.36 - 6.34 (m, 1H), 4.05 (s, 3H), 3.48 (s, 3H); MS (ESI): mass calcd. for C₂₅H₁₇Cl₂F₃N₄O₂S, 564.0; m/z found, 565.0 [M+H]⁺ and the second eluting enantiomer was **Example 14c:** ¹H NMR (400 MHz, CD₃OD) δ 8.80 - 8.76 (m, 1H), 8.21 (d, *J* = 2.1 Hz, 1H), 8.05 - 8.00 (m, 1H), 7.91 (d, *J* = 8.8 Hz, 1H), 7.84 (d, *J* = 8.2 Hz, 1H), 7.75 (s, 1H), 7.74 - 7.70 (m, 1H), 7.08 - 7.06 (m, 1H), 7.06 - 7.03 (m, 1H), 6.37 - 6.34 (m, 1H), 4.05 (s, 3H), 3.48 (s, 3H); MS (ESI): mass calcd. for C₂₅H₁₇Cl₂F₃N₄O₂S, 564.0; m/z found, 565.0 [M+H]⁺.

### Example 15: [4-Chloro-2-methoxy-3-(2-methoxypyrimidin-5-yl)quinolin-6-yl](1-methyl-1H-imidazol-5-yl)[6-(trifluoromethyl)pyridin-3-yl]methanol•TFA

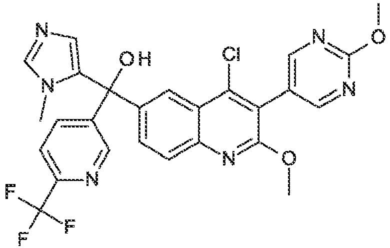

The title compound was prepared using (2-methoxypyrimidin-5-yl)boronic acid in place of (5-chlorothiophen-2-yl)boronic acid using the procedure described for Example 14a. ¹H NMR (400 MHz, CD₃OD) δ 9.05 - 9.02 (m, 1H), 8.84 - 8.80 (m, 1H), 8.63 (s, 2H), 8.28 (d, *J* = 2.2 Hz, 1H), 8.12 - 8.08 (m, 1H), 8.00 (d, *J* = 8.7 Hz, 1H), 7.90 (d, *J* = 8.3 Hz, 1H), 7.78 - 7.73 (m, 1H), 7.11 - 7.08 (m, 1H), 4.09 (s, 3H), 4.05 (s, 3H), 3.72 (s, 3H); MS (ESI): mass calcd. for C₂₆H₂₀ClF₃N₆O₃, 556.1; m/z found, 557.1 [M+H]⁺.

### Example 16:4-(4-Chloro-6-{hydroxy(1-methyl-1H-imidazol-5-yl)[6-(trifluoromethyl)pyridin-3-yl]methyl}-2-methoxyquinolin-3-yl)benzonitrile•TFA

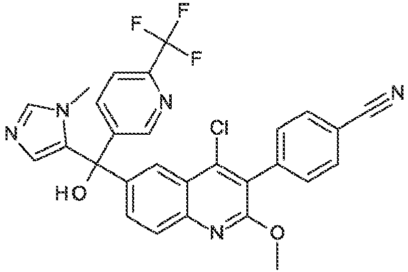

The title compound was prepared using (4-cyanophenyl)boronic acid in place of (5-chlorothiophen-2-yl)boronic acid using the procedure described for Example 14a. ¹H NMR (400 MHz, CD₃OD) δ 9.05 - 9.03 (m, 1H), 8.84 - 8.80 (m, 1H), 8.27 (d, *J* = 2.3 Hz, 1H), 8.11 - 8.07 (m, 1H), 8.00 (d, *J* = 8.7 Hz, 1H), 7.90 (d, *J* = 8.3 Hz, 1H), 7.87 - 7.83 (m, 2H), 7.74 (dd, *J* = 8.8, 2.2 Hz, 1H), 7.57 - 7.53 (m, 2H), 7.10 - 7.07 (m, 1H), 4.00 (s, 3H), 3.72 (s, 3H); MS (ESI): mass calcd. for C₂₈H₁₉ClF₃N₅O₂, 549.1; m/z found, 550.2 [M+H]⁺.

### Example 17: N-[4-(4-Chloro-6-{hydroxy(1-methyl-1H-imidazol-5-yl)[6-(trifluoromethyl)pyridin-3-yl]methyl}-2-methoxyquinolin-3-yl)phenyl]methamesulfonamide•TFA

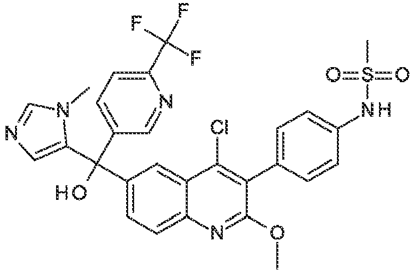

The title compound was prepared using (4-(methylsulfonamido)phenyl)boronic acid in place of (5-chlorothiophen-2-yl)boronic acid using the procedure described for Example 14a. ¹H NMR (400 MHz, CD₃OD) δ 9.05 - 9.03 (m, 1H), 8.84 - 8.81 (m, 1H), 8.25 (d, *J* = 2.1 Hz, 1H), 8.11 - 8.06 (m, 1H), 7.97 (d, *J* = 8.6 Hz, 1H), 7.90 (d, *J* = 8.3 Hz, 1H), 7.73 - 7.69 (m, 1H), 7.37 - 7.30 (m, 4H), 7.09 - 7.06 (m, 1H), 4.00 (s, 3H), 3.72 (s, 3H), 3.04 (s, 3H); MS (ESI): mass calcd. for C₂₈H₂₃ClF₃N₅O₄S, 617.1; m/z found, 618.2 [M+H]⁺.

### Example 18a: 6-(Hydroxy(1-methyl-1H-imidazol-5-yl)(pyridin-2-yl)methyl)-3-phenylquinoline-2,4-dicarbonitrile

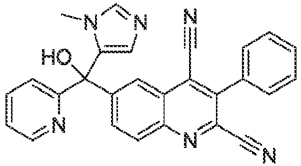

(2,4-Dichloro-3-phenylquinolin-6-yl)(1-methyl-1*H*-imidazol-5-yl)(pyridin-2-yl)methanol (227.8 mg, 0.494 mmol, Example 64), zinc cyanide (73.8 mg, 0.628 mmol), zinc dust (8.8 mg, 0.135 mmol), dppf (27.7 mg, 0.05 mmol), and Pd₂(dba)₃ (22.6 mg, 0.0247 mmol) were charged to an oven-dried microwave vial. The vial was evacuated and back-filled with nitrogen. Dimethylacetamide (3 mL) was added to the mixture via syringe. Nitrogen was bubbled through the reaction mixture for 5 minutes and the mixture was stirred and heated at 120 °C for 1 hour, followed by 100 °C for 3 hours under a positive pressure of nitrogen. The mixture was allowed to cool to ambient temperature, filtered through Celite®, and rinsed with ethyl acetate. The filtrate was basified with 5 M aqueous ammonium hydroxide, the layers were separated and the aqueous layer was extracted with excess ethyl acetate. The combined organic layers were dried (Na₂SO₄), filtered, and concentrated to dryness. Due to an incomplete reaction as shown by LCMS (starting material and mono-cynated product present), the crude material was subjected to additional reaction conditions. In an oven-dried microwave vial, the dry crude material was mixed with zinc cyanide (80.1 mg, 0.682 mmol), zinc dust (8.7 mg, 0.133 mmol), X-Phos (32.7 mg, 0.0665 mmol), and Pd₂(dba)₃ (62.2 mg, 0.0679 mmol). The vial was evacuated and back-filled with nitrogen. Dimethylacetamide (3.4 mL) was added to the mixture via syringe. Nitrogen was bubbled through the reaction mixture for 5 minutes and the mixture was stirred and heated at 120 °C for 1.25 hours under a positive pressure of nitrogen. The mixture was allowed to cool to ambient temperature, filtered through Celite®, and rinsed with ethyl acetate. The filtrate was basified with 5 M aqueous ammonium hydroxide, the layers were separated and the aqueous layer was extracted with excess ethyl acetate. The combined organic layers were dried (Na₂SO₄), filtered, and concentrated to dryness. The crude product was purified by flash column chromatography (silica gel, 0-5% MeOH-DCM) followed by reverse phase chromatography (acetonitrile/H₂O + 0.05% TFA). Product fractions were basified with saturated aqueous sodium bicarbonate and extracted with DCM, before being dried (Na₂SO₄), filtered, and concentrated to provide the title compound. ¹H NMR (500 MHz, CDCl₃) δ 8.66 (d, *J* = 4.5 Hz, 1H), 8.41 (d, *J* = 1.8 Hz, 1H), 8.27 (d, *J* = 8.9 Hz, 1H), 8.06 (dd, *J* = 8.9, 1.9 Hz, 1H), 7.78 (td, *J* = 7.7, 1.7 Hz, 1H), 7.64 - 7.58 (m, 5H), 7.48 (s, 1H), 7.38 - 7.35 (m, 1H), 7.30 (d, *J* = 7.9 Hz, 1H), 6.84 (s, 1H), 6.40 (s, 1H), 3.41 (s, 3H); MS m/e 443.2 [M+H]⁺.
6-(Hydroxy(1-methyl-1*H*-imidazol-5-yl)(pyridin-2-yl)methyl)-3-phenylquinoline-2,4-dicarbonitrile was purified by chiral SFC (ChiralPak AD, 100 % ethanol) to provide two pure enantiomers. The first eluting enantiomer was **Example 18b:** ¹H NMR (500 MHz, CDCl₃) δ 8.66 (d, *J* = 4.3 Hz, 1H), 8.41 (d, *J* = 1.7 Hz, 1H), 8.28 (d, *J* = 8.9 Hz, 1H), 8.06 (dd, *J* = 8.9, 1.9 Hz, 1H), 7.78 (td, *J* = 7.7, 1.7 Hz, 1H), 7.66 - 7.57 (m, 5H), 7.51 (s, 1H), 7.37 (ddd, *J* = 7.5, 4.9, 0.8 Hz, 1H), 7.30 (d, *J* = 7.9 Hz, 1H), 6.82 (s, 1H), 6.41 (s, 1H), 3.42 (s, 3H); MS m/e 443.2 [M+H]⁺. The second eluting enantiomer was **Example 18c:** ¹H NMR (500 MHz, CDCl₃) δ 8.66 (d, *J* = 4.8 Hz, 1H), 8.41 (d, *J* = 1.8 Hz, 1H), 8.28 (d, *J* = 8.9 Hz, 1H), 8.06 (dd, *J* = 8.9, 1.9 Hz, 1H), 7.78 (td, *J* = 7.7, 1.6 Hz, 1H), 7.66 - 7.58 (m, 5H), 7.48 (s, 1H), 7.37 (dd, *J* = 7.1, 5.2 Hz, 1H), 7.29 (d, *J* = 7.9 Hz, 1H), 6.79 (s, 1H), 6.40 (s, 1H), 3.41 (s, 3H); MS m/e 443.2 [M+H]⁺.

### Example 19a: 6-(Hydroxy(1-methyl-1H-imidazol-5-yl)(6-(trifluoromethyl)pyridin-3-yl)methyl)-2-methoxy-8-methyl-3-(4-(trifluoromethoxy)phenyl)quinoline-4-carbonitrile

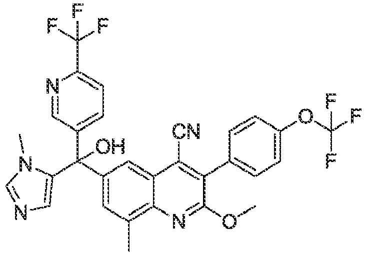

To a round bottom flask containing (4-chloro-2-methoxy-8-methyl-3-(4-(trifluoromethoxy)phenyl)quinolin-6-yl)(1-methyl- 1*H*-imidazol-5-yl)(6-(trifluoromethyl)pyridin-3-yl)methanol (0.19 g, 0.305 mmol, Example 63) was added Zn(CN)₂ (64.47 mg, 0.596 mmol), Pd₂(dba)₃ (41.9 mg, 0.046 mmol), zinc nanopowder (5.9 mg, 0.092 mmol), and dicyclohexyl(2',4',6'-triisopropyl-[1,1'-biphenyl]-2-yl)phosphine (X-Phos, 29.9 mg, 0.061 mmol). The flask was evacuated and re-filled with argon (3x). Dimethylacetamide (1.5 mL, degassed) was then added and the mixture was heated at 120 °C for 4 hours. The mixture was cooled to room temperature, diluted with EtOAc and filtered through Celite® rinsing with EtOAc. The filtrate was diluted with saturated aqueous NH₄Cl and H₂O and layers were separated. The aqueous layer was again extracted with EtOAc. The combined EtOAc extract was washed with brine, dried over Na₂SO₄, filtered and evaporated *in vacuo* to provide an oil. LC/MS showed product plus a significant amount of starting material. The mixture was re-subjected to the conditions above and heated in a 120 °C oil bath for an additional 4 hours. After work-up (same as above) the crude product was purified by chromatography (10% MeOH in DCM on a gradient) to provide the title compound.
6-(Hydroxy(1-methyl-1*H*-imidazol-5-yl)(6-(trifluoromethyl)pyridin-3-yl)methyl)-2-methoxy-8-methyl-3-(4-(trifluoromethoxy)phenyl)quinoline-4-carbonitrile was purified by chiral HPLC (Chiralpak (IC); solvent A = CO₂; solvent B = methanol + 0.2% IPA; flow rate 50 mL/min; wavelength 254 nM; temperature 40 degrees) to give 2 enantiomers. The first eluting enantiomer was **Example 19b:** ¹H NMR (400 MHz, CDCl₃) δ 8.74 - 8.87 (m, 1H), 8.06 - 8.16 (m, 1H), 7.91 - 8.02 (m, 1H), 7.65 - 7.74 (m, 1H), 7.54 - 7.65 (m, 2H), 7.42 - 7.52 (m, 2H), 7.32 - 7.41 (m, 2H), 6.44 - 6.60 (m, 1H), 4.09 (s, 3H), 3.28 - 3.51 (s, 3H), 2.67 (s, 3H); MS (ESI) 614 (M+H)⁺. The second eluting enantiomer was **Example 19c:** ¹H NMR (400 MHz, CDCl₃) δ 8.75 - 8.86 (m, 1H), 8.05 - 8.16 (m, 1H), 7.89 - 8.02 (m, 1H), 7.65 - 7.77 (m, 1H), 7.54 - 7.63 (m, 2H), 7.42 - 7.50 (m, 2H), 7.32 - 7.42 (m, 2H), 6.40 - 6.60 (m, 1H), 4.08 (s, 3H), 3.35 - 3.56 (broad s, 3H), 2.68 (s, 3H); MS (ESI) 614 (M+H)⁺.

### Example 20: 1-(4-((2,4-Dichloro-8-methyl-3-phenylquinolin-6-yl)(hydroxy)(phenyl)methyl)piperidin-1-yl)ethanone

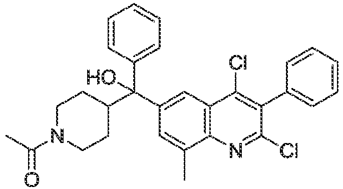

A solution of *n*-butyllithium (1.6 M in hexanes, 0.5 mL, 0.8 mmol) was added dropwise by syringe to a solution of 6-bromo-2,4-dichloro-8-methyl-3-phenylquinoline (0.32 g, 0.88 mmol, Intermediate 3) in dry deoxygenated THF (10 mL) at -78 °C. After 2 minutes, a solution of 1-(4-benzoylpiperidin-1-yl)ethanone (0.203 g, 0.88 mmol, Intermediate 4) in dry THF (4 mL) was added dropwise by syringe. An additional 2 mL of THF was used to complete the quantitative addition. After 10 minutes, the flask was removed from the dry-ice bath and placed into an ice-water bath. After 1 hour, the reaction was quenched with saturated aqueous ammonium chloride solution and the mixture was partitioned between water and EtOAc. The layers were separated and the aqueous phase was further extracted with EtOAc and washed with saturated aqueous NaCl solution. The organic phase was dried (MgSO₄), filtered, and concentrated to dryness. The crude product was purified by flash column chromatography (silica gel, 0-100% EtOAc-DCM) to provide the title compound. MS m/e 519.1 (M+H)⁺.

### Example 21a: 6-((1-Acetylpiperidin-4-yl)(hydroxy)(phenyl)methyl)-8-methyl-3-phenylquinoline-2,4-dicarbonitrile

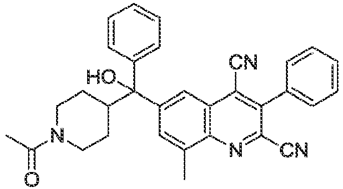

A microwave vial was charged with 1-(4-((2,4-dichloro-8-methyl-3-phenylquinolin-6-yl)(hydroxy)(phenyl)methyl)piperidin-1-yl)ethanone (240 mg, 0.46 mmol, Example 20), Zn(CN)₂ (55.7 mg, 0.480 mmol), Pd₂(dba)₃ (43.3 mg, 0.047 mmol), zinc dust (6.05 mg, 0.0926 mmol), and dicyclohexyl(2',4',6'-triisopropyl-[1,1'-biphenyl]-2-yl)phosphine (X-Phos, 22.1 mg, 0.046 mmol). Dimethylacetamide (2.4 mL) was then added and the mixture was sparged with nitrogen for 10 minutes and placed in a pre-heated aluminum block at 120 °C for 2 hours. The mixture was cooled to room temperature and was filtered through Celite®, and washed with EtOAc. The filtrate was washed sequentially with 2 M aqueous NH₄OH and aqueous NaCl solution. The organic phase was dried (MgSO₄), filtered, and concentrated. The residue was purified by flash column chromatography (silica gel, 0 to 100% EtOAc-DCM). MS (ESI) 501.3 (M+H)⁺.

6-((1-Acetylpiperidin-4-yl)(hydroxy)(phenyl)methyl)-8-methyl-3-phenylquinoline-2,4-dicarbonitrile was purified by chiral HPLC (Chiralcel OD, 80% heptane/20% ethanol) to give 2 enantiomers. The 2 enantiomers were further purified by reverse-phase HPLC (5-85% CH₃CN-H₂O, 0.05% TFA). The product was converted to the free base (neutralized with saturated aqueous NaHCO₃ and extracted with EtOAc) and the organic fractions were concentrated to afford the title compound. The first eluting enantiomer was **Example 21b:** ¹H NMR (600 MHz, CDCl₃, mixture of rotamers) δ ppm 8.35-8.32 (m, 1H), 7.84-7.81 (m, 1H), 7.66-7.59 (m, 5H), 7.58-7.55 (m, 2H), 7.41-7.37 (m, 2H), 7.31-7.24 (m, 1H), 4.75 (d, *J* = 13.4 Hz, 0.5H), 4.69 (d, *J* = 13.5 Hz, 0.5H), 3.89 (d, *J* = 13.7 Hz, 0.5H), 3.82 (d, *J* = 13.7 Hz, 0.5H), 3.16 (t, *J* = 12.2 Hz, 0.5H), 3.12-3.03 (m, 0.5H), 2.89-2.83 (m, 1H), 2.82 (s, 1.5H), 2.80 (s, 1.5H), 2.67-2.53 (m, 1H), 2.38 (s, 0.5H), 2.36 (s, 0.5H), 2.07 (s, 1.5H), 2.06 (s, 1.5H), 1.46-1.21 (m, 4H); MS m/e 501.2 [M+H]⁺ and the second eluting enantiomer was **Example 21c:** ¹H NMR (600 MHz, CDCl₃, mixture of rotamers) δ ppm 8.35-8.32 (m, 1H), 7.84-7.81 (m, 1H), 7.65-7.59 (m, 5H), 7.58-7.55 (m, 2H), 7.41-7.37 (m, 2H), 7.31-7.24 (m, 1H), 4.75 (d, *J* = 13.5 Hz, 0.5H), 4.69 (d, *J* = 13.5 Hz, 0.5H), 3.89 (d, *J* = 13.7 Hz, 0.5H), 3.82 (d, *J* = 13.6 Hz, 0.5H), 3.18-3.14 (m, 0.5H), 3.11-3.07 (m, 0.5H), 2.86-2.83 (m, 1H), 2.82 (s, 1.5H), 2.80 (s, 1.5H), 2.68-2.54 (m, 1H), 2.37 (s, 0.5H), 2.35 (s, 0.5H), 2.07 (s, 1.5H), 2.06 (s, 1.5H), 1.47-1.21 (m, 4H); MS m/e 501.2 [M+H]⁺.

### Example 22: (4-Chlorophenyl)(2,4-dichloro-8-methyl-3-phenylquinofin-6-yl)(1-methyl-1H-imidazol-5-yl)methanol

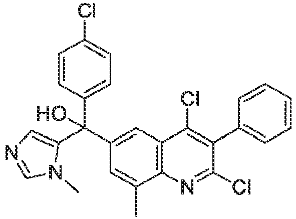

The title compound was prepared analogously to the method described in Example 20 using (4-chlorophenyl)(1-methyl-1*H*-imidazol-5-yl)methanone (Intermediate 8: step b) in place of 1-(4-benzoylpiperidin-1-yl)ethanone (Intermediate 4). MS m/e 508.1 (M+H)⁺.

### Example 23a: 6-((4-Chlorophenyl)(hydroxy)(1-methyl-1H-imidazol-5-yl)methyl)-8-methyl-3-phenylquinoline-2,4-dicarbonitrile

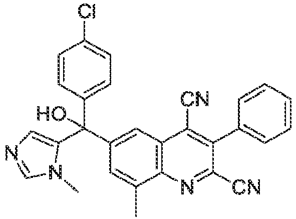

The title compound was prepared analogously to the method described in Example 21a using (4-chlorophenyl)(2,4-dichloro-8-methyl-3-phenylquinolin-6-yl)(1-methyl-1*H*-imidazo1-5-yl)methanol (Example 22) in place of 1-(4-((2,4-dichloro-8-methyl-3-phenylquinolin-6-yl)(hydroxy)(phenyl)methyl)piperidin-1-yl)ethanone (Example 20). MS m/e 490.2 (M+H)⁺.

6-((4-Chlorophenyl)(hydroxy)(1-methyl-1*H*-imidazol-5-yl)methyl)-8-methyl-3-phenylquinoline-2,4-dicarbonitrile was purified by chiral HPLC (Chiralcel OD, 50% heptane/20% ethanol) to give 2 enantiomers. The 2 enantiomers were further purified by reverse-phase HPLC (5-85% CH₃CN-H₂O, 0.05% TFA). The product was converted to the free base (neutralized with saturated aqueous NaHCO₃ and extracted with EtOAc) and the organic fractions were concentrated to afford the title compound. The first eluting enantiomer was **Example 23b:** ¹H NMR (600 MHz, CDCl₃) δ ppm 8.21 (s, 1H), 7.62-7.49 (m, 6H), 7.24 (s, 5H), 6.29 (s, 1H), 3.32 (s, 3H), 2.70 (s, 3H); MS m/e 490.2 [M+H]⁺ and the second eluting enantiomer was **Example 23c:** ¹H NMR (600 MHz, CDCl₃) δ ppm 8.29 (d, *J* = 1.5 Hz, 1H), 7.67-7.56 (m, 6H), 7.35-7.28 (m, 4H), 7.14 (s, 1H), 6.27 (s, 1H), 3.35 (s, 3H), 2.75 (s, 3H); MS m/e 490.2 [M+H]⁺.

### Example 24: (2,4-Dichloro-8-methyl-3-phenylquinolin-6-yl)(1-methyl-1H-imidazol-5-yl)(6-(trifluoromethyl)pyridin-3-yl)methanol

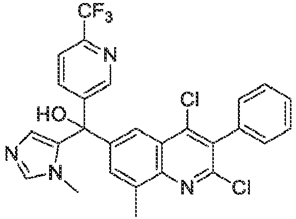

The title compound was prepared analogously to the method described in Example 20 using (1-methyl-1*H*-imidazol-5-yl)(6-(trifluoromethyl)pyridin-3-yl)methanone (Intermediate 2: step c) in place of 1-(4-benzoylpiperidin-1-yl)ethanone (Intermediate 4). MS m/e 543.1 (M+H)⁺.

### Example 25a: 6-(Hydroxy(1-methyl-1H-imidazol-5-yl)(6-(trifluoromethyl)pyridin-3-yl)methyl)-8-methyl-3-phenylquinoline-2,4-dicarbonitrile

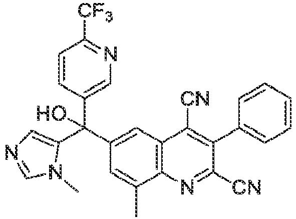

The title compound was prepared analogously to the method described in Example 21a using (2,4-dichloro-8-methyl-3-phenylquinolin-6-yl)(1-methyl-1*H*-imidazol-5-yl)(6-(trifluoromethyl)pyridin-3-yl)methanol (Example 24) in place of 1-(4-((2,4-dichloro-8-methyl-3-phenylquinolin-6-yl)(hydroxy)(phenyl)methyl)piperidin-1-yl)ethanone (Example 20). MS m/e 525.2 (M+H)⁺.

6-(Hydroxy(1-methyl-1*H*-imidazol-5-yl)(6-(trifluoromethyl)pyridin-3-yl)methyl)-8-methyl-3-phenylquinoline-2,4-dicarbonitrile was purified by chiral HPLC (Chiralcel OD, 80% heptane/20% ethanol) to give 2 enantiomers. The first eluting enantiomer was **Example 25b:** ¹H NMR (600 MHz, CDCl₃) δ ppm 8.81 (d, *J* = 2.1 Hz, 1H), 8.33 (d, *J* = 1.9 Hz, 1H), 7.98 (dd, *J* = 8.3, 2.2 Hz, 1H), 7.71 (d, *J* = 8.2 Hz, 1H), 7.66-7.59 (m, 6H), 7.37 (s, 1H), 6.45 (s, 1H), 5.29 (s, 1H), 3.41 (s, 3H), 2.80 (s, 3H); MS m/e 525.2 (M+H)⁺ and the second eluting enantiomer was **Example 25c:** ¹H NMR (600 MHz, CDCl₃) δ ppm 8.80 (d, *J* = 2.1 Hz, 1H), 8.33 (dd, *J* = 2.2, 0.7 Hz, 1H), 7.97 (dd, *J* = 8.3, 2.2 Hz, 1H), 7.70 (dd, *J* = 8.2, 0.8 Hz, 1H), 7.67-7.58 (m, 6H), 7.31 (s, 1H), 6.38 (d, *J* = 1.2 Hz, 1H), 5.94 (s, 1H), 3.39 (s, 3H), 2.79 (s, 3H); MS m/e 525.2 (M+H)⁺.

### Example 26: 1-(4-((2,4-Dichloro-8-methyl-3-phenylquinolin-6-yl)(hydroxy)(6-(trifluoromethyl)pyridin-3-yl)methyl)piperidin-1-yl)ethanone

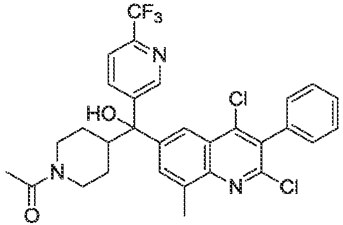

The title compound was prepared analogously to the method described in Example 20 using 1-(4-(6-(trifluoromethyl)nicotinoyl)piperidin-1-yl)edianone (Intermediate 14: step d) in place of 1-(4-benzoylpiperidin-1-yl)ethanone (Intermediate 4). MS m/e 588.2 (M+H)⁺.

### Example 27a: 6-((1-Acetylpiperidin4-yl)(hydroxy)(6-(trifluoromethyl)pyridin-3-yl)methyl)-8-methyl-3-phenylquinoline-2,4-dicarbonitrile

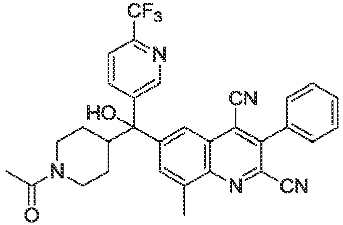

The title compound was prepared analogously to the method described in Example 21a using 1-(4-((2,4-dichloro-8-methyl-3-phenylquinolin-6-yl)(hydroxy)(6-(trifluoromethyl)pyridin-3-yl)methyl)piperidin-1-yl)ethanone (Example 26). MS m/e 570.3 (M+H)⁺.

6-((1-Acetylpiperidin-4-yl)(hydroxy)(6-(trifluoromethyl)pyridin-3-yl)methyl)-8-methyl-3-phenylquinoline-2,4-dicarbonitrile was purified by chiral SFC (Chiralpak AD-H, 5 µm, 250 x 20 mm, mobile phase: 75% CO₂, 25% mixture of methanol-isopropanol 50/50 v/v). The first eluting enantiomer was **Example 27b:** ¹H NMR (600 MHz, CDCl₃, mixture of rotamers) δ ppm 8.98-8.94 (m, 1H), 8.41-8.32 (m, 1H), 8.16-8.13 (m, 1H), 7.87 (s, 0.5H), 7.79 (s, 0.5H), 7.71-7.66 (m, 1H), 7.65-7.56 (m, 5H), 4.74-4.68 (m, 1H), 3.96-3.64 (m, 2H), 3.21-3.08 (m, 1H), 2.93-2.88 (m, 1H), 2.84 (s, 1.5H), 2.83 (s, 1.5H), 2.65-2.60 (m, 1H), 2.03 (s, 1.5H), 2.02 (s, 1.5H), 1.58-1.34 (m, 4H); MS m/e 570.3 (M+H)⁺ and the second eluting enantiomer was **Example 27c:** ¹H NMR (600 MHz, CDCl₃, mixture of rotamers) δ ppm 9.01-8.93 (m, 1H), 8.39-8.36 (m, 1H), 8.16-8.13 (m, 1H), 7.87 (s, 0.5H), 7.79 (s, 0.5H), 7.69-7.67 (m, 1H), 7.66-7.58 (m, 5H), 4.74-4.68 (m, 1H), 3.97-3.64 (m, 2H), 3.22-3.06 (m, 1H), 2.93-2.88 (m, 1H), 2.84 (s, 1.5H), 2.83 (s, 1.5H), 2.70-2.53 (m, 1H), 2.03 (s, 1.5H), 2.02 (s, 1.5H) 1.58-1.34 (m, 4H); MS m/e 570.3 (M+H)⁺.

### Example 28: 6-((4-Cyanophenyl)(hydroxy)(1-methyl-1H-imidazol-5-yl)methyl)-3-phenyl-2-(trifluoromethyl)quinoline-4-carbonitrile•TFA

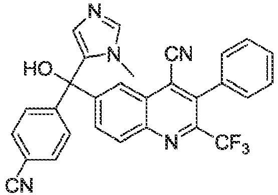

The title compound was isolated from the reaction that formed Example 31a. ¹H NMR (400 MHz, CD₃OD) δ 9.04 (s, 1H), 8.40 (d, *J* = 9.09 Hz, 1H), 8.34 (d, *J=* 2.02 Hz, 1H), 8.06 (dd, *J=* 2.27, 8.84 Hz, 1H), 7.84 (d, *J* = 8.59 Hz, 2H), 7.70 (d, *J* = 8.59 Hz, 2H), 7.54 - 7.60 (m, 3H), 7.43 - 7.49 (m, 2H), 7.09 (d, *J=* 1.52 Hz, 1H), 3.70 (s, 3H); MS m/e 510.1 [M+H]⁺.

### Example 29: 1-(4-((4-Chloro-3-phenyl-2-(trifluoromethyl)quinolin-6-yl)(hydroxy)(pyridin-3-yl)methyl)piperidin-1-yl)ethanone•TFA

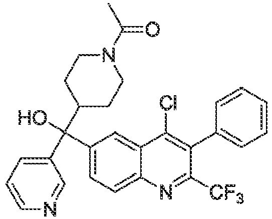

A mixture of (4-chloro-3-phenyl-2-(trifluoromethyl)quinolin-6-yl)(piperidin-4-yl)(pyridin-3-yl)methanol (198 mg, 0.400 mmol, Example 70), acetyl chloride (0.060 mL, 0.84 mmol), and Et₃N (0.16 mL, 1.15 mmol) in CH₂Cl₂ (4 mL) was stirred at room temperature for 1.5 hours and then concentrated to dryness. The residue was purified by reverse phase HPLC (water/acetonitrile/0.1% TFA) to provide the title compound as a white solid. ¹H NMR (400 MHz, CD₃OD) δ 9.13 (s, 1H), 8.82 (t, *J* = 8.08 Hz, 1H), 8.69 - 8.75 (m, 2H), 8.27 (d, *J* = 9.09 Hz, 1H), 8.18 - 8.24 (m, 1H), 7.94 - 8.02 (m, 1H), 7.49 - 7.55 (m, 3H), 7.26 - 7.34 (m, 2H), 4.55 - 4.65 (m, 1H), 3.91 - 4.05 (m, 1H), 3.15 - 3.27 (m, 2H), 2.67 - 2.77 (m, 1H), 2.08 (d, *J* = 4.04 Hz, 3H), 1.52 (m, 4H); MS m/e 540.3 [M+H]⁺.

### Example 30a: 6-((1-Acetylpiperidin4-yl)(hydroxy)(pyridin-3-yl)methyl)-3-phenyl-2-(trifluoromethyl)quinoline4-carbonitrile•TFA

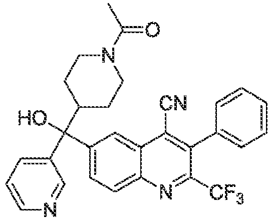

The title compound was prepared using 1-(4-((4-chloro-3-phenyl-2-(trifluoromethyl)quinolin-6-yl)(hydroxy)(pyridin-3-yl)methyl)piperidin-1-yl)ethanone-TFA (Example 29) in place of (4-chloro-3-phenyl-2-(trifluoromethyl)quinolin-6-yl)(4-chlorophenyl)(1-methyl-1*H*-imidazol-5-yl)methanol•TFA (Example 62) using the procedure described for Example 31a, with the exception that the reaction mixture was heated at 120 °C for 10 hours. ¹H NMR (400 MHz, CD₃OD) δ 9.17 (s, 1H), 8.87 - 8.93 (m, 1H), 8.74 (d, *J* = 5.56 Hz, 1H), 8.62 (dd, *J* = 2.02, 5.56 Hz, 1H), 8.37 (d, *J* = 9.09 Hz, 1H), 8.25 - 8.32 (m, 1H), 8.00 - 8.07 (m, 1H), 7.53 - 7.62 (m, 3H), 7.41 - 7.50 (m, 2H), 4.55 - 4.66 (m, 1H), 3.92 - 4.03 (m, 1H), 3.16 - 3.28 (m, 2H), 2.68 - 2.77 (m, 1H), 2.08 (d, *J* = 5.05 Hz, 3H), 1.41 - 1.67 (m, 4H); MS m/e 531.3 [M+H]⁺.
6-((1-Acetylpiperidin-4-yl)(hydroxy)(pyridin-3-yl)methyl)-3-phenyl-2-(trifluoromethyl)quinoline-4-carbonitrile was neutralized by partitioning between saturated NaHCO₃ aqueous solution and DCM (x1) followed by EtOAc (x3). The organic layer was dried (Na₂SO₄), filtered, concentrated to dryness, and purified by chiral HPLC (Chiralcel OD, CH₃CN) to give two pure enantiomers. **Example 30b:** (first enantiomer to elute off chiral column) ¹H NMR (400 MHz, CDCl₃, ∼1:1 mixture of rotamers) δ 8.77 - 8.94 (m, 1H), 8.54 (dd, *J* = 1.52,

12.13 Hz, 1H), 8.38 - 8.50 (m, 1H), 8.29 (t, *J* = 9.09 Hz, 1H), 8.07 (dd, *J* = 2.02, 9.09 Hz, 0.5H), 7.99 (dd, *J* = 2.02, 9.09 Hz, 0.5H), 7.95 (d, *J* = 8.08 Hz, 1H), 7.47 - 7.65 (m, 3H), 7.36 - 7.42 (m, 2H), 7.19 - 7.34 (m, 1H), 5.55 - 5.84 (m, 2H), 5.08 - 5.18 (m, 1H), 4.56 - 4.77 (m, 1H), 3.71 - 3.93 (m, 1H), 3.00 - 3.21 (m, 1H), 2.78 - 2.96 (m, 1H), 2.52 - 2.66 (m, 1H), 1.37 - 1.75 (m, 3H), 1.22 - 1.35 (m, 1H); MS m/e 531.3 [M+H]⁺. **Example 30c:** (second enantiomer to elute off chiral column) ¹H NMR (400 MHz, CDCl₃, ∼1:1 mixture of rotamers) δ 8.79 - 8.93 (m, 1H), 8.54 (dd, *J* = 1.52, 11.62 Hz, 1H), 8.40 - 8.50 (m, 1H), 8.29 (t, *J* = 8.34 Hz, 1H), 8.06 (dd, *J* = 2.02,9.09 Hz, 0.5H), 7.99 (dd, *J* = 2.02, 9.09 Hz, 0.5H), 7.94 (d, *J* = 8.08 Hz, 1H), 7.51 - 7.59 (m, 3H), 7.36 - 7.44 (m, 2H), 7.25 - 7.34 (m, 1H), 5.62 - 5.95 (m, 2H), 4.93 - 5.03 (m, 1H), 4.59 - 4.76 (m, 1H), 3.76 - 3.90 (m, 1H), 3.03 - 3.20 (m, 1H), 2.80 - 2.93 (m, 1H), 2.52 - 2.68 (m, 1H), 1.36 - 1.74 (m, 3H), 1.20 - 1.36 (m, 1H); MS m/e 531.3 [M+H]⁺.

### Example 31a: 6-((4-Chlorophenyo(hydroxy)(1-methyl-1H-imidazol-5-yl)methyl)-3-phenyl-2-(trifluoromethyl)quinoline4-carbonitrile•TFA

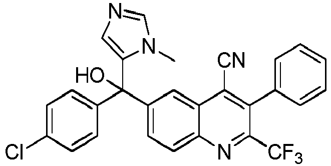

A pressure tube containing (4-chloro-3-phenyl-2-(trifluoromethyl)quinolin-6-yl)(4-chlorophenyl)(1-methyl-1*H*-imidazol-5-yl)methanol•TFA (145 mg, 0.190 mmol, Example 62), Pd₂(dba)₃ (18 mg, 0.020 mmol), dicyclohexyl(2',4',6'-triisopropyl-[1,1'-biphenyl]-2-yl)phosphine (X-Phos, 10 mg, 0.021 mmol), zinc cyanide (11.5 mg, 0.098 mmol), and zinc nanopowder (2.5 mg, 0.038 mmol) in *N,N*-dimethylacetamide (1 mL) was sparged with nitrogen for 8 minutes, and then heated at 120 °C for 2.5 hours. The mixture was allowed to cool to room temperature and filtered through a syringe filter. The filtrate was concentrated *in vacuo,* EtOAc and saturated NH₄Cl aqueous solution were added. The organic layer was separated, and the aqueous layer was extracted with EtOAc. The combined organic layers were dried (Na₂SO₄), filtered, and concentrated to dryness. The residue was purified by reverse phase HPLC (water/acetonitrile/0.1 % TFA) to provide the title compound as a white solid. ¹H NMR (400 MHz, CD₃OD) δ 9.02 (s, 1H), 8.39 (d, *J* = 8.59 Hz, 1H), 8.34 (d, *J* = 2.02 Hz, 1H), 8.05 (dd, *J* = 2.27, 8.84 Hz, 1H), 7.52 - 7.63 (m, 3H), 7.42 - 7.50 (m, 6H), 7.04 (s, 1H), 3.71 (s, 3H); MS m/e 519.2 [M+H]⁺.

6-((4-Chlorophenyl)(hydroxy)(1-methyl-1*H*-imidazol-5-yl)methyl)-3-phenyl-2-(trifluoromethyl)quinoline-4-carbonitrile was neutralized by partitioning between saturated NaHCO₃ aqueous solution and DCM. The organic layer was dried (Na₂SO₄), filtered, concentrated to dryness, and purified by chiral HPLC (Chiralcel OD, 90% heptanes/10% EtOH) to give two pure enantiomers. **Example 31b:** (first enantiomer to elute off chiral column) ¹H NMR (400 MHz, CDCl₃) δ 8.46 (s, 1H), 8.23 (d, *J* = 9.09 Hz, 1H), 7.78 (dd, *J* = 2.02, 9.09 Hz, 1H), 7.52 - 7.62 (m, 3H), 7.36 - 7.43 (m, 2H), 7.28 - 7.36 (m, 4H), 7.18 - 7.28 (m, 1H), 6.29 (s, 1H), 3.37 (s, 3H); MS m/e 519.2 [M+H]⁺. **Example 31c:** (second enantiomer to elute off chiral column) ¹H NMR (400 MHz, CDCl₃) δ 8.45 (s, 1H), 8.23 (d, *J* = 9.09 Hz, 1H), 7.78 (d, *J* = 9.09 Hz, 1H), 7.50 - 7.64 (m, 3H), 7.35 - 7.43 (m, 2H), 7.27 - 7.35 (m, 4H), 7.19 - 7.28 (m, 1H), 6.28 (s, 1H), 3.37 (s, 3H); MS m/e 519.2 [M+H]⁺.

### Example 32a: (4-Methoxy-3-phenyl-2-(trifluoromethyl)quinolin-6-yl)(1-methyl-1H-imidazol-5-yl)(pyridin-2-yl)methanol•TFA (Reference)

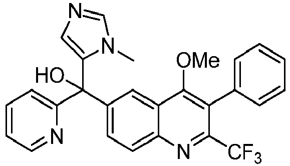

A mixture of (4-chloro-3-phenyl-2-(trifluoromethyl)quinolin-6-yl)(1-methyl-1*H*-imidazol-5-yl)(pyridin-2-yl)methanol•TFA (53 mg, 0.073 mmol, Example 61) and 0.5 M NaOMe in MeOH (0.80 mL, 0.40 mmol) in a sealed tube was heated at 70 °C for 2 hours. The solvent was evaporated, and water was added. The mixture was extracted with EtOAc, dried (Na₂SO₄), filtered, and concentrated. The residue was purified by reverse phase HPLC (water/acetonitrile/0.1% TFA) to provide the title compound as a white solid. ¹H NMR (400 MHz, CD₃OD) δ 8.96 (s, 1H), 8.62 (d, *J* = 4.04 Hz, 1H), 8.41 (d, *J* = 2.02 Hz, 1H), 8.19 (d, *J* = 9.09 Hz, 1H), 8.02 (dd, *J* = 2.02, 9.09 Hz, 1H), 7.93 (td, *J* = 2.02, 8.08 Hz, 1H), 7.79 (d, *J* = 8.08 Hz, 1H), 7.47 - 7.52 (m, 3H), 7.36 - 7.45 (m, 3H), 7.08 (d, *J* = 1.52 Hz, 1H), 3.63 (s, 3H), 3.55 (s, 3H); MS m/e 491.2 [M+H]⁺.

The racemate was neutralized by partitioning between saturated NaHCO₃ aqueous solution and DCM. The organic layer was dried (Na₂SO₄), filtered, concentrated to dryness, and purified by chiral HPLC (Chiralcel OD, 90% heptanes/10% EtOH) to give two enantiomers. The first eluting enantiomer was **Example 32b:** ¹H NMR (400 MHz, CDCl₃) δ 8.64 (d, *J* = 4.55 Hz, 1H), 8.18 - 8.24 (m, 2H), 7.88 (dd, *J* = 2.02, 9.09 Hz, 1H), 7.72 (td, *J* = 1.52, 7.58 Hz, 1H), 7.50 (s, 1H), 7.43 - 7.49 (m, 3H), 7.30 - 7.40 (m, 3H), 7.23 (d, *J* = 8.08 Hz, 1H), 6.37 (s, 1H), 3.50 (s, 3H), 3.44 (s, 3H); MS m/e 491.2 [M+H]⁺.

### Example 33a: (4-Chlorophenyl)(4-methoxy-3-phenyl-2-(trifluoromethyl)quinolin-6-yl)(1-methyl-1H-imidazol-5-yl)methanol•TFA (Reference)

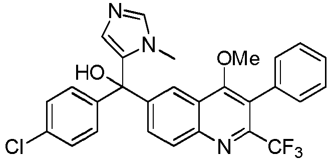

A mixture of (4-chloro-3-phenyl-2-(trifluoromethyl)quinolin-6-yl)(4-chlorophenyl)(1-methyl-1*H-*imidazol-5-yl)methanol•TFA (80 mg, 0.11 mmol, Example 62) and 0.5 M NaOMe in MeOH (0.50 mL, 0.25 mmol) in a sealed tube was heated at 70 °C for 7 hours. More 0.5 M NaOMe in MeOH (0.35 mL, 0.18 mmol) was added and the mixture was heated at the same temperature for another hour. The solvent was evaporated, and DMSO was added. After filtering through a syringe filter, the filtrate was purified by reverse phase HPLC (water/acetonitrile/0.1% TFA) to provide the title compound as a white solid. ¹H NMR (400 MHz, CD₃OD) δ 9.00 (s, 1H), 8.25 (d, *J* = 2.02 Hz, 1H), 8.23 (d, *J* = 9.09 Hz, 1H), 7.88 (dd, *J* = 2.02, 9.09 Hz, 1H), 7.47 - 7.52 (m, 4H), 7.41 - 7.47 (m, 3H), 7.36 - 7.41 (m, 2H), 6.97 (s, 1H), 3.71 (s, 3H), 3.53 (s, 3H); MS m/e 524.3 [M+H]⁺. (4-Chlorophenyl)(4-methoxy-3-phenyl-2-(trifluoromethyl)quinolin-6-yl)(1-methyl-1*H*-imidazol-5-yl)methanol was neutralized by partitioning between saturated NaHCO₃ aqueous solution and DCM. The organic layer was dried (Na₂SO₄), filtered, concentrated to dryness, and purified by chiral HPLC (Chiralpak OJ, 100% MeOH) to give two enantiomers. The first eluting enantiomer was **Example 33b:** ¹H NMR (400 MHz, CDCl₃) δ 8.14 - 8.20 (m, 2H), 7.77 (dd, *J* = 2.27, 8.84 Hz, 1H), 7.44 - 7.55 (m, 3H), 7.34 - 7.40 (m, 2H), 7.29 - 7.34 (m, 4H), 7.24 - 7.29 (m, 1H), 6.34 (br. s., 1H), 3.48 (s, 3H), 3.36 (s, 3H); MS m/e 524.3 [M+H]⁺ and the second eluting enantiomer was **Example 33c:** ¹H NMR (400 MHz, CDCl₃) δ 8.14 - 8.20 (m, 2H), 7.77 (dd, *J* = 2.02, 9.09 Hz, 1H), 7.44 - 7.50 (m, 3H), 7.36 (d, *J* = 4.04 Hz, 2H), 7.29 - 7.34 (m, 4H), 7.23 - 7.29 (m, 1H), 6.32 - 6.36 (m, 1H), 3.48 (s, 3H), 3.37 (s, 3H); MS m/e 524.1 [M+H]⁺.

### Example 34a: 1-(4-((4-Chloro-3-phenyl-2-(trifluoromethyl)quinolin-6-yl)(hydroxy)(1-methyl-1H-imidazol-5-yl)methyl)piperidin-1-yl)ethanone

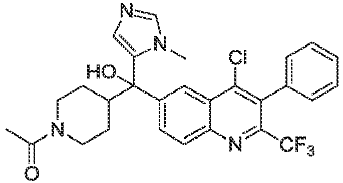

The racemic title compound was prepared using 1-(4-(1-methyl-1*H*-imidazole-5-carbonyl)piperidin-1-yl)ethanone (Intermediate 1: step c) in place of (4-chlorophenyl)(1-methyl-1*H*-imidazol-5-yl)methanone (Intermediate 8: step b) using the procedure described for Example 62, with the exception that the reaction was carried out at -78 °C all the time. ¹H NMR (400 MHz, CD₃OD, ∼1:1 mixture of retainers) δ 8.86 (s, 1H), 8.56 (d, *J* = 4.04 Hz, 1H), 8.27 (d, *J* = 9.09 Hz, 1H), 8.09 (s, 1H), 7.81 (d, *J* = 9.09 Hz, 1H), 7.45 - 7.58 (m, 3H), 7.31 (d, *J* = 2.53 Hz, 2H), 4.66 (d, *J* = 13.14 Hz, 0.5H), 4.46 (d, *J* = 12.63 Hz, 0.5H), 4.04 (d, *J* = 13.64 Hz, 0.5H), 3.84 (d, *J* = 13.64 Hz, 0.5H), 3.60 (s, 1.5H), 3.58 (s, 1.5H), 3.25 - 3.30 (overlap with solvent, m, 0.5H), 3.05 (td, *J* = 2.53, 13.14 Hz, 0.5H), 2.67 - 2.86 (m, 1.5H), 2.55 (td, *J* = 3.03, 12.88 Hz, 0.5H), 2.09 - 2.28 (m, 1H), 2.08 (s, 1.5H), 2.03 (s, 1.5H), 1.41 - 1.63 (m, 1H), 1.27 - 1.41 (m, 1H), 1.05 - 1.27 (m, 1H); MS m/e 543.2 [M+H]⁺.
The racemate was neutralized by partitioning between saturated NaHCO₃ aqueous solution and DCM. The organic layer was dried (Na₂SO₄), filtered, concentrated to dryness, and purified by chiral HPLC (Chiralcel OD, 100% EtOH) to give two pure enantiomers. **Example 34b:** (first enantiomer to elute off chiral column) ¹H NMR (400 MHz, CDCl₃, ∼1.5:1 mixture of rotamers) δ 8.45 (d, *J* = 13.64 Hz, 1H), 8.20 (dd, *J* = 4.04, 8.59 Hz, 1H), 7.68 (t, *J* = 10.11 Hz, 1H), 7.48 - 7.53 (m, 3H), 7.24 - 7.34 (m, 2H), 7.10 - 7.24 (m, 2H), 4.65 (d, *J* = 12.63 Hz, 0.6H), 4.56 (d, *J* = 13.14 Hz, 0.4H), 3.91 (d, *J* = 13.14 Hz, 0.4H), 3.66 - 3.79 (m, 0.6H), 3.31 (s, 1.8H), 3.29 (s, 1.2H), 3.18 (t, *J* = 12.38 Hz, 0.4H), 2.97 (t, *J* = 12.13 Hz, 0.6H), 2.38 - 2.69 (m, 2H), 2.34 (d, *J* = 13.14 Hz, 0.4H), 2.22 (d, *J* = 12.63 Hz, 0.6H), 2.01 (s, 1.8H), 1.95 (s, 1.2H), 1.13 - 1.55 (m, 2.4H), 1.09 (d, *J* = 12.63 Hz, 0.6H); MS m/e 543.2 [M+H]⁺. **Example 34c:** (second enantiomer to elute off chiral column) ¹H NMR (400 MHz, CDCl₃, ∼1.5:1 mixture of rotamers) δ 8.46 (d, *J* = 12.63 Hz, 1H), 8.20 (dd, *J* = 3.79, 8.84 Hz, 1H), 7.68 (t, *J* = 10.11 Hz, 1H), 7.46 - 7.53 (m, 3H), 7.24 - 7.35 (m, 2H), 7.10 - 7.23 (m, 2H), 4.64 (d, *J* = 13.14 Hz, 0.6H), 4.55 (d, *J* = 12.63 Hz, 0.4H), 3.90 (d, *J* = 13.14 Hz, 0.4H), 3.72 (d, *J* = 13.14 Hz, 0.6H), 3.31 (s, 1.8H), 3.29 (s, 1.2H), 3.18 (t, *J* = 12.38 Hz, 0.4H), 2.97 (t, *J* = 12.38 Hz, 0.6H), 2.38 - 2.68 (m, 2H), 2.34 (d, *J* = 12.63 Hz, 0.4H), 2.21 (d, *J* = 13.14 Hz, 0.6H), 2.01 (s, 1.8H), 1.94 (s, 1.2H), 1.12 - 1.58 (m, 2.4H), 1.08 (d, *J* = 12.63 Hz, 0.6H); MS m/e 543.2 [M+H]⁺.

### Example 35: (4-Chloro-2-methoxy-3-phenylquinolin-6-yl)(2,4-dimethylthiazol-5-yl)(1-methyl-1H-1,2,3-triazol-5-yl)methnol•TFA

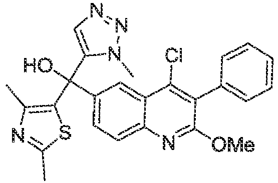

To a flask containing (2,4-dichloro-3-phenylquinolin-6-yl)(2,4-dimethylthiazol-5-yl)(1-methyl-1*H*-1,2,3-triazol-5-yl)methanol (100 mg, 0.2 mmol, Example 67) was added MeOH (6 mL) followed by solid NaOMe (50 mg, 0.88 mmol, 95% purity) at room temperature. The reaction mixture was heated to reflux for 24 hours, then allowed to cool to room temperature, filtered through Celite® and rinsed with MeOH. The MeOH was removed under reduced pressure and the residue was diluted with water and extracted with EtOAc (3 x 40 mL). The combined organics were washed with brine, dried over MgSO₄, filtered and concentrated to dryness. Purification by RP-HPLC (water / acetonitrile / 0.05% TFA) afforded the title compound. ¹H NMR (500 MHz, CDCl₃) δ 8.23 (d, *J* = 2.1 Hz, 1H), 7.88 (d, *J* = 8.8 Hz, 1H), 7.57 - 7.39 (m, 4H), 7.38 - 7.30 (m, 2H), 7.17 (s, 1H), 4.75 (s, 1H), 4.02 (s, 3H), 3.92 (s, 3H), 2.56 (s, 3H), 2.12 (s, 3H); MS (ESI): mass calcd. for C₂₅H₂₂ClN₅O₂S, 491.1, m/z found 492.0 [M+H]⁺.

### Example 36: (2-Chloro-4-methoxy-3-phenylquinolin-6-yl)(2,4-dimethylthiazol-5-yl)(1-methyl-1H-1,2,3-triazol-5-yl)methano]•TFA

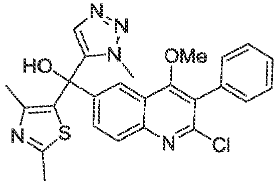

To a flask containing (2,4-dichloro-3-phenylquinolin-6-yi)(2,4-dimethylthiazol-5-yl)(1-methyl-1*H*-1,2,3-triazol-5-yl)methanol (100 mg, 0.2 mmol, Example 67) was added MeOH (6 mL) followed by solid NaOMe (50 mg, 0.88 mmol, 95% purity) at room temperature. The reaction mixture was heated to reflux for 24 hours, then allowed to cool to room temperature, filtered through Celite® and rinsed with MeOH. The MeOH was removed under reduced pressure and the residue was diluted with water and extracted with EtOAc (3 x 40 mL). The combined organics were washed with brine, dried over MgSO₄, filtered and concentrated to dryness. Purification by RP-HPLC (water / acetonitrile / 0.05% TFA) afforded the title compound. ¹H NMR (500 MHz, CDCl₃) δ 8.13 (d, *J* = 2.1 Hz, 1H), 8.04 (d, *J* = 8.9 Hz, 1H), 7.68 (dd, *J* = 8.9, 2.3 Hz, 1H), 7.54 - 7.44 (m, 3H), 7.43 - 7.39 (m, 2H), 7.23 (s, 1H), 3.95 (br. s, 1H), 3.93 (s, 3H), 3.48 (s, 3H), 2.59 (s, 3H), 2.16 (s, 3H); MS (ESI): mass calcd. for C₂₅H₂₂CN₅O₂S, 491.1, m/z found 492.0 [M+H]⁺.

### Example 37: (4-Chloro-2-methoxy-3-phenylquinolin-6-yl)(2,4-dimethylthiazol-5-yl)(1-methyl-1H-imidazol-5-yl)methanol•TFA

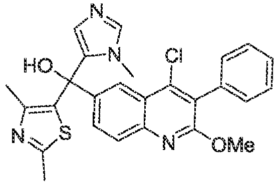

To a microwave vial containing (2,4-dichloro-3-phenylquinolin-6-yl)(2,4-dimethylthiazol-5-yl)(1-methyl-1*H*-imidazol-5-yl)methanol (120 mg, 0.24 mmol, Example 68) was added MeOH (4 mL) followed by solid NaOMe (60 mg, 1.11 mmol, 95% purity) at room temperature. The vial was sealed and evacuated and the mixture was heated to 75 °C for 5.5 hours. The MeOH was removed under reduced pressure and the residue was diluted with water and extracted with EtOAc (4 x 25 mL). The combined organics were washed with brine, dried over MgSO₄, filtered and concentrated to dryness. Purification by RP-HPLC (water / acetonitrile / 0.05% TFA) afforded the title compound. ¹H NMR (500 MHz, CD₃OD) δ 9.00 (s, 1H), 8.30 (d, *J* = 2.0 Hz, 1H), 8.01 (d, *J* = 8.9 Hz, 1H), 7.81 (dd, *J* = 8.9, 2.1 Hz, 1H), 7.60 - 7.37 (m, 5H), 7.26 (s, 1H), 3.75 (s, 3H), 3.55 (s, 3H), 2.62 (s, 3H), 2.25 (s, 3H); MS (ESI): mass calcd. for C₂₆H₂₃ClN₄O₂S, 490.1; m/z found 491.1 [M+H]⁺.

### Example 38: (2-Chloro-4-methoxy-3-phenylquinolin-6-yl)(2,4-dimethylthiazol-5-yl)(1-methyl-1H-imidazol-5-yl)methanol•TFA

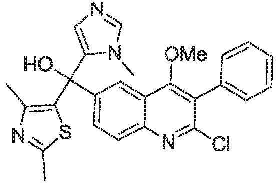

To a microwave vial containing (2,4-dichloro-3-phenylquinolin-6-yl)(2,4-dimethylthiazol-5-yl)(1-methyl-1*H*-imidazol-5-yl)methanol (120 mg, 0.24 mmol, Example 68) was added MeOH (4 mL) followed by solid NaOMe (60 mg, 1.11 mmol, 95% purity) at room temperature. The vial was sealed and evacuated and the mixture was heated to 75 °C for 5.5 hours. The MeOH was removed under reduced pressure and the residue was diluted with water and extracted with EtOAc (4 x 25 mL). The combined organics were washed with brine, dried over MgSO₄, filtered and concentrated to dryness. Purification by RP-HPLC (water / acetonitrile / 0.05% TFA) afforded the title compound. ¹H NMR (500 MHz, CD₃OD) δ 9.00 (s, 1H), 8.28 (d, *J* = 2.1 Hz, 1H), 7.98 (d, *J* = 8.8 Hz, 1H), 7.74 (dd, *J* = 8.8, 2.2 Hz, 1H), 7.51 - 7.38 (m, 3H), 7.35 - 7.27 (m, 2H), 7.24 (s, 1H), 4.00 (s, 3H), 3.75 (s, 3H), 2.62 (s, 3H), 2.25 (s, 3H); MS (ESI): mass calcd. for C₂₆H₂₃ClN₄O₂S, 490.1; m/z found 491.1 [M+H]⁺.

### Example 39: (2,4-Dichloro-3-phenylquinolin-6-yl)(1,2-dhnethyl-1H-imidazol-5-yl)(1-methyl-1H-1,2,3-triazol-5-yl)methanol

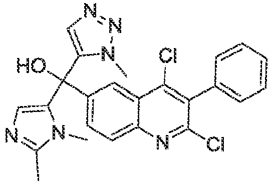

To a flask containing 1-methyl-1*H*-1,2,3-triazole (125 mg, 1.5 mmol) was added THF (10 mL) and the colorless solution was cooled to -45 °C. Then, *n*-BuLi (2.5 M in hexanes, 0.6 mL, 1.5 mmol) was added affording a suspension. The suspension was stirred between -40 °C and -10 °C for 30 minutes, then a THF solution of (2,4-dichloro-3-phenylquinolin-6-yl)(1,2-dimethyl-1*H-*imidazol-5-yl)methanone (500 mg, 1.26 mmol, Intermediate 18: step b, in 5 mL THF) was introduced and the mixture was allowed to warm up to room temperature. After 1 hour, the reaction mixture was heated to 40 °C for 3 hours and then quenched with aqueous NH₄Cl solution. The aqueous portion was extracted with EtOAc (3 x 50 mL) and then with DCM (3 x 50 mL). The individual organic portions were washed with brine, dried over MgSO₄, filtered, combined and concentrated to dryness. Chromatography on silica gel (5% MeOH-DCM increasing to 10% MeOH) provided material which was re-purified by preparative TLC (5% 2 M-NH₃-MeOH-EtOAc) to provide the title compound as light tan solid. ¹H NMR (500 MHz, CDCl₃) δ 8.45 - 8.36 (m, 1H), 7.94 (d, *J* = 8.8 Hz, 1H), 7.66 - 7.46 (m, 4H), 7.46 - 7.30 (m, 2H), 7.01 (s, 1H), 5.94 (s, 1H), 3.91 (s, 3H), 3.34 (s, 3H), 2.17 (s, 3H). MS (ESI): mass calcd. for C_{2A}H₂₀Cl₂N₆O, 478.1, m/z found 479.1 [M+H]⁺.

### Example 40a: (2-Chloro-4-metboxy-3-phenylquinolin-6-yl)(1,2-dimethyl-1H-imidazol-5-yl)(1-methyl-1H-1,2,3-triazol-5-yl)methanol•TFA

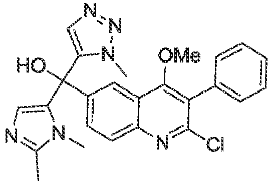

To a flask containing (2,4-dichloro-3-phenylquinolin-6-yl)(1,2-dimefhyl-1*H*-imidazol-5-yl)(1-methyl-1*H*-1,2,3-triazol-5-yl)methanol (1.1 g, 2.29 mmol, Example 39) was added MeOH (30 mL) followed by solid NaOMe (505 mg, 9.35 mmol, 95% purity) at room temperature. The mixture was heated to 80 °C for 5 hours, then cooled back to room temperature and the MeOH was removed under reduced pressure. The crude material was passed through a plug of silica gel (5% MeOH-DCM) to afford a light brown solid. This material was further purified by RP-HPLC (water / acetonitrile / 0.05% TFA) to afford the title compound. ¹H NMR (500 MHz, CD₃OD) δ 8.34 (d, *J* = 1.8 Hz, 1H), 8.01 (d, *J* = 8.8 Hz, 1H), 7.67 (dd, *J* = 8.8, 2.1 Hz, 1H), 7.58 - 7.36 (m, 6H), 6.90 (s, 1H), 4.01 (s, 3H), 3.69 (s, 3H), 3.55 (s, 3H), 2.66 (s, 3H); MS (ESI): mass calcd. for C₂₅H₂₃ClN₆O₂, 474.2, m/z found 475.1 [M+H]⁺.

The racemic mixture was separated by chiral chromatography using [Chiralpak AD, 1000A, 20 µM (Diacel), heptane:2-propanol (80:20 with 0.2% TEA), to give two enantiomers. The first eluting enantiomer was **Example 40b** and the second eluting enantiomer was **Example 40c.**

### Example 41a: (4-Chloro-2-methoxy-3-phenylquinolin-6-yl)(1,2-dimethyl-1H-imidazol-5-yl)(1-methyl-1H-1,2,3-triazol-5-yl)methanol•TFA

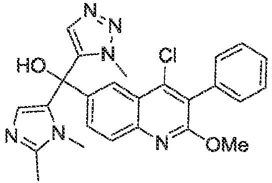

To a flask containing (2,4-dichloro-3-phenylquinolin-6-yl)(1,2-dimethyl-1*H*-imidazol-5-yl)(1-methyl-1*H*-1,2,3-triazol-5-yl)methanol (1.1 g, 2.29 mmol, Example 39) was added MeOH (30 mL) followed by solid NaOMe (505 mg, 9.35 mmol, 95% purity) at room temperature. The mixture was heated to 80 °C for 5 hours, then cooled back to room temperature and the MeOH was removed under reduced pressure. The crude material was passed through a plug of silica gel (5% MeOH-DCM) to afford a light brown solid. This material was further purified by RP-HPLC (water / acetonitrile / 0.05% TFA) to afford the title compound. ¹H NMR (500 MHz, CD₃OD) δ 8.32 (d, *J* = 2.1 Hz, 1H), 7.98 (d, *J* = 8.8 Hz, 1H), 7.60 (dd, *J* = 8.8, 2.2 Hz, 1H), 7.50 - 7.37 (m, 4H), 7.34 - 7.25 (m, 2H), 6.87 (s, 1H), 4.00 (s, 3H), 3.99 (s, 3H), 3.70 (s, 3H), 2.67 (s, 3H); MS (ESI): mass calcd. for C₂₅H₂₃ClN₆O₂, 474.2, m/z found 475.1 [M+H]⁺.
The racemic mixture was separated by chiral chromatography using [Chiralpak AD, 1000A, 20 µM (Diacel), heptane:2-propanol (75:25 with 0.2% TEA), to give two enantiomers. The first eluting enantiomer was **Example 41b** and the second eluting enantiomer was **Example 41c.**

### Example 42: (2,4-Bis(methylthio)-3-phenylquinolin-6-yl)(2,4-dimethylthiazol-5-yl)(1-methyl-1H-imidazol-5-yl)methanol•TFA

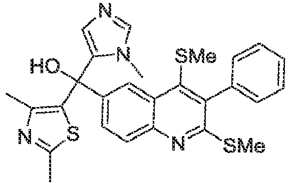

To a flask containing (2,4-dichloro-3-phenylquinolin-6-yi)(2,4-dimethylthiazol-5-yl)(1-methyl-1*H*-imidazol-5-yl)methanol (198 mg, 0.4 mmol, Example 68) was added DMF (67.5 mL) followed by sodium methanethiolate (37 mg, 0.53 mmol) at room temperature. After 15 minutes, the reaction mixture was concentrated and chromatographed directly on silica gel (2% MeOH-DCM increasing to 5% MeOH) which afforded a mixture of two products. This material was re-purified by RP-HPLC (water / acetonitrile / 0.05% TFA) to provide the title compound. ¹H NMR (500 MHz, CDCl₃) δ 8.72 (s, 1H), 8.60 (s, 1H), 8.25 (d, *J =* 8.3 Hz, 1H), 7.77 (d, *J* = 8.3 Hz, 1H), 7.59 - 7.45 (m, 3H), 7.39 - 7.28 (m, 2H), 7.20 (s, 1H), 3.71 (s, 3H), 2.80 (s, 3H), 2.68 (s, 3H), 2.32 (s, 3H), 1.95 (s, 3H); MS (ESI): mass calcd. for C₂₇H₂₆N₄OS₃, 518.1, m/z found 519.1 [M+H]⁺.

### Example 43: (4-Chloro-2-(methylthio)-3-phenylquinolin-6-yl)(2,4-dimethylthiazol-5-yl)(1-methyl-1H-imidazol-5-yl)methanol•TFA

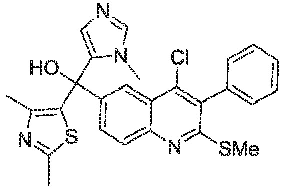

To a flask containing (2,4-dichloro-3-phenylquinolin-6-yl)(2,4-dimethylthiazol-5-yl)(1-methyl-1*H*-imidazol-5-yl)methanol (198 mg, 0.4 mmol, Example 68) was added DMF (67.5 mL) followed by sodium methanethiolate (37 mg, 0.53 mmol) at room temperature. After 15 minutes, the reaction mixture was concentrated to dryness and chromatographed directly on silica gel (2% MeOH-DCM increasing to 5% MeOH) which afforded a mixture of two products. This material was re-purified by RP-HPLC (water / acetonitrile / 0.05% TFA) to provide the title compound. ¹H NMR (500 MHz, CDCl₃) δ 8.70 (s, 1H), 8.63 (s, 1H), 8.12 (d, *J* = 8.9 Hz, 1H), 7.78 (d, *J* = 8.7 Hz, 1H), 7.56 - 7.43 (m, 3H), 7.34 (d, *J* = 7.1 Hz, 2H), 7.20 (s, 1H), 3.71 (s, 3H), 2.78 (s, 3H), 2.34 (s, 3H), 1.94 (s, 3H); MS (ESI): mass calcd. for C₂₆H₂₃ClN₄OS₂, 506.1, m/z found 507.0 [M+H]⁺.

### Example 44: [2-(2-Aziridin-1-ylethoxy)-4-chloro-3-phenylquinolin-6-yl](4-chlorophenyl)(1-methyl-1H-imidazol-5-yl)methanol

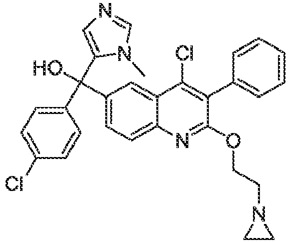

(4-Chlorophenyl)(2,4-dichloro-3-phenylquinolin-6-yl)(1-methyl-1*H*-imidazol-5-yl)methanol (100 mg, 0.202 mmol, Example 65), 2-(aziridin-1-yl)ethanol (16.2 µL, 0.20 mmol), toluene (2 mL), and sodium hydride (60% dispersion in mineral oil, 20.2 mg, 0.51 mmol) were combined in a round bottom flask under an N₂ atmosphere. The reaction solution was heated to reflux and refluxed overnight, cooled and stirred at room temperature an additional night before workup. Reaction contents were transferred to a separatory funnel with EtOAc dilution, and extracted with saturated, aqueous NH₄Cl then saturated, aqueous NaHCO₃ solutions. The organic phase was separated then dried over MgSO₄, filtered and concentrated under reduced pressure. The crude product was purified by flash column chromatography (silica gel, 0-10% DCM / MeOH) to afford the title compound. MS (ESI): mass calcd. for C₃₀H₂₆Cl₂N₄O₂, 544.1; m/z found, 545.1 [M+H]⁺; ¹H NMR (600 MHz, CD₃OD) δ ppm 8.05 (d, *J* = 2.0 Hz, 1H), 7.74 (d, *J* = 8.7 Hz, 1H), 7.61 - 7.57 (m, 2H), 7.40 - 7.35 (m, 2H), 7.35 - 7.31 (m, 1H), 7.28 - 7.23 (m, 6H), 6.18 (d, *J* = 1.1 Hz, 1H), 4.51 - 4.44 (m, 2H), 3.37 (s, 3H), 2.50 - 2.39 (m, 2H), 1.48 - 1.37 (m, 2H), 0.97 - 0.94 (m, 2H).

### Example 45: (4-Chlorophenyl)[4-chloro-3-phenyl-2-(2,2,2-trifluoroethoxy)quinolin-6-yl](1-methyl-1H-imidazol-5-yl)methanol•TFA

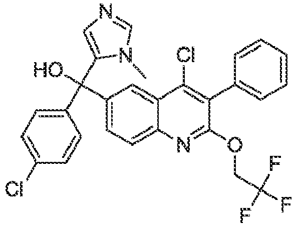

(4-Chlorophenyl)(2,4-dichloro-3-phenylquinolin-6-yl)(1-methyl-1*H*-imidazol-5-yl)methanol (100 mg, 0.202 mmol, Example 65), 2,2,2-trifluoroethanol (14.5 µL, 0.202 mmol), toluene (2 mL), and sodium hydride (60% dispersion in mineral oil, 20 mg, 0.51 mmol) were combined in a round bottom flask under an N₂ atmosphere. The reaction solution was heated to reflux and refluxed overnight. The reaction solution was cooled to room temperature then transferred to a separatory funnel with EtOAc dilution, and extracted with saturated, aqueous NH₄Cl then saturated, aqueous NaHCO₃ solutions. The organic phase was separated then dried over MgSO₄, filtered and concentrated under reduced pressure. The crude product was purified by flash column chromatography (silica gel, 0-10% DCM / MeOH) then via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to provide the title compound. MS (ESI): mass calcd. for C₂₈H₂₀Cl₂F₃N₃O₂, 557.1; m/z found, 558.3 [M+H]⁺; ¹H NMR (600 MHz, CDCl₃) δ ppm 8.38 (s, 1H), 8.17 (d, *J* = 2.0 Hz, 1H), 7.87 (d, *J* = 8.8 Hz, 1H), 7.60 (dd, *J* = 8.8, 2.1 Hz, 1H), 7.52 - 7.43 (m, 3H), 7.38 - 7.31 (m, 6H), 6.56 (s, 1H), 4.92 (q, *J* = 8.4 Hz, 2H), 3.62 (s, 3H).

### Example 46: 2-[{4-Chloro-6-[(4-chlorophenyl)(hydroxy)(1-methyl-1H-imidazol-5-yl)methyl]-3-phenylquinolin-2-yl}(methyl)amino]ethanol•TFA

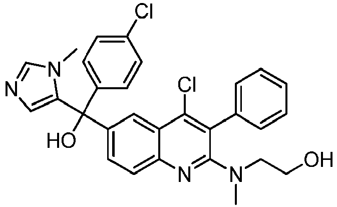

(4-Chlorophenyl)(2,4-dichloro-3-phenylquinolin-6-yl)(1-methyl-1*H*-imidazol-5-yl)methanol (100 mg, 0.202 mmol, Example 65), 2-(methylamino)ethanol (16.2 µL, 0.202 mmol), toluene (2 mL), and sodium hydride (60% dispersion in mineral oil, 28.3 mg, 0.707 mmol) were combined in a round bottom flask and heated at reflux for 48 hours under an N₂ atmosphere. The reaction solution was then cooled to room temperature, diluted with EtOAc, transferred to a separatory funnel, extracted with saturated, aqueous NH₄Cl then saturated, aqueous NaHCO₃ solutions. The organic phase was separated then dried over MgSO₄, filtered and concentrated under reduced pressure. The crude product was purified by flash column chromatography (silica gel, 0-10% DCM / MeOH) then further purified via reverse phase chromatography using acetonitrile with 0.05% trifluoroacetic acid in water as eluent, then lyophilized to afford the title compound as a trifluoroacetate salt. MS (ESI): mass calcd. for C₂₉H₂₆Cl₂N₄O₂, 532.1; m/z found, 533.2 [M+H]⁺; ¹H NMR (600 MHz, CD₃OD) δ ppm 8.98 (s, 1H), 8.22 (s, 1H), 7.83 (d, *J=* 8.8 Hz, 1H), 7.79 (d, *J=* 8.9 Hz, 1H), 7.55 (t, *J* = 7.4 Hz, 2H), 7.50 (t, *J=* 7.4 Hz, 1H), 7.47 - 7.39 (m, 6H), 6.93 (d, *J* = 1.4 Hz, 1H), 3.80 - 3.73 (m, 2H), 3.69 (s, 3H), 3.61 - 3.57 (m, 2H), 2.75 (s, 3H).

### Example 47a: 6-((2,4-Dimethylthiazol-5-yl)(hydroxy)(1-methyl-1H-1,2,3-triazol-5-yl)methyl)-3-phenylquinoline-2,4-dicarbonitrile (Reference)

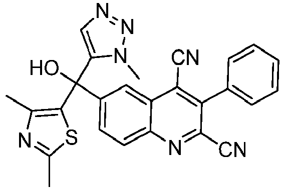

(2,4-Dichloro-3-phenylquinolin-6-yl)(2,4-dimethylthiazol-5-yl)(1-methyl-1*H*-1,2,3-triazol-5-yl)methanol (100 mg, 0.2 mmol, Example 67), zinc cyanide (75 mg, 0.64 mmol), X-Phos (25 mg, 0.052 mmol), and Pd₂(dba)₃ (60 mg, 0.066 mmol) were all added to a large microwave vial. DMA (3 mL) was added and the vial was sealed and evacuated and the mixture was heated to 120 °C in an oil bath. After 1.5 hours, the reaction mixture was filtered while still warm through Celite® and rinsed with EtOAc. The effluent was concentrated and the DMA was partially removed under high vacuum. The crude material was chromatographed on silica gel (3% MeOH-DCM increasing to 8% MeOH) which provided the title compound as a pale yellow foam. ¹H NMR (500 MHz, CDCl₃) δ 8.53 (s, 1H), 8.26 (d, *J* = 8.9 Hz, 1H), 7.79 (d, *J=* 9.0 Hz, 1H), 7.71 - 7.54 (m, 5H), 7.10 (s, 1H), 6.10 (s, 1H), 4.00 (s, 3H), 2.57 (s, 3H), 2.14 (s, 3H); MS (ESI): mass calcd. for C₂₆H₁₉N₇OS, 477.1, m/z found 478.1 [M+H]⁺.
The racemic mixture was separated by chiral chromatography using [Chiralcel OJ, 1000A, 20 µM (Diacel), and 100% MeOH], to give two enantiomers. The first eluting enantiomer was **Example 47b** and the second eluting enantiomer was **Example 47c.**

### Example 48: [4-Chloro-2-(2-methoxyethoxy)-3-phenylquinolin-6-yl](4-chlorophenyl)(1-methyl-1H-imidazol-5-yl)methanol

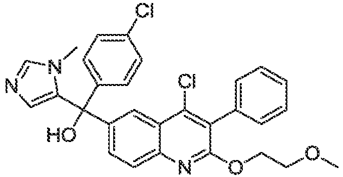

(4-Chlorophenyl)(2,4-dichloro-3-phenylquinolin-6-yl)(1-methyl-1*H*-imidazol-5-yl)methanol (100 mg, 0.202 mmol, Example 65), 2-methoxyethanol (16.0 µL, 0.202 mmol), toluene (2 mL), and sodium hydride (60% dispersion in mineral oil, 20.2 mg, 0.505 mmol) were combined in a round bottom flask under an N₂ atmosphere. The contents were heated to reflux and refluxed overnight. The reaction solution turned from a heterogeneous white mixture to slightly yellowish with a moderate amount of precipitate. The contents were cooled to room temperature then transferred to a separatory funnel with EtOAc dilution, and extracted with saturated, aqueous NH₄Cl and saturated, aqueous NaHCO₃ solutions. The organic phase was separated then dried over MgSO₄, filtered and concentrated under reduced pressure. The crude product was purified by flash column chromatography (silica gel, 0-10% DCM / (10% of a 2 M NH₃ MeOH in DCM)) then further purified via reverse phase chromatography using acetonitrile with ammonium hydroxide in water as eluent to afford the title compound. MS (ESI): mass calcd. for C₂₉H₂₅Cl₂N₃O₃, 533.1; m/z found, 534.2 [M+H]⁺; ¹H NMR (600 MHz, CD₃OD) δ ppm 8.96 (d, *J* = 0.9 Hz, 1H), 8.19 (d, *J* = 1.9 Hz, 1H), 7.91 (d, *J* = 8.8 Hz, 1H), 7.70 (dd, *J* = 8.8, 2.2 Hz, 1H), 7.48 - 7.44 (m, 4H), 7.43 - 7.39 (m, 3H), 7.36 - 7.33 (m, 2H), 6.90 (d, *J* = 1.6 Hz, 1H), 4.59 - 4.54 (m, 2H), 3.70 (s, 3H), 3.68 - 3.63 (m, 2H), 3.25 (s, 3H).

### Example 49a: (4-Chloro-2-methoxy-3-pheny]quinolin-6-yl)(4-chlorophenyl)(1-methyl-1H-imidazol-5-yl)methanole•TFA

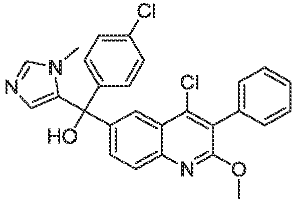

(4-Chlorophenyl)(2,4-dichloro-3-phenylquinolin-6-yl)(1-methyl-1*H*-imidazol-5-yl)methanol (100 mg, 0.202 mmol, Example 65), toluene (2 mL), and sodium methoxide (109 mg, 2.02 mmol) were combined in a round bottom flask equipped with a stirbar and condenser under an N₂ atmosphere. The reaction solution was heated to reflux and refluxed overnight. Analysis showed an incomplete reaction, so additional sodium methoxide (109 mg, 2.02 mmol) was added and contents were refluxed for an additional day. The reaction was cooled to room temperature and contents were transferred to a separatory funnel with EtOAc dilution, and extracted with saturated, aqueous NH₄Cl then saturated, aqueous NaHCO₃ solutions. The organic phase was separated, dried over MgSO₄, filtered and concentrated under reduced pressure. The crude material was purified via reverse phase chromatography using acetonitrile with 0.05% trifluoroacetic acid in water as eluent. The fractions from the purification containing the desired product were lyophilized to provide the title compound as a trifluoroacetate salt. MS (ESI): mass calcd. for C₂₇H₂₁Cl₂N₃O₂, 489.1; m/z found, 490.1 [M+H]⁺; ¹H NMR (600 MHz, CD₃OD) δ ppm 8.97 (d, *J* = 0.9 Hz, 1H), 8.18 (d, *J* = 1.9 Hz, 1H), 7.94 (d, *J* = 8.9 Hz, 1H), 7.71 (dd, *J* = 8.8, 2.2 Hz, 1H), 7.48 - 7.44 (m, 4H), 7.43 - 7.40 (m, 3H), 7.32 - 7.28 (m, 2H), 6.90 (d, *J=* 1.6 Hz, 1H), 3.98 (s, 3H), 3.70 (s, 3H).
(4-Chloro-2-methoxy-3-phenylquinolin-6-yl)(4-chlorophenyl)(1-methyl-1*H*-imidazol-5-yl)methanol**•**TFA was purified on a chiralcel AD column (8 cm) with ethanol to provide two enantiomers. The first eluting enantiomer was **Example 49b:** MS (ESI): mass calcd. for C₂₇H₂₁Cl₂N₃O₂, 489.1; m/z found, 490.2 [M+H]⁺; ¹H NMR (600 MHz, CD₃OD) δ ppm 8.14 (d, *J* = 2.0 Hz, 1H), 7.94 (s, 1H), 7.87 (d, *J=* 8.8 Hz, 1H), 7.69 (dd, *J=* 8.8, 2.1 Hz, 1H), 7.45 (dd, *J=* 11.4, 4.4 Hz, 2H), 7.42 - 7.34 (m, 5H), 7.33 - 7.27 (m, 2H), 6.40 (s, 1H), 3.97 (s, 3H), 3.51 (s, 3H) and the second eluting enantiomer was **Example 49c:** MS (ESI): mass calcd. for C₂₇H₂₁Cl₂N₃O₂, 489.1; m/z found, 490.1 [M+H]⁺; ¹H NMR (600 MHz, CD₃OD) δ ppm 8.20 - 8.14 (m, 2H), 7.88 (d, *J=* 8.8 Hz, 1H), 7.69 (dd, *J=* 8.8, 2.1 Hz, 1H), 7.47 - 7.42 (m, 2H), 7.42 - 7.35 (m, 5H), 7.31 - 7.27 (m, 2H), 6.51 (s, 1H), 3.96 (s, 3H), 3.55 (s, 3H).

### Example 50: {4-Chloro-2-[(2-methoxyethyl)(methyl)amino]-3-phenylquinolin-6-yl}(4-chlorophenyl)(1-methyl-1H-imidazol-5-yl)methanol

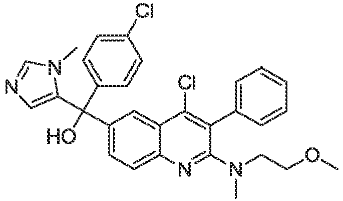

(4-Chlorophenyl)(2,4-dichloro-3-phenylquinolin-6-yl)(1-methyl-1*H*-imidazol-5-yl)methanol (100 mg, 0.202 mmol, Example 65), *N*-(2-methoxymethyl)methylamine (900 µL, 10.1 mmol), and methanol (2 mL) were combined in a reaction tube, which was then sealed and heated to 100 °C for 72 hours. The contents were then cooled to room temperature, transferred to a round bottom flask and the solvent was removed via reduced pressure distillation. The crude residue was then taken up into EtOAc, transferred to a separatory funnel, extracted twice with a saturated, aqueous NH₄Cl solution. The organic phase was separated, dried over MgSO₄, filtered and concentrated under reduced pressure. The crude product was purified by flash column chromatography (silica gel, 0-10% DCM / (10% of a 2 M NH₃ MeOH in DCM)) then further purified via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to afford the title compound. MS (ESI): mass calcd. for C₃₀H₂₈Cl₂N₄O₂, 546.2; m/z found, 547.3 [M+H]⁺; ¹H NMR (600 MHz, CD₃OD) δ ppm 8.05 (d, *J* = 2.0 Hz, 1H), 7.97 (s, 1H), 7.75 (d, *J* = 8.8 Hz, 1H), 7.60 (dd, *J* = 8.8, 2.1 Hz, 1H), 7.45 (dd, *J* = 10.3, 4.6 Hz, 2H), 7.38 (ddd, *J* = 8.6, 4.5, 1.2 Hz, 1H), 7.36 - 7.31 (m, 6H), 6.42 (s, 1H), 3.51 (s, 3H), 3.31 - 3.28 (m, 2H), 3.24 (dd, *J* = 8.7, 3.0 Hz, 2H), 3.17 (s, 3H), 2.74 (s, 3H).

### Example 51: N-[2-({4-Chloro-6-[(4-chlorophenyl)(hydroxy)(1-methyl-1H-imidazol-5-yl)methyl]-3-phenylquinolin-2-yl}oxy)ethyl]propanamide

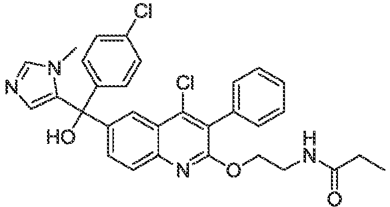

(4-Chlorophenyl)(2,4-dichloro-3-phenylquinolin-6-yl)(1-methyl-1*H*-imidazol-5-yl)methanol (100 mg, 0.202 mmol, Example 65), *N*-(2-hydroxylethyl)propionamide (34.2 µL, 0.303 mmol), toluene (2 mL), and sodium hydride (60% dispersion in mineral oil, 32.3 mg, 0.808 mmol) were combined in a round bottom flask under an N₂ atmosphere. The reaction solution was heated to reflux and refluxed overnight. The reaction solution was cooled to room temperature then transferred to a separatory funnel with EtOAc dilution, and extracted with saturated, aqueous NH₄Cl then saturated, aqueous NaHCO₃ solutions. The organic phase was separated, then dried over MgSO₄, filtered and concentrated under reduced pressure. The crude product was purified by flash column chromatography (silica gel, 0-10% DCM / MeOH) then via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to provide the title compound. MS (ESI): mass calcd. for C₃₁H₂₈Cl₂N₄O₃, 574.2; m/z found, 575.2 [M+H]⁺; ¹H NMR (600 MHz, CD₃OD) δ ppm 8.14 (s, 1H), 7.83 (d, *J* = 8.8 Hz, 1H), 7.78 (s, 1H), 7.68 (dd, *J* = 8.8, 1.7 Hz, 1H), 7.47 - 7.41 (m, 2H), 7.41 - 7.37 (m, 1H), 7.38 - 7.34 (m, 4H), 7.34 - 7.30 (m, 2H), 6.32 (s, 1H), 4.52 (t, *J=* 5.6 Hz, 2H), 3.53 - 3.44 (m, 5H), 2.11 (q, *J* = 7.6 Hz, 2H), 1.04 (t, *J* = 7.6 Hz, 3H).

### Example 52: [2-(2-Aminoethoxy)4-chloro-3-phenylquinolin-6-yl](4-chlorophenyl)(1-methyl-1H-imidazol-5-yl)methanol

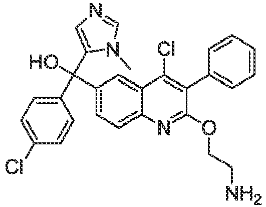

(4-Chlorophenyl)(2,4-dichloro-3-phenylquinolin-6-yl)(1-methyl-1*H*-imidazol-5-yl)methanol (100 mg, 0.202 mmol, Example 65), N-(2-hydroxyethyl)-2,2,2-trifluoroacetamide (49 mg, 0.30 mmol), toluene (2 mL), and sodium hydride (60% dispersion in mineral oil, 32.3 mg, 0.808 mmol) were combined in a round bottom flask and heated at reflux for 48 hours under an N₂ atmosphere. Analysis after overnight reaction showed only partial conversion, so additional N-(2-hydroxyethyl)-2,2,2-trifluoroacetamide (33 mg, 0.21 mmol) and sodium hydride (60% dispersion in mineral oil, 25 mg, 1.03 mmol) were added and the vessel was resealed and heated at reflux for an additional 48 hours. The reaction solution was then cooled to room temperature, diluted with EtOAc, transferred to a separatory funnel, and extracted with saturated, aqueous NH₄Cl then saturated, aqueous NaHCO₃ solutions. The organic phase was separated then dried over MgSO₄, filtered and concentrated under reduced pressure. The crude product was purified via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to afford the title compound. MS (ESI): mass calcd. for C₂₈H₂₄Cl₂N₄O₂, 518.1; m/z found, 519.2 [M+H]⁺; ¹H NMR (600 MHz, CD₃OD) δ ppm 8.17 (d, *J=* 2.0 Hz, 1H), 7.87 (d, *J=* 8.8 Hz, 1H), 7.72 (dd, *J* = 8.8,2.1 Hz, 1H), 7.69 (s, 1H), 7.50 - 7.45 (m, 2H), 7.44 - 7.40 (m, 1H), 7.39 - 7.33 (m, 6H), 6.26 (s, 1H), 4.67 - 4.60 (m, 2H), 3.47 (s, 3H), 3.20 (t, *J=* 5.3 Hz, 2H).

### Example 53: {4-Chloro-2-[(2-methoxyethyl)(methyl)amino]-3-phenylquinolin-6-yl}(1-methyl-1H-imidazol-5-yl)[6-(trifluoromethyl)pyridin-3-yl]methanol

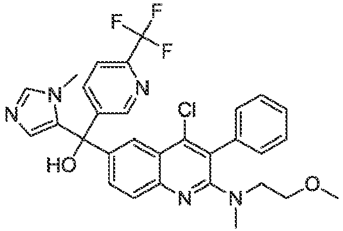

(2,4-Dichloro-3-phenylquinolin-6-yl)(1-methyl-1*H*-imidazol-5-yl)[6-(trifluoromethyl) pyridin-3-yl]methanol (200 mg, 0.378 mmol, Example 66), *N*-(2-methoxymethyl)methylamine (842 µL, 9.45 mmol), and methanol (2 mL) were combined in a reaction tube, which was then sealed and heated to 100 °C for 48 hours. The contents were then cooled to room temperature, transferred to a round bottom flask and the solvent was removed via reduced pressure distillation. The crude residue was then taken up into EtOAc, transferred to a separatory funnel and extracted twice with a saturated, aqueous NH₄Cl solution. The organic phase was separated, dried over MgSO₄, filtered and concentrated under reduced pressure. The crude product was purified via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to afford the title compound. MS (ESI): mass calcd. for C₃₀H₂₇ClF₃N₅O₂, 581.2; m/z found, 582.2 [M+H]⁺; ¹H NMR (600 MHz, CD₃OD) δ ppm 8.77 (d, *J* = 1.8 Hz, 1H), 8.08 (d, *J* = 2.0 Hz, 1H), 8.01 (dd, *J* = 8.2, 1.9 Hz, 1H), 7.92 (s, 1H), 7.84 (d, *J=* 8.2 Hz, 1H), 7.80 (d, *J=* 8.8 Hz, 1H), 7.61 (dd, *J=* 8.8, 2.1 Hz, 1H), 7.49 (t, *J=* 7.5 Hz, 2H), 7.42 (ddd, *J* = 6.7, 2.5, 1.2 Hz, 1H), 7.39 - 7.35 (m, 2H), 6.44 (s, 1H), 3.52 (s, 3H), 3.35 - 3.32 (m, 2H), 3.30 - 3.27 (m, 2H), 3.19 (s, 3H), 2.78 (s, 3H).

### Example 54: [4-Chloro-3-phenyl-2-(2,2,2-trifluoroethoxy)quinolin-6-yl](1-methyl-1H-imidazol-5-yl)[6-(trifluoromethyl)pyridin-3-yl]methanol

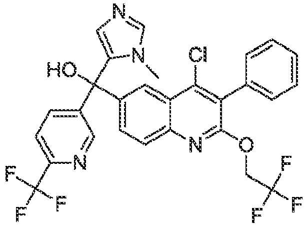

(2,4-Dichloro-3-phenylquinolin-6-yl)(1-methyl-1H-imidazol-5-yl)[6-(trifluoromethyl) pyridin-3-yl]methanol (200 mg, 0.378 mmol, Example 66), 2,2,2-trifluoroethanol (14.0 µL, 0.19 mmol), toluene (2 mL), and sodium hydride (60% dispersion in mineral oil, 19 mg, 0.47 mmol) were combined in a round bottom flask under an N₂ atmosphere. The reaction solution was heated to reflux and refluxed overnight. Analysis showed the reaction had only progressed a moderate amount, so additional reagents were added, 2,2,2-trifluoroethanol (14.0 µL, 0.19 mmol) and sodium hydride (60% dispersion in mineral oil, 6 mg, 0.19 mmol) and contents were heated at reflux for an additional 48 hours. The contents were cooled to room temperature then transferred to a separatory funnel with EtOAc dilution, and extracted with saturated, aqueous NH₄Cl then saturated, aqueous NaHCO₃ solutions. The organic phase was separated then dried over MgSO₄, filtered and concentrated under reduced pressure. The crude material was purified via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to afford the title compound. MS (ESI): mass calcd. for C₂₈H₁₉ClF₆N₄O₂, 592.1; m/z found, 593.2 [M+H]⁺; ¹H NMR (600 MHz, CD₃OD) δ ppm 8.80 (d, *J* = 2.1 Hz, 1H), 8.28 (d, *J* = 1.9 Hz, 1H), 8.17 (s, 1H), 8.05 (dd, *J* = 8.2, 2.0 Hz, 1H), 7.97 (d, *J=* 8.7 Hz, 1H), 7.86 (dd, *J=* 8.3, 0.4 Hz, 1H), 7.75 (dd, *J=* 8.8, 2.2 Hz, 1H), 7.51 - 7.42 (m, 3H), 7.37 - 7.34 (m, 2H), 6.60 (s, 1H), 5.00 (q, *J=* 8.6 Hz, 2H), 3.56 (s, 3H).

### Example 55: (2,4-Dichloro-3-phenylquinolin-6-yl)(1-methyl-1H-imidazol-5-yl)pyrimidin-2-ylmethanol

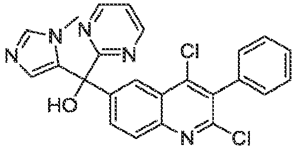

6-Bromo-2,4-dichloro-3-phenylquinoline (0.895 g, 2.54 mmol, Intermediate 7: step c) and (1-methyl-1*H*-imidazol-5-yl)(pyrimidin-2-yl)methanone (500 mg, 2.66 mmol, Intermediate 15: step b) were dissolved in THF (250 mL) in a dry round bottom flask under an N₂ atmosphere, then cooled to -78 °C in dry ice acetone bath. *n*-BuLi (2.5 M in hexanes, 0.966 mL, 2.42 mmol) was then added dropwise via syringe over approximately 2 minutes. The reaction contents were stirred at -78 °C for approximately 1.5 hours, then the dry ice bath was removed and allowed to warm to room temperature and stir for approximately 1 hour. The reaction was then re-cooled to 0 °C and quenched with a saturated, aqueous NH₄Cl solution, then transferred to a separatory funnel with EtOAc. The organic phase was separated, then the aqueous layer was back extracted with EtOAc, then the combined organic phases were dried over MgSO₄, filtered and concentrated under reduced pressure. The crude product was purified by flash column chromatography (silica gel, 0-10% DCM / (10% of a 2M NH₃ MeOH in DCM)) then further purified via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to afford the title compound. MS (ESI): mass calcd. for C₂₄H₁₇Cl₂N₅O, 461.1; m/z found, 462.1 [M+H]⁺; ¹H NMR (600 MHz, CD₃OD) δ ppm 8.88 (d, *J=* 4.9 Hz, 2H), 8.57 (d, *J* = 1.8 Hz, 1H), 8.08 (dd, *J=* 8.9,2.0 Hz, 1H), 7.99 (d, *J* = 8.9 Hz, 1H), 7.80 (s, 1H), 7.54 - 7.45 (m, 4H), 7.36 - 7.32 (m, 2H), 6.49 (d, *J=* 1.0 Hz, 1H), 3.43 (s, 3H).

### Example 56: {4-Chloro-2-[methoxy(methyl)amino]-3-phenylquinolin-6-yl}(1-methyl-1H-imidazol-5-yl)[6-(trifluoromethyl)pyridin-3-yl]methanol

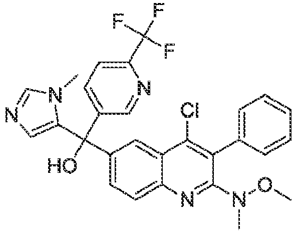

(2,4-Dichloro-3-phenylquinolin-6-yl)(1-methyl-1*H*-imidazol-5-yl)[6-(trifluoromethyl)pyridin-3-yl] methanol (200 mg, 0.378 mmol, Example 66), *N*,*O*-dimethylhydroxylamine hydrochloride (376 mg, 3.78 mmol), and dimethylformamide (2 mL) were combined in a reaction tube which was then sealed and heated to 100 °C for 48 hours. Analysis shows desired product, but also the presence of starting material. Additional *N*,*O*-dimethylhydroxylamine hydrochloride (190 mg, 0.195 mmol) was added and the contents were heated for an additional 24 hours. The contents were then cooled, transferred to a separatory funnel, diluted with EtOAc and extracted with a saturated, aqueous NH₄Cl solution, followed by deionized water (4 x). The organic phase was separated, dried over MgSO₄, filtered and concentrated under reduced pressure. The crude material was purified via reverse phase chromatography using acetonitrile with ammonium hydroxide in water as eluent to afford the title compound. MS (ESI): mass calcd. for C₂₈H₂₃ClF₃N₅O₂, 553.1; m/z found, 554.2 [M+H]⁺; ¹H NMR (400 MHz, CD₃OD) δ ppm 8.79 (d, *J=* 2.1 Hz, 1H), 8.19 (d, *J* = 1.9 Hz, 1H), 8.02 (dd, *J* = 8.2, 1.9 Hz, 1H), 7.96 (d, *J* = 8.8 Hz, 1H), 7.83 (d, *J* = 8.2 Hz, 1H), 7.75 - 7.68 (m, 2H), 7.48 (t, *J* = 7.4 Hz, 2H), 7.40 (ddd, *J* = 7.4, 3.9, 1.3 Hz, 1H), 7.32 - 7.24 (m, 2H), 6.35 (s, 1H), 3.48 (s, 3H), 3.07 (s, 3H), 2.77 (s, 3H).

### Example 57: {2,4-Bis[methoxy(methyl)amino]-3-phenylquinolin-6-yl}(1-methyl-1H-imidazol-5-yl)[6-(trifluoromethyl)pyridin-3-yl]methanol

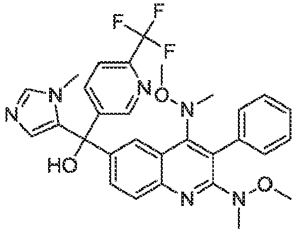

Purification of the crude product from the synthesis of {4-chloro-2-[methoxy(methyl)amino]-3-phenylquinolin-6-yl}(1-methyl-1*H*-imidazol-5-yl)[6-(trifluoromethyl)pyridin-3-yl]methanol (Example 56) also provided the title compound. MS (ESI): mass calcd. for C₃₀H₂₉F₃N₆O₃, 578.2; m/z found, 579.2 [M+H]⁺; ¹H NMR (400 MHz, CD₃OD) δ ppm 8.75 (d, *J=* 2.0 Hz, 1H), 8.36 (d, *J* = 2.0 Hz, 1H), 8.02 (dd, *J* = 8.3, 1.8 Hz, 1H), 7.97 (d, *J* = 8.9 Hz, 1H), 7.84 (d, *J* = 8.2 Hz, 1H), 7.78 - 7.70 (m, 2H), 7.44 - 7.37 (m, 2H), 7.36 - 7.29 (m, 1H), 7.24 (d, *J* = 7.3 Hz, 2H), 6.33 (s, 1H), 3.50 (s, 3H), 3.26 (s, 3H), 3.02 (s, 3H), 2.86 (s, 3H), 2.78 (s, 3H).

### Example 58: {4-Chloro-2-[methoxy(methyl)amino]-3-phenylquinolin-6-yl}(4-chlorophenyl)(1-methyl-1H-imidazol-5-yl)methanol

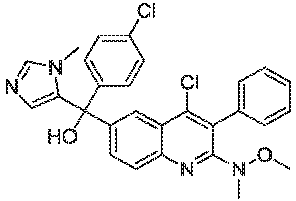

(4-Chlorophenyl)(2,4-dichloro-3-phenylquinolin-6-yl)(1-methyl-1*H*-imidazol-5-yl)methanol (250 mg, 0.505 mmol, Example 65), *N*,*O*-dimethylhydroxylamine hydrochloride (1.01 g, 10.1 mmol), and dimethylformamide (2 mL) were combined in a reaction tube, which was then sealed and heated to 100 °C for 48 hours. The contents were then cooled, transferred to a separatory funnel, diluted with EtOAc and extracted with saturated, aqueous NH₄Cl solution, followed by deionized water (4 x). The organic phase was separated and dried over MgSO₄, filtered and concentrated under reduced pressure. The crude material was purified via reverse phase chromatography using acetonitrile with ammonium hydroxide in water as eluent to afford the title compound. MS (ESI): mass calcd. for C₂₈H₂₄Cl₂N₄O₂, 518.1; m/z found, 519.2 [M+H]⁺; ¹H NMR (400 MHz, CD₃OD) δ ppm 8.14 (d, *J=* 1.9 Hz, 1H), 7.91 (d, *J=* 8.8 Hz, 1H), 7.70 (dd, *J* = 8.8,2.1 Hz, 1H), 7.67 (s, 1H), 7.47 (dd, *J* = 11.4,4.3 Hz, 2H), 7.39 (dt, *J=* 4.4, 1.8 Hz, 1H), 7.36 (d, *J* = 4.1 Hz, 4H), 7.26 (dd, *J* = 8.2, 1.3 Hz, 2H), 6.28 (s, 1H), 3.46 (s, 3H), 3.05 (s, 3H), 2.76 (s, 3H).

### Example 59: {2,4-Bis[methoxy(methyl)amino]-3-phenylquinolin-6-yl}(4-chlorophenyl)(1-methyl-1H-imidazol-5-yl)methanol

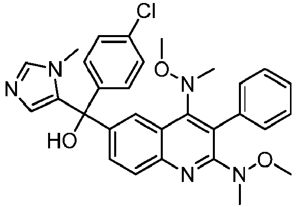

Purification of the crude product from the synthesis of {4-chloro-2-[methoxy(methyl)amino]-3-phenylquinolin-6-yl}(4-chlorophenyl)(1-methyl-1*H*-imidazol-5-yl)methanol (Example 58) also provided the title compound. MS (ESI): mass calcd. for C₃₀H₃₀ClN₅O₃, 543.2; m/z found, 544.3 [M+H]⁺; ¹H NMR (400 MHz, CD₃OD) δ ppm 8.36 (d, *J=* 1.8 Hz, 1H), 7.96 - 7.89 (m, 1H), 7.71 (dd, *J* = 8.9, 2.2 Hz, 1H), 7.66 (s, 1H), 7.42 - 7.28 (m, 7H), 7.24 (d, *J* = 6.6 Hz, 2H), 6.28 (d, *J* = 1.0 Hz, 1H), 3.48 (s, 3H), 3.25 (s, 3H), 3.01 (s, 3H), 2.85 (s, 3H), 2.79 (s, 3H).

### Example 60a: (4-Chloro-2-methoxy-3-phenylquinolin-6-yl)(1-methyl-1H-imidazol-5-yl)pyrimidin-2-ylmethanol (Reference)

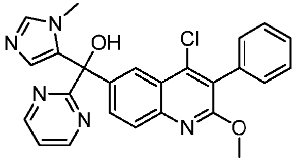

(2,4-Dichloro-3-phenylquinolin-6-yl)(1-methyl-1*H*-imidazol-5-yl)pyrimidin-2-ylmethanol (156 mg, 0.337 mmol, Example 55), toluene (2 mL), and sodium methoxide (365 mg, 6.75 mmol) were combined in a round bottom flask equipped with a stirbar and condenser under an N₂ atmosphere. The contents were heated to reflux and refluxed overnight. The reaction was cooled and the contents were transferred to a separatory funnel with EtOAc dilution, and extracted with saturated, aqueous NH₄Cl then saturated, aqueous NaHCO₃ solutions. The organic phase was separated, dried over MgSO₄, filtered and concentrated under reduced pressure. The crude material was purified via reverse phase chromatography using acetonitrile with 0.05% trifluoroacetic acid in water as eluent. The fractions from the purification containing the desired product were transferred to a separatory funnel with EtOAc and extracted with a saturated, aqueous NaHCO₃ solution. The aqueous layer was separated, extracted with EtOAc, then the combined organic phases were dried over MgSO₄, filtered and concentrated under reduced pressure to provide the title compound. MS (ESI): mass calcd. for C₂₃H₂₀ClN₃O₂, 457.1; m/z found, 458.2 [M+H]⁺; ¹H NMR (400 MHz, CDCl₃) δ ppm 8.81 (d, *J* = 4.9 Hz, 2H), 8.48 (d, *J* = 1.8 Hz, 1H), 7.92 - 7.81 (m, 2H), 7.54-7.39 (m, 4H), 7.36 - 728 (m, 3H), 6.55 (s, 1H), 6.15 (s, 1H), 3.98 (s, 3H), 3.40 (s, 3H).

(4-Chloro-2-methoxy-3-phenylquinolin-6-yl)(1-methyl-1*H*-imidazol-5-yl)pyrimidin-2-ylmethanol was purified on a chiralcel OD column (20 µm, Diacel) with methanol to provide two enantiomers. The first eluting enantiomer was **Example 60b:** MS (ESI): mass calcd. for C₂₅H₂₀ClN₅O₂, 457.1; m/z found, 458.2 [M+H]⁺; ¹H NMR (600 MHz, CD₃OD) δ ppm 8.86 (d, *J* = 4.9 Hz, 2H), 8.41 (d, *J* = 2.0 Hz, 1H), 7.90 (dd, *J* = 8.8, 2.0 Hz, 1H), 7.82 (d, *J* = 8.8 Hz, 1H), 7.63 (s, 1H), 7.46 - 7.41 (m, 3H), 7.41 - 7.37 (m, 1H), 7.29 (d, *J* = 7.0 Hz, 2H), 6.41 (s, 1H), 3.95 (s, 3H), 3.39 (s, 3H).

### Example 61: (4-Chloro-3-phenyl-2-(trifluoromethyl)quinolin-6-yl)(1-methyl-1H-imidazol-5-yl)(pyridin-2-yl)methanol•TFA (Reference)

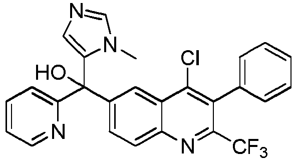

The title compound was prepared using (1-methyl-1*H*-imidazol-5-yl)(pyridin-2-yl)methanone (Intermediate 12: step b) in place of *tert*-butyl 4-nicotinoylpiperidine-1-carboxylate using the procedure described for Example 69. ¹H NMR (400 MHz, CD₃OD) δ 8.97 (s, 1H), 8.58 - 8.65 (m, 2H), 8.28 (d, *J* = 9.09 Hz, 1H), 8.11 (dd, *J* = 2.02,9.09 Hz, 1H), 7.88 - 7.97 (m, 1H), 7.80 (d, *J* = 7.58 Hz, 1H), 7.48 - 7.55 (m, 3H), 7.38 - 7.46 (m, 1H), 7.26 - 7.34 (m, 2H), 7.11 (s, 1H), 3.63 (s, 3H); MS m/e 495.3 [M+H]⁺.

### Example 62: (4-Chloro-3-phenyl-2-(trifluoromethyl)quinolin-6-yl)(4-chlorophenyl)(1-methyl-1H-imidazol-5-yl)methanol•TFA (Reference)

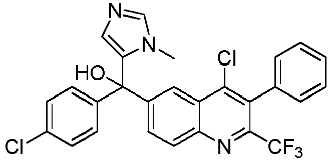

A mixture of 6-bromo-4-chloro-3-phenyl-2-(trifluoromethyl)quinoline (864 mg, 2.23 mmol, Intermediate 19: step b), (4-chlorophenyl)(1-methyl-1*H*-imidazol-5-yl)methanone (490 mg, 2.22 mmol, Intermediate 8: step b) and 22 mL of THF was purged with N₂ and cooled to -78 °C. To the mixture was added *n*-BuLi (1.6 M in hexanes, 1.8 mL, 2.9 mmol) dropwise and the color changed to orange then almost black. The reaction mixture was stirred at -78 °C to 0 °C for 70 minutes, then allowed to warm up to room temperature overnight. Saturated NH₄Cl (aqueous) was added and the organic layer was separated. The aqueous layer was extracted with dichloromethane. The combined organic phases were dried (Na₂SO₄), filtered, and concentrated. The crude was purified by flash column chromatography (silica gel, 5-10% MeOH in DCM) followed by reverse phase HPLC (water/acetonitrile/0.1% TFA) to provide the title compound as a white solid. ¹H NMR (400 MHz, CD₃OD) δ 9.00 (s, 1H), 8.42 (d, *J=* 2.02 Hz, 1H), 8.31 (d, *J* = 8.59 Hz, 1H), 7.97 (dd, *J* = 2.02, 9.09 Hz, 1H), 7.47 - 7.54 (m, 4H), 7.41 - 7.47 (m, 3H), 7.27 - 7.34 (m, 2H), 6.99 (d, *J=* 1.52 Hz, 1H), 3.71 (s, 3H); MS m/e 528.2 [M+H]⁺.

### Example 63: (4-Chloro-2-methoxy-8-methyl-3-(4-(trifluoromethoxy)phenyl)quinolin-6-yl)(1-methyl-1H-imidazol-5-yl)(6-(trifluoromethyl)pyridin-3-yl)methanol (Reference)

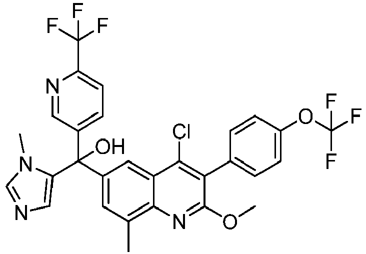

*n*-Butyllithium (2.0 mL, 3.202 mmol) was added to a -78 °C mixture of 6-bromo-4-chloro-2-methoxy-8-methyl-3-(4-(trifluoromethoxy)phenyl)quinoline (1.1 g, 2.463 mmol, Intermediate 13: step d) and (1-methyl-1*H*-imidazol-5-yl)(6-(trifluoromethyl)pyridin-3-yl)methanone (0.691 g, 2.709 mmol, Intermediate 2: step c) in dry THF (25 mL) over a 2 minute period. After complete addition stirring was continued at -78 °C for 10 minutes then the reaction was warmed up to 0 °C and stirred for 1 hour. Saturated aqueous NH₄Cl was added and the reaction mixture slowly warmed to room temperature. Water was added and the layers were separated. The aqueous layer was extracted with EtOAc. The combined organic extracts were dried (Na₂SO₄), filtered, evaporated *in vacuo* and chromatographed (DCM/10% MeOH in EtOAc) to provide the product.

Further purification using reverse phase HPLC (acetonitrile / water + 0.1 % TFA) provided the title compound. MS (ESI) 623.1 (M+H)⁺.

### Example 64: (2,4-Dichloro-3-phenylquinolin-6-yl)(1-methyl-1H-imidazol-5-yl)(pyridin-2-yl)methanol (Reference)

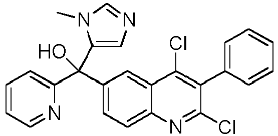

A solution of *n*-BuLi (2.5 M in hexanes, 0.34 mL, 0.85 mmol) was added dropwise by syringe to a solution of 6-bromo-2,4-dichloro-3-phenylquinoline (305.4 mg, 0.865 mmol, Intermediate 7: step c) in dry THF (4.4 mL) at -78 °C. After 1.5 minutes, a solution of (1-methyl-1*H*-imidazol-5-yl)(pyridin-2-yl)methanone (0.175 g, 0.936 mmol, Intermediate 12: step b) in dry THF (1.8 mL) was added dropwise. The reaction mixture was stirred for 5 minutes at -78 °C, then the reaction flask was placed into an ice-water bath. After 10 minutes, the mixture was warmed to room temperature and the reaction was quenched with methanol and water. The mixture was partitioned between water and DCM. The separated aqueous phase was further extracted with DCM. The organic phase was dried (Na₂SO₄), filtered, and concentrated. The crude product was purified by flash column chromatography (silica gel, 0-10% MeOH-DCM) to provide the title compound as a white solid. ¹H NMR (500 MHz, CDCl₃) δ 8.65 (ddd, *J* = 4.9, 1.6, 1.0 Hz, 1H), 8.30 (d, *J* = 1.7 Hz, 1H), 8.06 (d, *J* = 8.8 Hz, 1H), 7.86 (dd, *J* = 8.8,2.0 Hz, 1H), 7.73 (td, *J* = 7.7, 1.7 Hz, 1H), 7.55 - 7.47 (m, 4H), 7.36 - 7.29 (m, 3H), 7.23 (d, *J* = 8.0 Hz, 1H), 6.37 (d, *J* = 1.1 Hz, 1H), 3.44 (s, 3H); MS m/e 461.1 [M+H]⁺.

### Example 65: (4-Chlorophenyl)(2,4-dichloro-3-phenylquinolin-6-yl)(1-methyl-1H-imidazol-5-yl)methanol (Reference)

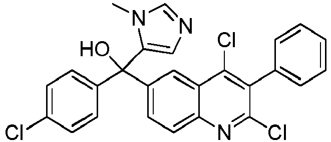

To (4-chlorophenyl)(1-methyl-1*H*-imidazol-5-yl)methanone (830 mg, 3.76 mmol, Intermediate 8: step b) under an atmosphere of nitrogen was added THF (30 mL) and the mixture was heated until a solution was obtained. To 6-bromo-2,4-dichloro-3-phenylquinoline (121 g, 3.42 mmol, Intermediate 7: step c) under an atmosphere of nitrogen was added THF (25 mL). The resulting colorless solution was cooled in a dry ice/acetone bath. *n*-BuLi (1.6 M in hexane, 2.35 mL, 3.76 mmol) was added dropwise. The mixture was stirred for 5 minutes before addition of the THF solution of (4-chlorophenyl)(1-methyl-1*H*-imidazol-5-yl)methanone via cannula. The reaction mixture was stirred in the dry ice/acetone bath for 30 minutes, then in an ice bath for 50 minutes and at room temperature for 15 minutes, then was quenched by addition of saturated, aqueous NH₄Cl solution. The mixture was diluted with water and extracted three times with EtOAc. The organic phase was dried (Na₂SO₄), filtered, and concentrated and the residue was purified by flash column chromatography (silica gel, 0-4% MeOH-DCM) to afford the title compound. ¹H NMR (400 MHz, CDCl₃) δ ppm 8.32 (d, *J=* 1.96 Hz, 1H), 8.02 (d, *J=* 8.80 Hz, 1H), 7.72 (dd, *J* = 2.20, 8.80 Hz, 1H), 7.48 - 7.56 (m, 3H), 7.30 - 7.38 (m, 7H), 6.40 (d, *J* = 1.22 Hz, 1H), 3.39 (s, 3H); MS m/e 494.1 (M+H)⁺.

### Example 66: (2,4-Dichloro-3-phenylquinolin-6-yl)(1-methyl-1H-imidazol-5-yl)[6-(trifluoromethyl)pyridin-3-yl]methanol•TFA (Reference)

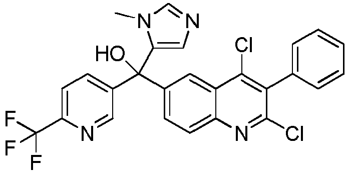

(1-Methyl-1*H*-imidazol-5-yl)(6-(trifluoromethyl)pyridin-3-yl)methanone (3.00 g, 8.50 mmol, Intermediate 2: step c) and 6-bromo-2,4-dichloro-3-phenylquinoline (2.32 g, 9.08 mmol, Intermediate 7: step c) were dissolved in THF (250 mL) under an N₂ atmosphere in a dry round bottom flask, then cooled to -78 °C in dry ice / acetone bath. *n*-BuLi (2.5 M in hexanes, 3.24 mL, 8.09 mmol) was then added dropwise via syringe over approximately 2 minutes. The contents were stirred at -78 °C for approximately 2.5 hours, then the dry ice bath was removed and the contents were allowed to warm to room temperature. The reaction was then cooled to 0 °C in an ice water bath and quenched with a saturated, aqueous NH₄Cl solution, then transferred to a separatory funnel with EtOAc dilution. The organic phase was separated, then the aqueous layer was back extracted twice with EtOAc, then the combined organic phases were dried over MgSO₄, filtered and concentrated under reduced pressure. The crude product was purified by RP-HPLC (40-80% CH₃CN-H₂O, 0.1% TFA) to afford the title compound as a yellow solid. MS (ESI): mass calcd. for C₂₆H₁₇Cl₂F₃N₄O, 528.1; m/z found, 529.4 [M+H]⁺; ¹H NMR (600 MHz, CD₃OD) δ ppm 8.80 (d, *J* = 2.1 Hz, 1H), 8.35 (d, *J* = 1.8 Hz, 1H), 8.07 - 8.03 (m, 2H), 7.88 (dd, *J* = 8.9, 2.1 Hz, 1H), 7.85 (d, *J=* 8.3 Hz, 1H), 7.76 (s, 1H), 7.54 - 7.50 (m, 3H), 7.37 - 7.33 (m, 2H), 6.39 (d, *J=* 1.1 Hz, 1H), 3.48 (s, 3H).

### Example 67: (2,4-Dichloro-3-phenylquinolin-6-yl)(2,4-dimethylthiazol-5-yl)(1-methyl-1H-1,2,3-triazol-5-yl)methanol (Reference)

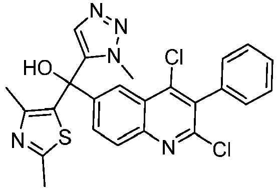

To a flask containing 6-bromo-2,4-dichloro-3-phenylquinoline (750 mg, 2.12 mmol, Intermediate 7: step c) was added THF (30 mL) to give a homogeneous clear solution. The solution was cooled in a dry ice bath and *n*-BuLi (2.5 M in hexanes, 0.840 mL, 2.1 mmol) was introduced. After 2 minutes, a solution of (2,4-dimethylthiazol-5-yl)(1-methyl-1*H*-1,2,3-triazol-5-yl)methanone (600 mg, 2.7 mmol, Intermediate 16: step b) in 18 mL THF was added. The dry-ice bath was replaced with a 0 °C ice-bath and after 45 minutes the reaction mixture was quenched with NH₄Cl solution and the aqueous portion was extracted with EtOAc (4 x 50 mL). The combined organics were washed with brine, dried over MgSO₄, filtered and concentrated. The crude material was triturated with Et₂O to afford the title compound as a light tan solid. The mother liquors were chromatographed on silica gel (2% MeOH/DCM increasing to 5% MeOH) which provided additional title compound. ¹H NMR (500 MHz, CDCl₃) δ 8.32 (d, *J* = 2.0 Hz, 1H), 8.09 (d, *J* = 8.9 Hz, 1H), 7.73 (dd, *J* = 8.9,2.2 Hz, 1H), 7.58 - 7.46 (m, 3H), 7.20-7.33 (m, 2H), 7.20 (s, 1H), 4.38 (s, 1H), 3.94 (s, 3H), 2.58 (s, 3H), 2.16 (s, 3H); MS (ESI); mass calcd. for C₂₄H₁₉Cl₂N₅OS, 495.1, m/z found 496.1 [M+H]⁺.

### Example 68: (2,4-Dichloro-3-phenylquinolin-6-yl)(2,4-dimethylthiazol-5-yl)(1-methyl-1H-imidazol-5-yl)methanol (Reference)

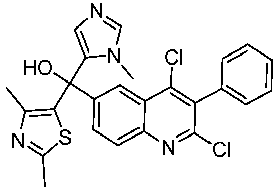

To a flask containing 6-bromo-2,4-dichloro-3-phenylquinoline (450 mg, 1.27 mmol, Intermediate 7: step c) was added THF (15 mL) to give a homogeneous clear solution. The solution was cooled in a dry ice bath and *n*-BuLi (2.5 M in hexanes, 0.45 mL, 1.13 mmol) was added. After 2 minutes, a solution of (2,4-dimethylthiazol-5-yl)(1-methyl-1*H*-imidazol-5-yl)methanone (350 mg, 1.58 mmol, Intermediate 17: step b) in 4 mL THF was introduced. The dry-ice bath was replaced with a 0 °C bath and after 35 minutes the reaction mixture was quenched with aqueous NH₄Cl solution and the aqueous portion was extracted with EtOAc (4 x 50 mL). The combined organics were washed with brine, dried over MgSO₄, filtered and concentrated. Chromatography on silica gel (20% acetone-DCM increasing to 5% MeOH-DCM) afforded the title compound as a pale yellowish solid. ¹H NMR (500 MHz, CDCl₃) δ 8.35 (d, *J=* 1.9 Hz, 1H), 8.03 (d, *J=* 8.8 Hz, 1H), 7.79 (dd, *J* = 8.8, 2.1 Hz, 1H), 7.59 - 7.41 (m, 3H), 7.40 - 7.31 (m, 2H), 7.28 (d, *J=* 6.4 Hz, 1H), 6.44 (s, 1H), 5.46 (s, 1H), 3.48 (s, 3H), 2.56 (s, 3H), 2.13 (s, 3H); MS (ESI): mass calcd. for C₂₅H₂₀Cl₂N₄OS, 494.1, m/z found 495.0 [M+H]⁺.

### Example 69: tert-Butyl 4-((4-chloro-3-phenyl-2-(trifluoromethyl)quinolin-6-yl)(hydroxy)(pyridin-3-yl)methyl)piperidine-1-carboxylate

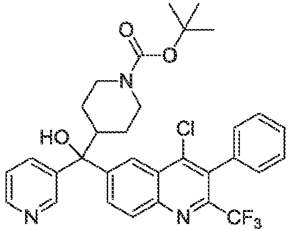

To a solution of 4-chloro-6-iodo-3-phenyl-2-(trifluoromethyl)quinoline (Intermediate 20: step b, containing about 13% molar of 4-chloro-3-phenyl-2-(trifluoromethyl)quinoline as impurity, 340 mg) in THF (4 mL) at -78 °C under N₂ was added *i*PrMgCl (2.0 M in THF, 0.40 mL, 0.8 mmol). After stirring for 8 minutes, the cooling bath was removed, and stirring was continued for 20 minutes, then *tert*-butyl 4-nicotinoylpiperidine-1-carboxylate (225 mg, 0.770 mmol, Intermediate 21) was added in neat. After stirring at room temperature overnight, the mixture became clear brown and was heated at 50 °C for 1.5 hours. The mixture was quenched with saturated NH₄Cl aqueous solution, and extracted with EtOAc. The extracts were dried over Na₂SO₄, filtered, and concentrated. The crude mixture was purified by flash column chromatography (silica gel, 20-100% EtOAc in heptanes) to provide the title compound. ¹H NMR (400 MHz, CDCl₃) δ 9.17 - 9.35 (m, 1H), 8.50 - 8.88 (m, 2H), 8.29 (d, *J* = 7.58 Hz, 1H), 8.24 (d, *J* = 8.59 Hz, 1H), 7.82 - 8.00 (m, 1H), 7.47 - 7.57 (m, 4H), 7.21 - 7.33 (m, 2H), 4.08 - 4.26 (m, 2H), 2.69 - 2.99 (m, 3H), 1.82 - 1.92 (m, 1H), 1.53 - 1.80 (m, 3H), 1.47 (s, 4.5H), 1.41 (s, 4.5H); MS m/e 598.3 [M+H]⁺.

### Example 70: (4-Chloro-3-phenyl-2-(trifluoromethyl)quinolin-6-yl)(piperidin4-yl)(pyridin-3-yl)methanol

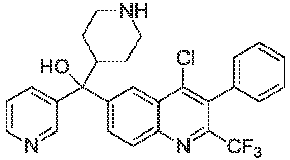

A solution of *tert*-butyl 4-((4-chloro-3-phenyl-2-(trifluoromethyl)quinolin-6-yl)(hydroxy)(pyridin-3-yl)methyl)piperidine-1-carboxylate (313 mg, 0.520 mmol, Example 69) in DCM (6 mL) was treated with 1.8 mL of TFA, stirred for 3 hours, and concentrated. The residue was partitioned between saturated NaHCO₃ aqueous solution and DCM. The organic layer was dried (Na₂SO₄), filtered and concentrated to dryness to provide the title compound as a light brown solid. ¹H NMR (400 MHz, CDCl₃) δ 8.86 (s, 1H), 8.55 (s, 1H), 8.44 (d, *J=* 4.55 Hz, 1H), 8.21 (d, *J=* 9.09 Hz, 1H), 7.89 - 7.96 (m, 2H), 7.46 - 7.53 (m, 4H), 7.23 -7.32 (m, 2H), 3.23 - 3.36 (m, 2H), 2.77 - 2.91 (m, 3H), 1.66 - 1.86 (m, 4H).

### Example 71: 4-Chloro-6-((4-chlorophenyl)(1-methyl-1H-imidazol-5-yl)methyl)-N,N-dimethyl-3-phenylquinolin-2-amine

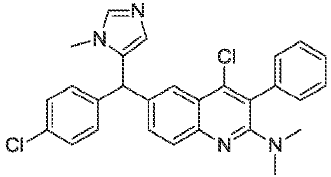

To (4-chloro-2-(dimethylamino)-3-phenylquinolin-6-yl)(4-chlorophenyl)(1-methyl-1*H*-imidazol-5-yl)methanol (83.6 mg, 0.166 mmol, Example 90) in NMP (1.5 mL), was added copper (I) cyanide (16.4 mg, 0.183 mmol). The mixture was heated by microwave irradiation for 10 minutes at 120 °C. The mixture was partitioned between MTBE and saturated aqueous NH₄OH. The organic phase was washed with water. The organic phase was dried (Na₂SO₄), filtered, and concentrated *in vacuo.* The residue was purified by RP-HPLC (10-90% CH₃CN-H₂O, 0.1% TFA) and fractions were converted to the corresponding free base by extraction from saturated aqueous NaHCO₃ with DCM. Further purification by flash column chromatography (silica gel, 10-70% acetone-DCM) afforded the title compound. ¹H NMR (400 MHz, CDCl₃) δ 7.82 (d, *J* = 1.96 Hz, 1H), 7.76 (d, *J* = 8.80 Hz, 1H), 7.55 (br. s., 1H), 7.43 - 7.50 (m, 2H), 7.34 - 7.42 (m, 4H), 7.30 (d, *J=* 8.56 Hz, 2H), 7.12 (d, *J=* 8.56 Hz, 2H), 6.48 (br. s., 1H), 5.45 (s, 1H), 3.37 (s, 3H), 2.71 (s, 6H); MS m/e 487.0 (M+H)⁺.

### Example 72: 4-Chloro-6-((4-chlorophenyl)(1-methyl-1H-imidazol-5-yl)methyl)-3-phenylquinoline-2-carbonitrile•TFA

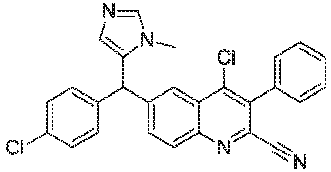

Copper (I) cyanide (44.4 mg, 0.496 mmol) was added to a colorless solution of (4-chlorophenyl)(2,4-dichloro-3-phenylquinolin-6-yl)(1-methyl-1*H*-imidazol-5-yl)methanol (123 mg, 0.248 mmol, Example 65) in NMP (1 mL). The mixture was stirred until CuCN dissolved, then was heated by microwave irradiation at 145 °C for 40 minutes. The mixture was partitioned between saturated aqueous NH₄OH and MTBE. The aqueous phase was extracted twice with MTBE. The organic phases were combined, dried (Na₂SO₄), filtered, and concentrated *in vacuo.* The residue was purified by RP-HPLC (10-90% CH₃CN-H₂O, 0.1% TFA) to afford the title compound. ¹H NMR (400 MHz, CDCl₃) δ 8.68 (s, 1H), 8.26 (d, *J* = 8.80 Hz, 1H), 8.05 (d, *J* = 1.96 Hz, 1H), 7.66 (dd, *J* = 1.96, 8.80 Hz, 1H), 7.52 - 7.62 (m, 3H), 7.37 - 7.49 (m, 4H), 7.12 (d, *J* = 8.31 Hz, 2H), 6.78 (s, 1H), 5.63 (s, 1H), 3.62 (s, 3H); MS m/e 469.0 (M+H)⁺.

### Example 73: (2,4-Dichloro-3-(2-chlorophenyl)quinolin-6-yl)(phenyl)(pyridin-4-yl)methanol

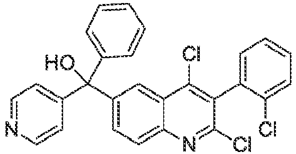

To a solution of 6-bromo-2,4-dichloro-3-(2-chlorophenyl)quinoline (387.5 mg, 1 mmol, Intermediate 32: step c) in THF (15 mL) at -78 °C was added *n*BuLi (2.5 M in hexanes, 0.52 mL, 1.3 mmol) dropwise and the resulting mixture stirred at -78 °C for 15 minutes. Then, phenyl(pyridin-4-yl)methanone (183 mg, 1 mmol) was added, and the resulting mixture stirred at -78 °C for 15 minutes. The dry ice - acetone bath was then removed and the mixture was allowed to warm to room temperature over 2 hours. The reaction was quenched by the addition of water (10 mL) and then extracted with DCM (2 x 20 mL). The organics were combined, dried (MgSO₄), filtered and concentrated to dryness. The residue was purified by FCC (1:20 MeOH/DCM) to provide the title compound. MS (ESI): mass calcd. for C₂₇H₁₇Cl₃N₂O, 490.0, m/z found 491.0 [M+H]⁺. ¹H NMR (300 MHz, CD₃OD) δ 8.54 (dd, *J* = 4.7, 1.7 Hz, 2H), 8.25 - 8.22 (m, 1H), 8.06 - 8.02 (m, 1H), 7.93 - 7.88 (m, 1H), 7.64 - 7.60 (m, 1H), 7.57 - 7.52 (m, 1H), 7.52 - 7.50 (m, 1H), 7.50 - 7.47 (m, 2H), 7.42 - 7.33 (m, 6H).

### Example 74: (2,4-Dichloro-3-(2-chlorophenyl)quinolin-6-yl)(oxazol-2-yl)(phenyl)methanol

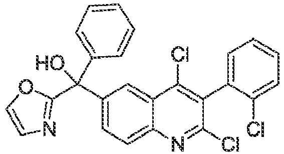

To a solution of 6-bromo-2,4-dichloro-3-(2-chlorophenyl)quinoline (387.5 mg, 1 mmol, Intermediate 32: step c) in THF (15 mL) at -78 °C was added nBuLi (2.5 M in hexanes, 0.52 mL, 1.3 mmol) dropwise and the resulting mixture stirred at -78 °C for 15 minutes. Then, phenyl(pyridin-4-yl)methanone (183 mg, 1 mmol) was added, and the resulting mixture stirred at -78 °C for 15 minutes. The dry ice - acetone bath was then removed and the mixture was allowed to warm to room temperature over 2 hours. The reaction was quenched by the addition of water (10 mL) and then extracted with DCM (2 x 20 mL). The organics were combined, dried (MgSO₄), filtered and concentrated to dryness. The residue was purified by FCC (1:5 EtOAc/petroleum ether) to provide the title compound. MS (ESI): mass calcd. for C₂₅H₁₅Cl₃N₂O₂, 480.0, m/z found 481.0 [M+H]⁺. ¹H NMR (300 MHz, CD₃OD) δ 8.34 (d, *J* = 1.8 Hz, 1H), 8.03-7.89 (m, 3H), 7.64-7.57 (m, 1H), 7.56-7.44 (m, 2H), 7.42-7.29 (m, 6H), 7.23 (s, 1H).

### Example 75: 6-((3-Chlorophenyl)(hydroxy)(6-(trifluoromethyl)pyridin-3-yl)methyl)-3-phenylquinoline-2-carbonitrile

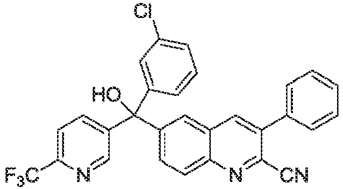

A pressure tube containing (3-chlorophenyl)(2,4-dichloro-3-phenylquinolin-6-yl)(6-(trifluoromethyl)pyridin-3-yl)methanol (94 mg, 0.17 mmol, Intermediate 22: step b), Pd₂(dba)₃ (8.0 mg, 0.0087 mmol), 1,1'-bis(diphenylphosphino)ferrocene (dppf, 10 mg, 0.018 mmol), zinc cyanide (25 mg, 0.21 mmol), and zinc nanopowder (3.0 mg, 0.046 mmol) in *N,N-*dimethylacetamide (1 mL) was sparged with nitrogen for 5 minutes, and then heated at 120 °C for 1 hour followed by 100 °C for 3 hours. The mixture was allowed to cool to room temperature and filtered through a syringe filter. The filtrate was concentrated *in vacuo,* then EtOAc and NH₄OH (aqueous) were added. The organic layer was separated, and the aqueous layer was extracted with EtOAc. The combined organic layers were dried (Na₂SO₄), filtered, and concentrated. The residue was purified by flash column chromatography (silica gel column, 30-50% EtOAc in heptane), and then by reverse phase HPLC (water/acetonitrile/0.1% TFA) to provide the title compound as a by-product. ¹H NMR (400 MHz, CDCl₃) δ 8.76 (d, *J* = 2.02 Hz, 1H), 8.25 (s, 1H), 8.20 (d, *J* = 9.60 Hz, 1H), 7.93 (dd, *J* = 2.02, 8.08 Hz, 1H), 7.75 - 7.79 (m, 2H), 7.71 (d, *J* = 8.08 Hz 1H), 7.60 - 7.65 (m, 2H), 7.51 - 7.58 (m, 3H), 7.32 - 7.40 (m, 3H), 7.15 (dt, *J* = 1.52, 7.58 Hz, 1H).

### Example 76: 6-((3-Chlorophenyl)(hydroxy)(2-(trifluoromethyl)pyridin4-yl)methyl)-3-phenylquinoline-2,4-dicarbonitrile

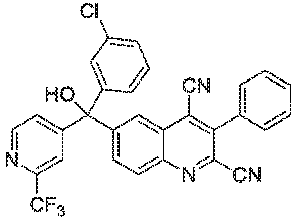

The title compound was prepared using (3-chlorophenyl)(2,4-dichloro-3-phenylquinolin-6-yl)(2-(trifluoromethyl)pyridin-4-yl)methanol (Example 77) in place of (3-chlorophenyl)(2,4-dichloro-3-phenylquinolin-6-yl)(6-(trifluoromethyl)pyridin-3-yl)methanol (Intermediate 22: step b) according to the procedure described in Example 75. ¹H NMR (400 MHz, CDCl₃) δ 8.81 (d, *J* = 5.56 Hz, 1H), 8.46 (d, *J* = 1.52 Hz, 1H), 8.31 (d, *J* = 9.09 Hz, 1H), 7.90 (d, *J* = 1.52 Hz, 1H), 7.84 (dd, *J* = 2.02, 8.59 Hz, 1H), 7.60 - 7.67 (m, 5H), 7.58 (dd, *J* = 1.52, 5.05 Hz, 1H), 7.35 - 7.46 (m, 2H), 7.23 - 7.27 (m, 1H), 7.10 (dt, *J=* 1.52, 8.08 Hz, 1H).

### Example 77: (3-Chlorophenyl)(2,4-dichloro-3-phenylquinolin-6-yl)(2-(trifluoromethyl)pyridin-4-yl)methanol

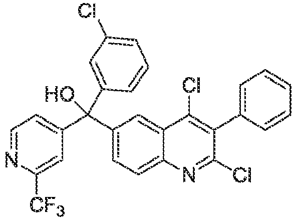

To a solution of 6-bromo-2,4-dichloro-3-phenylquinoline (286 mg, 0.810 mmol, Intermediate 7: step c) and (3-chlorophenyl)(2-(trifluoromethyl)pyridin-4-yl)methanone (231 mg, 0.810 mmol, Intermediate 23: step b) in THF (6 mL) at -78 °C was added 1.6 M *n*-BuLi in hexane (0.76 mL, 1.22 mmol). The mixture was stirred at -78 °C to 10 °C for 2 hours and then quenched with NH₄Cl (aqueous). The organic layer was separated, and the aqueous layer was extracted with CH₂Cl₂. The combined organic phases were dried (Na₂SO₄), filtered, concentrated, and purified by flash column chromatography (40 g silica gel column, 10 - 40% EtOAc in heptane) to afford the title compound as a yellow solid. ¹H NMR (400 MHz, CDCl₃) δ 8.74 (d, *J* = 5.56 Hz, 1H), 8.23 (d, *J* = 2.53 Hz, 1H), 8.09 (d, *J* = 9.09 Hz, 1H), 7.84 (d, *J* = 1.52 Hz, 1H), 7.68 (dd, *J =* 2.02, 8.59 Hz, 1H), 7.46 - 7.56 (m, 4H), 7.30 - 7.40 (m, 4H), 7.25 - 7.28 (m, 1H), 7.13 (dt, *J* = 1.52, 7.58 Hz, 1H).

### Example 78: (2,4-Dichloro-3-phenylquinolin-6-yl)(1-methylpiperidin4-yl)(pyridin-3-yl)methanol•TFA

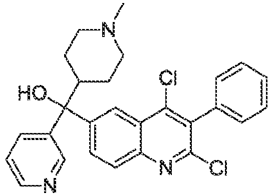

To a mixture of (2,4-dichloro-3-phenylquinolin-6-yl)(piperidin-4-yl)(pyridin-3-yl)methanol•TFA (15 mg, 0.022 mmol, Example 91), formaldehyde (0.010 mL, 0.13 mmol, 37% in water), and MeOH (1 mL) was added NaCNBH₃ (4.0 mg, 0.063 mmol). After stirring at room temperature overnight, the mixture was concentrated and purified by reverse phase HPLC (water / acetonitrile / 0.1% TFA) to provide the title compound as a white solid. ¹H NMR (400 MHz, CD₃OD) δ 8.97 (s, 1H), 8.56 - 8.62 (m, 2H), 8.44 (d, *J* = 8.31 Hz, 1H), 8.03 (s, 2H), 7.70 - 7.76 (m, 1H), 7.47 - 7.57 (m, 3H), 7.33 (d, *J* = 6.85 Hz, 2H), 3.50 - 3.60 (m, 2H), 3.03 - 3.19 (m, 3H), 2.86 (s, 3H), 1.82 - 1.98 (m, 2H), 1.64 - 1.77 (m, 2H); MS m/e 478.0 [M+H]⁺.

### Example 79: (3-Chlorophenyl)(2-isopropoxy-3-phenylquinolin-6-yl)(pyridin-3-yl)methanol•TFA

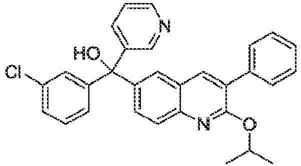

A mixture of (3-chlorophenyl)(2,4-dichloro-3-phenylquinolin-6-yl)(pyridin-3-yl)methanol (21 mg, 0.043 mmol, Intermediate 24) and NaO*i*Pr (11 mg, 0.13 mmol) in *i*PrOH (0.4 mL) was heated in a sealed tube at 80 °C for 5 hours. More NaO*i*Pr (15 mg, 0.18 mmol) was added and the mixture was heated at the same temperature for 21 hours. After cooling to room temperature, the mixture was purified by reverse phase HPLC (water / acetonitrile / 0.1% TFA) to provide the title compound as a by-product. ¹H NMR (400 MHz, CD₃OD) δ 8.86 (s, 1H), 8.75 - 8.81 (m, 1H), 8.52 (d, *J=* 8.31 Hz, 1H), 8.03 (s, 1H), 7.97 - 8.03 (m, 1H), 7.82 (d, *J=* 8.80 Hz, 1H), 7.65 (d, *J=* 2.20 Hz, 1H), 7.56 - 7.63 (m, 3H), 7.46 - 7.48 (m, 1H), 7.33 - 7.45 (m, 5H), 7.25 - 7.29 (m, 1H), 5.59 - 5.66 (m, 1H), 1.38 (d, *J* = 6.36 Hz, 6H).

### Example 80: (4-Butyl-2-isopropoxy-3-phenylquinolin-6-yl)(1-methyl-1H-imidazol-5-yl)(pyridin4-yl)methanol•TFA

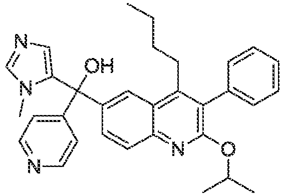

A mixture of (4-butyl-2-chloro-3-phenylquinolin-6-yl)(1-methyl-1*H*-imidazol-5-yl)(pyridin-4-yl)methanol**•**TFA (16 mg, 0.023 mmol, Intermediate 26) and NaO*i*Pr (19 mg, 0.23 mmol) in *i*PrOH (0.4 mL) was heated in a sealed tube at 80 °C for 17 hours. More NaO*i*Pr (7.0 mg, 0.085 mmol) was added and the mixture was heated at the same temperature for 64 hours. After cooling to room temperature, the mixture was purified by reverse phase HPLC (water / acetonitrile / 0.1% TFA) to provide the title compound. ¹H NMR (400 MHz, CD₃OD) δ 9.06 (s, 1H), 8.78 (d, *J* = 6.57 Hz, 2H), 7.86 - 7.90 (m, 4H), 7.67 (dd, *J=* 2.02, 9.09 Hz, 1H), 7.36 - 7.47 (m, 3H), 7.16 - 7.20 (m, 2H), 7.12 (d, *J* = 1.52 Hz, 1H), 5.44 - 5.52 (m, 1H), 3.72 (s, 3H), 2.66 - 2.74 (m, 2H), 1.34 - 1.44 (m, 2H), 1.22 (d, *J* = 6.06 Hz, 6H), 1.12 - 1.19 (m, 2H), 0.73 (t, *J =* 7.33 Hz, 3H); MS m/e 507.3 [M+H]⁺.

### Example 81: (3-Chlorophenyl)(2,4-diethoxy-3-phenylquinolin-6-yl)(pyridin-3-yl)methanol•TFA

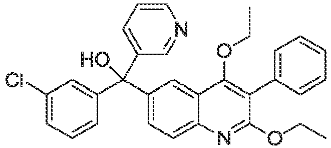

A mixture of (3-chlorophenyl)(2,4-dichloro-3-phenylquinolin-6-yl)(pyridin-3-yl)methanol (27 mg, 0.055 mmol, Intermediate 24) and NaOEt (0.30 mL, 3.8 mmol, 21% wt. in EtOH) was heated in a sealed tube at 82 °C for 24 hours. After cooling to room temperature, the mixture was purified by reverse phase HPLC (water / acetonitrile / 0.1% TFA) to provide the title compound. ¹H NMR (400 MHz, CD₃OD) δ 8.89 (s, 1H), 8.81 (s, 1H), 8.55 (d, *J=* 8.07 Hz, 1H), 8.05 (br. s., 1H), 7.87 (s, 1H), 7.84 (d, *J* = 8.80 Hz, 1H), 7.61 (d, *J* = 7.09 Hz, 1H), 7.49 (s, 1H), 7.33 - 7.45 (m, 7H), 7.29 (br. s., 1H), 4.47 (q, *J* = 7.09 Hz, 2H), 3.51 - 3.63 (m, 2H), 1.30 (t, *J* = 6.97 Hz, 3H), 0.95 (t, *J* = 7.09 Hz, 3H).

### Example 82: (4-Chloro-2-(methyl(2-(methylamino)ethyl)amino)-3-phenylquinolin-6-yl)(1-methyl-1H-inddazol-5-yl)(pyridin4-yl)methanol•TFA

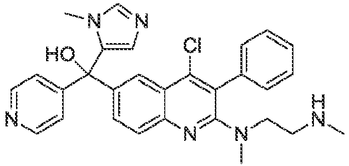

A mixture of (2,4-dichloro-3-phenylquinolin-6-yl)(1-methyl-1*H*-imidazol-5-yl)(pyridin-4-yl)methanol•TFA (18 mg, 0.026 mmol, Intermediate 25: step c) and *N*¹,*N*²-dimethylethane-1,2-diamine (500 mg, 5.67 mmol) was heated in a sealed tube at 80 °C for 24 hours. After cooling to room temperature, the mixture was purified by reverse phase HPLC (water / acetonitrile / 0.1% TFA) to provide the title compound. ¹H NMR (400 MHz, CD₃OD) δ 9.09 (s, 1H), 8.87 (br. s., 2H), 8.25 (d, *J=* 2.02 Hz, 1H), 8.09 (d, *J=* 6.57 Hz, 2H), 7.90 (d, *J=* 9.09 Hz, 1H), 7.72 (dd, *J=* 2.02, 8.59 Hz, 1H), 7.49 - 7.59 (m, 2H), 7.43 - 7.49 (m, 1H), 7.35 - 7.43 (m, 2H), 7.21 (d, *J* = 1.52 Hz, 1H), 3.76 - 3.88 (m, 2H), 3.70 (s, 3H), 3.22 - 3.29 (m, 2H), 2.76 (s, 3H), 2.51 (s, 3H); MS m/e 513.0 [M+H]⁺.

### Example 83: 2-((4-Chloro-6-(hydroxy(1-methyl-1H-imidazol-5-yl)(pyridin4-yl)methyl)-3-phenylquinolin-2-yl)(methyl)amino)ethano]•TFA

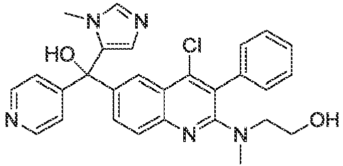

A mixture of (2,4-dichloro-3-phenylquinolin-6-yl)(1-methyl-1*H*-imidazol-5-yl)(pyridin-4-yl)methanol**•**TFA (20 mg, 0.029 mmol, Intermediate 25: step c) and 2-(methylamino)ethanol (408 mg, 5.43 mmol) was heated in a sealed tube at 80 °C for 16 hours. After cooling to room temperature, the mixture was purified by reverse phase HPLC (water / acetonitrile / 0.1% TFA) to provide the title compound. ¹H NMR (400 MHz, CD₃OD) δ 9.08 (s, 1H), 8.81 (br. s., 2H), 8.37 (s, 1H), 7.97 (d, *J* = 6.06 Hz, 2H), 7.89 (s, 2H), 7.59 - 7.64 (m, 1H), 7.52 - 7.58 (m, 2H), 7.42 - 7.47 (m, 2H), 7.21 (d, *J=* 1.52 Hz, 1H), 3.81 (t, *J=* 4.55 Hz, 2H), 3.69 (s, 3H), 3.64 (t, *J=* 4.55 Hz, 2H), 2.78 (s, 3H); MS m/e 500.0 [M+H]⁺.

### Example 84: (4-Chloro-2-(dimethylamino)-3-phenylquinolin-6-yl)(pyridin-2-yl)(pyridin4-yl)methanol•TFA

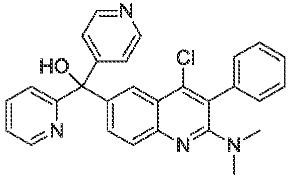

A mixture of (2,4-dichloro-3-phenylquinolin-6-yl)(pyridin-2-yl)(pyridin-4-yl)methanol**•**TFA (35 mg, 0.051 mmol, Intermediate 27) and dimethylamine (0.8 mL, 1.6 mmol, 2.0 M in MeOH) was heated in a sealed tube at 80 °C for 4.5 days. After cooling to room temperature, the mixture was purified by reverse phase HPLC (water / acetonitrile / 0.1% TFA) to provide the title compound. ¹H NMR (400 MHz, CD₃OD) δ 8.81 (d, *J* = 7.09 Hz, 2H), 8.57 - 8.61 (m, 1H), 8.20 - 8.26 (m, 3H), 8.06 (d, *J* = 8.80 Hz, 1H), 7.92 - 7.98 (m, 1H), 7.85 - 7.92 (m, 2H), 7.50 - 7.61 (m, 3H), 7.37 - 7.47 (m, 3H), 3.00 (s, 6H).

### Example 85: (2-Chloro-4-(dimethylamino)-3-phenylquinolin-6-yl)(1-methyl-1H-imidazol-5-yl)(pyridin-2-yl)methanol•TFA

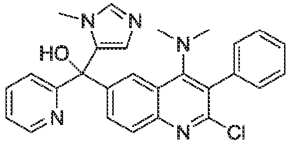

A mixture of (2,4-dichloro-3-phenylquinolin-6-yl)(1-methyl-1*H*-imidazol-5-yl)(pyridin-2-yl)methanol (28 mg, 0.41 mmol, Example 64) and dimethylamine (0.8 mL, 1.6 mmol, 2.0 M in MeOH) was heated in a sealed tube at 80 °C for 4.5 days. After cooling to room temperature, the mixture was purified by reverse phase HPLC (water / acetonitrile / 0.1% TFA) to afford the title compound. ¹H NMR (400 MHz, CD₃OD) δ 8.94 (s, 1H), 8.59 - 8.64 (m, 1H), 8.18 - 8.20 (m, 1H), 7.87 - 7.96 (m, 3H), 7.77 (d, *J* = 8.07 Hz, 1H), 7.41 - 7.53 (m, 4H), 7.25 - 7.32 (m, 2H), 7.03 (d, *J* = 1.71 Hz, 1H), 3.64 (s, 3H), 2.69 (s, 6H); MS m/e 470.0 [M+H]⁺.

### Example 86: (2,4-Dichloro-3-phenylquinolin-6-yl)(2-fluoropyridin4-yl)(1-methyl-1H-imidazol-2-yl)methanol

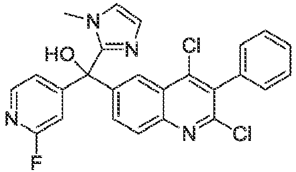

The title compound was prepared using (2-fuoropyridin-4-yl)(1-methyl-1*H*-imidazol-2-yl)methanone (Intermediate 28: step b) in place of (3-chlorophenyl)(2-(trifluoromethyl)pyridin-4-yl)methanone (Intermediate 23: step b) according to the procedure described in Example 77. ¹H NMR (400 MHz, CDCl₃) δ 8.18 (d, *J* = 1.96 Hz, 1H), 8.15 (d, *J* = 5.38 Hz, 1H), 8.05 (d, *J* = 8.80 Hz, 1H), 7.73 (dd, *J* = 2.20, 8.80 Hz, 1H), 7.49 - 7.53 (m, 3H), 7.27 - 7.34 (m, 2H), 7.21 (dt, *J* = 1.59,5.38 Hz, 1H), 6.99 (s, 1H), 6.91 (d, *J* = 1.22 Hz, 1H), 6.88 (d, *J* = 1.22 Hz, 1H), 3.39 (s, 3H); MS m/e 478.8 [M+H]⁺.

### Example 87: (4-Chlorophenyl)(2,4-dichloro-3-phenylquinolin-6-yl)(2,4-dimethylthiazol-5-yl)methanol

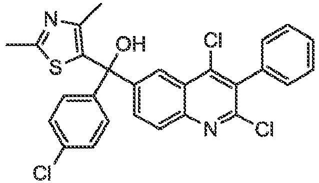

To a flask containing 6-bromo-2,4-dichloro-3-phenylquinoline (255 mg, 0.72 mmol, Intermediate 7: step c) was added THF (8 mL) to give a homogeneous clear solution. The solution was cooled in a -78 °C bath and then n-BuLi (2.5 M in hexanes, 0.26 mL, 0.65 mmol) was added which resulted in an immediate orange homogeneous solution. After approximately 2 minutes, (4-chlorophenyl)(2,4-dimethylthiazol-5-yl)methanone (210 mg, 0.83 mmol, Intermediate 30, in 3 mL THF) was added. The reaction mixture was maintained at -75 °C for 5 minutes then replaced with a 0 °C ice-bath. After 45 minutes, the reaction mixture was quenched with aqueous NH₄Cl solution. The aqueous portion was extracted with EtOAc (3 x 50 mL) and the combined organics were washed with brine, dried over MgSO₄, filtered and concentrated to dryness. Chromatography on silica gel (100% DCM increasing to 20% EtOAc-DCM) provided the title compound as a white amorphous solid. ¹H NMR (500 MHz, CD₂Cl₂) δ 8.33 (s, 1H), 8.01 (d, *J* = 8.8 Hz, 1H), 7.82 (dd, *J* = 8.9, 2.1 Hz, 1H), 7.59 - 7.46 (m, 3H), 7.46 - 7.28 (m, 6H), 2.54 (s, 3H), 2.03 (s, 3H). MS (ESI): mass calcd. for C₂₇H₁₉Cl₃N₂OS, 524.0, m/z found 525.0 [M+H]⁺.

### Example 88: (2,4-Dimethoxy-3-phenylquinolin-6-yl)(1-methyl-1H-imidazol-5-yl)(pyrimidin-2-yl)methanol

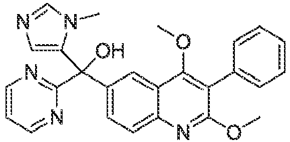

Purification of the crude reaction mixture from the synthesis of (4-chloro-2-methoxy-3-phenylquinolin-6-yl)(1-methyl-1*H*-imidazol-5-yl)pyrimidin-2-ylmethanol (Example 60a) also afforded the title compound as a regioisomer. MS (ESI): mass calcd. for C₂₆H₂₃N₅O₃, 453.2; m/z found, 454.3 [M+H]⁺. ¹H NMR (400 MHz, CD₃OD) δ 8.86 (d*, J* = 4.9 Hz, 2H), 8.26 (d, *J* = 1.7 Hz, 1H), 7.86 - 7.75 (m, 3H), 7.47 - 7.36 (m, 6H), 6.52 (s, 1H), 3.95 (s, 3H), 3.48 (s, 3H), 3.44 (s, 3H).

### Example 89: (2-Chloro-3-phenylquinolin-6-yl)(4-chlorophenyl)(1-methyl-1H-imidazol-5-yl)methanol

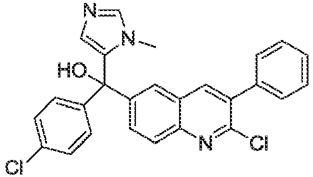

To a 50-mL round-bottom flask containing a solution of 6-bromo-2-chloro-3-phenylquinoline (210 mg, 0.66 mmol, Intermediate 31: step b) in tetrahydrofuran (10 mL) was added 2.5 M *n-*BuLi in hexanes (0.29 mL, 0.72 mmol) dropwise with stirring at -78 °C. After 45 minutes at - 78 °C, a solution of (4-chlorophenyl)(1-methyl-1*H*-imidazol-5-yl)methanone (132 mg, 0.60 mmol, Intermediate 8: step b) in tetrahydrofuran (2 mL) was added dropwise. The resulting solution was stirred at -78 °C for an additional 2 hours, then quenched with 30 mL of aqueous NH₄Cl and extracted with ethyl acetate (2 x 30 mL). The combined organic layers were dried over anhydrous sodium sulfate and concentrated under vacuum. The residue was purified by FCC (20:1 DCM/MeOH) to provide the title compound as a white solid. MS (ES): mass calcd. for C₂₆H₁₉Cl₂N₃O, 459.1; m/z found, 460.0 [M+H]⁺. ¹H NMR (400 MHz, CD₃OD) δ 8.96 (d, *J* = 3.9 Hz, 1H), 8.33 (s, 1H), 8.05 (d, *J* = 6.0 Hz, 1H), 7.94 (s, 1H), 7.88 (d, *J* = 6.6 Hz, 1H), 7.42-7.56 (m, 9H), 6.93 (s, 1H), 3.71 (s, 3H).

### Example 90: (4-Chloro-2-(dimethylamino)-3-phenylquinolin-6-yl)(4-chlorophenyl)(1-methyl-1H-imidazol-5-yl)methanol

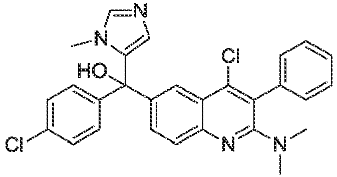

(4-Chlorophenyl)(2,4-dichloro-3-phenylquinolin-6-yl)(1-methyl-1*H*-imidazol-5-yl)methanol (124 mg, 0.251 mmol, Example 65) was treated with dimethylamine (2 M in MeOH, 2 mL, 4 mmol) and the resulting suspension was heated in a sealed tube in an 85 °C oil bath for 2 days. The mixture was concentrated *in vacuo* and the residue was purified by flash column chromatography (Biotage NH column, 0-1% MeOH-DCM) to provide the title compound as a cream-colored foam. ¹H NMR (400 MHz, CDCl₃) δ 8.10 (d, *J* = 2.20 Hz, 1H), 7.72 (d, *J* = 8.80 Hz, 1H), 7.43 - 7.52 (m, 3H), 7.34 - 7.43 (m, 3H), 7.22 - 7.34 (m, 6H), 6.33 (s, 1H), 3.36 (s, 3H), 2.72 (s, 6H).

### Example 91: (2,4-Dichloro-3-phenylquinolin-6-yl)(piperidin-4-yl)(pyridin-3-yl)methanol•TFA

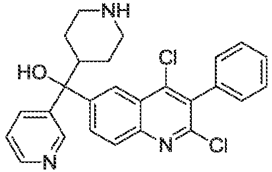

A solution of *tert-butyl* 4-((2,4-dichloro-3-phenylquinolin-6-yl)(hydroxy)(pyridin-3-yl)methyl)piperidine-1-carboxylate (149 mg, 0.260 mmol, Intermediate 29) and TFA (1 mL) was stirred at room temperature for 1 hour and concentrated to provide the title compound. ¹H NMR (400 MHz, CD₃OD) δ 9.12 (s, 1H), 8.69 - 8.77 (m, 2H), 8.62 (s, 1H), 8.02 - 8.11 (m, 2H), 7.97 (dd, *J* = 5.56, 8.08 Hz, 1H), 7.49 - 7.58 (m, 3H), 7.30 - 7.36 (m, 2H), 3.40 - 3.50 (m, 2H), 3.17 - 3.27 (m, 1H), 3.05 - 3.17 (m, 2H), 1.75 - 1.94 (m, 2H), 1.60 -1.74 (m, 2H).

### IN VITRO BIOLOGICAL DATA

### ThermoFluor® Assay

ThermoFluor® is a fluorescence based assay that estimates ligand binding affinities by measuring the effect of a ligand on protein thermal stability (Pantoliano, M. W., Petrella, E. C., Kwasnoski, J. D., Lobanov, V. S., Myslik, J., Graf, E., Carver, T., Asel, E., Springer, B. A., Lane, P., and Salemme, F. R. (2001) High-density miniaturized thermal shift assays as a general strategy for drug discovery. J Biomol Screen 6, 429-40, and Matulis, D., Kranz, J. K., Salemme, F. R., and Todd, M. J. (2005) Thermodynamic stability of carbonic anhydrase: measurements of binding affinity and stoichiometry using ThermoFluor. Biochemistry 44, 5258-66). This approach is applicable to a wide variety of systems, and rigorous in theoretical interpretation through quantitation of equilibrium binding constants *(K_{D}).*

In a ThermoFluor® experiment where protein stability is monitored as the temperature is steadily increased, an equilibrium binding ligand causes the midpoint of an unfolding transition (*Tₘ*) to occur at a higher temperature. The shift in the melting point described as a Δ*Tₘ* is proportional to the concentration and affinity of the ligand. The compound potency may be compared as a rank order of either Δ*Tₘ* values at a single compound concentration or in terms of *K_{D}* values, estimated from concentration response curves.

### RORγt ThermoFluor® Assay Construct

For the RORγt construct used in the ThermoFluor® assay, numbering for the nucleotide sequences was based on the reference sequence for human RORγt, transcript variant 2, NCBI Accession: NM_001001523.1 (SEQ ID NO:1). Nucleotides 850-1635 (SEQ ID NO:2) coding for the wild type human RORγt ligand binding domain (RORγt LBD) were cloned into the pHIS1 vector, a modified pET *E. coli* expression vector (Accelagen, San Diego), containing an in-frame N-terminal His-tag and a TurboTEV protease cleavage site (ENLYFQG, SEQ ID NO:3) upstream of the cloned insert sequence. The amino acid sequence for the RORγt construct used in the Thermofluor assay is shown as SEQ ID NO:4.

ThermoFluor® experiments were carried out using instruments owned by Janssen Research and Discovery, L.L.C. through its acquisition of 3-Dimensional Pharmaceuticals, Inc. 1,8-ANS (Invitrogen) was used as a fluorescent dye. Protein and compound solutions are dispensed into black 384-well polypropylene PCR microplates (Abgene) and overlayed with silicone oil (1 µL, Fluka, type DC 200) to prevent evaporation.

Bar-coded assay plates are robotically loaded onto a thermostatically controlled PCR-type thermal block and then heated at a typical ramp-rate of 1 °C/min for all experiments. Fluorescence was measured by continuous illumination with UV light (Hamamatsu LC6) supplied via fiber optic and filtered through a band-pass filter (380-400 nm; >6 OD cutoff). Fluorescence emission of the entire 384-well plate was detected by measuring light intensity using a CCD camera (Sensys, Roper Scientific) filtered to detect 500 ± 25 nm, resulting in simultaneous and independent readings of all 384 wells. Images were collected at each temperature, and the sum of the pixel intensity in a given area of the assay plate was recorded versus temperature. Reference wells contained RORγt without compounds, and the assay conditions were as follows:
0.065 mg/mL RORγt
60 µM 1,8-ANS
100 mM Hepes, pH 7.0
10 mM NaCl
2.5 mM GSH
0.002% Tween-20

Project compounds were arranged in a pre-dosed mother plate (Greiner Bio-one) wherein compounds are serially diluted in 100% DMSO by 1:2 from a high concentration of 10 mM over 12 columns within a series (column 12 is a reference well containing DMSO, no compound). The compounds were robotically dispensed directly into assay plates (1x = 46 nL) using a Hummingbird capillary liquid handling instrument (Digilab). Following compound dispense, protein and dye in buffer was added to achieve the final assay volume of 3 µL, followed by 1 µL of silicone oil.

The binding affinity was estimated as described previously (Matulis, D., Kranz, J. K., Salemme, F. R., and Todd, M. J. (2005) Thermodynamic stability of carbonic anhydrase: measurements of binding affinity and stoichiometry using ThermoFluor®. Biochemistry 44, 5258-66) using the following thermodynamic parameters of protein unfolding:
Reference RORγt *Tₘ*: 47.8 °C
ΔH_{(T*m*)} = 115 kcal/mol
ΔC_{p(T*m*)} = 3 kcal/mol

### CELL BASED BIOLOGICAL DATA

### RORγt Reporter Assay

A reporter assay was used to test functional activity of RORγt modulatory compounds on transcriptional activation driven by the RORγt LBD. Cells used in the assay were co-transfected with two constructs. The first construct, pBIND-RORγt LBD, contained the wild type human RORγt LBD fused to the DNA binding domain of the GAL4 protein. The second construct, pGL4.31 (Promega Cat no. C935A), contained multiple GAL4 responsive DNA elements upstream of firefly luciferase. To generate a background control, cells were similarly co-transfected with two constructs, but in the first construct the AF2 amino acid motif in the RORγt LBD was changed from LYKELF (SEQ ID NO:5) to LFKELF (SEQ ID NO:6). The AF2 mutation has been shown to prevent co-activator binding to the RORγt LBD, thus preventing transcription of firefly luciferase. The mutant construct was called pBIND-RORγt-AF2.

For the RORγt constructs used in the reporter assay, numbering for the nucleotide sequences was also based on the reference sequence for human RORγt, transcript variant 2, NCBI Accession: NM_001001523.1 (SEQ ID NO:1). For the wild type human RORγt LBD construct, pBIND-RORγt LBD, nucleotides 850-1635 (SEQ ID NO:2) coding for the wild type human RORγt LBD were cloned into EcoRI and NotI sites in the pBIND vector (Promega cat. No E245A). The pBIND vector contains the GAL4 DNA Binding Domain (GAL4 DBD) and the renilla luciferase gene under control of the SV40 promoter. Renilla luciferase expression serves as a control for transfection efficiency and cell viability. For the background control construct, pBIND-RORγt-AF2, the AF2 domain of RORγt LBD was mutated using the Quik Change II Site Directed Mutagenesis System (Stratagene Cat. No. 200519). The nucleotide sequence coding for the RORγt LBD sequence with the mutated AF2 domain is shown as SEQ ID NO:7. The amino acid sequences for the wild type RORγt LBD and RORγt LBD with the mutated AF2 domain are shown as SEQ ID NO:8 and SEQ ID NO:9, respectively.

The reporter assay was performed by transiently transfecting HEK293T cells with 5 µg of pBIND-RORγt LBD or pBIND-RORγt LBD-AF2 and 5 µg pGL4.31 (Promega Cat no. C935A) using Fugene 6 (Invitrogen Cat no. E2691) at a 1:6 ratio of DNA: Fugene 6 in a T-75 flask in which cells were at least 80% confluent. Twenty four hours after bulk transfection, cells were plated into 96-well plates at 50,000 cells/well in phenol-red free DMEM containing 5% Lipid Reduced FCS and Pen/Strep. Six hours after plating, cells were treated with compounds for 24 hours. Media was removed and cells were lysed with 50 µL 1x Glo Lysis Buffer (Promega). Dual Glo Luciferase Reagent (50 µL/well) was then added and firefly luciferase luminescence was read on an Envision after a ten minute incubation. Finally, Stop and Glo reagent (50 µL/well) was added and renilla luciferase luminescence was read on an Envision instrument after a ten minute incubation. To calculate the effect of compounds on RORγt activity, the ratio of firefly to renilla luciferase was determined and plotted against compound concentration. Agonist compounds increase RORγt-driven luciferase expression, and antagonist or inverse agonist compounds decrease luciferase expression.

### Human Th17 Assay

The human Th17 assay tests the effect of RORγt modulatory compounds on IL-17 production by CD4 T cells under conditions which favor Th17 differentiation. Total CD4⁺ T cells were isolated from the peripheral blood mononuclear cells (PBMC) of healthy donors using a CD4⁺ T cell isolation kit II, following the manufacturer's instructions (Miltenyi Biotec). Cells were resuspended in a medium of RPMI-1640 supplemented with 10% fetal bovine serum, penicillin, streptomycin, glutamate, and β-mercaptoethanol and were added to 96-well plates at 1.5x10⁵ per 100 µL per well. 50 µL of compound at titrated concentrations in DMSO were added into each well at final DMSO concentration at 0.2%. Cells were incubated for 1 hour, then 50 µL of Th17 cell differentiation medium was added to each well. The final concentrations of antibodies and cytokines (R&D Systems) in differentiation medium were: 3x10⁶/mL anti-CD3/CD28 beads (prepared using human T cell activation/expansion kit, Miltenyi Biotec), 10 µg/mL anti-IL4, 10 µg/mL anti-IFNγ, 10 ng/mL IE1β, 10 ng/mL IL23, 50 ng/mL IL6, 3 ng/mL TGFβ and 20 U/mL IL2. Cells were cultured at 37 °C and 5% CO₂ for 3 days. Supernatants were collected and the accumulated IL-17 in culture was measured by using MULTI-SPOT® Cytokine Plate following manufacture's instruction (Meso Scale Discovery). The plate was read using Sector Imager 6000, and IL-17 concentration was extrapolated from the standard curve. The IC50s were determined by GraphPad.

**Table 1**

| Example Number | ThermoFluor® Assay, Kd (µM) | RORγt reporter Assay, IC50 (µM) | RORγt reporter Assay, % inhibition @ 6 µM | Human Th17 Assay, IC₅₀ (µM) |
|---|---|---|---|---|
| 1a | ND | ND | ND | ND |
| 1b | 0.13 | 0.16 | 89 | 0.4 |
| 1c | 0.033 | 0.11 | 95 | 0.31 |
| 2a | ND | ND | ND | ND |
| 2b | 0.13 | 0.2 | 91 | 2.9 |
| 2c | 0.14 | 0.17 | 95 | 0.67 |
| 3a | ND | ND | ND | ND |
| 3b | 0.00056 | 0.003 | 96 | 0.013 |
| 3c | 0.0023 | 0.0066 | 98 | 0.043 |
| 4a | ND | ND | ND | ND |
| 4b | 0.0044 | 0.0095 | 98 | 0.061 |
| 4c | 0.0014 | 0.011 | 98 | 0.031 |
| 5* | 0.62 | 1.7 | 87 | ND |
| 6* | 0.94 | 1.9 | 92 | ND |
| 7a | 0.072 | >6 | -60 | ND |
| 7b | 0.037 | >6 | -78 | >6 |
| 7c | 0.77 | >6 | -20 | >6 |
| 8 | 0.27 | 0.89 | 87 | ND |
| 9a | 0.068 | 0.27 | 90 | ^{∼}0.9 |
| 9b | 0.7 | 1.2 | 95 | ND |
| 9c | 0.032 | 0.31 | 96 | ND |
| 10 | 0.2 | 0.32 | 89 | 1.5 |
| 11 | 0.64 | >6 | 32 | ND |
| 12 | 0.65 | ^{∼}2 | 90 | ND |
| 13 | 2 | >6 | -52 | ND |
| 14a | 0.0067 | 1.1 | 55 | ND |
| 14b | 0.0052 | 0.24 | 53 | 0.079 |
| 14c | 0.14 | 0.51 | 87 | 0.31 |
| 15 | 0.021 | >6 | -29 | ND |
| 16 | 0.027 | 0.2 | 86 | 0.081 |
| 17 | 0.026 | 0.53 | 87 | ND |
| 18a | ND | ND | ND | ND |
| 18b | 14 | >6 | 40 | ND |
| 18c | 0.33 | 0.85 | 95 | ND |
| 19a | ND | ND | ND | ND |
| 19b | 0.36 | 0.3 | 94 | 0.4 |
| 19c | 0.017 | >6 | 35 | ND |
| 20 | ND | ND | ND | ND |
| 21a | ND | ND | ND | ND |
| 21b | 1.3 | 1.6 | 87 | ND |
| 21c | 0.11 | 0.19 | 96 | 0.18 |
| 22 | ND | ND | ND | ND |
| 23a | ND | ND | ND | ND |
| 23b | 15 | ^{∼}4 | 60 | ND |
| 23c | 0.15 | 0.23 | 100 | 0.4 |
| 24 | ND | ND | ND | ND |
| 25a | ND | ND | ND | ND |
| 25b | 3.5 | ^{∼}2 | 87 | ND |
| 25c | 0.061 | 0.16 | 95 | 0.16 |
| 26 | ND | ND | ND | ND |
| 27a | ND | ND | ND | ND |
| 27b | 15 | ^{∼}4 | 80 | ND |
| 27c | 0.82 | 2.1 | 96 | ND |
| 28 | 0.046 | 0.024, ^{∼}0.04 | 96 | ND |
| 29 | 0.026 | 0.085 | 97 | ND |
| 30a | 0.023 | 0.11 | 91 | ND |
| 30b | 0.3 | 0.049 | 95 | 0.057 |
| 30c | 0.009 | 0.14 | 93 | 2.2 |
| 31a | ND | 0.15 | 102 | ND |
| 31b | 0.005 | 0.042 | 103 | 0.02 |
| 31c | 3.3 | 1.6 | 85 | ND |
| 32a* | ND | ND | ND | ND |
| 32b* | 0.77 | 1.2 | 92 | ND |
| 33a* | 0.16 | 0.65 | 98 | ND |
| 33b* | 13 | ^{∼}4 | 64 | ND |
| 33c* | 0.077 | 0.13 | 99 | 0.29 |
| 34a | ND | ND | ND | ND |
| 34b | 0.45 | 1 | 90 | ND |
| 34c | 0.1 | 0.27 | 96 | 0.26 |
| 35 | 0.018 | 0.16 | 101 | ND |
| 36 | 0.00089 | 0.011, ^{∼}0.04 | 97 | ND |
| 37 | 0.0072 | 0.26 | 90 | ND |
| 38 | 0.00026 | 0.03 | 93 | ND |
| 39 | 0.0052 | 0.034 | 103 | ND |
| 40a | 0.0015 | 0.023 | 104 | ND |
| 40b | 0.15 | 0.68 | 97 | ND |
| 40c | 0.12 | 0.19 | 103 | 0.21 |
| 41a | 0.089 | 0.32 | 98 | ND |
| 41b | 0.0014 | 0.0086 | 102 | 0.007 |
| 41c | 0.0065 | 0.27 | 102 | 0.21 |
| 42 | 0.00051 | 0.017 | 103 | ND |
| 43 | 0.0035 | 0.039 | 98 | ND |
| 44 | 5.4 | >6 | 31 | ND |
| 45 | 0.77 | 0.57 | 93 | ND |
| 46 | 0.23 | 0.83 | 106 | ND |
| 47a* | 0.0083 | 0.04 | 98 | ND |
| 47b* | 0.93 | ^{∼}2 | 80 | ND |
| 47c* | 0.0019 | 0.025 | 100 | 0.054 |
| 48 | 0.81 | 0.67 | 103 | ND |
| 49a | 0.1 | 0.22 | 104 | ND |
| 49b | 10 | ^{∼}5 | 71 | ND |
| 49c | 0.065 | 0.23 | 100 | 0.28 |
| 50 | 0.09 | 0.031 | 99 | ND |
| 51 | 1.1 | 1.1 | 96 | ND |
| 52 | 3.5 | ^{∼}6 | 52 | ND |
| 53 | 0.0072 | ND | ND | ND |
| 54 | 0.38 | 0.44 | 102 | ND |
| 55 | 3.3 | ^{∼}3 | 70 | ND |
| 56 | 0.06 | 0.023 | 102 | ND |
| 57 | 0.58 | 0.69 | 100 | ND |
| 58 | 0.22 | 0.15 | 99 | ND |
| 59 | 1.2 | 0.54 | 98 | ND |
| 60a* | ND | ND | ND | ND |
| 60b* | 0.9 | ^{∼}2 | 87 | ND |
| 61* | 0.45 | 0.13 | 105 | 0.48 |
| 62* | 0.057 | 0.12 | 110 | 0.1 |
| 63* | 0.0093 | >6 | 26 | ND |
| 64* | 0.96 | 1 | 93 | ND |
| 65* | 0.16 | 0.26 | 101 | 1.8 |
| 66* | 0.031 | 0.18 | 100 | ND |
| 67* | 0.0035 | 0.02 | 106 | ND |
| 68* | 0.0011 | 0.01 | 104 | 0.02 |
| 69 | ND | ND | ND | ND |
| 70 | ND | ND | ND | ND |
| 71 | 2.2 | 1.4 | 84 | ND |
| 72 | 0.89 | 0.63 | 100 | ND |
| 73 | 20 | 0.7 | 80 | ^{∼}6 |
| 74 | 11 | >6 | 60 | ND |
| 75 | 14 | 2.9 | 99 | ND |
| 76 | 18 | ^{∼}2 | 80 | ND |
| 77 | 12 | >6 | 48 | ND |
| 78 | 14 | >6 | 33 | ND |
| 79 | 17 | ND | ND | ND |
| 80 | 12 | >6 | 72 | ND |
| 81 | 14 | ND | ND | ND |
| 82 | 11 | >6 | 18 | ND |
| 83 | 15 | 1.9 | 73 | ND |
| 84 | 17 | 3.5 | 93 | ND |
| 85 | 20 | 3.5 | 81 | ND |
| 86 | 20 | >6 | 37 | ND |
| 87 | 12 | >6 | 56 | ND |
| 88 | 20 | >6 | 32 | ND |
| 89 | 10 | >4 | 57 | ND |
| 90 | ND | ND | ND | ND |
| 91 | 21 | >6 | 0 | ND |

| | | | | |
|---|---|---|---|---|
| * = reference | | | | |

All data shown in Table 1 is either the value of one data point or the average of more than one data point. In cases where more than one value is shown in a table cell, values with qualifiers such as ∼, > or < shown on the right side of the table cell could not be included in the averaging calculation for the value shown on the left side of the table cell. ND - no data.

While the foregoing specification teaches the principles of the present invention, with examples provided for the purpose of illustration, it will be understood that the practice of the invention encompasses all of the usual variations, adaptations and/or modifications as come within the scope of the following claims.

## Claims

1. The compounds of Formula I: 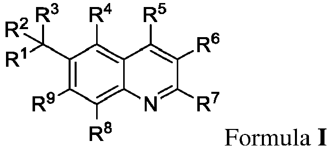 wherein:
R¹ is azetidinyl, pyrrolyl, pyrazolyl, imidazolyl, triazolyl, thiazolyl, pyridyl, pyridyl *N-*oxide, pyrazinyl, pyrimidinyl, pyridazyl, piperidinyl, tetrahydropyranyl, phenyl, oxazolyl, isoxazolyl, thiophenyl, benzoxazolyl, or quinolinyl; wherein said piperidinyl, pyridyl, pyridyl *N-*oxide, imidazolyl, phenyl, thiophenyl, benzoxazolyl, and pyrazolyl are optionally substituted with SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃, or OCH₂OCH₃; and optionally substituted with up to two additional substituents independently selected from the group consisting of Cl, OCH₃, and CH₃; and wherein said triazolyl, oxazolyl, isoxazolyl, and thiazolyl are optionally substituted with one or two CH₃ groups; and wherein said azetidinyl is optionally substituted with CO₂C(CH₃)₃, C(O)NH₂, CH₃, SO₂CH₃, or C(O)CH₃;
R² is 1-methyl-1,2,3-triazolyl, pyridyl, pyridyl-*N*-oxide, 1-methyl pyrazol-4-yl, pyrimidin-5-yl, pyridazyl, pyrazin-2-yl, oxazolyl, isoxazolyl, *N*-acetyl-azetidin-3-yl, *N-*methylsulfonyl-azetidin-3-yl, *N*-Boc-azetidin-3-yl, *N*-methyl-azetidin-3-yl, *N*-acetamidyl-azetidin-3-yl, *N*-acetyl piperidinyl, 1-*H*-piperidinyl, *N*-Boc-piperidinyl, *N*-C₍₁₋₂₎alkyl-piperidinyl, thiazol-5-yl, 1-methyl imidazol-2-yl, 1-(3-methoxypropyl)-imidazol-5-yl, or 1-C₍₁₋₂₎alkyl imidazol-5-yl; wherein said 1-C₍₁₋₂₎alkyl imidazol-5-yl is optionally substituted with up to two additional CH₃ groups, or one substituent selected from the group consisting of SCH₃, and Cl; and said pyridyl, and pyridyl-*N*-oxide are optionally substituted with up to two substituents independently selected from the group consisting of C(O)NH₂, -CN, OCH₃, CF₃, Cl, and CH₃; and said thiazol-5-yl, oxazolyl, and isoxazolyl are optionally substituted with up to two CH₃ groups; and said 1-methyl pyrazol-4-yl is optionally substituted with up to two additional CH₃ groups;
R³ is H, OH, OCH₃, NHCH₃, N(CH₃)₂, or NH₂;
R⁴ is H, or F;
R⁵ is H, Cl, -CN, CF₃, SCH₃, OC₍₁₋₃₎alkyl, OH, C₍₁₋₄₎alkyl, N(CH₃)OCH₃, NH(C₍₁₋₂₎alkyl), N(C₍₁₋₂₎alkyl)₂, NH-cyclopropyl, OCHF₂, 4-hydroxy-piperidinyl, azetidin-1-yl, or fur-2-yl;
R⁶ is 2-chloro-thiophen-5-yl, 1-methyl-pyrazol-4-yl, phenyl, pyrimidinyl, or pyridyl, wherein said phenyl, pyrimidinyl, and pyridyl are optionally substituted with SO₂CH₃, NHSO₂CH₃, CF₃, F, Cl, -CN, OCH₃, or OCF₃;
R⁷ is H, Cl, -CN, C₍₁₋₄₎alkyl, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, OC₍₁₋₃₎alkyl, OCH₂-(1-methyl)-imidazol-2-yl, imidazol-2-yl, fur-2-yl, pyrazol-4-yl, pyrid-3-yl, or pyrimidin-5-yl; thiophen-3-yl, 1-methyl-indazol-5-yl, 1-methyl-indazol-6-yl, phenyl, or 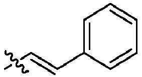 wherein said imidazolyl or pyrazolyl can be optionally substituted with a CH₃ group ;
A¹ is H or C₍₁₋₄₎alkyl;
A² is C₍₁₋₄₎alkyl, cyclopropyl, C₍₁₋₄₎alkylOC₍₁₋₄₎alkyl, C₍₁₋₄₎alkylOH, C(O)C₍₁₋₂₎alkyl, or OCH₃; or A¹ and A² may be taken together with their attached nitrogen to form a ring selected from the group consisting of: 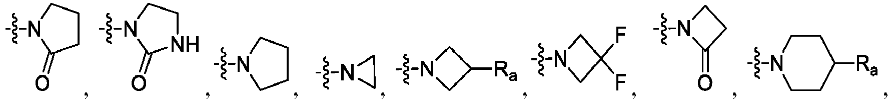 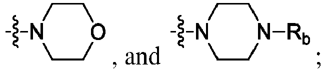
Rₐ is H, F, OCH₃, or OH;
R_{b} is CH₃, or phenyl;
R⁸ is H, CH₃, OCH₃, or F;
R⁹ is H, or F;
and wherein:
(i) R¹ is azetidinyl, wherein said azetidinyl is optionally substituted with CO₂C(CH₃)₃, C(O)NH₂, CH₃, SO₂CH₃, or C(O)CH₃; or
(ii) R² is *N*-acetyl-azetidin-3-yl, *N*-methylsulfonyl-azetidin-3-yl, *N*-Boc-azetidin-3-yl, *N*-methyl-azetidin-3-yl, or *N*-acetamidyl-azetidin-3-yl; or
(iii) R³ is NHCH₃, or N(CH₃)₂; or
(iv) R⁵ is NH-cyclopropyl, OCHF₂, azetidin-1-yl, or fur-2-yl; or
(v) R⁶ is phenyl substituted with NHSO₂CH₃, pyrimidinyl substituted with NHSO₂CH₃, or pyridyl substituted with NHSO₂CH₃; or
(vi) R⁷ is SO₂CH₃, 1-methyl-indazol-5-yl, 1-methyl-indazol-6-yl, or 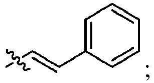 or
(vii) A² is cyclopropyl
and pharmaceutically acceptable salts thereof.

2. The compounds of claim 1, wherein:
R¹ is imidazolyl, thiazolyl, pyridyl, pyrimidinyl, or phenyl; wherein said pyridyl, and said phenyl are optionally substituted with -CN, CF₃, F, or Cl; and wherein said imidazolyl, and thiazolyl are optionally substituted with one or two CH₃ groups;
R² is 1-methyl-1,2,3-triazol-5-yl, *N*-acetyl piperidin-4-yl, *N*-Boc-piperidin-4-yl, *N*-methyl piperidin-4-yl, 1-*H*-piperidin-4-yl, oxazol-2-yl, 2,4-dimethyl thiazol-5-yl, 1-methyl imidazol-2-yl, 1-methyl-imidazol-5-yl, or pyridyl; wherein said pyridyl is optionally substituted with CF₃;
R³ is H, OH;
R⁴ is H;
R⁵ is H, Cl, -CN, C₍₁₋₄₎alkyl, OC₍₁₋₂₎alkyl, SCH₃, N(CH₃)₂, or N(CH₃)OCH₃;
R⁶ is phenyl substituted with NHSO₂CH₃, pyrimidinyl substituted with NHSO₂CH₃, or pyridyl substituted with NHSO₂CH₃;
R⁷ is Cl, -CN, CF₃, SCH₃, OCH₂CF₃, NA¹A², N(CH₃)CH₂CH₂NA¹A², OC₍₁₋₃)alkyl, OCH₂CH₂OCH₃, OCH₂CH₂NA¹A², or OCH₂CH₂NH₂;
A¹ is H, or CH₃;
A² is OCH₃, CH₃, CH₂CH₂OH, C(O)C₍₁₋₂₎alkyl, or CH₂CH₂OCH₃; or A¹ and A² may be taken together with their attached nitrogen to form a ring selected from the group consisting of: 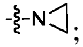
R⁸ is H, or CH₃;
R⁹ is H;
and pharmaceutically acceptable salts thereof.

3. A compound selected from the group consisting of: 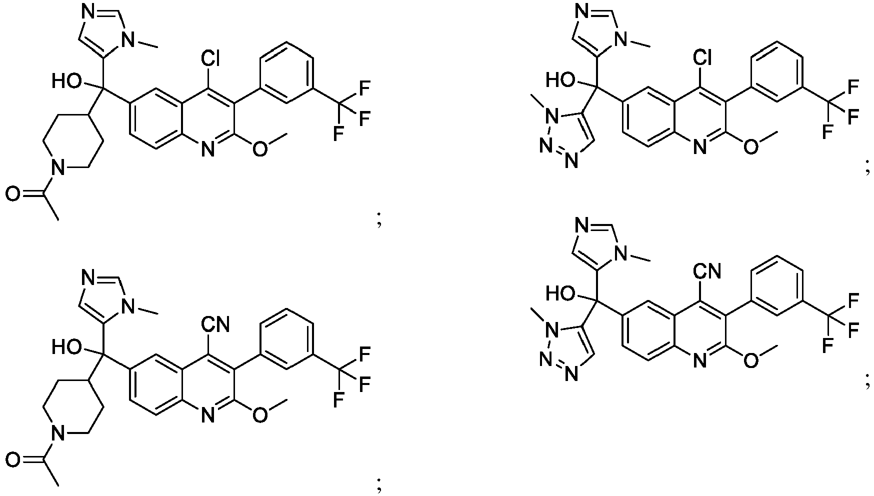 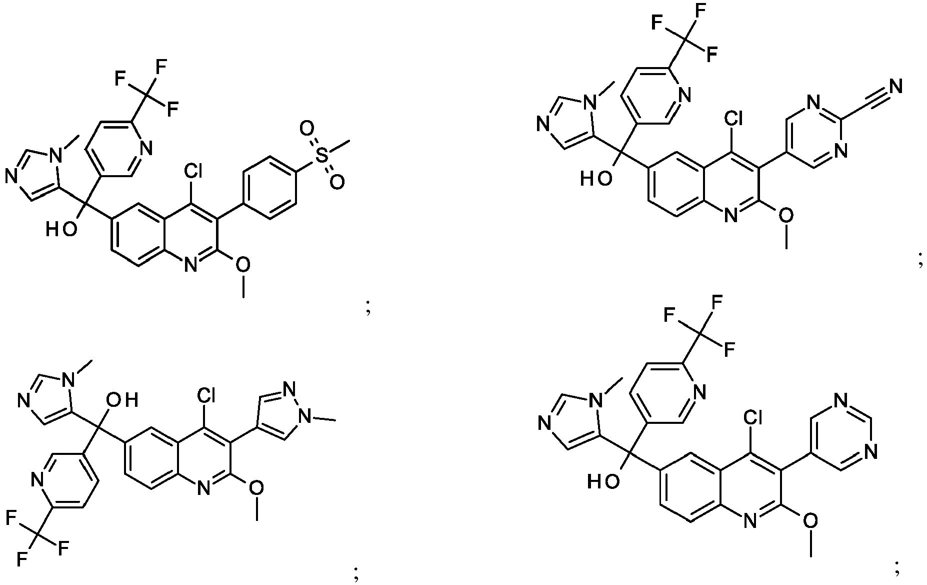 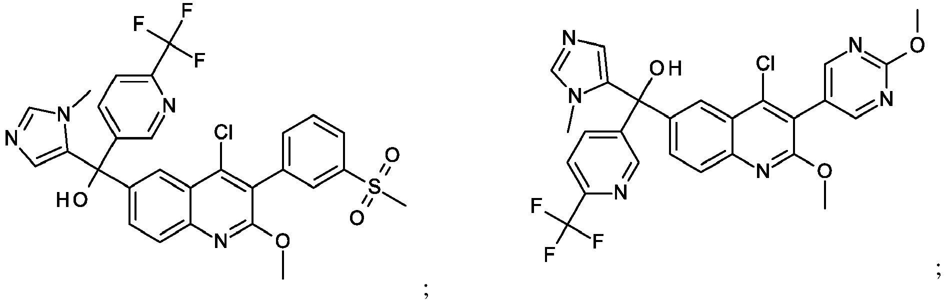 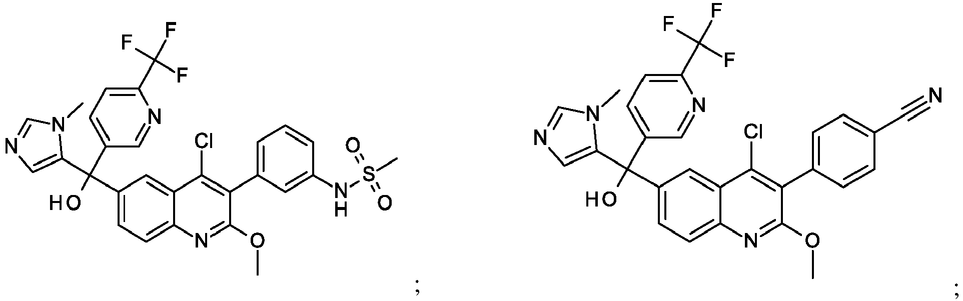 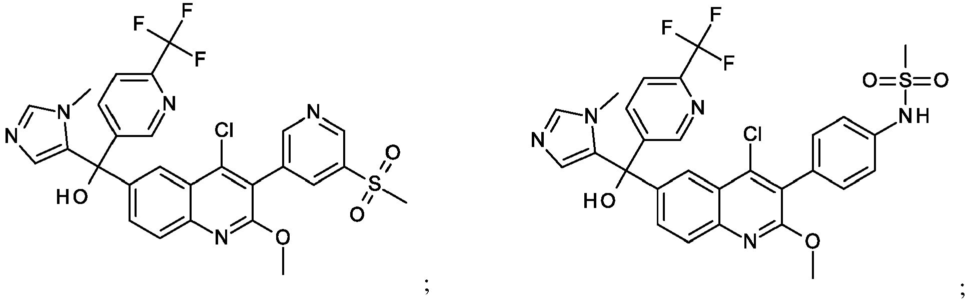 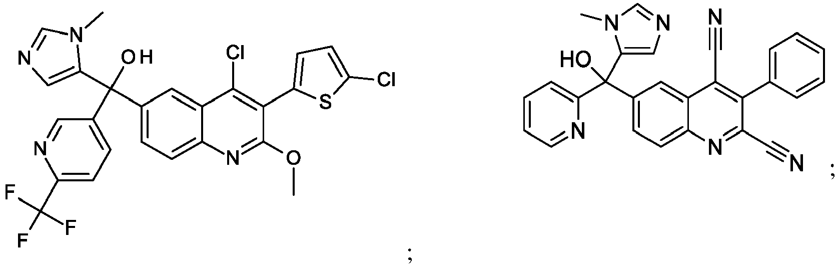 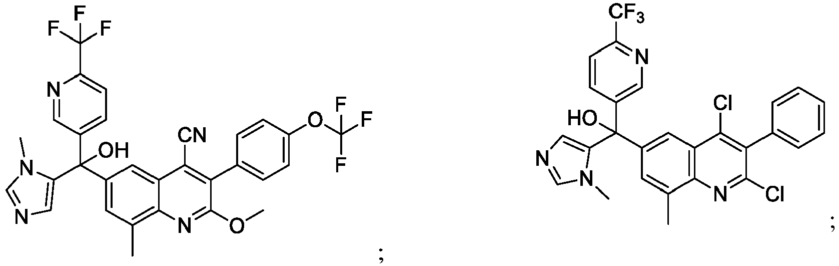 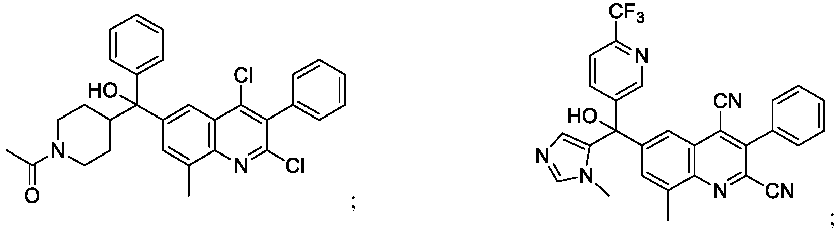 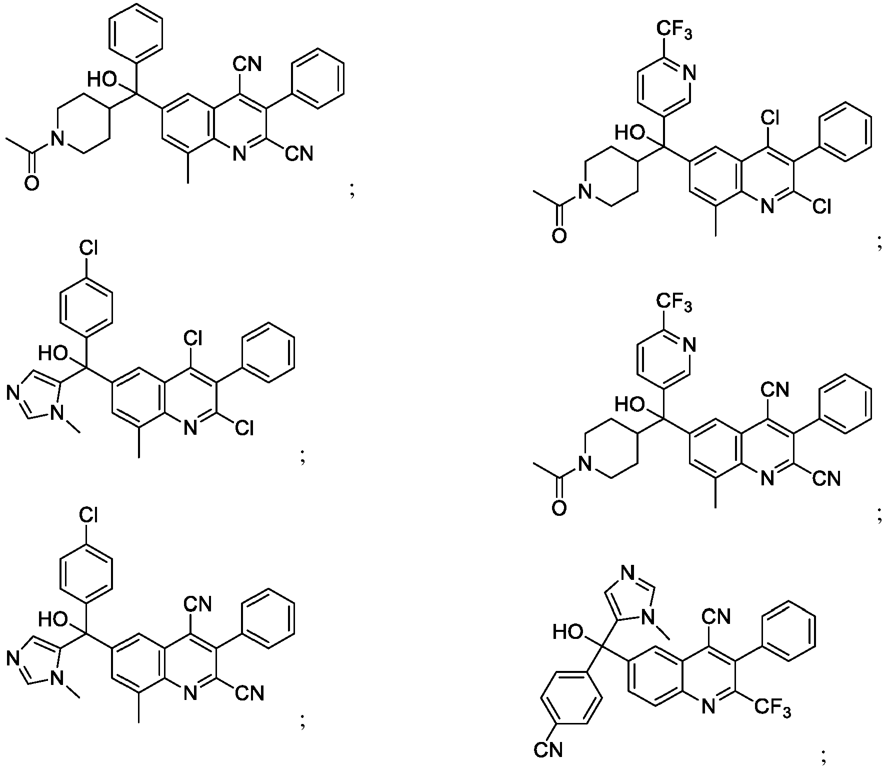 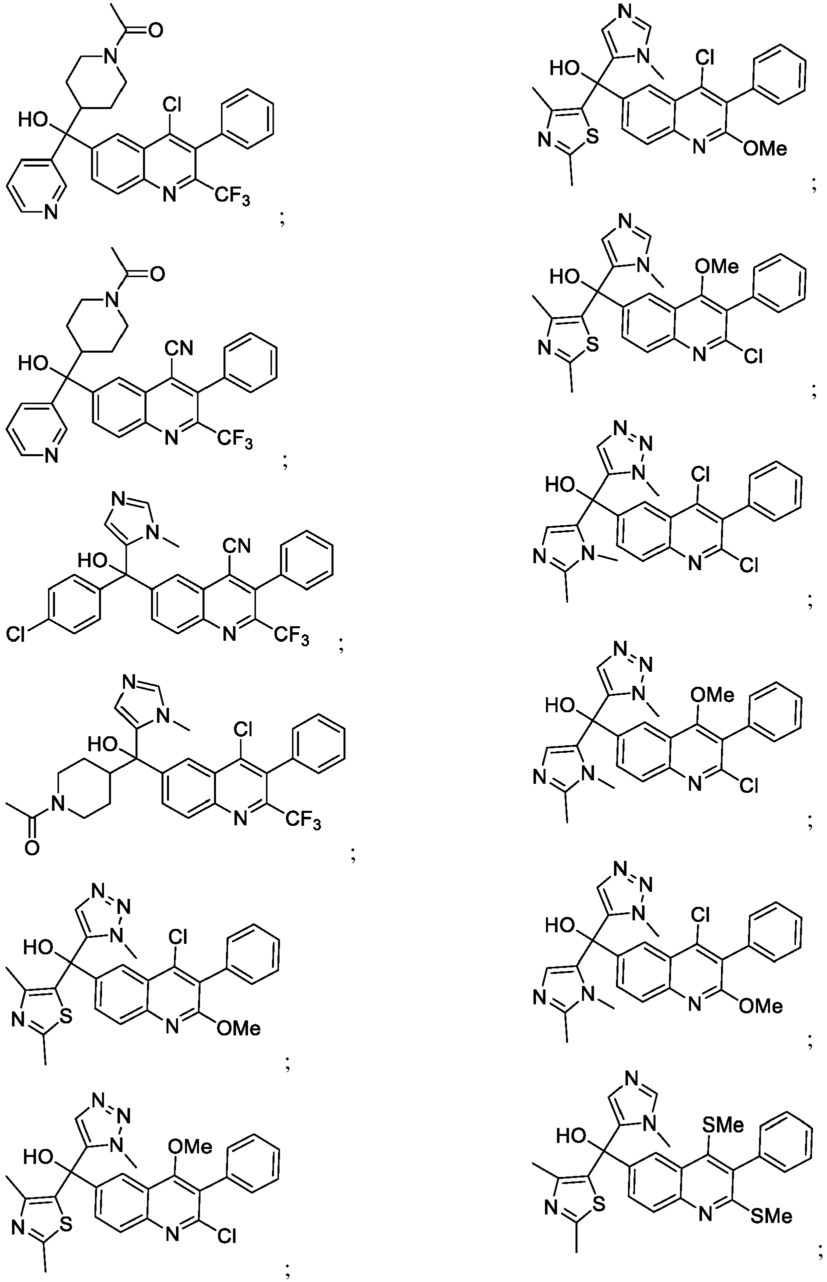 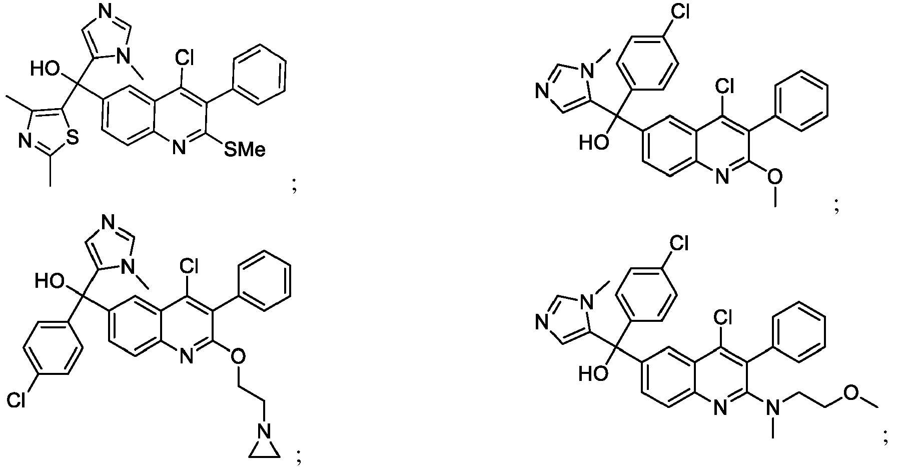 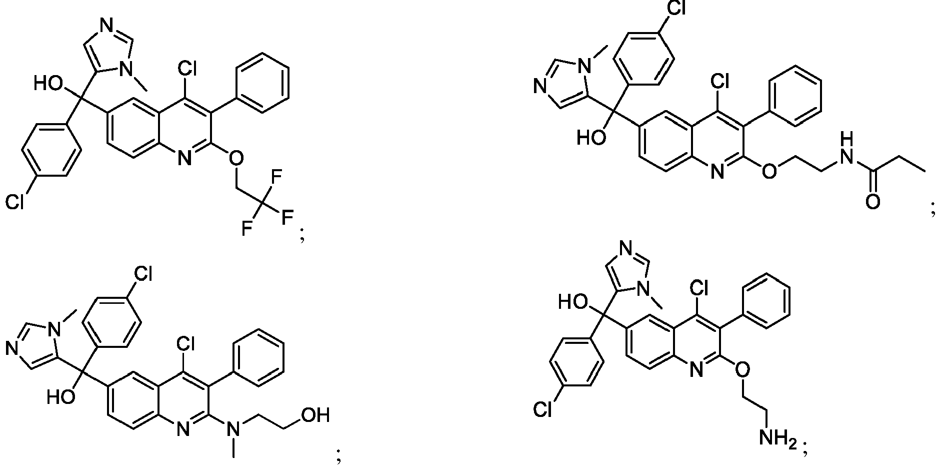 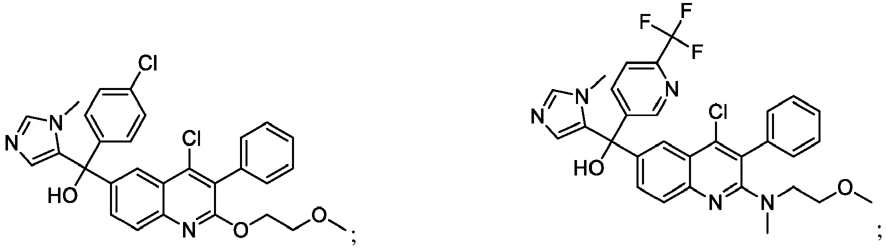 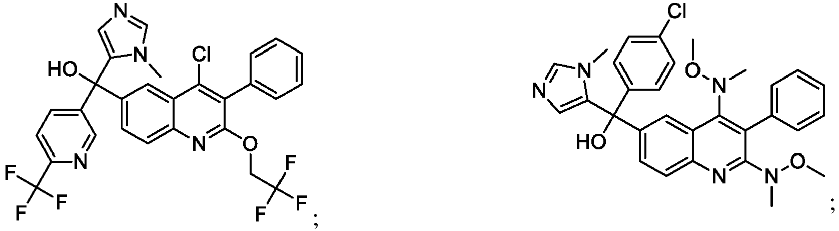 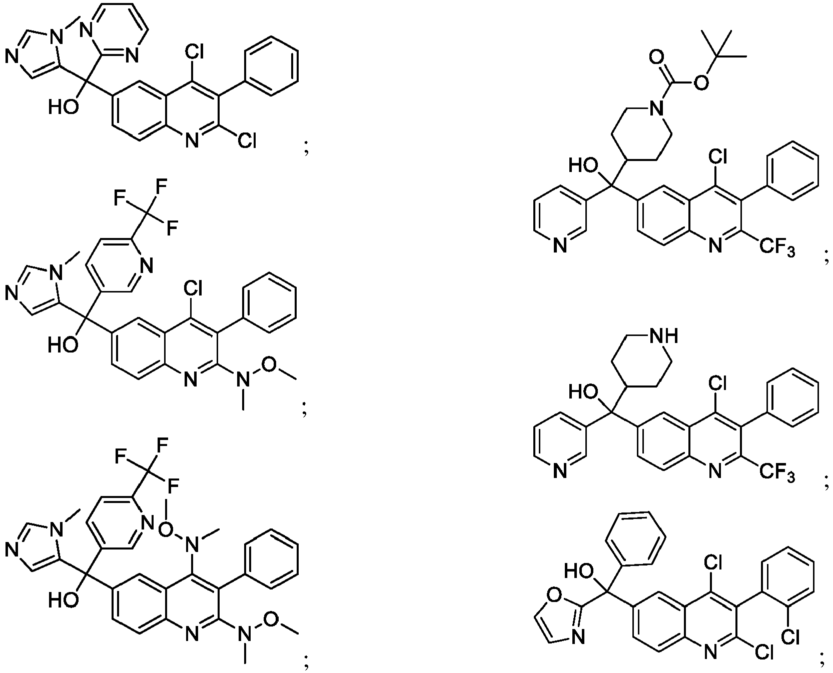 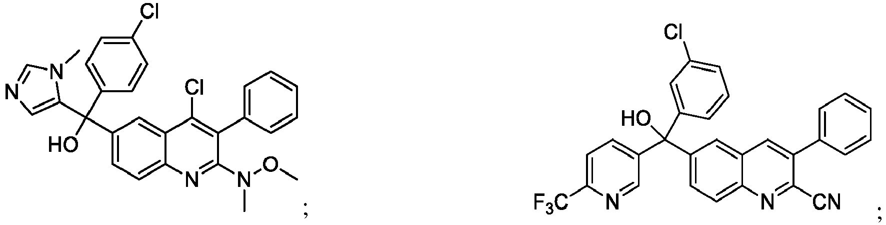 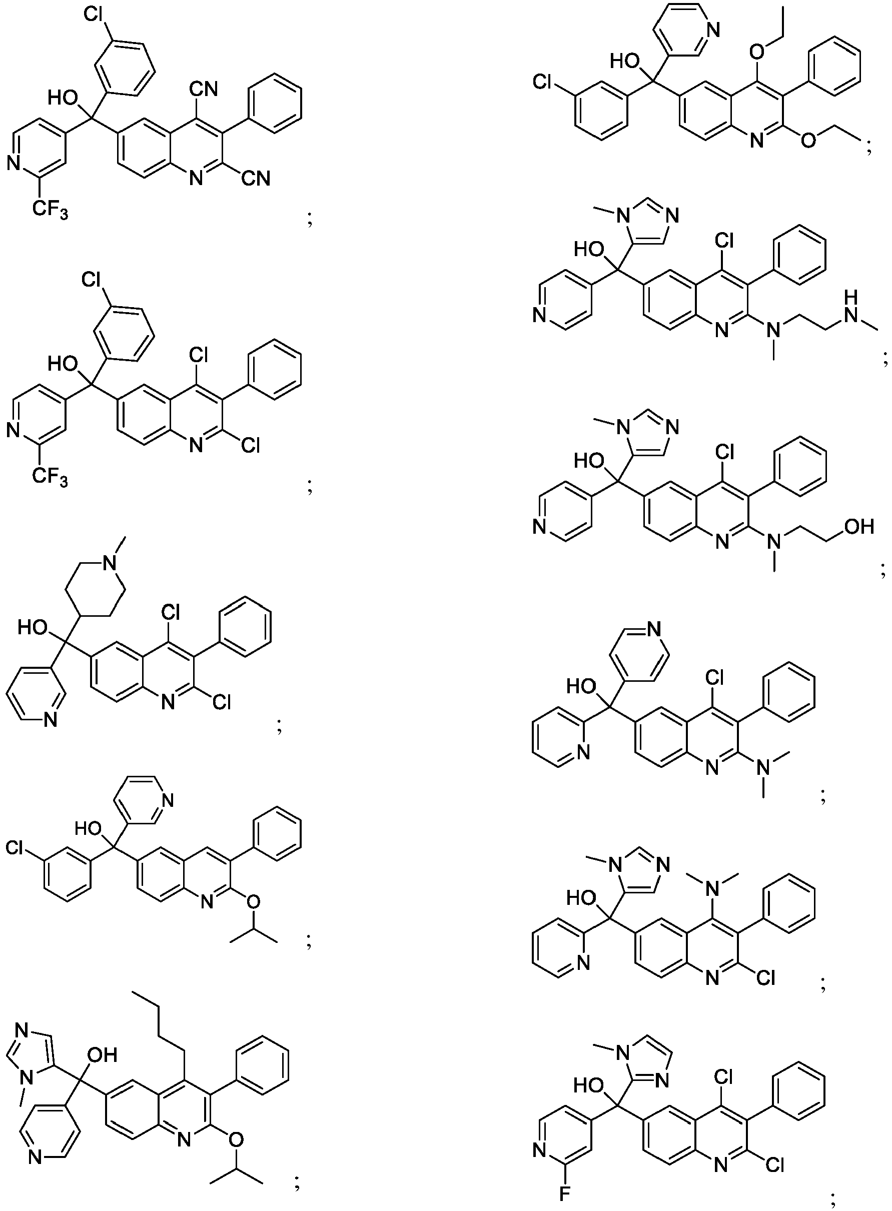 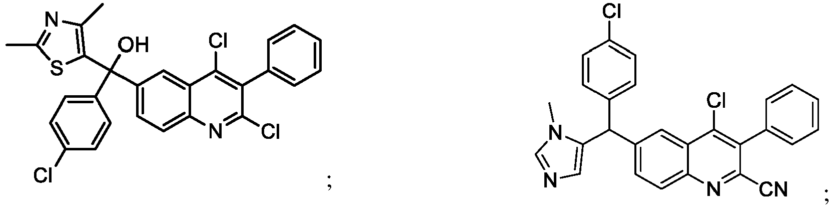 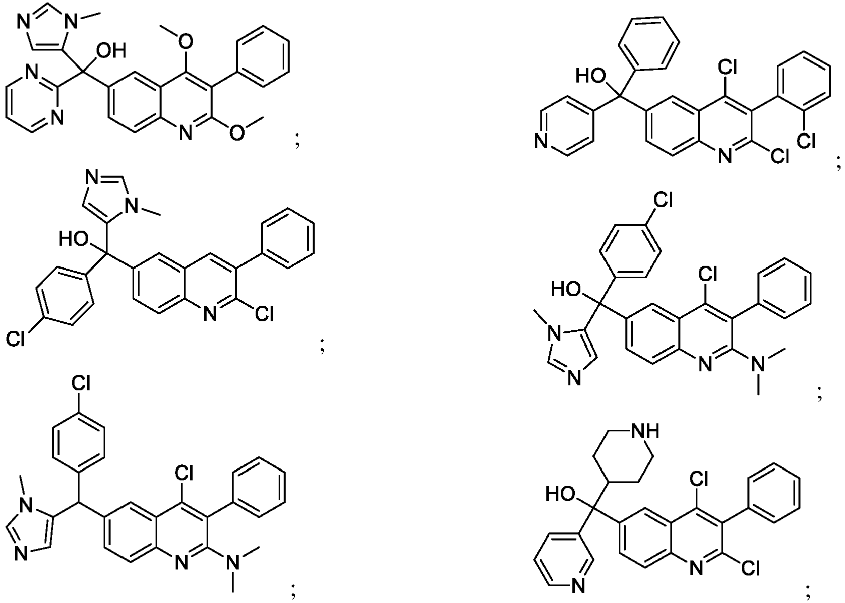 and pharmaceutically acceptable salts thereof.

4. A pharmaceutical composition, comprising a compound of any one of claims 1 to 3 and a pharmaceutically acceptable carrier.

5. A pharmaceutical composition made by mixing a compound of any one of claims 1 to 3 and a pharmaceutically acceptable carrier.

6. A process for making a pharmaceutical composition comprising mixing a compound of any one of claims 1 to 3 and a pharmaceutically acceptable carrier.

7. A compound of any one of claims 1 to 3 for use in a method for treating or ameliorating a RORyt mediated inflammatory syndrome, disorder or disease comprising administering to a subject in need thereof an effective amount of said compound of any one of claims 1 to 3.

8. The compound for use in a method according to claim 7, wherein the disease is selected from the group consisting of: inflammatory bowel diseases, rheumatoid arthritis, psoriasis, chronic obstructive pulmonary disorder, psoriatic arthritis, ankylosing spondylitis, neutrophilic asthma, steroid resistant asthma, multiple sclerosis, and systemic lupus erythematosus.

9. The compound for use in a method according to claim 7, wherein the disease is psoriasis.

10. The compound for use in a method according to claim 7, wherein the disease is rheumatoid arthritis.

11. The compound for use in a method according to claim 8, wherein the inflammatory bowel disease is ulcerative colitis.

12. The compound for use in a method according to claim 8, wherein the inflammatory bowel disease is Crohn's disease.

13. A compound of any one of claims 1 to 3 or composition or medicament thereof for use in a method of treating or ameliorating a syndrome, disorder or disease, in a subject in need thereof comprising administering to the subject an effective amount of said compound of any one of claims 1 to 3 or composition or medicament thereof in a combination therapy with one or more antiinflammatory agents, or immunosuppressive agents, wherein said syndrome, disorder or disease is selected from the group consisting of: rheumatoid arthritis, and psoriasis.

## Patentansprüche

1. Verbindungen der Formel I: 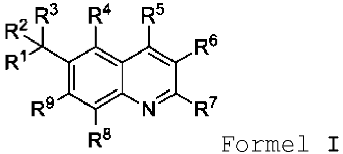 wobei:
R¹ für Azetidinyl, Pyrrolyl, Pyrazolyl, Imidazolyl, Triazolyl, Thiazolyl, Pyridyl, Pyridyl-N-oxid, Pyrazinyl, Pyrimidinyl, Pyridazyl, Piperidinyl, Tetrahydropyranyl, Phenyl, Oxazolyl, Isoxazolyl, Thiophenyl, Benzoxazolyl oder Chinolinyl steht, wobei das Piperidinyl, Pyridyl, Pyridyl-N-oxid, Imidazolyl, Phenyl, Thiophenyl, Benzoxazolyl und Pyrazolyl gegebenenfalls durch SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃ oder OCH₂OCH₃ substituiert ist, und gegebenenfalls durch bis zu zwei weitere, unabhängig voneinander aus der aus Cl, OCH₃ und CH₃ bestehenden Gruppe ausgewählte Substituenten substituiert sind, und wobei das Triazolyl, Oxazolyl, Isoxazolyl und Thiazolyl gegebenenfalls durch eine oder zwei CH₃-Gruppen substituiert ist, und wobei das Azetidinyl gegebenenfalls durch CO₂C(CH₃)₃, C(O)NH₂, CH₃, SO₂CH₃ oder C(O)CH₃ substituiert ist,
R² für 1-Methyl-1,2,3-triazolyl, Pyridyl, Pyridyl-*N*-oxid, 1-Methylpyrazol-4-yl, Pyrimidin-5-yl, Pyridazyl, Pyrazin-2-yl, Oxazolyl, Isoxazolyl, *N-*Acetyl-azetidin-3-yl, *N*-Methylsulfonylazetidin-3-yl, *N*-Boc-Azetidin-3-yl, *N*-Methylazetidin-3-yl, *N-*Acetamidylazetidin-3-yl, *N*-Acetylpiperidinyl, 1-*H-*Piperidinyl, *N*-Boc-Piperidinyl, *N*-C₍₁₋₂₎-Alkylpiperidinyl, Thiazol-5-yl, 1-Methylimidazol-2-yl, 1-(3-Methoxypropyl)-imidazol-5-yl oder 1-C₍₁₋₂₎-Alkylimidazol-5-yl steht, wobei das 1-C₍₁₋₂₎-Alkylimidazol-5-yl gegebenenfalls durch bis zu zwei weitere CH₃-Gruppen oder einen Substituent ausgewählt aus der Gruppe bestehend aus SCH₃ und Cl substituiert ist, und das Pyridyl und Pyridyl-*N-*oxid gegebenenfalls durch bis zu zwei unabhängig aus der Gruppe bestehend aus C(O)NH₂, -CN, OCH₃, CF₃, Cl und CH₃ ausgewählte Substituenten substituiert sind, und das Thiazol-5-yl, Oxazolyl und Isoxazolyl gegebenenfalls durch bis zu zwei CH₃-Gruppen substituiert sind, und das 1-Methylpyrazol-4-yl gegebenenfalls durch bis zu zwei weitere CH₃-Gruppen substituiert ist,
R³ für H, OH, OCH₃, NHCH₃, N(CH₃)₂ oder NH₂ steht,
R⁴ für H oder F steht,
R⁵ für H, Cl, -CN, CF₃, SCH₃, OC₍₁₋₃₎-Alkyl, OH, C₍₁₋₄₎-Alkyl, N(CH₃)OCH₃, NH(C₍₁₋₂₎-Alkyl), N(C₍₁₋₂₎-Alkyl)₂, NH-Cyclopropyl, OCHF₂, 4-Hydroxypiperidinyl, Azetidin-1-yl oder Fur-2-yl steht,
R⁶ für 2-Chlorthiophen-5-yl, 1-Methylpyrazol-4-yl, Phenyl, Pyrimidinyl oder Pyridyl steht, wobei das Phenyl, Pyrimidinyl und Pyridyl gegebenenfalls durch SO₂CH₃, NHSO₂CH₃, CF₃, F, Cl, -CN, OCH₃ oder OCF₃ substituiert ist,
R⁷ für H, Cl, -CN, C₍₁₋₄₎-Alkyl, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, OC₍₁₋₃₎-Alkyl, OCH₂-(1-Methyl)imidazol-2-yl, Imidazol-2-yl, Fur-2-yl, Pyrazol-4-yl, Pyrid-3-yl oder Pyrimidin-5-yl, Thiophen-3-yl, 1-Methylindazol-5-yl, 1-Methylindazol-6-yl, Phenyl oder 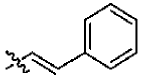 steht, wobei das Imidazolyl oder Pyrazolyl gegebenenfalls durch eine CH₃-Gruppe substituiert sein kann,
A¹ für H oder C₍₁₋₄₎-Alkyl steht
A² für C₍₁₋₄)-Alkyl, Cyclopropyl, C₍₁₋₄)-Alkyl-OC₍₁₋₄₎-alkyl, C₍₁₋₄₎-Alkyl-OH, C(O)C₍₁₋₂₎-Alkyl oder OCH₃ steht oder A¹ und A² zusammen mit dem Stickstoff, an den sie gebunden sind, einen aus der folgenden Gruppe ausgewählten Ring bilden: 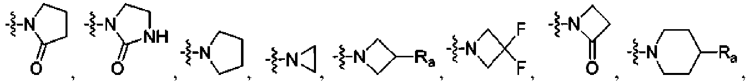 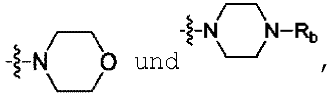
Rₐ für H, F, OCH₃ oder OH steht,
R_{b} für CH₃ oder Phenyl steht,
R⁸ für H, CH₃, OCH₃ oder F steht,
R⁹ für H oder F steht,
und wobei:
(i) R¹ für Azetidinyl steht, wobei das Azetidinyl gegebenenfalls durch CO₂C(CH₃)₃, C(O)NH₂, CH₃, SO₂CH₃ oder C(O)CH₃ substituiert ist, oder
(ii) R² für *N*-Acetylazetidin-3-yl, *N-*Methylsulfonylazetidin-3-yl, *N*-Boc-Azetidin-3-yl, *N*-Methylazetidin-3-yl oder *N*-Acetamidylazetidin-3-yl steht, oder
(iii) R³ für NHCH₃ oder N(CH₃)₂ steht, oder
(iv) R⁵ für NH-Cyclopropyl, OCHF₂, Azetidin-1-yl oder Fur-2-yl steht, oder
(v) R⁶ für Phenyl, das durch NHSO₂CH₃ substituiert ist, Pyrimidinyl, das durch NHSO₂CH₃ substituiert ist, oder Pyridyl steht, das durch NHSO₂CH₃ substituiert ist, oder
(vi) R⁷ für SO₂CH₃, 1-Methylindazol-5-yl, 1-Methylindazol-6-yl oder 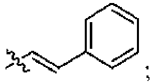 steht, oder
(vii) A² für Cyclopropyl steht,
und pharmazeutisch unbedenkliche Salze davon.

2. Verbindungen nach Anspruch 1, wobei:
R¹ für Imidazolyl, Thiazolyl, Pyridyl, Pyrimidinyl oder Phenyl steht, wobei das Pyridyl und das Phenyl gegebenenfalls durch -CN, CF₃, F oder Cl substituiert sind, und wobei das Imidazolyl und Thiazolyl gegebenenfalls durch eine oder zwei CH₃-Gruppen substituiert sind,
R² für 1-Methyl-1,2,3-triazol-5-yl, *N-*Acetylpiperidin-4-yl, *N*-Boc-Piperidin-4-yl, *N-*Methylpiperidin-4-yl, 1-*H*-Piperidin-4-yl, Oxazol-2-yl, 2,4-Dimethylthiazol-5-yl, 1-Methylimidazol-2-yl, 1-Methylimidazol-5-yl oder Pyridyl steht,
wobei das Pyridyl gegebenenfalls durch CF₃ substituiert ist,
R³ für H, OH steht,
R⁴ für H steht,
R⁵ für H, Cl, -CN, C₍₁₋₄₎-Alkyl, OC₍₁₋₂₎-Alkyl, SCH₃, N(CH₃)₂ oder N(CH₃)OCH₃ steht,
R⁶ für Phenyl, das durch NHSO₂CH₃ substituiert ist, Pyrimidinyl, das durch NHSO₂CH₃ substituiert ist, oder Pyridyl steht, das durch NHSO₂CH₃ substituiert ist,
R⁷ für Cl, -CN, CF₃, SCH₃, OCH₂CF₃, NA¹A², N(CH₃)CH₂CH₂NA¹A², OC₍₁₋₃₎-Alkyl, OCH₂CH₂OCH₃, OCH₂CH₂NA¹A² oder OCH₂CH₂NH₂ steht,
A¹ für H oder CH₃ steht,
A² für OCH₃, CH₃, CH₂CH₂OH, C(O)C₍₁₋₂₎-Alkyl oder CH₂CH₂OCH₃ steht, oder A¹ und A² zusammen mit dem Stickstoff, an den sie gebunden sind, einen aus der folgenden Gruppe ausgewählten Ring bilden: 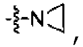
R⁸ für H oder CH₃ steht,
R⁹ für H steht,
und pharmazeutisch unbedenkliche Salze davon.

3. Verbindung ausgewählt aus der Gruppe bestehend aus: 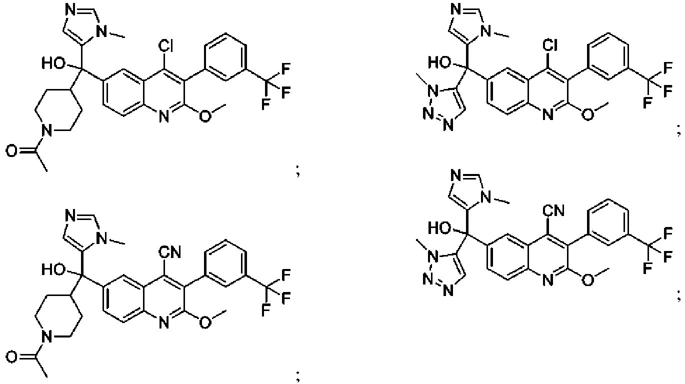 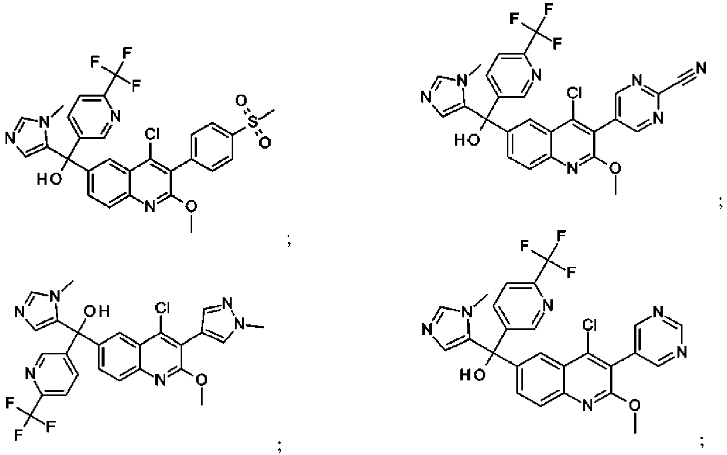 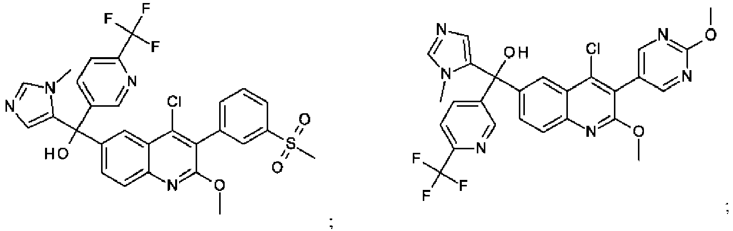 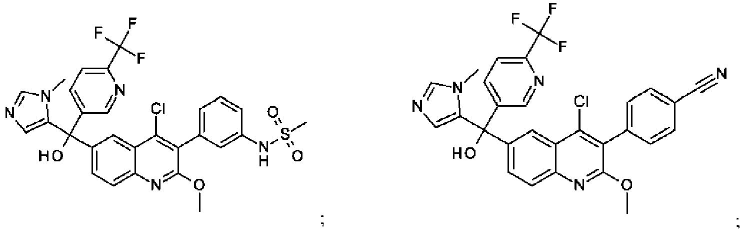 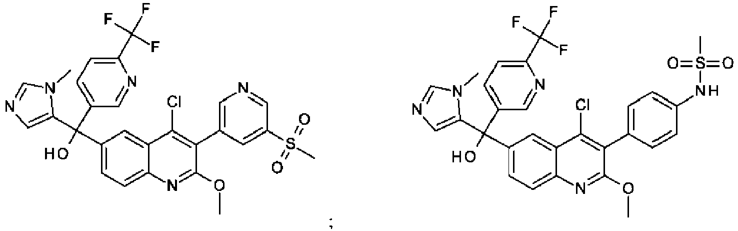 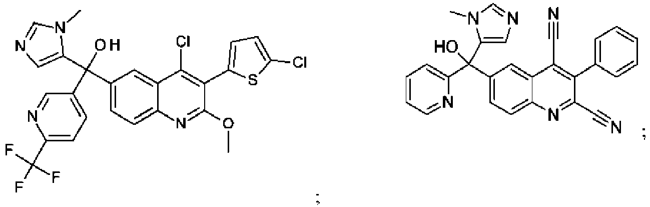 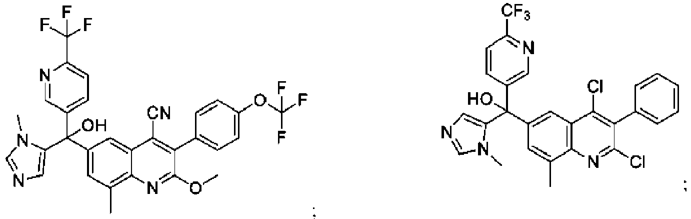 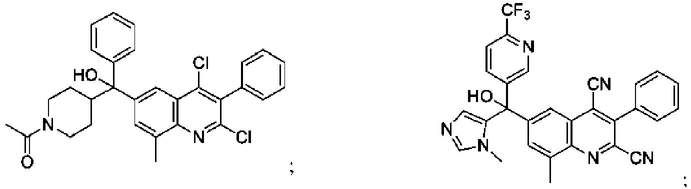 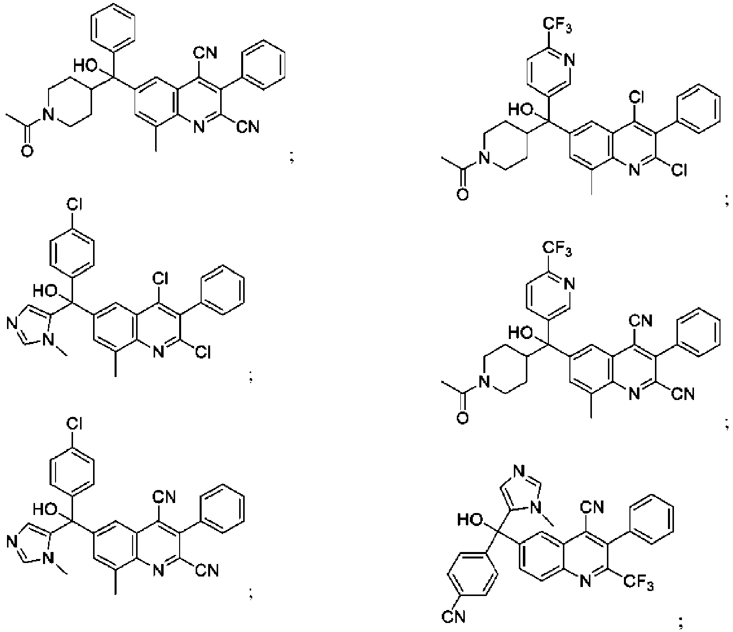 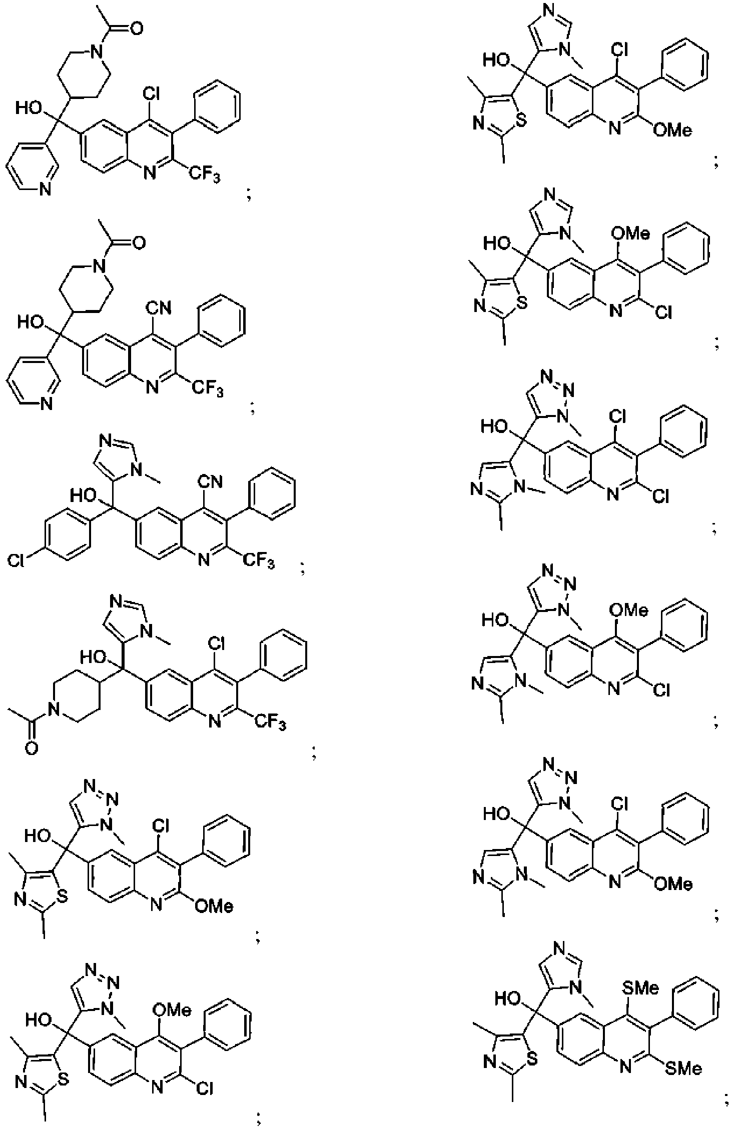 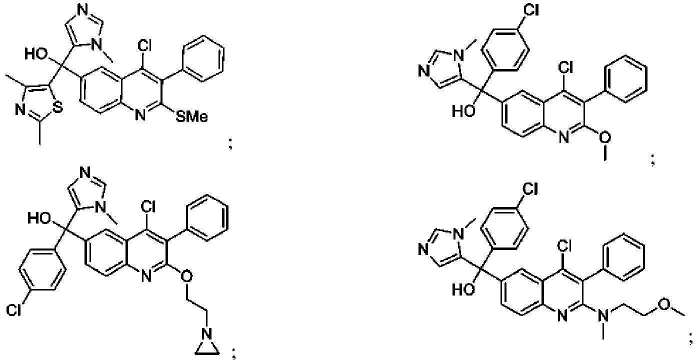 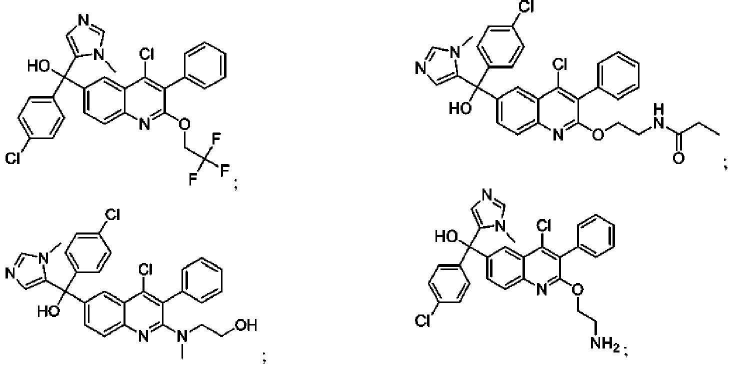 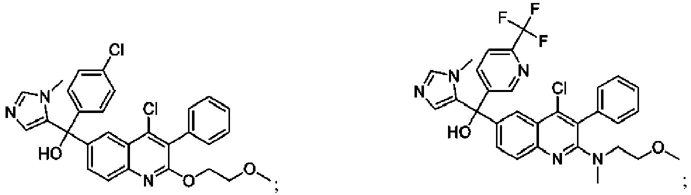 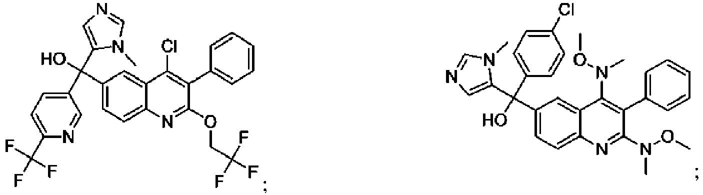 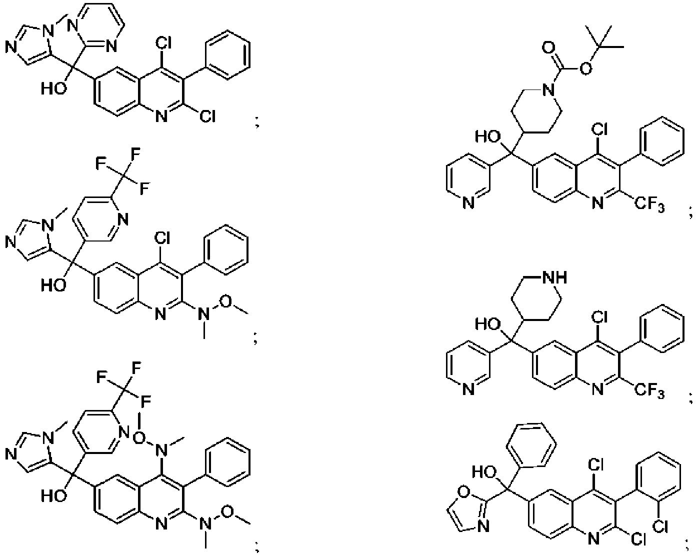 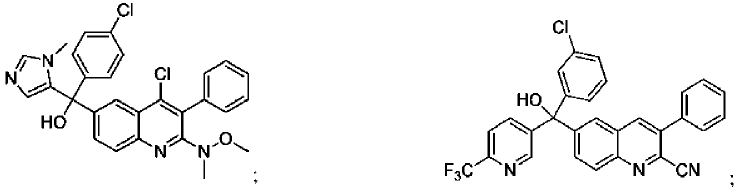 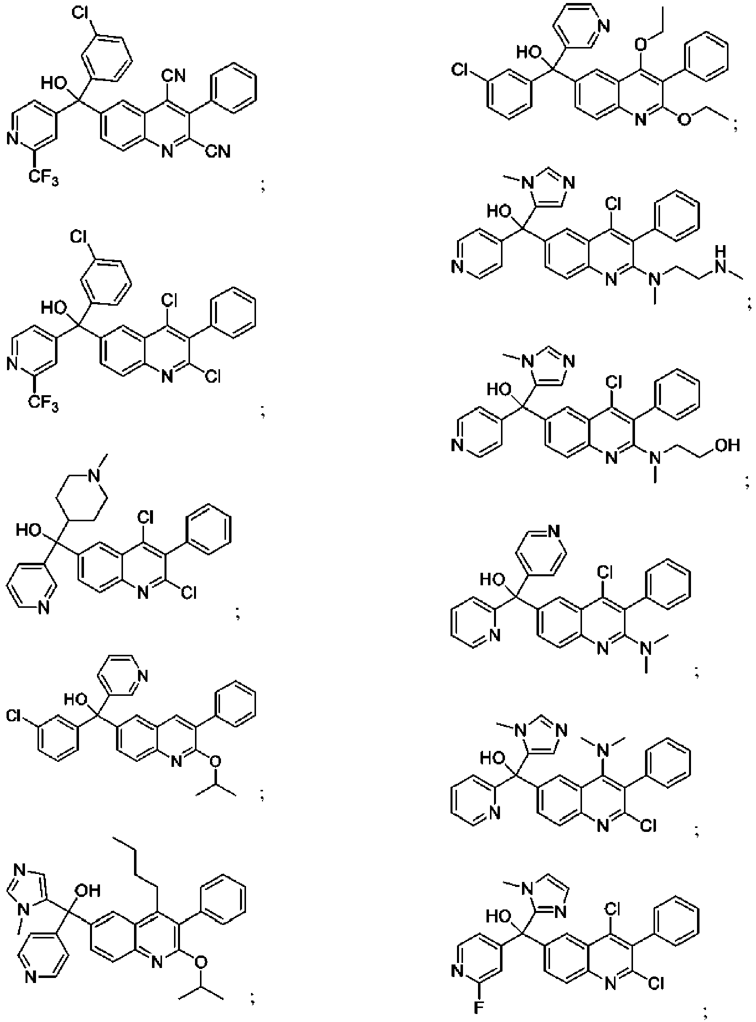 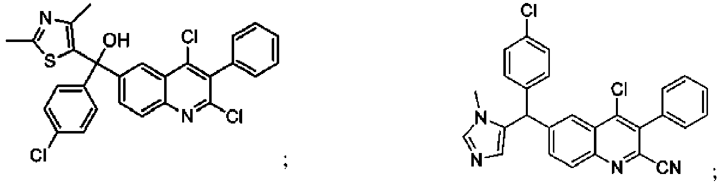 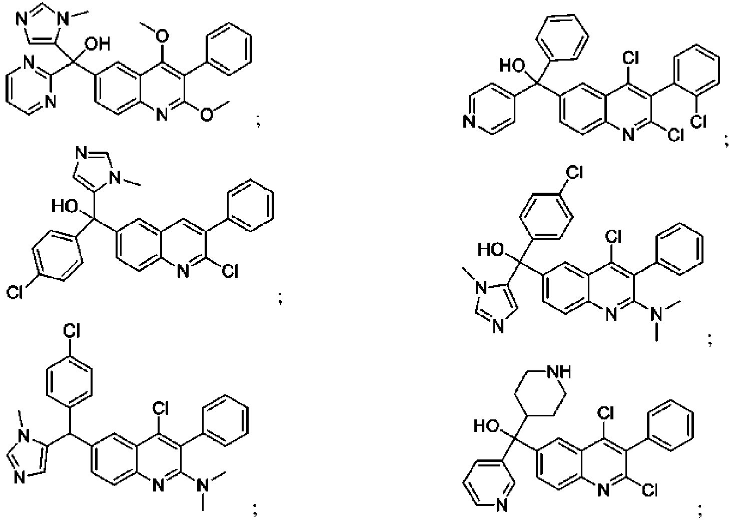 und pharmazeutisch unbedenkliche Salze davon.

4. Pharmazeutische Zusammensetzung, umfassend eine Verbindung nach einem der Ansprüche 1 bis 3 und einen pharmazeutisch unbedenklichen Träger.

5. Pharmazeutische Zusammensetzung, hergestellt durch Mischen einer Verbindung nach einem der Ansprüche 1 bis 3 und eines pharmazeutisch unbedenklichen Trägers.

6. Verfahren zur Herstellung einer pharmazeutischen Zusammensetzung, umfassend das Mischen einer Verbindung nach einem der Ansprüche 1 bis 3 und eines pharmazeutisch unbedenklichen Trägers.

7. Verbindung nach einem der Ansprüche 1 bis 3 zur Verwendung in einem Verfahren zur Behandlung oder Linderung eines entzündlichen Syndroms, einer entzündlichen Erkrankung bzw. einer entzündlichen Krankheit, das bzw. die durch RORyt vermittelt wird, indem einem Subjekt, das dessen bedarf, eine effektive Menge der Verbindung nach einem der Ansprüche 1 bis 3 verabreicht wird.

8. Verbindung zur Verwendung in einem Verfahren gemäß Anspruch 7, wobei die Krankheit ausgewählt ist aus der Gruppe bestehend aus: entzündlichen Darmerkrankungen, rheumatoider Arthritis, Psoriasis, chronischer obstruktiver Lungenerkrankung, Psoriasisarthritis, Spondylitis ankylosans, neutrophilem Asthma, steroidresistentem Asthma, multipler Sklerose, und systemischem Lupus erythematodes.

9. Verbindung zur Verwendung in einem Verfahren gemäß Anspruch 7, wobei es sich bei der Krankheit um Psoriasis handelt.

10. Verbindung zur Verwendung in einem Verfahren gemäß Anspruch 7, wobei es sich bei der Krankheit um rheumatoide Arthritis handelt.

11. Verbindung zur Verwendung in einem Verfahren gemäß Anspruch 8, wobei es sich bei der entzündlichen Darmerkrankung um Colitis ulcerosa handelt.

12. Verbindung zur Verwendung in einem Verfahren gemäß Anspruch 8, wobei es sich bei der entzündlichen Darmerkrankung um Morbus Crohn handelt.

13. Verbindung nach einem der Ansprüche 1 bis 3 oder Zusammensetzung oder Medikament davon zur Verwendung in einem Verfahren zur Behandlung oder Linderung eines Syndroms, einer Erkrankung oder einer Krankheit bei einem Subjekt, das dessen bedarf, wobei dem Subjekt eine effektive Menge der Verbindung nach einem der Ansprüche 1 bis 3 oder eine Zusammensetzung oder ein Medikament davon in einer Kombinationstherapie mit einem oder mehreren entzündungshemmenden Mitteln oder Immunsuppressiva verabreicht wird, wobei das Syndrom, die Erkrankung oder Krankheit ausgewählt ist aus der Gruppe bestehend aus: rheumatoider Arthritis und Psoriasis.

## Revendications

1. Composés de formule I : 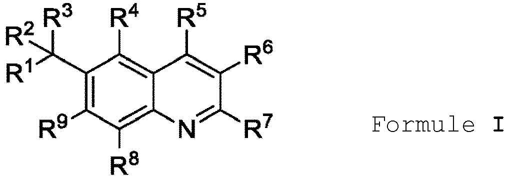 dans lesquels :
R¹ est azétidinyle, pyrrolyle, pyrazolyle, imidazolyle, triazolyle, thiazolyle, pyridyle, pyridyl-N-oxyde, pyrazinyle, pyrimidinyle, pyridazyle, pipéridinyle, tétrahydropyranyle, phényle, oxazolyle, isoxazolyle, thiophényle, benzoxazolyle ou quinolyle ; où lesdits pipéridinyle, pyridyle, pyridyl-N-oxyde, imidazolyle, phényle, thiophényle, benzoxazolyle et pyrazolyle sont facultativement substitués par SO₂CH₃, C(O)CH₃, C(O)NH₂, CH₃, CH₂CH₃, CF₃, Cl, F, -CN, OCH₃, N(CH₃)₂, - (CH₂)₃OCH₃, SCH₃, OH, CO₂H, CO₂C(CH₃)₃ ou OCH₂OCH₃ ; et facultativement substitués par jusqu'à deux substituants additionnels indépendamment choisis dans le groupe constitué de Cl, OCH₃ et CH₃ ; et où lesdits triazolyle, oxazolyle, isoxazolyle et thiazolyle sont facultativement substitués par un ou deux groupes CH₃ ; et où ledit azétidinyle est facultativement substitué par CO₂C(CH₃)₃, C(O)NH₂, CH₃, SO₂CH₃ ou C(O)CH₃ ;
R² est 1-méthyl-1,2,3-triazolyle, pyridyle, pyridyl-*N*-oxyde, 1-méthylpyrazol-4-yle, pyrimidin-5-yle, pyridazyle, pyrazin-2-yle, oxazolyle, isoxazolyle, *N-*acétylazétidin-3-yle, *N*-méthylsulfonylazétidin-3-yle, *N-*Boc-azétidin-3-yle, *N*-méthylazétidin-3-yle, *N-*acétamidylazétidin-3-yle, *N*-acétylpipéridinyle, *1-H-*pipéridinyle, *N*-Boc-pipéridinyl, *N*-(alkyl en C₁₋₂)pipéridinyle, thiazol-5-yle, 1-méthylimidazol-2-yle, 1-(3-méthoxypropyl)imidazol-5-yle ou 1-(alkyl en C₁₋₂)imidazol-5-yle ; où ledit 1-(alkyl en C₁₋₂)imidazol-5-yle est facultativement substitué par jusqu'à deux groupes CH₃ additionnels ou un substituant choisi dans le groupe constitué de SCH₃ et Cl ; et lesdits pyridyle et pyridyl-N-oxyde sont facultativement substitués par jusqu'à deux substituants indépendamment choisis dans le groupe constitué de C(O)NH₂, -CN, OCH₃, CF₃, Cl et CH₃ ; et lesdits thiazol-5-yle, oxazolyle et isoxazolyle sont facultativement substitués par jusqu'à deux groupes CH₃ ; et ledit 1-méthylpyrazol-4-yle est facultativement substitué par jusqu'à deux groupes CH₃ additionnels ;
R³ est H, OH, OCH₃, NHCH₃, N(CH₃)₂ ou NH₂ ;
R⁴ est H ou F ;
R⁵ est H, Cl, -CN, CF₃, SCH₃, O(alkyle en C₁₋₃), OH, alkyle en C₁₋₄, N(CH₃)OCH₃, NH(alkyle en C₁₋₂), N(alkyle en C₁₋₂)₂, NH-cyclopropyle, OCHF₂, 4-hydroxypipéridinyle, azétidin-1-yle ou fur-2-yle ;
R⁶ est 2-chlorothiophén-5-yle, 1-méthylpyrazol-4-yle, phényle, pyrimidinyle ou pyridyle, où lesdits phényle, pyrimidinyle et pyridyle sont facultativement substitués par SO₂CH₃, NHSO₂CH₃, CF₃, F, Cl, -CN, OCH₃ ou OCF₃ ;
R⁷ est H, Cl, -CN, alkyle en C₁₋₄, OCH₂CF₃, OCH₂CH₂OCH₃, CF₃, SCH₃, SO₂CH₃, OCHF₂, NA¹A², C(O)NHCH₃, N(CH₃)CH₂CH₂NA¹A², OCH₂CH₂NA¹A², OCH₂CH₂NH₂, O(alkyle en C₁₋₃), OCH₂-(1-méthyl)imidazol-2-yle, imidazol-2-yle, fur-2-yle, pyrazol-4-yl, pyrid-3-yle ou pyrimidin-5-yle ; thiophén-3-yle, 1-méthylindazol-5-yle, 1-méthylindazol-6-yle, phényle ou 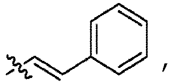 où ledit imidazolyle ou pyrazolyle peut être facultativement substitué par un groupe CH₃ ;
A¹ est H ou alkyle en C₁₋₄ ;
A² est alkyle en C₁₋₄, cyclopropyle, (alkyl en C₁₋₄)O(alkyle en C₁₋₄), (alkyl en C₁₋₄)-OH, C(O)(alkyle en C₁₋₂) ou OCH₃ ; ou A¹ et A² peuvent être pris conjointement avec leur azote lié pour former un cycle choisi dans le groupe constitué de : 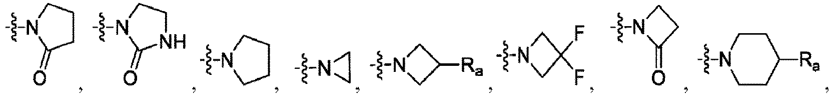 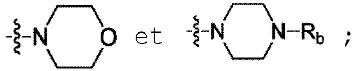
Rₐ est H, F, OCH₃ ou OH ;
R_{b} est CH₃ ou phényle ;
R⁸ est H, CH₃, OCH₃ ou F ;
R⁹ est H ou F ;
et dans lesquels :
(i) R¹ est azétidinyle, où ledit azétidinyle est facultativement substitué par CO₂C(CH₃)₃, C(O)NH₂, CH₃, SO₂CH₃ ou C(O)CH₃ ; ou
(ii) R² est N-acétylazétidin-3-yle, N-méthylsulfonylazétidin-3-yle, N-Boc-azétidin-3-yle, N-méthylazétidin-3-yle ou N-acétamidylazétidin-3-yle ; ou
(iii) R³ est NHCH₃ ou N(CH₃)₂ ; ou
(iv) R⁵ est NH-cyclopropyle, OCHF₂, azétidin-1-yle ou fur-2-yle ; ou
(v) R⁶ est phényle substitué par NHSO₂CH₃, pyrimidinyle substitué par NHSO₂CH₃ ou pyridyle substitué par NHSO₂CH₃ ; ou
(vi) R⁷ est SO₂CH₃, 1-méthylindazol-5-yle, 1-méthylindazol-6-yle ou 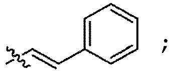 ou
(vii) A² est cyclopropyle
et sels pharmaceutiquement acceptables de ceux-ci.

2. Composés de la revendication 1, dans lesquels :
R¹ est imidazolyle, thiazolyle, pyridyle, pyrimidinyle ou phényle ; où ledit pyridyle et ledit phényle sont facultativement substitués par -CN, CF₃, F ou Cl ; et où lesdits imidazolyle et thiazolyle sont facultativement substitués par un ou deux groupes CH₃ ;
R² est 1-méthyl-1,2,3-triazol-5-yle, *N-*acétylpipéridin-4-yle, *N*-Boc-pipéridin-4-yle, *N-*méthylpipéridin-4-yle, 1-*H*-pipéridin-4-yle, oxazol-2-yle, 2,4-diméthylthiazol-5-yle, 1-méthylimidazol-2-yle, 1-méthylimidazol-5-yle ou pyridyle ; où ledit pyridyle est facultativement substitué par CF₃ ;
R³ est H, OH ;
R⁴ est H ;
R⁵ est H, Cl, -CN, alkyle en C₁₋₄, O(alkyle en C₁₋₂), SCH₃, N(CH₃)₂ ou N(CH₃)OCH₃ ;
R⁶ est phényle substitué par NHSO₂CH₃, pyrimidinyle substitué par NHSO₂CH₃ ou pyridyle substitué par NHSO₂CH₃ ;
R⁷ est Cl, -CN, CF₃, SCH₃, OCH₂CF₃, NA¹A², N(CH₃)CH₂CH₂NA¹A², O(alkyle en C₁₋₃), OCH₂CH₂OCH₃, OCH₂CH₂NA¹A² ou OCH₂CH₂NH₂ ;
A¹ est H ou CH₃ ;
A² est OCH₃, CH₃, CH₂CH₂OH, C(O) (alkyle en C₁₋₂) ou CH₂CH₂OCH₃ ; ou A¹ et A² peuvent être pris conjointement avec leur azote lié pour former un cycle choisi dans le groupe constitué de : 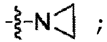
R⁸ est H ou CH₃ ;
R⁹ est H ;
et sels pharmaceutiquement acceptables de ceux-ci.

3. Composé choisi dans le groupe constitué de : 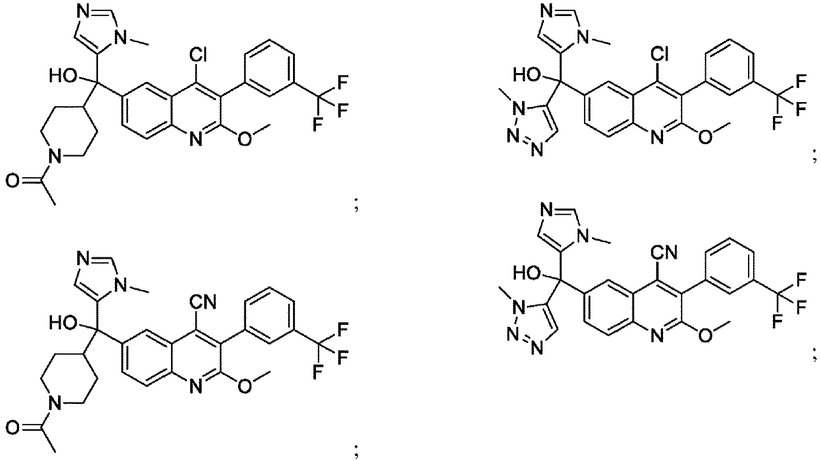 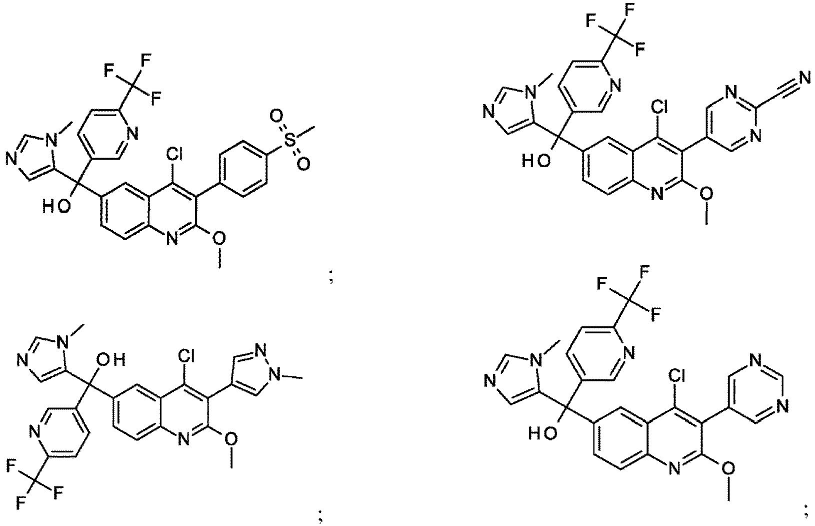 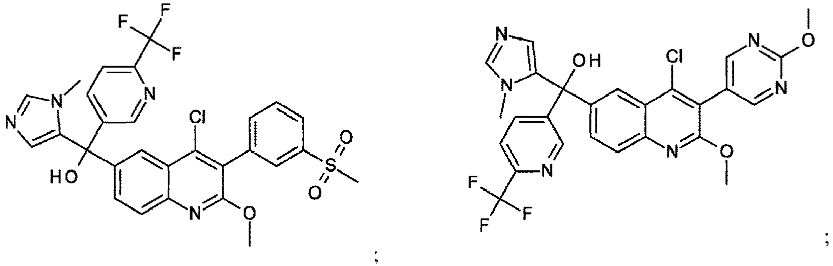 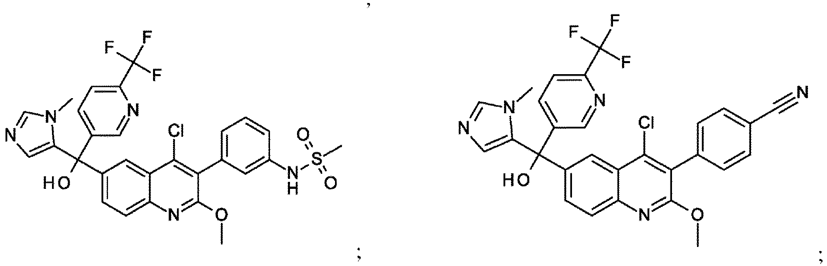 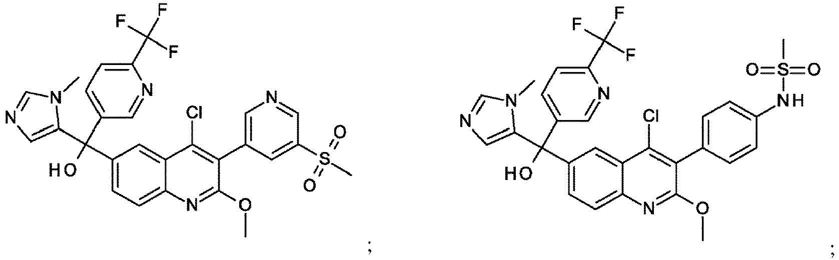 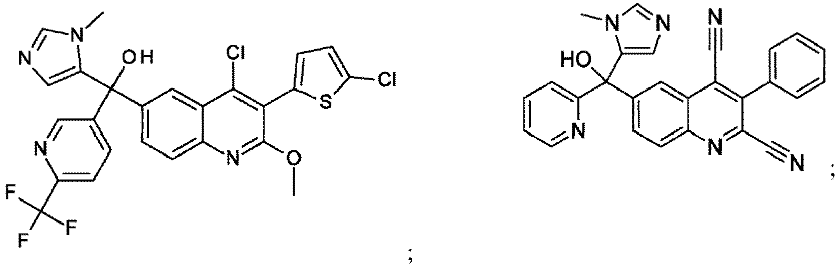 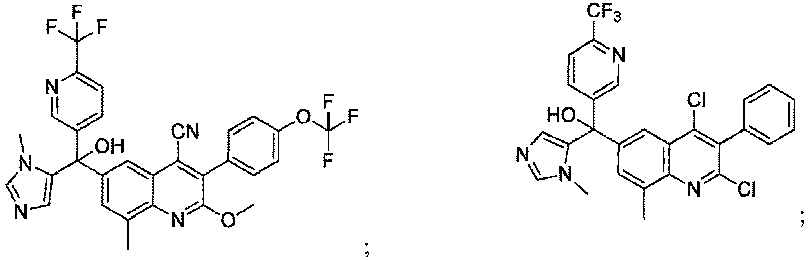 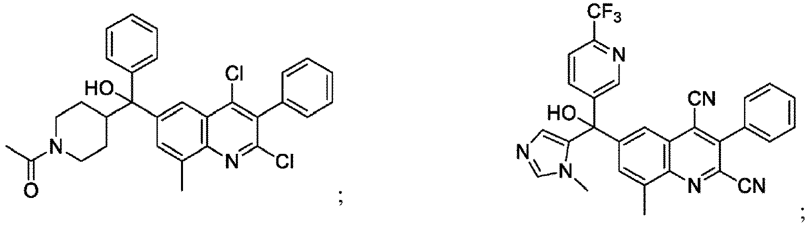 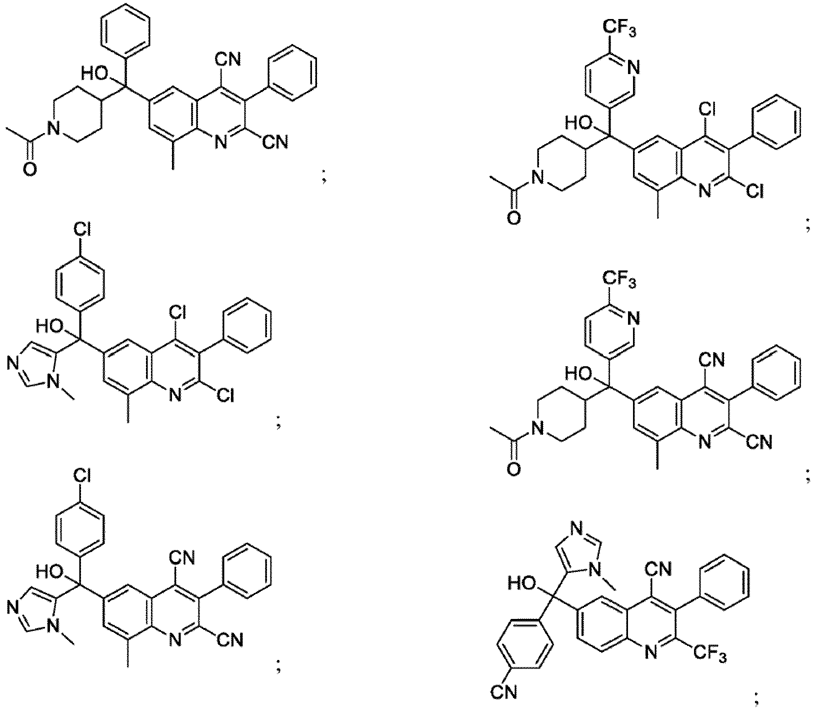 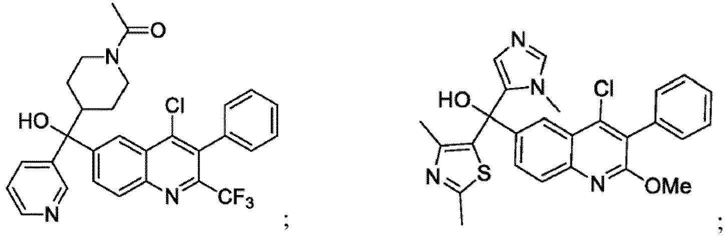 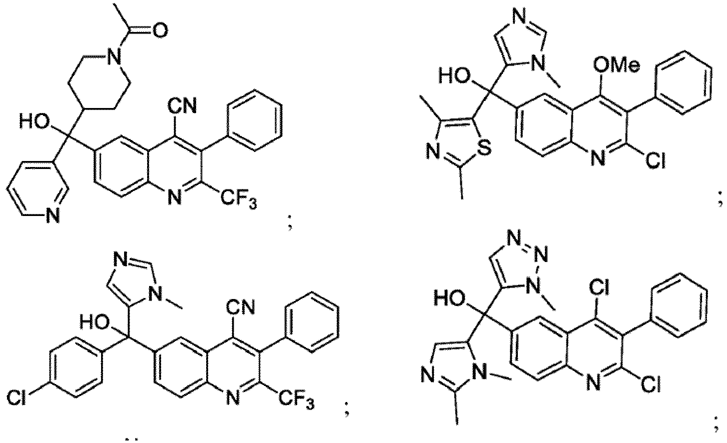 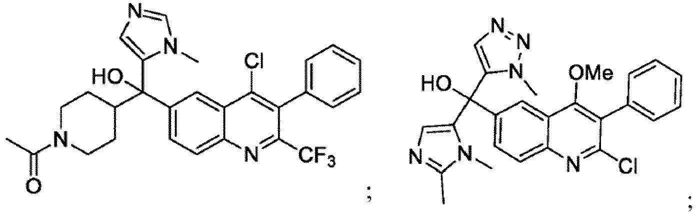 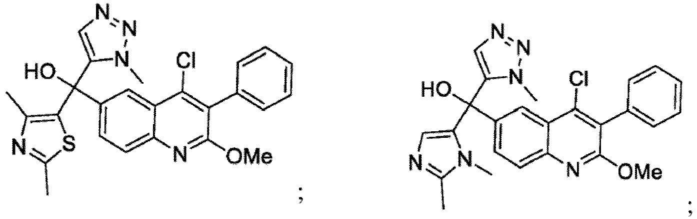 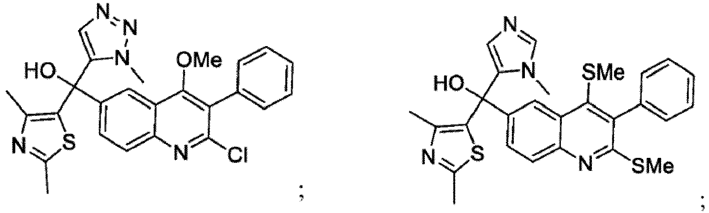 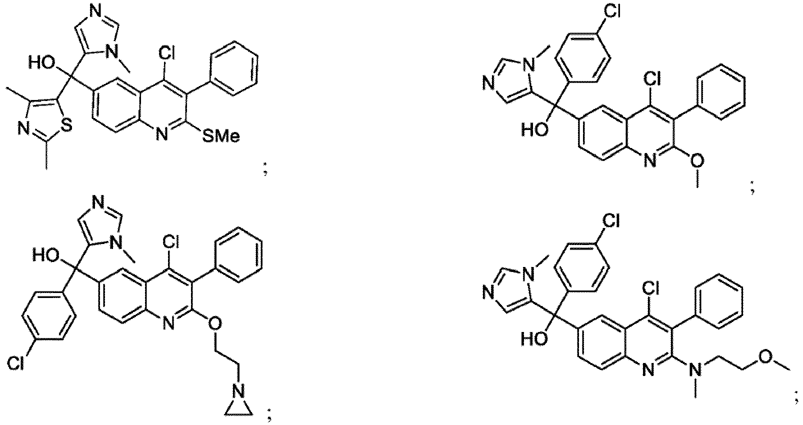 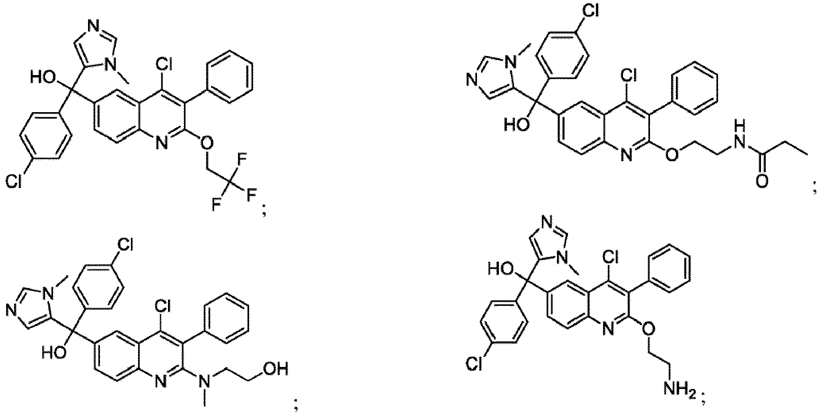 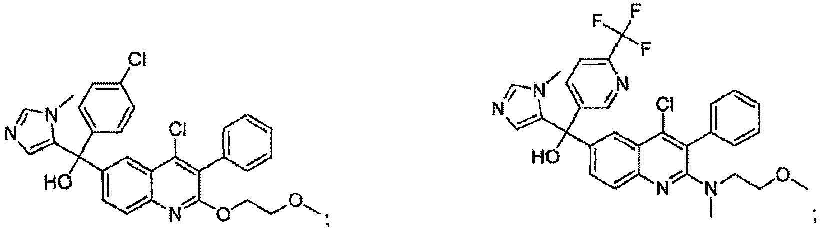 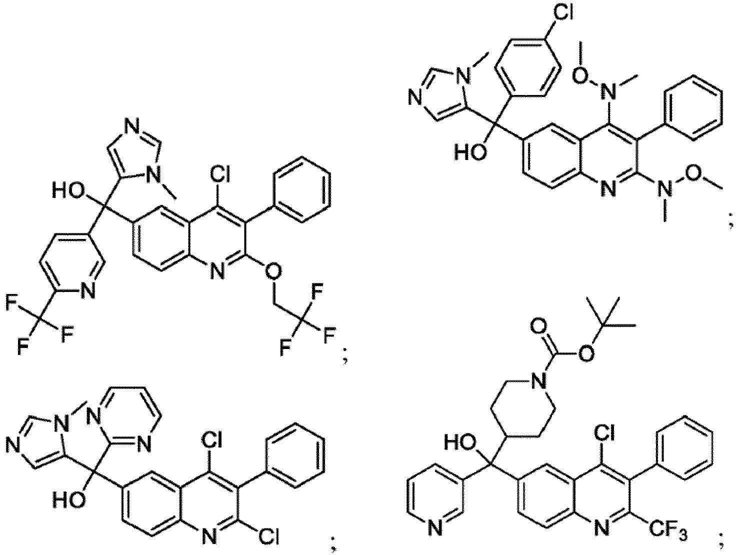 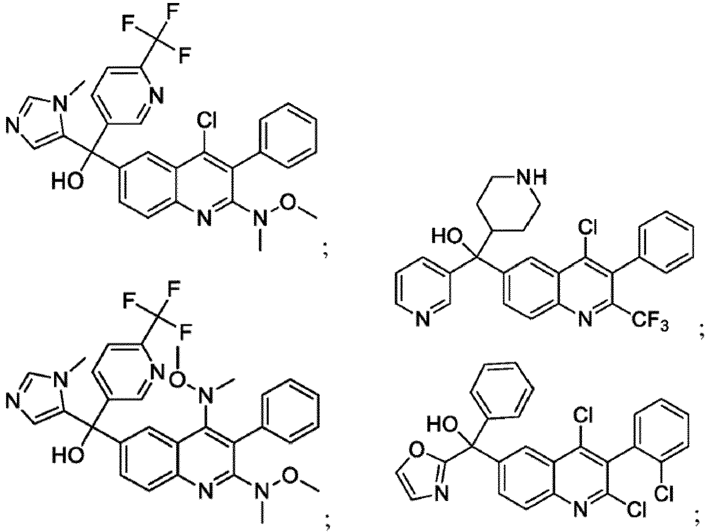 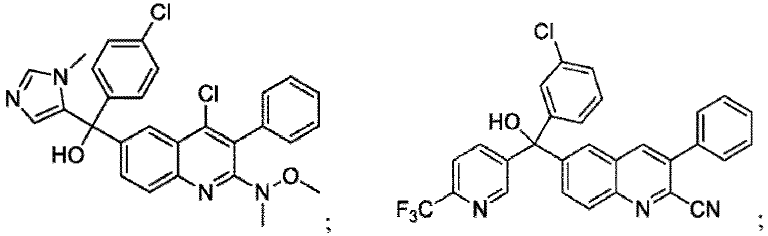 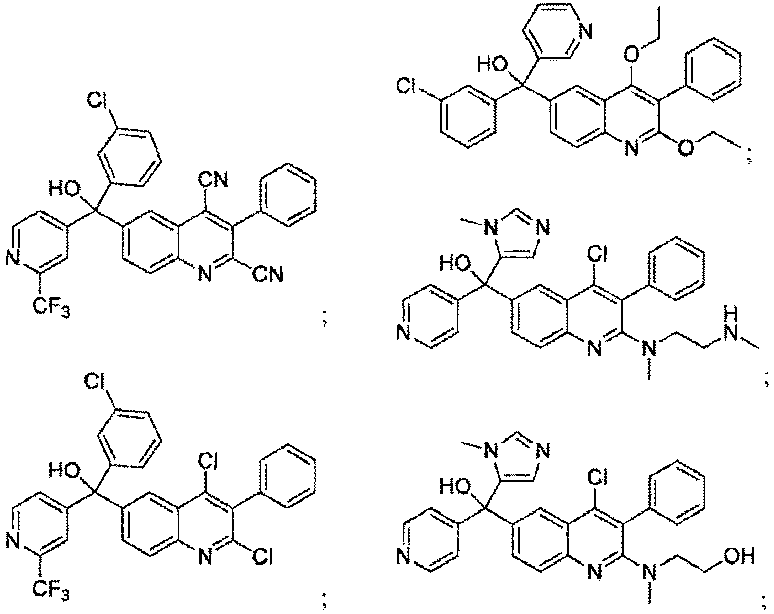 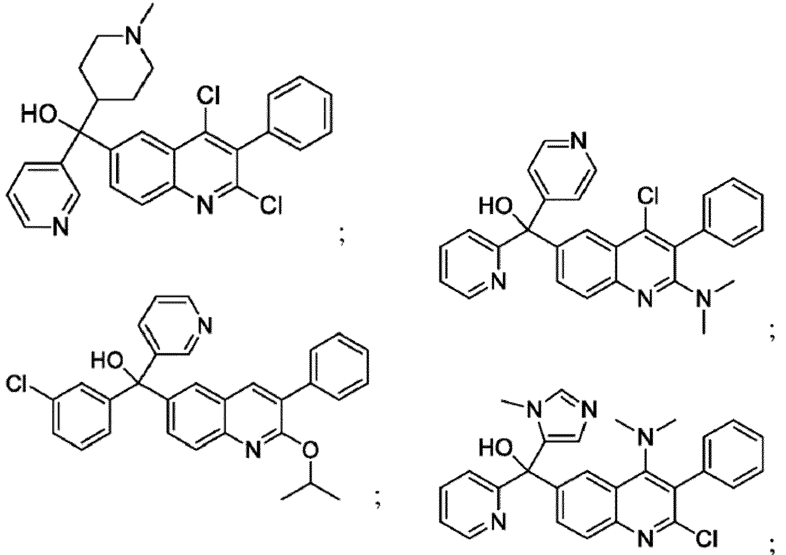 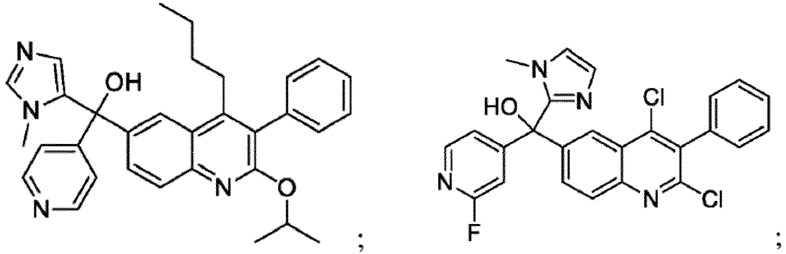 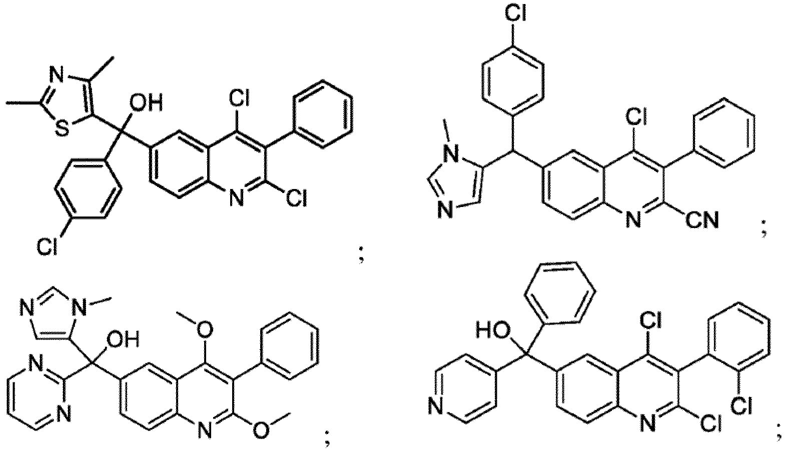 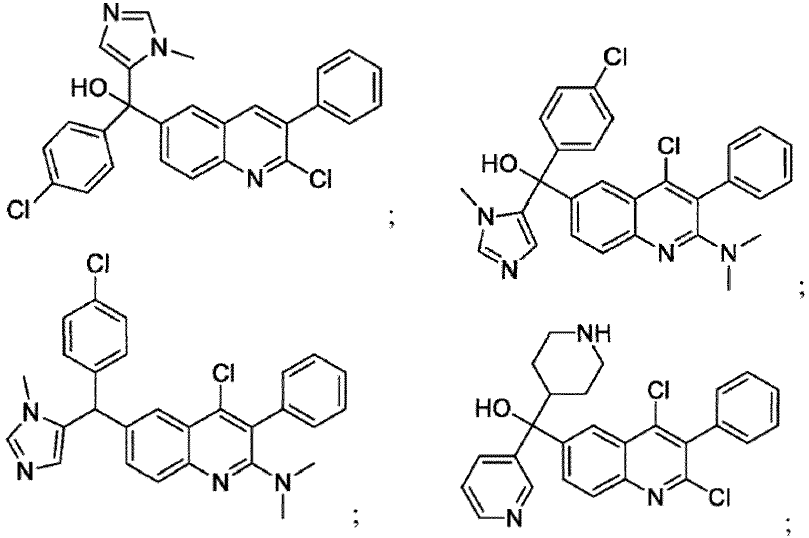 et sels pharmaceutiquement acceptables de ceux-ci.

4. Composition pharmaceutique, comprenant un composé de l'une quelconque des revendications 1 à 3 et un véhicule pharmaceutiquement acceptable.

5. Composition pharmaceutique fabriquée par mélange d'un composé de l'une quelconque des revendications 1 à 3 et d'un véhicule pharmaceutiquement acceptable.

6. Procédé de fabrication d'une composition pharmaceutique comprenant le mélange d'un composé de l'une quelconque des revendications 1 à 3 et d'un véhicule pharmaceutiquement acceptable.

7. Composé de l'une quelconque des revendications 1 à 3 pour utilisation dans une méthode de traitement ou d'amélioration d'un syndrome, d'un trouble ou d'une maladie inflammatoires médiés par RORγt comprenant l'administration à un sujet qui en a besoin d'une quantité efficace dudit composé de l'une quelconque des revendications 1 à 3.

8. Composé pour utilisation dans une méthode selon la revendication 7, la maladie étant choisie dans le groupe constitué de : les maladies intestinales inflammatoires, la polyarthrite rhumatoïde, le psoriasis, la bronchopneumopathie chronique obstructive, le rhumatisme psoriasique, la spondylarthrite ankylosante, l'asthme neutrophilique, l'asthme résistant aux stéroïdes, la sclérose en plaques et le lupus érythémateux disséminé.

9. Composé pour utilisation dans une méthode selon la revendication 7, la maladie étant le psoriasis.

10. Composé pour utilisation dans une méthode selon la revendication 7, la maladie étant la polyarthrite rhumatoïde.

11. Composé pour utilisation dans une méthode selon la revendication 8, la maladie intestinale inflammatoire étant la recto-colite hémorragique.

12. Composé pour utilisation dans une méthode selon la revendication 8, la maladie intestinale inflammatoire étant la maladie de Crohn.

13. Composé de l'une quelconque des revendications 1 à 3 ou composition ou médicament de celui-ci pour utilisation dans une méthode de traitement ou d'amélioration d'un syndrome, d'un trouble ou d'une maladie, chez un sujet qui en a besoin, comprenant l'administration au sujet d'une quantité efficace dudit composé de l'une quelconque des revendications 1 à 3 ou de ladite composition ou dudit médicament de celui-ci en thérapie d'association avec un ou plusieurs agents anti-inflammatoires ou agents immunosuppresseurs, ledit syndrome, ledit trouble ou ladite maladie étant choisis dans le groupe constitué de : la polyarthrite rhumatoïde et le psoriasis.
